(19) Europäisches Patentamt
European Patent Office
Office européen des brevets

(11) EP 4 090 661 B1

(12) # EUROPEAN PATENT SPECIFICATION

(45) Date of publication and mention of the grant of the patent:
**12.03.2025 Bulletin 2025/11**

(21) Application number: **21701423.2**

(22) Date of filing: **15.01.2021**

(51) International Patent Classification (IPC):
***C07D 471/04*** (2006.01) ***C07D 471/10*** (2006.01)
***C07D 471/14*** (2006.01) ***C07D 487/04*** (2006.01)
***C07D 498/04*** (2006.01) ***C07D 519/00*** (2006.01)
***A61P 5/44*** (2006.01) ***A61P 25/04*** (2006.01)
***A61P 29/00*** (2006.01) ***A61K 31/4985*** (2006.01)
***A61K 31/519*** (2006.01) ***A61K 31/4745*** (2006.01)
***A61K 31/4747*** (2006.01)

(52) Cooperative Patent Classification (CPC):
**C07D 487/04; A61P 5/44; A61P 25/04; A61P 29/00; C07D 471/04; C07D 471/10; C07D 471/14; C07D 498/04; C07D 519/00**

(86) International application number:
**PCT/EP2021/050841**

(87) International publication number:
**WO 2021/144440 (22.07.2021 Gazette 2021/29)**

(54) **QUINOXALINE DERIVATIVES AS MODULATORS OF THE GLUCOCORTICOID RECEPTOR**

QUINOXALINDERIVATE ALS MODULATOREN DES GLUCOCORTICOID REZEPTORS

DÉRIVÉS DE QUINOXALINE COMME DES MODULATEURS DU RECEPTEUR DE GLUCOCORTICOIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.01.2020 EP 20152469**

(43) Date of publication of application:
**23.11.2022 Bulletin 2022/47**

(73) Proprietor: **Grünenthal GmbH**
**52078 Aachen (DE)**

(72) Inventors:
- **ALEN, Jo**
  **3271 Averbode (BE)**
- **JAKOB, Florian**
  **52066 Aachen (DE)**
- **KRÜGER, Sebastian**
  **52062 Aachen (DE)**
- **BARBIE, Philipp**
  **10557 Berlin (DE)**
- **FRIEBE, Daniela**
  **40217 Düsseldorf (DE)**
- **HENNEN, Stephanie**
  **52066 Aachen (DE)**

(56) References cited:
**EP-A1- 1 995 242**
**EP-A1- 3 342 768**
**WO-A1-2020/016452**
**WO-A1-2020/016453**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

The present invention relates to compounds according to general formula (I)

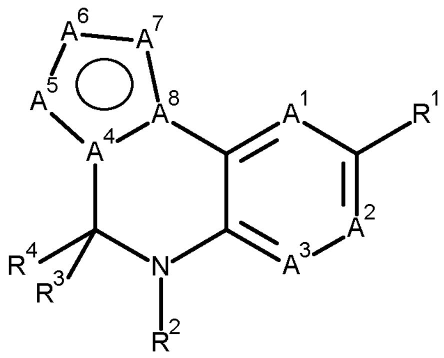

(I),

which act as modulators of the glucocorticoid receptor and can be used in the treatment and/or prophylaxis of disorders which are at least partially mediated by the glucocorticoid receptor.

Glucocorticoids (GC) exert strong anti-inflammatory, immunosuppressive and disease-modifying therapeutic effects mediated by the glucocorticoid receptor (GR). They have been widely used to treat inflammatory and immune diseases for decades and still represent the most effective therapy in those conditions. However, chronic GC treatment of inflammatory diseases is hampered by GC-associated adverse effects. These undesired side effects include insulin resistance, diabetes, hypertension, glaucoma, depression, osteoporosis, adrenal suppression and muscle wasting with osteoporosis and diabetes being the most severe ones from the physician's point of view (Hapgood JP. et al., Pharmacol Ther. 2016 Sep; 165: 93-113; Buttgereit F. el al, Clin Exp Rheumatol. 2015 Jul-Aug;33(4 Suppl 92):529-33; Hartmann K. et al, Physiol Rev. 2016 Apr;96(2):409-47).

One example of an oral glucocorticoid is prednisone which is frequently prescribed for the treatment of several inflammatory disorders (De Bosscher Ket al., Trends Pharmacol Sci. 2016 Jan;37(1):4-16; Buttgereit F. et al., JAMA. 2016;315(22):2442-2458). As GC cause adrenal suppression, prednisolone withdrawal symptoms can be severe if the drug is discontinued abruptly when all the signs of the disease have disappeared. Thus gradual GC tapering to physiological doses is frequently part of treatment protocols to reduce the risk of relapse and other withdrawal symptoms (Liu D. et al., Allergy Asthma Clin Immunol. 2013 Aug 15;9(1):30). Therefore, there is high medical need for novel potent anti-inflammatory drugs with less adverse effects.

Recent research has focused on the development of partial agonists or selective glucocorticoid receptor modulators which activate the pathways for the inhibition of inflammation but avoid targeting the pathways that lead to the GC-associated adverse effects. Most of these effects have been demonstrated to be mediated by different GR-dependent genomic mechanisms termed transactivation and transrepression. The anti-inflammatory actions of GC are mainly attributable to the transrepression of inflammatory genes while certain side effects are predominantly mediated via transactivation of several genes. According to the nature of a ligand the GR can be selectively modulated in a specific conformation which favors transrepression over transactivation resulting in an improved therapeutic benefit (De Bosscher K et al., Trends Pharmacol Sci. 2016 Jan;37(1):4-16). The concept of such dissociating ligands was already defined about two decades ago and several compounds have been identified and were evaluated in preclinical and clinical testing but none of them has as yet been approved for clinical use.

Compounds which are active as modulators of the glucocorticoid receptor are also known from WO 2009/035067, WO 2017/034006, EP 1 995 242, EP 3 342 768, WO 2020/016452 and WO 2020/016453.

It was an object of the present invention to provide novel compounds which are modulators of the glucocorticoid receptor and which preferably have advantages over the compounds of the prior art. The novel compounds should in particular be suitable for use in the treatment and/or prophylaxis of disorders or diseases which are at least partially mediated by the glucocorticoid receptor.

This object has been achieved by the subject-matter of the patent claims.

It was surprisingly found that the compounds according to the present invention are highly potent modulators of the glucocorticoid receptor.

The present invention relates to a compound according to general formula (I),

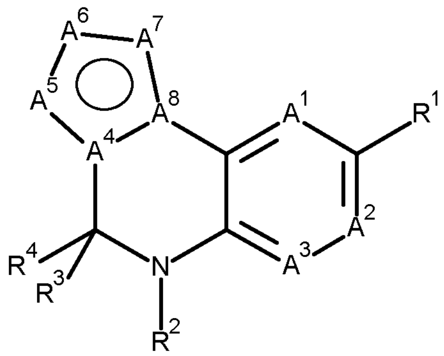

(I),

wherein

$R^1$ represents phenyl or 5 to 10-membered heteroaryl;

$R^2$ represents H;

$R^3$ and $R^4$ independently of one another represent H; $C_{1-10}$-alkyl; or together with the carbon atom joining them, form $C_{3-10}$-cycloalkyl;

$A^1$, $A^2$ and $A^3$ corresponds to embodiment a, b, c, or d:

| embodiment | $A^1$ | $A^2$ | $A^3$ |
|---|---|---|---|
| **a** | C-$R^5$ | C-$R^6$ | C-$R^7$ |
| **b** | C-$R^5$ | N | C-$R^7$ |
| **c** | C-$R^5$ | C-$R^6$ | N |
| **d** | N | C-$R^6$ | C-$R^7$ |

wherein $R^5$ represents H; F; Cl; Br; I; $C_{1-4}$-alkyl; $C_{3-10}$-cycloalkyl; O-$C_{1-10}$-alkyl; $R^6$ represents H; F; Cl; Br; I; $C_{1-4}$-alkyl; $C_{3-10}$-cycloalkyl; O-$C_{1-10}$-alkyl; $R^7$ represents H; F; Cl; Br; I; $C_{1-4}$-alkyl; $C_{3-10}$-cycloalkyl; O-$C_{1-10}$-alkyl;

$A^4$ represents C or N;

$A^5$ represents O, N, N-$R^8$ or C-$R^8$, wherein $R^8$ represents H; $C_{1-10}$-alkyl; $C_{3-10}$-cycloalkyl; 3 to 7 membered heterocycloalkyl; $S(O)_2$-$C_{1-6}$-alkyl; or $S(O)_2$-$C_{3-10}$-cycloalkyl, wherein $C_{3-10}$-cycloalkyl, or 3 to 7 membered heterocycloalkyl, can optionally be bridged via $C_{1-6}$-alkylene;

$A^6$ represents O, N, N-$R^9$ or C-$R^9$, wherein $R^9$ represents H; $C_{1-10}$-alkyl; $C_{3-10}$-cycloalkyl; 3 to 7 membered heterocycloalkyl; $S(O)_2$-$C_{1-6}$-alkyl; or $S(O)_2$-$C_{3-10}$-cycloalkyl, wherein $C_{3-10}$-cycloalkyl, or 3 to 7 membered heterocycloalkyl, can optionally be bridged via $C_{1-6}$-alkylene;

$A^7$ represents O, N, N-$R^{10}$ or C-$R^{10}$, wherein $R^{10}$ represents H; $C_{1-10}$-alkyl; $C_{3-10}$-cycloalkyl; 3 to 7 membered heterocycloalkyl; $S(O)_2$-$C_{1-6}$-alkyl; or $S(O)_2$-$C_{3-10}$-cycloalkyl, wherein $C_{3-10}$-cycloalkyl, or 3 to 7 membered heterocycloalkyl can optionally be bridged via $C_{1-6}$-alkylene;

$A^8$ represents C or N;

wherein $A^4$, $A^5$, $A^6$, $A^7$ and $A^8$ form a heteroaromatic system; and

wherein if $A^4$ represents C and each of $A^5$, $A^6$ and $A^8$ represent N and $A^7$ represents C-$R^{10}$; then one of $A^1$, $A^2$ and $A^3$ represents N;

wherein $C_{1-4}$-alkyl, $C_{1-6}$-alkyl, $C_{1-10}$-alkyl and $C_{1-6}$-alkylene in each case independently from one another is linear or branched, saturated or unsaturated;

wherein $C_{1-4}$-alkyl, $C_{1-6}$-alkyl, $C_{1-10}$-alkyl, $C_{1-6}$-alkylene, $C_{3-10}$-cycloalkyl and 3 to 7 membered heterocycloalkyl in each case independently from one another are unsubstituted or mono- or polysubstituted with one or more substituents selected from F; Cl; Br; I; CN; $C_{1-6}$-alkyl; $CF_3$; $CF_2H$; $CFH_2$; $CF_2Cl$; $CFCl_2$; C(O)-$C_{1-6}$-alkyl; C(O)-OH; C(O)-O$C_{1-6}$-alkyl; C(O)-$NH_2$; C(O)-N(H)($C_{1-6}$-alkyl); C(O)-N($C_{1-6}$-alkyl)$_2$; OH; =O; $OCF_3$; $OCF_2H$; $OCFH_2$; $OCF_2Cl$; $OCFCl_2$; O-$C_{1-6}$-alkyl; O-C(O)-$C_{1-6}$-alkyl; O-C(O)-O-$C_{1-6}$-alkyl; O-(CO)-N(H)($C_{1-6}$-alkyl); O-C(O)-N($C_{1-6}$-alkyl)$_2$; O-$S(O)_2$-$NH_2$; O-$S(O)_2$-N(H)($C_{1-6}$-alkyl); O-$S(O)_2$-N($C_{1-6}$-alkyl)$_2$; $NH_2$; N(H)($C_{1-6}$-alkyl); N($C_{1-6}$-alkyl)$_2$; N(H)-C(O)-$C_{1-6}$-alkyl; N(H)-C(O)-O-$C_{1-6}$-alkyl; N(H)-C(O)-$NH_2$; N(H)-C(O)-N(H)($C_{1-6}$-alkyl); N(H)-C(O)-N($C_{1-6}$-alkyl)$_2$; N($C_{1-6}$-alkyl)-C(O)-$C_{1-6}$-alkyl; N($C_{1-6}$-alkyl)-C(O)-O-$C_{1-6}$-alkyl; N($C_{1-6}$-alkyl)-C(O)-$NH_2$; N($C_{1-6}$-alkyl)-C(O)-N(H)($C_{1-6}$-alkyl); N($C_{1-6}$-alkyl)-C(O)-N($C_{1-6}$-alkyl)$_2$; N(H)-$S(O)_2$OH; N(H)-$S(O)_2$-$C_{1-6}$-alkyl; N(H)-$S(O)_2$-O-$C_{1-6}$-alkyl; N(H)-$S(O)_2$-$NH_2$; N(H)-$S(O)_2$-N(H)($C_{1-6}$-alkyl); N(H)-$S(O)_2$N($C_{1-6}$-alkyl)$_2$; N($C_{1-6}$-alkyl)-$S(O)_2$-OH; N($C_{1-6}$-alkyl)-$S(O)_2$-$C_{1-6}$-alkyl; N($C_{1-6}$-alkyl)-$S(O)_2$-O-$C_{1-6}$-alkyl; N($C_{1-6}$-alkyl)-$S(O)_2$-$NH_2$; N($C_{1-6}$-alkyl)-$S(O)_2$-N(H)($C_{1-6}$-alkyl); N($C_{1-6}$-alkyl)-$S(O)_2$-N($C_{1-6}$-alkyl)$_2$; $SCF_3$; $SCF_2H$;

$SCFH_2$; S-$C_{1-6}$-alkyl; S(O)-$C_{1-6}$-alkyl; $S(O)_2$-$C_{1-6}$-alkyl; $S(O)_2$-OH; $S(O)_2$-O-$C_{1-6}$-alkyl; $S(O)_2$-$NH_2$; $S(O)_2$-N(H)($C_{1-6}$-alkyl); $S(O)_2$-N($C_{1-6}$-alkyl)$_2$; $C_{3-6}$-cycloalkyl; 3 to 7-membered heterocycloalkyl; phenyl; 5 or 6-membered heteroaryl; O-$C_{3-6}$-cycloalkyl; O-(3 to 7-membered heterocycloalkyl); O-phenyl; O-(5 or 6-membered heteroaryl); C(O)-$C_{3-6}$-cycloalkyl; C(O)-(3 to 7-membered heterocycloalkyl); C(O)-phenyl; C(O)-(5 or 6-membered heteroaryl); $S(O)_2$-($C_{3-6}$-cycloalkyl); $S(O)_2$-(3 to 7-membered heterocycloalkyl); $S(O)_2$-phenyl or $S(O)_2$-(5 or 6-membered heteroaryl); wherein phenyl, 5 to 10-membered heteroaryl in each case independently from one another are unsubstituted or mono- or polysubstituted with one or more substituents selected from F; Cl; Br; I; CN; $C_{1-6}$-alkyl; $C_{1-6}$-alkenyl; $C_{1-6}$-alkynyl; $C_{1-6}$-alkynyl-C(H)(OH)$CH_3$; $_{1-6}$-alkynyl-C($CH_3$)$_2$OH; $CF_3$; $CF_2H$; $CFH_2$; $CF_2Cl$; $CFCl_2$; $C_{1-6}$-alkylene-$CF_3$; $C_{1-6}$-alkylene-$CF_2H$; $C_{1-6}$-alkylene-$CFH_2$; $C_{1-6}$-alkylene-OH; $C_{1-6}$-alkylene-$OCH_3$; C(O)-$C_{1-6}$-alkyl; C(O)-OH; C(O)-O$C_{1.}$ $_6$-alkyl; C(O)-N(H)(OH); C(O)-$NH_2$; C(O)-N(H)($C_{1-6}$-alkyl); C(O)-N($C_{1-6}$-alkyl)$_2$; OH; $OCF_3$; $OCF_2H$; $OCFH_2$; $OCF_2Cl$; $OCFCl_2$; O-$C_{1-6}$-alkyl; O-$C_{3-6}$-cycloalkyl; O-(3 to 7-membered heterocycloalkyl); $NH_2$; N(H)($C_{1-6}$-alkyl); N($C_{1-6}$-alkyl)$_2$; N(H)-C(O)-$C_{1-6}$-alkyl; N($C_{1-6}$-alkyl)-C(O)-$C_{1-6}$-alkyl; N(H)-C(O)-$NH_2$; N(H)-C(O)-N(H)($C_{1-6}$-alkyl); N(H)-C(O)-N($C_{1-6}$-alkyl)$_2$; N($C_{1-6}$-alkyl)-C(O)-N(H)($C_{1-6}$-alkyl); N($C_{1-6}$-alkyl)-C(O)-N($C_{1-6}$-alkyl)$_2$; N(H)-$S(O)_2$-$C_{1-6}$-alkyl; $SCF_3$; S-$C_{1-6}$-alkyl; S(O)-$C_{1-6}$-alkyl; $S(O)_2$-$C_{1-6}$-alkyl; $S(O)_2$-$C_{3-6}$-cycloalkyl; $S(O)_2$-$C_{1-6}$-alkylene-$C_{3-6}$-cycloalkyl; $S(O)_2$-$NH_2$; $S(O)_2$-N(H)($C_{1-6}$-alkyl); $S(O)_2$-N($C_{1-6}$-alkyl)$_2$; $C_{3-6}$-cycloalkyl; $C_{1-6}$-alkylene-$C_{3-6}$-cycloalkyl; 3 to 7-membered heterocycloalkyl; $C_{1-6}$-alkylene-(3 to 7-membered heterocycloalkyl); phenyl or 5 or 6-membered heteroaryl;
in the form of the free compound or a physiologically acceptable salt thereof.

In a preferred embodiment,

- $C_{1-4}$-alkyl, $C_{1-6}$-alkyl, $C_{1-10}$-alkyl, $C_{1-6}$-alkylene, $C_{3-10}$-cycloalkyl and 3 to 7 membered heterocycloalkyl in each case independently from one another are unsubstituted or mono- or polysubstituted with one or more substituents selected from F; Cl; Br; I; CN; $C_{1-6}$-alkyl; $CF_3$; $CF_2H$; $CFH_2$; $CF_2Cl$; $CFCl_2$; OH; =O; $OCF_3$; $OCF_2H$; $OCFH_2$; $OCF_2Cl$; $OCFCl_2$; O-$C_{1-6}$-alkyl; $C_{3-6}$-cycloalkyl; or 3 to 7-membered heterocycloalkyl; and/or

- phenyl, and 5 to 10-membered heteroaryl in each case independently from one another are unsubstituted or mono- or polysubstituted with one or more substituents selected from F; Cl; Br; I; CN; $C_{1-6}$-alkyl; $C_{2-6}$-alkynyl, preferably -C≡C-$CH_3$; $CF_3$; $CF_2H$; $CFH_2$; $CF_2Cl$; $CFCl_2$; $C_{1-6}$-alkylene-$CF_3$; $C_{1-6}$-alkylene-$CF_2H$; $C_{1-6}$-alkylene-$CFH_2$; C(O)-$C_{1-6}$-alkyl; C(O)-OH; C(O)-O$C_{1-6}$-alkyl; OH; $C_{1-6}$-alkylene-OH; $OCF_3$; $OCF_2H$; $OCFH_2$; $OCF_2Cl$; $OCFCl_2$; O-$C_{1-6}$-alkyl; O-$C_{3-6}$-cycloalkyl; O-(3 to 7-membered heterocycloalkyl); $SCF_3$; S-$C_{1-6}$-alkyl; S(O)-$C_{1-6}$-alkyl; $S(O)_2$-$C_{1-6}$-alkyl; $S(O)_2$-$C_{1-6}$-alkylene-$C_{3-6}$-cycloalkyl; $S(O)_2$-$NH_2$; $S(O)_2$-N(H)($C_{1-6}$-alkyl); $S(O)_2$-N($C_{1-6}$-alkyl)$_2$; $C_{3-6}$-cycloalkyl; $C_{1-6}$-alkylene-$C_{3-6}$-cycloalkyl; 3 to 7-membered heterocycloalkyl; $C_{1-6}$-alkylene-(3 to 7-membered heterocycloalkyl); phenyl or 5 or 6-membered heteroaryl.

In another preferred embodiment, $R^3$ and $R^4$ independently of one another represent H or $CH_3$; or $R^3$ and $R^4$, together with the carbon atom joining them, form $C_{3-10}$-cycloalkyl, preferably cyclobutyl.

In another preferred embodiment, $R^1$ represents phenyl or 5 to 10-membered heteroaryl which is selected from the group consisting of indolyl, indazolyl, pyridyl, preferably 2-pyridyl, 3-pyridyl or 4-pyridyl, pyrazolyl, pyrazolopyrimidinyl, pyrrolopyridinyl, pyrimidinyl, pyridazinyl, pyrazinyl, pyrrolyl, imidazolyl, oxazolyl, isoxazolyl, thiazolyl, isothiazolyl, furanyl, thienyl (thiophenyl), triazolyl, thiadiazolyl, 4,5,6,7-tetrahydro-2H-indazolyl, 2,4,5,6-tetrahydrocyclo-penta[c]pyrazolyl, benzofuranyl, benzoimidazolyl, benzothienyl, benzothiadiazolyl, benzothiazolyl, benzotriazolyl, benzooxazolyl, benzooxadiazolyl, quinazolinyl, quinoxalinyl, carbazolyl, quinolinyl, dibenzofuranyl, dibenzothienyl, imidazothiazolyl, indolizinyl, isoquinolinyl, naphthyridinyl, oxazolyl, oxadiazolyl, phenazinyl, phenothiazinyl, phthalazinyl, purinyl, phenazinyl, tetrazolyl and triazinyl; and/or (i) $R^3$ and $R^4$, together with the carbon atom joining them, form $C_{3-10}$-cycloalkyl; or (ii) $R^3$ and $R^4$ independently of one another represent H or $C_{1-10}$-alkyl, preferably -$CH_3$.

In another preferred embodiment, $R^1$ represents phenyl, unsubstituted or mono- or polysubstituted with one or more substituents selected from F; Cl; Br; I; -$CH_3$; -$CH_2$-$CH_3$; O-$CH_3$; -$CF_3$; -$C_{3-10}$-cycloalkyl; -$CH_2$-$C_{3-10}$-cycloalkyl; S(=O)$_2$-$C_{3-10}$-cycloalkyl; S(=O)$_2$-$CH_2$-$C_{3-10}$-cycloalkyl; S(=O)$_2$-$CH_3$; S(=O)$_2$-$CH_2$-$CH_3$; -$CH_2$-$CH_2$-O-$CH_2$- (i.e. oxolanyl);, -C≡C-$CH_3$; C(=O)-$CH_3$; -CH=$CH_2$; $NH_2$; or -$CH_2$-$CH_2$-OH; or any of the following structures (II), (III), (IV), (V) or (VI), with the proviso that with respect to structures (II), (III), (IV) and (V) at least one of X and Z is a heteroatom:

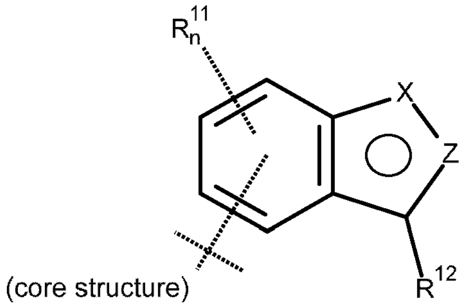

(II)

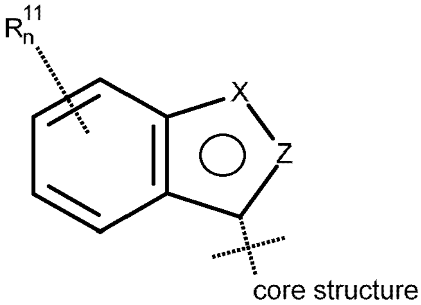

(III)

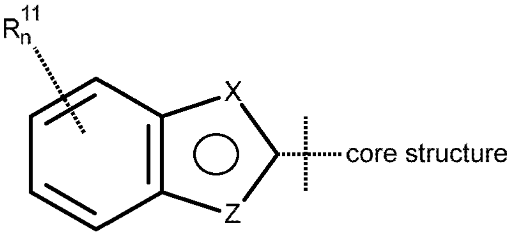

(IV)

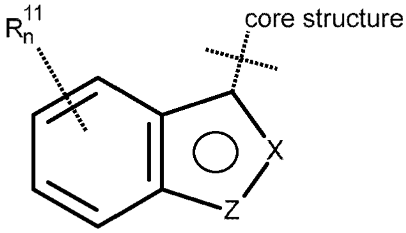

(V)

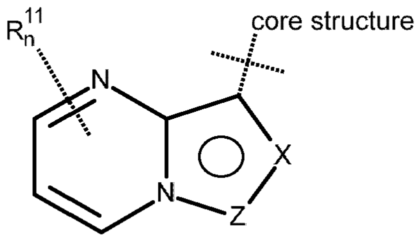

(VI)

wherein X represents N, N-$R^{13}$ or C-$R^{13}$; Z represents N, N-$R^{13}$ or C-$R^{13}$; $R^{11}$, $R^{12}$ and $R^{13}$ represent, independently from one another, H; F; Cl; Br; I; CN; $C_{1-10}$-alkyl; $C_{1-6}$-alkenyl; $C_{2-6}$-alkynyl; $C_{3-10}$-cycloalkyl; 3 to 7 membered heterocycloalkyl; S(O)-($C_{1-10}$-alkyl); S(O)-($C_{3-10}$-cycloalkyl); S(O)-(3 to 7-membered heterocycloalkyl); $S(O)_2$-($C_{1-10}$-alkyl); $S(O)_2$-($C_{3-10}$-cycloalkyl); $S(O)_2$-(3 to 7-membered heterocycloalkyl); P(O)-$(C_{1-10}\text{-alkyl})_2$; P(O)($C_{1-10}$-alkyl)($C_{3-10}$-cycloalkyl); P(O)($C_{1-10}$-alkyl)(3 to 7-membered heterocycloalkyl); P(O)-$(O\text{-}C_{1-10}\text{-alkyl})_2$; P(O)(O-$C_{1-10}$-alkyl)(O-$C_{3-10}$-cycloalkyl); P(O)(O-$C_{1-10}$-alkyl)(O-(3 to 7-membered heterocycloalkyl)); O-$C_{1-10}$-alkyl; S-$C_{1-10}$-alkyl; N(H)($C_{1-10}$-alkyl), N$(C_{1-10}\text{-alkyl})_2$; C(O)-$C_{1-10}$-alkyl; C(O)-O-$C_{1-10}$-alkyl; C(O)-$NH_2$; C(O)-N(H)($C_{1-10}$-alkyl); C(O)-N$(C_{1-10}\text{-alkyl})_2$; O-$C_{3-10}$-cycloalkyl; N(H)($C_{3-10}$-cycloalkyl), N($C_{1-10}$-alkyl)($C_{3-10}$-cycloalkyl); C(O)-$C_{3-10}$-cycloalkyl; C(O)-O-$C_{3-10}$-cycloalkyl; C(O)-N(H)($C_{3-10}$-cycloalkyl); C(O)-N($C_{1-10}$-alkyl)($C_{3-10}$-cycloalkyl); O-3 to 7-membered heterocycloalkyl; N(H)(3 to 7-membered heterocycloalkyl), N($C_{1-10}$-alkyl)(3 to 7-membered heterocycloalkyl); C(O)-3 to 7-membered heterocycloalkyl; C(O)-O-(3 to 7-membered heterocycloalkyl); C(O)-N(H)(3 to 7-membered heterocycloalkyl) or C(O)-N($C_{1-10}$-alkyl)(3 to 7-membered heterocycloalkyl); wherein $C_{3-10}$-cycloalkyl and 3 to 7 membered heterocycloalkyl can optionally be bridged via $C_{1-6}$-alkylene; and n represents 0, 1, 2 or 3.

In another preferred embodiment, $R^{11}$, $R^{12}$ and $R^{13}$ represent, independently from one another, H; F; Cl; Br; I; -$CH_3$; O-$CH_3$; -$CF_3$; -$C_{3-10}$-cycloalkyl; -$CH_2$-$C_{3-10}$-cycloalkyl; $S(=O)_2$-$CH_2$-$C_{3-10}$-cycloalkyl; $S(=O)_2$-$CH_3$; -$CH_2$-$CH_2$-O-$CH_2$- (i.e. oxolanyl); -C≡C-$CH_3$; C(=O)-$CH_3$; -$CH_2$-$CH_2$-OH; and n represents 0, 1, 2 or 3.

Together with the carbon atom carrying residue $R^{12}$ on the one hand and the two carbon atoms of the adjacent phenyl moiety on the other hand, X and Z according to structure (II) form an aromatic system.

Together with the carbon atom connecting structures (III), (IV) and (V) to the core structure on the one hand and the two carbon atoms of the adjacent phenyl moiety on the other hand, X and Z form an aromatic system.

Together with the carbon atom connecting structure (VI) to the core structure on the one hand and the carbon and nitrogen atoms of the adjacent pyrimidinyl moiety on the other hand, X and Z form an aromatic system.

In another preferred embodiment, none of $A^1$, $A^2$ and $A^3$ represents N.

According to the invention, $A^1$, $A^2$ and $A^3$ corresponds to embodiment a, b, c, or d:

| embodiment | $A^1$ | $A^2$ | $A^3$ |
|---|---|---|---|
| **a** | C-$R^5$ | C-$R^6$ | C-$R^7$ |
| **b** | C-$_{RS}$ | N | C-$R^5$ |
| **c** | C-$R^5$ | C-$R^6$ | N |
| **d** | N | C-$R^6$ | C-$R^7$ |

In another preferred embodiment, $A^7$ does not represent C-$R^{10}$; or none of $A^5$, $A^6$ and $A^7$ represents C-$R^8$, C-$R^9$ or C-$R^{10}$, respectively, and/or at most one of $A^5$, $A^6$ and $A^7$ represents O; or at least one of $A^5$, $A^6$ and $A^7$ represents C-$R^8$, C-$R^9$ or C-$R^{10}$, respectively, and/or at most one of $A^5$, $A^6$ and $A^7$ represents O; or at least one of $A^5$, $A^6$ and $A^7$ represents N, respectively, and/or at most one of $A^5$, $A^6$ and $A^7$ represents O; or at least one of $A^5$, $A^6$ and $A^7$ represents N-$R^8$, N-$R^9$ or N-$R^{10}$, respectively, and/or at most one of $A^5$, $A^6$ and $A^7$ represents O.

In another preferred embodiment, the definition of $A^5$, $A^6$ and $A^7$ corresponds to embodiment e, f, g, h, i, j, k, l or m:

| embodiment | $A^5$ | $A^6$ | $A^7$ |
|---|---|---|---|
| **e** | N | C-$R^9$ | C-$R^{10}$ |
| **f** | C-$R^8$ | C-$R^9$ | C-$R^{10}$ |
| **g** | N | N | C-$R^{10}$ |
| **h** | N | N | N |
| **i** | N | N | N-$R^{10}$ |
| **j** | C-$R^8$ | N | N-$R^{10}$ |
| **k** | N | C-$R^9$ | N-$R^{10}$ |
| **l** | C-$R^8$ | N-$R^9$ | N |
| **m** | N | C-$R^9$ | O |

In another preferred embodiment, the definition of $A^4$, $A^5$, $A^6$, $A^7$ and $A^8$ corresponds to embodiment n, o, p, q, r, s, t, u, v, w, x or y:

| embodiment | $A^4$ | $A^5$ | $A^6$ | $A^7$ | $A^8$ |
|---|---|---|---|---|---|
| **n** | C | N | C-$R^9$ | C-$R^{10}$ | N |
| **0** | N | N | C-$R^9$ | C-$R^{10}$ | C |
| **p** | C | C-$R^8$ | C-$R^9$ | C-$R^{10}$ | N |
| **q** | C | N | N | C-$R^{10}$ | N |
| **r** | N | N | N | C-$R^{10}$ | C |
| **s** | C | N | N | N | N |
| **t** | C | N | N | N-$R^{10}$ | C |
| **u** | C | C-$R^8$ | N | N-$R^{10}$ | C |
| **v** | N | N | C-$R^9$ | N-$R^{10}$ | C |
| **w** | C | N | C-$R^9$ | N-$R^{10}$ | C |
| **x** | C | C-$R^8$ | N-$R^9$ | N | C |
| **y** | C | N | C-$R^9$ | O | C |

In another preferred embodiment, $R^5$, $R^6$ and $R^7$ independently from one another, represent $CH_3$, $CH_2CH_3$; F, Cl, $CF_3$, cyclopropyl, cyclobutyl, O-$CH_3$ O-$CH_2CH_3$ or H; more preferably $CH_3$, F, Cl, $CF_3$, or H; and/or $R^8$, $R^9$ and $R^{10}$ independently from one another, represent $S(O)_2$-$CH_3$, $CH_3$, $CH_2CH_3$, F, $CF_3$, $CH_2$-cyclopropyl, or H.

In a preferred embodiment, the compound according to the present invention is present in form of the free

compound. For the purpose of specification, "free compound" preferably means that the compound according to the present invention is not present in form of a salt. Methods to determine whether a chemical substance is present as the free compound or as a salt are known to the skilled artisan such as $^{14}N$ or $^{15}N$ solid state NMR, x-ray diffraction, x-ray powder diffraction, IR, Raman, XPS. $^{1}H$-NMR recorded in solution may also be used to consider the presence of protonation.

In another preferred embodiment, the compound according to the present invention is present in form of a physiologically acceptable salt. For the purposes of this specification, the term "physiologically acceptable salt" preferably refers to a salt obtained from a compound according to the present invention and a physiologically acceptable acid or base.

According to the present invention, the compound according to the present invention may be present in any possible form including solvates, cocrystals and polymorphs. For the purposes of this specification, the term "solvate" preferably refers to an adduct of (i) a compound according to the present invention and/or a physiologically acceptable salt thereof with (ii) distinct molecular equivalents of one or more solvents.

Further, the compound according to the present invention may be present in form of the racemate, enantiomers, diastereomers, tautomers or any mixtures thereof.

The present invention also includes isotopic isomers of a compound of the invention, wherein at least one atom of the compound is replaced by an isotope of the respective atom which is different from the naturally predominantly occurring isotope, as well as any mixtures of isotopic isomers of such a compound. Preferred isotopes are $^{2}H$ (deuterium), $^{3}H$ (tritium), $^{13}C$ and $^{14}C$. Isotopic isomers of a compound of the invention can generally be prepared by conventional procedures known to a person skilled in the art.

According to the present invention, the terms "$C_{1-10}$-alkyl", "$C_{1-8}$-alkyl", "$C_{1-6}$-alkyl" and "$C_{1-4}$-alkyl" preferably mean acyclic saturated or unsaturated aliphatic (i.e. non-aromatic) hydrocarbon residues, which can be linear (i.e. unbranched) or branched and which can be unsubstituted or mono- or polysubstituted (e.g. di- or trisubstituted), and which contain 1 to 10 (i.e. 1, 2, 3, 4, 5, 6, 7, 8, 9 or 10), 1 to 8 (i.e. 1, 2, 3, 4, 5, 6, 7 or 8), 1 to 6 (i.e. 1, 2, 3, 4, 5 or 6) and 1 to 4 (i.e. 1, 2, 3 or 4) carbon atoms, respectively. In a preferred embodiment, $C_{1-10}$-alkyl, $C_{1-8}$-alkyl, $C_{1-6}$-alkyl and $C_{1-4}$-alkyl are saturated. In another preferred embodiment, $C_{1-10}$-alkyl, $C_{1-8}$-alkyl, $C_{1-6}$-alkyl and $C_{1-4}$-alkyl are not saturated. According to this embodiment, $C_{1-10}$-alkyl, $C_{1-8}$-alkyl, $C_{1-6}$-alkyl and $C_{1-4}$-alkyl comprise at least one C-C double bond (a C=C-bond) or at least one C-C triple bond (a C≡C-bond). In still another preferred embodiment, $C_{1-10}$-alkyl, $C_{1-8}$-alkyl, $C_{1-6}$-alkyl and $C_{1-4}$-alkyl are (i) saturated or (ii) not saturated, wherein $C_{1-10}$-alkyl, $C_{1-8}$-alkyl, $C_{1-6}$-alkyl and $C_{1-4}$-alkyl comprise at least one, preferably one, C-C triple bond (a C≡C-bond). Preferred $C_{1-10}$-alkyl groups are selected from methyl, ethyl, ethenyl (vinyl), n-propyl, 2-propyl, 1-propynyl, 2-propynyl, propenyl ($-CH_2CH=CH_2$, $-CH=CH-CH_3$, $-C(=CH_2)-CH_3$), n-butyl, 1-butynyl, 2-butynyl, 1-butenyl, 2-butenyl, isobutyl, sec-butyl, tert-butyl, n-pentyl, 2-pentyl, 3-pentyl, 1-pentenyl, 2-pentenyl, 1-pentynyl, 2-pentynyl, 2-methylbutyl, 3-methylbutyl, 3-methylbut-2-yl, 2-methylbut-2-yl, 3-methylbut-1-ynyl, 2,2-dimethylpropyl, n-hexyl, 2-hexyl, 3-hexyl, 2-methylpentyl, 4-methylpentyl, 4-methylpent-2-yl, 2-methylpent-2-yl, 3,3-dimethylbutyl, 3,3-dimethylbut-2-yl, 3-methylpentyl, 3-methylpent-2-yl and 3-methylpent-3-yl; more preferably methyl, ethyl, n-propyl, 2-propyl, 1-propynyl, 2-propynyl, propenyl ($-CH_2CH=CH_2$, $-CH=CH-CH_3$, $-C(=CH_2)-CH_3$), n-butyl, 1-butynyl, 2-butynyl, 1-butenyl, 2-butenyl, isobutyl, sec-butyl, tert-butyl, n-pentyl, 2-pentyl, 3-pentyl, 1-pentenyl, 2-pentenyl, 1-pentynyl, 2-pentynyl, 2-methylbutyl, 3-methylbutyl, 3-methylbut-2-yl, 2-methylbut-2-yl, 3-methylbut-1-ynyl, 2,2-dimethylpropyl, n-hexyl, n-heptyl, n-octyl, n-nonyl and n-decyl. Preferred $C_{1-8}$-alkyl groups are selected from methyl, ethyl, ethenyl (vinyl), n-propyl, 2-propyl, 1-propynyl, 2-propynyl, propenyl ($-CH_2CH=CH_2$, $-CH=CH-CH_3$, $-C(=CH_2)-CH_3$), n-butyl, 1-butynyl, 2-butynyl, 1-butenyl, 2-butenyl, isobutyl, sec-butyl, tert-butyl, n-pentyl, 2-pentyl, 3-pentyl, 1-pentenyl, 2-pentenyl, 1-pentynyl, 2-pentynyl, 2-methylbutyl, 3-methylbutyl, 3-methylbut-2-yl, 2-methylbut-2-yl, 3-methylbut-1-ynyl, 2,2-dimethylpropyl, n-hexyl, 2-hexyl, 3-hexyl, 2-methylpentyl, 4-methylpentyl, 4-methylpent-2-yl, 2-methylpent-2-yl, 3,3-dimethylbutyl, 3,3-dimethylbut-2-yl, 3-methylpentyl, 3-methylpent-2-yl and 3-methylpent-3-yl; more preferably methyl, ethyl, n-propyl, 2-propyl, 1-propynyl, 2-propynyl, propenyl ($-CH_2CH=CH_2$, $-CH=CH-CH_3$, $-C(=CH_2)-CH_3$), n-butyl, 1-butynyl, 2-butynyl, 1-butenyl, 2-butenyl, isobutyl, sec-butyl, tert-butyl, n-pentyl, 2-pentyl, 3-pentyl, 1-pentenyl, 2-pentenyl, 1-pentynyl, 2-pentynyl, 2-methylbutyl, 3-methylbutyl, 3-methylbut-2-yl, 2-methylbut-2-yl, 3-methylbut-1-ynyl, 2,2-dimethylpropyl, n-hexyl, n-heptyl and n-octyl. Preferred $C_{1-6}$-alkyl groups are selected from methyl, ethyl, ethenyl (vinyl), n-propyl, 2-propyl, n-butyl, isobutyl, sec-butyl, tert-butyl, n-pentyl, 2-pentyl, 3-pentyl, 2-methylbutyl, 3-methylbutyl, 3-methylbut-2-yl, 2-methylbut-2-yl, 2,2-dimethylpropyl, n-hexyl, 2-hexyl, 3-hexyl, 2-methylpentyl, 4-methylpentyl, 4-methylpent-2-yl, 2-methylpent-2-yl, 3,3-dimethylbutyl, 3,3-dimethylbut-2-yl, 3-methylpentyl, 3-methylpent-2-yl and 3-methylpent-3-yl; more preferably methyl, ethyl, n-propyl, 2-propyl, 1-propynyl, 2-propynyl, propenyl ($-CH_2CH=CH_2$, $-CH=CH-CH_3$, $-C(=CH_2)-CH_3$), n-butyl, 1-butynyl, 2-butynyl, 1-butenyl, 2-butenyl, isobutyl, sec-butyl, tert-butyl, n-pentyl, 2-pentyl, 3-pentyl, 1-pentenyl, 2-pentenyl, 1-pentynyl, 2-pentynyl, 2-methylbutyl, 3-methylbutyl, 3-methylbut-2-yl, 2-methylbut-2-yl, 3-methylbut-1-ynyl, 2,2-dimethylpropyl, n-hexyl. Particularly preferred $C_{1-6}$-alkyl groups are selected from $C_{1-4}$-alkyl groups. Preferred $C_{1-4}$-alkyl groups are selected from methyl, ethyl, ethenyl (vinyl), n-propyl, 2-propyl, 1-propynyl, 2-propynyl, propenyl ($-CH_2CH=CH_2$, $-CH=CH-CH_3$, $-C(=CH_2)-CH_3$), n-butyl, 1-butynyl, 2-butynyl, 1-butenyl, 2-butenyl, isobutyl, sec-butyl, tert-butyl and 3-methylbut-1-ynyl.

Further according to the present invention, the terms "$C_{1-6}$-alkylene"; "$C_{1-4}$-alkylene" and "$C_{1-2}$-alkylene" relate to a linear or branched, preferably linear, and preferably saturated aliphatic residues which are preferably selected from the

group consisting of methylene ($-CH_2-$), ethylene ($-CH_2CH_2-$), propylene ($-CH_2CH_2CH_2-$ or $-C(CH_3)_2-$), butylene ($-CH_2CH_2CH_2CH_2-$), pentylene ($-CH_2CH_2CH_2CH_2CH_2-$) and hexylene ($-CH_2CH_2CH_2CH_2CH_2CH_2-$); more preferably methylene ($-CH_2-$) and ethylene ($-CH_2CH_2-$) and most preferably methylene ($-CH_2-$). Preferably, $C_{1-6}$-alkylene is selected from $C_{1-4}$-alkylene, more preferably from $C_{1-2}$-alkylene.

Still further according to the present invention, the terms "$C_{3-10}$-cycloalkyl" and "$C_{3-6}$-cycloalkyl" preferably mean cyclic aliphatic hydrocarbons containing 3, 4, 5, 6, 7, 8, 9 or 10 carbon atoms and 3, 4, 5 or 6 carbon atoms, respectively, wherein the hydrocarbons in each case can be saturated or unsaturated (but not aromatic), unsubstituted or mono- or polysubstituted. Preferably, $C_{3-10}$-cycloalkyl and $C_{3-6}$-cycloalkyl are saturated. The $C_{3-10}$-cycloalkyl and $C_{3-6}$-cycloalkyl can be bound to the respective superordinate general structure via any desired and possible ring member of the cycloalkyl group. The $C_{3-10}$-cycloalkyl and $C_{3-6}$-cycloalkyl groups can also be condensed with further saturated, (partially) unsaturated, (hetero)cyclic, aromatic or heteroaromatic ring systems, i.e. with cycloalkyl, heterocyclyl, phenyl or heteroaryl residues, which in each case can in turn be unsubstituted or mono- or polysubstituted. Further, $C_{3-10}$-cycloalkyl and $C_{3-6}$-cycloalkyl can be singly or multiply bridged such as, for example, in the case of adamantyl, bicyclo[2.2.1]heptyl or bicyclo[2.2.2]octyl. However, preferably, $C_{3-10}$-cycloalkyl and $C_{3-6}$-cycloalkyl are neither condensed with further ring systems nor bridged. More preferably, $C_{3-10}$-cycloalkyl and $C_{3-6}$-cycloalkyl are neither condensed with further ring systems nor bridged and are saturated. Preferred $C_{3-10}$-cycloalkyl groups are selected from the group consisting of cyclopropyl, cyclobutyl, cyclopentyl, cyclohexyl, cyclopentenyl, cyclohexenyl, cycloheptyl, cyclooctyl, cyclononyl, cyclodecyl, adamantly, cyclopentenyl, cyclohexenyl, cycloheptenyl, cyclooctenyl, bicyclo[2.2.1]heptyl and bicyclo[2.2.2]octyl. Particularly preferred $C_{3-10}$-cycloalkyl groups are selected from $C_{3-6}$-cycloalkyl groups. Preferred $C_{3-6}$-cycloalkyl groups are selected from the group consisting of cyclopropyl, cyclobutyl, cyclopentyl, cyclohexyl, cyclopentenyl and cyclohexenyl. Particularly preferred $C_{3-6}$-cycloalkyl groups are selected from the group consisting of cyclopropyl, cyclobutyl, cyclopentyl and cyclohexyl, most preferably cyclopropyl.

According to the present invention, the term "3 to 7-membered heterocycloalkyl" preferably mean heterocycloaliphatic saturated or unsaturated (but not aromatic) residues having 3 to 7, i.e. 3, 4, 5, 6 or 7 ring members, wherein in each case at least one, if appropriate also two or three carbon atoms are replaced by a heteroatom or a heteroatom group each selected independently of one another from the group consisting of O, S, S(=O), $S(=O)_2$, N, NH and N($C_{1-4}$-alkyl) such as $N(CH_3)$, wherein the carbon atoms of the ring can be unsubstituted or mono- or polysubstituted. Preferably, 3 to 7-membered heterocycloalkyl is saturated. The 3 to 7-membered heterocycloalkyl groupcan also be condensed with further saturated or (partially) unsaturated cycloalkyl or heterocyclyl, aromatic or heteroaromatic ring systems. However, more preferably, 3 to 7-membered heterocycloalkyl is not condensed with further ring systems. Still more preferably, 3 to 7-membered heterocycloalkyl isnot condensed with further ring systems and are saturated. The 3 to 7-membered heterocycloalkyl group can be bound to the superordinate general structure via any desired and possible ring member of the heterocycloaliphatic residue if not indicated otherwise. In a preferred embodiment, 3 to 7-membered heterocycloalkyl are bound to the superordinate general structure via a carbon atom.

Preferred 3 to 7-membered heterocycloalkyl groups are selected from the group consisting of azepanyl, dioxepanyl, oxazepanyl, diazepanyl, thiazolidinyl, tetrahydrothiophenyl, tetrahydropyridinyl, thiomorpholinyl, tetrahydropyranyl, oxetanyl, oxiranyl, tetrahydrofuranyl, morpholinyl, pyrrolidinyl, 4-methylpiperazinyl, morpholinonyl, azetidinyl, aziridinyl, dithiolanyl, dihydropyrrolyl, dioxanyl, dioxolanyl, dihydropyridinyl, dihydrofuranyl, dihydroisoxazolyl, dihydrooxazolyl, imidazolidinyl, isoxazolidinyl, oxazolidinyl, piperazinyl, piperidinyl, pyrazolidinyl, pyranyl; tetrahydropyrrolyl, dihydroquinolinyl, dihydroisoquinolinyl, dihydroindolinyl, dihydroisoindolyl, tetrahydroquinolinyl, tetrahydroisoquinolinyl and tetrahydroindolinyl. More preferably, 3 to 7-membered heterocycloalkyl groups are selected from the group consisting of tetrahydropyranyl, oxetanyl, oxiranyl, tetrahydrofuranyl, thiazolidinyl, tetrahydrothiophenyl, tetrahydropyridinyl, thiomorpholinyl, morpholinyl, pyrrolidinyl, 4-methylpiperazinyl, morpholinonyl, azetidinyl, aziridinyl, dithiolanyl, dihydropyrrolyl, dioxanyl, dioxolanyl, dihydropyridinyl, dihydrofuranyl, dihydroisoxazolyl, dihydrooxazolyl, imidazolidinyl, isoxazolidinyl, oxazolidinyl, piperazinyl, piperidinyl, pyrazolidinyl, pyranyl, tetrahydropyrrolyl, dihydroindolinyl, dihydroisoindolyl and tetrahydroindolinyl. Particularly preferred 3 to 7-membered heterocycloalkyl groups are selected from the group consisting of tetrahydropyranyl, oxetanyl, oxiranyl, and tetrahydrofuranyl.

According to the present invention, the terms "5- to 10-membered heteroaryl" and "5- to 6-membered heteroaryl", respectively, preferably mean a mono- or bicyclic aromatic residue containing at least 1, if appropriate also 2, 3, 4 or 5 heteroatoms, wherein the heteroatoms are each selected independently of one another from the group S, N and O and the heteroaryl residue can be unsubstituted or mono- or polysubstituted, if not indicated otherwise. In the case of substitution on the heteroaryl, the substituents can be the same or different and be in any desired and possible position of the heteroaryl. The binding to the superordinate general structure can be carried out via any desired and possible ring member of the heteroaryl residue if not indicated otherwise. Preferably, the 5- to 6-membered heteroaryl and 5-to 6-membered heteroaryl, respectively, is bound to the suprordinate general structure via a carbon atom of the heterocycle. In another preferred embodiment, the 5- to 10-membered heteroaryl and 5- to 6-membered heteroaryl, respectively, is bound to the suprordinate general structure via a heteroatom of the heterocycle. The heteroaryl can also be part of a bi- or polycyclic system having up to 14 ring members, wherein the ring system can be formed with further saturated or (partially)

unsaturated cycloalkyl or heterocycloalkyl, aromatic or heteroaromatic ring systems, which can in turn be unsubstituted or mono- or polysubstituted, if not indicated otherwise. In a preferred embodiment, the heteroaryl is part of a bi- or polycyclic, preferably bicyclic, system. In another preferred embodiment, the heteroaryl is not part of a bi- or polycyclic system. Preferably, the 5- to 6-membered heteroaryl is selected from the group consisting of indolyl, indazolyl, pyridyl, preferably 2-pyridyl, 3-pyridyl or 4-pyridyl, pyrazolyl, pyrazolopyrimidinyl, pyrrolopyridinyl, pyrimidinyl, pyridazinyl, pyrazinyl, pyrrolyl, imidazolyl, oxazolyl, isoxazolyl, thiazolyl, isothiazolyl, furanyl, thienyl (thiophenyl), triazolyl, thiadiazolyl, 4,5,6,7-tetrahydro-2H-indazolyl, 2,4,5,6-tetrahydrocyclo-penta[c]pyrazolyl, benzofuranyl, benzoimidazolyl, benzothienyl, benzothiadiazolyl, benzothiazolyl, benzotriazolyl, benzooxazolyl, benzooxadiazolyl, quinazolinyl, quinoxalinyl, carbazolyl, quinolinyl, dibenzofuranyl, dibenzothienyl, imidazothiazolyl, indolizinyl, isoquinolinyl, naphthyridinyl, oxazolyl, oxadiazolyl, phenazinyl, phenothiazinyl, phthalazinyl, purinyl, phenazinyl, tetrazolyl and triazinyl.

The compounds according to the present invention are defined by substituents, for example by $R^1$ and $R^3$ ($1^{st}$ generation substituents) which may optionally be for their part themselves be substituted ($2^{nd}$ generation substituents). Depending on the definition, these substituents of the substituents can optionally be for their part resubstituted ($3^{rd}$ generation substituents). If, for example, $R^3$ = a $C_{1-10}$-alkyl ($1^{st}$ generation substituent), then the $C_{1-10}$-alkyl can for its part be substituted, for example with a N(H)($C_{1-6}$-alkyl) ($2^{nd}$ generation substituent). This produces the functional group $R^3$ = ($C_{1-10}$-alkyl-NH-$C_{1-6}$-alkyl). The NH-$C_{1-6}$-alkyl can then for its part be resubstituted, for example with Cl ($3^{rd}$ generation substituent). Overall, this produces the functional group $R^3$ = $C_{1-10}$-alkyl-NH-$C_{1-6}$-alkyl, wherein the $C_{1-6}$-alkyl of the NH-$C_{1-6}$-alkyl is substituted by Cl. However, in a preferred embodiment, the $3^{rd}$ generation substituents may not be resubstituted, i.e. there are then no $4^{th}$ generation substituents. More preferably, the $2^{nd}$ generation substituents may not be resubstituted, i.e. there are no $3^{rd}$ generation substituents.

If a residue occurs multiply within a molecule, then this residue can have respectively different meanings for various substituents: if, for example, both $R^3$ and $R^4$ denote $C_{1-6}$-alkyl, then $C_{1-6}$-alkyl can e.g. represent ethyl for $R^3$ and can represent methyl for $R^4$.

In connection with the terms "$C_{1-10}$-alkyl", "$C_{1-6}$-alkyl", "$C_{1-4}$-alkyl","$C_{3-10}$-cycloalkyl". "$C_{3-6}$-cycloalkyl", "3 to 7 membered heterocycloalkyl", "$C_{1-6}$-alkylene", "$C_{1-4}$-alkylene" and "$C_{1-2}$-alkylene", the term "substituted" refers in the sense of the present invention, with respect to the corresponding residues or groups, to the single substitution (monosubstitution) or multiple substitution (polysubstitution), e.g. disubstitution or trisubstitution; more preferably to monosubstitution or disubstitution; of one or more hydrogen atoms each independently of one another by at least one substituent. In case of a multiple substitution, i.e. in case of polysubstituted residues, such as di- or trisubstituted residues, these residues may be polysubstituted either on different or on the same atoms, for example trisubstituted on the same carbon atom, as in the case of $CF_3$, $CH_2CF_3$ or disubstituted as in the case of 1,1-difluorocyclohexyl, or at various points, as in the case of CH(OH)-CH=CH-$CHCl_2$ or 1-chloro-3-fluorocyclohexyl. The multiple substitution can be carried out using the same or using different substituents.

In relation to the terms "phenyl", "heteroaryl" and "5- to 10-membered heteroaryl", the term "substituted" refers in the sense of this invention to the single substitution (monosubstitution) or multiple substitution (polysubstitution), e.g. disubstitution or trisubstitution, of one or more hydrogen atoms each independently of one another by at least one substituent. The multiple substitution can be carried out using the same or using different substituents.

According to the present invention, preferably $C_{1-10}$-alkyl, $C_{1-6}$-alkyl, $C_{1-4}$-alkyl, $C_{3-10}$-cycloalkyl, $C_{3-6}$-cycloalkyl, 3 to 7 membered heterocycloalkyl, $C_{1-6}$-alkylene, $C_{1-4}$-alkylene and $C_{1-2}$-alkylene in each case independently from one another are unsubstituted or mono- or polysubstituted with one or more substituents selected from F; Cl; Br; I; CN; $C_{1-6}$-alkyl; $CF_3$; $CF_2H$; $CFH_2$; $CF_2Cl$; $CFCl_2$; C(O)-$C_{1-6}$-alkyl; C(O)-OH; C(O)-O$C_{1-6}$-alkyl; C(O)-$NH_2$; C(O)-N(H)($C_{1-6}$-alkyl); C(O)-N($C_{1-6}$-alkyl)$_2$; OH; =O; $OCF_3$; $OCF_2H$; $OCFH_2$; $OCF_2Cl$; $OCFCl_2$; O-$C_{1-6}$-alkyl; O-C(O)-$C_{1-6}$-alkyl; O-C(O)-O-$C_{1-6}$-alkyl; O-(CO)-N(H)($C_{1-6}$-alkyl); O-C(O)-N($C_{1-6}$-alkyl)$_2$; O-S(O)$_2$-$NH_2$; O-S(O)$_2$-N(H)($C_{1-6}$-alkyl); O-S(O)$_2$-N($C_{1-6}$-alkyl)$_2$; $NH_2$; N(H)($C_{1-6}$-alkyl); N($C_{1-6}$-alkyl)$_2$; N(H)-C(O)-$C_{1-6}$-alkyl; N(H)-C(O)-O-$C_{1-6}$-alkyl; N(H)-C(O)-$NH_2$; N(H)-C(O)-N(H)($C_{1-6}$-alkyl); N(H)-C(O)-N($C_{1-6}$-alkyl)$_2$; N($C_{1-6}$-alkyl)-C(O)-$C_{1-6}$-alkyl; N($C_{1-6}$-alkyl)-C(O)-O-$C_{1-6}$-alkyl; N($C_{1-6}$-alkyl)-C(O)-$NH_2$; N($C_{1-6}$-alkyl)-C(O)-N(H)($C_{1-6}$-alkyl); N($C_{1-6}$-alkyl)-C(O)-N($C_{1-6}$-alkyl)$_2$; N(H)-S(O)$_2$OH; N(H)-S(O)$_2$-$C_{1-6}$-alkyl; N(H)-S(O)$_2$-O-$C_{1-6}$-alkyl; N(H)-S(O)$_2$-$NH_2$; N(H)-S(O)$_2$-N(H)($C_{1-6}$-alkyl); N(H)-S(O)$_2$N($C_{1-6}$-alkyl)$_2$; N($C_{1-6}$-alkyl)-S(O)$_2$-OH; N($C_{1-6}$-alkyl)-S(O)$_2$-$C_{1-6}$-alkyl; N($C_{1-6}$-alkyl)-S(O)$_2$-O-$C_{1-6}$-alkyl; N($C_{1-6}$-alkyl)-S(O)$_2$-$NH_2$; N($C_{1-6}$-alkyl)-S(O)$_2$-N(H)($C_{1-6}$-alkyl); N($C_{1-6}$-alkyl)-S(O)$_2$-N($C_{1-6}$-alkyl)$_2$; $SCF_3$; $SCF_2H$; $SCFH_2$; S-$C_{1-6}$-alkyl; S(O)-$C_{1-6}$-alkyl; S(O)$_2$-$C_{1-6}$-alkyl; S(O)$_2$-OH; S(O)$_2$-O-$C_{1-6}$-alkyl; S(O)$_2$-$NH_2$; S(O)$_2$-N(H)($C_{1-6}$-alkyl); S(O)$_2$-N($C_{1-6}$-alkyl)$_2$; $C_{3-6}$-cycloalkyl; 3 to 7-membered heterocycloalkyl; phenyl; 5 or 6-membered heteroaryl; O-$C_{3-6}$-cycloalkyl; O-(3 to 7-membered heterocycloalkyl); O-phenyl; O-(5 or 6-membered heteroaryl); C(O)-$C_{3-6}$-cycloalkyl; C(O)-(3 to 7-membered heterocycloalkyl); C(O)-phenyl; C(O)-(5 or 6-membered heteroaryl); S(O)$_2$-($C_{3-6}$-cycloalkyl); S(O)$_2$-(3 to 7-membered heterocycloalkyl); S(O)$_2$-phenyl and S(O)$_2$-(5 or 6-membered heteroaryl).

Preferred substituents of $C_{1-10}$-alkyl, $C_{1-6}$-alkyl, $C_{1-4}$-alkyl, $C_{3-10}$-cycloalkyl, $C_{3-6}$-cycloalkyl, 3 to 7 membered heterocycloalkyl, $C_{1-6}$-alkylene and $C_{1-4}$-alkylene are selected from the group consisting of F; Cl; Br; I; CN; $C_{1-6}$-alkyl; $CF_3$; $CF_2H$; $CFH_2$; C(O)-$NH_2$; C(O)-N(H)($C_{1-6}$-alkyl); C(O)-N($C_{1-6}$-alkyl)$_2$; OH; $OCF_3$; $OCF_2H$; $OCFH_2$; O-$C_{1-6}$-alkyl; $NH_2$; N(H)($C_{1-6}$-alkyl); N($C_{1-6}$-alkyl)$_2$; $SCF_3$; $SCF_2H$; $SCFH_2$; S-$C_{1-6}$-alkyl; S(O)-$C_{1-6}$-alkyl; S(O)$_2$-$C_{1-6}$-alkyl; $C_{3-6}$-cycloalkyl; 3 to 7-membered heterocycloalkyl; phenyl and 5 or 6-membered heteroaryl; and particularly preferably F, CN, $CH_3$, $CH_2CH_3$, $CF_3$; $CF_2H$; $CFH_2$; C(O)-$NH_2$; C(O)-N(H)($CH_3$); C(O)-N($CH_3$)$_2$; OH, $NH_2$, $OCH_3$, $SCH_3$, S(O)$_2$($CH_3$), S(O)($CH_3$), N($CH_3$)$_2$, cyclopropyl and oxetanyl. According to this embodiment, $C_{1-10}$-alkyl, $C_{1-6}$-alkyl, $C_{1-4}$-alkyl, $C_{3-10}$-cycloalkyl, $C_{3-6}$-cycloalkyl, and 3 to 7 membered heterocycloalkyl, are preferably each independently from one another unsubstituted, mono- di- or trisubstituted, more preferably unsubstituted or monosubstituted or disubstituted with a substituent selected from the group consisting of F; Cl; Br; I; CN; $C_{1-6}$-alkyl; $CF_3$; $CF_2H$; $CFH_2$; C(O)-$NH_2$; C(O)-N(H)($C_{1-6}$-alkyl); C(O)-N($C_{1-6}$-alkyl)$_2$; OH; $OCF_3$; $OCF_2H$; $OCFH_2$; O-$C_{1-6}$-alkyl; $NH_2$; N(H)($C_{1-6}$-alkyl); N($C_{1-6}$-alkyl)$_2$; $SCF_3$; $SCF_2H$; $SCFH_2$; S-$C_{1-6}$-alkyl; S(O)-$C_{1-6}$-alkyl; S(O)$_2$-$C_{1-6}$-alkyl; $C_{3-6}$-cycloalkyl; 3 to 7-membered heterocycloalkyl; phenyl and 5 or 6-membered heteroaryl. Preferably, $C_{1-6}$-alkylene groups and $C_{1-4}$-alkylene groups are unsubstituted.

According to the present invention, preferably phenyl and 5 to 10-membered heteroaryl in each case independently from one another are unsubstituted or mono- or polysubstituted with one or more substituents selected from F; Cl; Br; I; CN; $C_{1-6}$-alkyl; $C_{1-6}$-alkenyl; $C_{1-6}$-alkynyl; $C_{1-6}$-alkynyl-C(H)(OH)$CH_3$; $C_{1-6}$-alkynyl-C($CH_3$)$_2$OH; $CF_3$; $CF_2H$; $CFH_2$; $CF_2Cl$; $CFCl_2$; $C_{1-4}$-alkylene-$CF_3$; $C_{1-4}$-alkylene-$CF_2H$; $C_{1-4}$-alkylene-$CFH_2$; $C_{1-6}$-alkylene-OH; $C_{1-6}$-alkylene-$OCH_3$; C(O)-$C_{1-6}$-alkyl; C(O)-OH; C(O)-O$C_{1-6}$-alkyl; C(O)-N(H)(OH); C(O)-$NH_2$; C(O)-N(H)($C_{1-6}$-alkyl); C(O)-N($C_{1-6}$-alkyl)$_2$; OH; $OCF_3$; $OCF_2H$; $OCFH_2$; $OCF_2Cl$; $OCFCl_2$; O-$C_{1-6}$-alkyl; O-$C_{3-6}$-cycloalkyl; O-(3 to 7-membered heterocycloalkyl); $NH_2$; N(H)($C_{1-6}$-alkyl); N($C_{1-6}$-alkyl)$_2$; N(H)-C(O)-$C_{1-6}$-alkyl; N($C_{1-6}$-alkyl)-C(O)-$C_{1-6}$-alkyl; N(H)-C(O)-$NH_2$; N(H)-C(O)-N(H)($C_{1-6}$-alkyl); N(H)-C(O)-N($C_{1-6}$-alkyl)$_2$; N($C_{1-6}$-alkyl)-C(O)-N(H)($C_{1-6}$-alkyl); N($C_{1-6}$-alkyl)-C(O)-N($C_{1-6}$-alkyl)$_2$; N(H)-S(O)$_2$-$C_{1-6}$-alkyl; $SCF_3$; S-$C_{1-6}$-alkyl; S(O)-$C_{1-6}$-alkyl; S(O)$_2$-$C_{1-6}$-alkyl; S(O)$_2$-$C_{3-6}$-cycloalkyl; S(O)$_2$-$C_{1-6}$-alkylene-$C_{3-6}$-cycloalkyl; S(O)$_2$-$NH_2$; S(O)$_2$-N(H)($C_{1-6}$-alkyl); S(O)$_2$-N($C_{1-6}$-alkyl)$_2$; $C_{3-6}$-cycloalkyl; $C_{1-4}$-alkylene-$C_{3-6}$-cycloalkyl; 3 to 7-membered heterocycloalkyl; $C_{1-4}$-alkylene-(3 to 7-membered heterocycloalkyl); phenyl or 5 or 6-membered heteroaryl. According to this embodiment, phenyl and 5 to 10-membered heteroaryl are preferably each independently from one another unsubstituted, mono- di- or trisubstituted, more preferably unsubstituted or monosubstituted or disubstituted.

In a particularly preferred embodiment, the compound according to the present invention is selected from the group consisting of

| | |
|---|---|
| **1** | 7-fluoro-8-(3-fluoro-5-methylphenyl)-1,4,4,9-tetramethyl-5H-imidazo[1,2-a]quinoxaline |
| **2** | 7,9-difluoro-1,4,4-trimethyl-8-(1H-pyrazol-3-yl)-5H-pyrrolo[1,2-a]quinoxaline |
| **3** | 7,9-difluoro-8-(1H-indol-4-yl)-1,4,4-trimethyl-5H-pyrrolo[1,2-a]quinoxaline |
| **4** | 7,9-difluoro-1,4,4-trimethyl-8-pyrazolo[1,5-a]pyrimidin-3-yl-5H-pyrrolo[1,2-a]quinoxaline |
| **5** | 7,9-difluoro-8-(6-fluoro-1H-indol-4-yl)-1,4,4-trimethyl-5H-imidazo[1,2-a]quinoxaline |
| **6** | 7-fluoro-8-[2-methoxy-5-(trifluoromethyl)pyridin-3-yl]-1,4,4,9-tetramethyl-5H-imidazo[1,2-a]quinoxaline |
| **7** | 7-fluoro-1,4,4,9-tetramethyl-8-[6-(trifluoromethyl)-1H-indol-4-yl]-5H-imidazo[1,2-a]quinoxaline |
| **8** | 8-[1-(cyclopropylmethyl)indol-4-yl]-7-fluoro-1,4,4,9-tetramethyl-5H-imidazo[1,2-a]quinoxaline |
| **9** | 8-[1-(cyclopropylmethylsulfonyl)indol-4-yl]-7-fluoro-1,4,4,9-tetramethyl-5H-imidazo[1,2-a]quinoxaline |
| **10** | 8-(1-cyclopropylindol-4-yl)-7-fluoro-1,4,4,9-tetramethyl-5H-imidazo[1,2-a]quinoxaline |
| **11** | 9-fluoro-1,4,4-trimethyl-8-(3-methyl-1H-indol-7-yl)-4,5-dihydropyrido[3,4-e][1,2,4]triazolo[4,3-a]pyrazine |
| **12** | 7,9-difluoro-1,4,4-trimethyl-8-(1H-pyrrolo[2,3-b]pyridin-4-yl)-5H-pyrrolo[1,2-a]quinoxaline |
| **13** | 8-(5-fluoro-3-methyl-1H-indol-7-yl)-1,4,4,9-tetramethyl-4,5-dihydropyrido[2,3-e] [1,2,4]triazolo[4,3-a]pyrazine |
| **14** | 7-fluoro-8-(6-fluoro-1-methylsulfonylindol-4-yl)-1,4,4,9-tetramethyl-5H-imidazo[1,2-a]quinoxaline |
| **15** | 8-(5-chloro-2-methoxypyridin-3-yl)-7-fluoro-1,4,4,9-tetramethyl-5H-imidazo[1,2-a]quinoxaline |
| **16** | 7-fluoro-8-[5-fluoro-3-(oxolan-3-yl)-1H-indol-7-yl]-1,4,4,9-tetramethyl-5H-imidazo[1,2-a]quinoxaline |
| **17** | 7-fluoro-8-(5-fluoro-3-prop-1-ynyl-1H-indol-7-yl)-1,4,4,9-tetramethyl-5H-imidazo[1,2-a]quinoxaline |
| **18** | 9-fluoro-8-(6-fluoro-1-(methylsulfonyl)-1H-indol-4-yl)-1,4,4-trimethyl-4,5-dihydropyrido[3,4-e][1,2,4]triazolo [4,3-a]pyrazine |
| **19** | 7-fluoro-1,4,4,9-tetramethyl-8-(1-methylsulfonylindazol-4-yl)-5H-imidazo[1,2-a]quinoxaline |
| **20** | 7,9-difluoro-1,4,4-trimethyl-8-(1-methylsulfonylindazol-4-yl)-5H-pyrrolo[1,2-a]quinoxaline |
| **21** | 1-[4-(7,9-difluoro-1,4,4-trimethyl-5H-pyrrolo[1,2-a]quinoxalin-8-yl)indol-1-yl]ethanone |
| **22** | 8-(3-cyclopropyl-1H-indol-7-yl)-7,9-difluoro-4,4-dimethyl-5H-tetrazolo[1,5-a]quinoxaline |
| **23** | 7-fluoro-8-(6-fluoro-1-methylsulfonylindol-4-yl)-4,4-dimethyl-9-(trifluoromethyl)-5H-tetrazolo[1,5-a]quinoxaline |
| **24** | 7-fluoro-8-(6-fluoro-1-methylsulfonylindol-4-yl)-1,4,4,9-tetramethyl-5H-triazolo[4,5-c]quinoline |
| **25** | 7-fluoro-8-(5-fluoro-3-methyl-1H-indol-7-yl)-1,4,4,9-tetramethyl-5H-triazolo[4,5-c]quinoline |
| **26** | 2-[6-fluoro-4-(7-fluoro-1,4,4,9-tetramethyl-5H-triazolo[4,5-c]quinolin-8-yl)indol-1-yl]ethanol |
| **27** | 8-fluoro-9-(6-fluoro-l-methylsulfonylindol-4-yl)-1,5,5,10-tetramethyl-6H-pyrazolo[1,5-c]quinazoline |

(continued)

| | |
|---|---|
| **28** | 8,10-difluoro-9-(6-fluoro-1-methylsulfonylindol-4-yl)-5,5-dimethyl-6H-pyrazolo[1,5-c]quinazoline |
| **29** | 2-(6-fluoro-1-(methylsulfonyl)-1H-indol-4-yl)-6,6,9-trimethyl-5,6-dihydropyrido[3,2-e][1,2,4]triazolo[4,3-a]pyrazine |
| **30** | 3-fluoro-6,6,9-trimethyl-2-(3-methyl-1H-indol-7-yl)-5,6-dihydropyrido[3,2-e][1,2,4]triazolo[4,3-a]pyrazine |
| **31** | 1,4,4,9-tetramethyl-8-(3-methyl-1H-indol-7-yl)-4,5-dihydropyrido[3,4-e][1,2,4]triazolo[4,3-a]pyrazine |
| **32** | 6-fluoro-8-(6-fluoro-1-methylsulfonylindol-4-yl)-1,4,4,9-tetramethyl-5H-pyrazolo[4,3-c]quinoline |
| **33** | 6-fluoro-1,4,4,9-tetramethyl-8-(1-methylsulfonylindol-4-yl)-5H-pyrazolo[4,3-c]quinoline |
| **34** | 6-fluoro-8-(6-fluoro-1-methylsulfonylindazol-4-yl)-1,4,4,9-tetramethyl-5H-pyrazolo[4,3-c]quinoline |
| **35** | 7-fluoro-9-(6-fluoro-1-methylsulfonylindol-4-yl)-1,5,5,10-tetramethyl-6H-triazolo[1,5-c]quinazoline |
| **36** | 7-fluoro-9-(6-fluoro-1-methylsulfonylindazol-4-yl)-1,5,5,10-tetramethyl-6H-triazolo[1,5-c]quinazoline |
| **37** | 6-fluoro-8-(6-fluoro-1-methylsulfonylindol-4-yl)-1,9-dimethylspiro[5H-pyrazolo[4,3-c]quinoline-4,1-cyclobutane] |
| **38** | 6-fluoro-1,9-dimethyl-8-(1-methylsulfonylindazol-4-yl)spiro[5H-pyrazolo[4,3-c]quinoline-4,1-cyclobutane] |
| **39** | 6-fluoro-8-(5-fluoro-3-methyl-1H-indol-7-yl)-1,9-dimethylspiro[5H-pyrazolo[4,3-c]quinoline-4,1-cyclobutane] |
| **40** | 1-ethyl-6-fluoro-8-(6-fluoro-1-methylsulfonylindazol-4-yl)-4,4,9-trimethyl-5H-pyrazolo[4,3-c]quinoline |
| **41** | 1-ethyl-6-fluoro-8-(6-fluoro-1-methylsulfonylindol-4-yl)-4,4,9-trimethyl-5H-pyrazolo[4,3-c]quinoline |
| **42** | 1-(cyclopropylmethyl)-6-fluoro-8-(6-fluoro-1-methylsulfonylindol-4-yl)-4,4,9-trimethyl-5H-pyrazolo[4,3-c]quinoline |
| **43** | 1-(cyclopropylmethyl)-6-fluoro-8-(5-fluoro-3-methyl-1H-indol-7-yl)-4,4,9-trimethyl-5H-pyrazolo[4,3-c]quinoline |
| **44** | 1-(cyclopropylmethyl)-6-fluoro-8-(6-fluoro-1-methylsulfonylindazol-4-yl)-4,4,9-trimethyl-5H-pyrazolo[4,3-c]quinoline |
| **45** | 7-fluoro-9-(6-fluoro-1-methylsulfonylindazol-4-yl)-5,5,10-trimethyl-6H-pyrazolo[1,5-c]quinazoline |
| **46** | 7-fluoro-1,4,4,9-tetramethyl-8-(1-methylsulfonylindol-4-yl)-5H-pyrazolo[4,3-c]quinoline |
| **47** | 6-fluoro-8-(6-fluoro-1-methylsulfonylindol-4-yl)-1,4,4,9-tetramethyl-5H-imidazo[4,5-c]quinoline |
| **48** | 6-fluoro-8-(6-fluoro-1-methylsulfonylindazol-4-yl)-1,4,4,9-tetramethyl-5H-imidazo[4,5-c]quinoline |
| **49** | 6-fluoro-8-(5-fluoro-3-methyl-1<I>H</I>-indol-7-yl)-1,4,4,9-tetramethyl-5H-imidazo[4,5-c]quinoline |
| **50** | 7-fluoro-8-(6-fluoro-1-methylsulfonylindazol-4-yl)-1,3,4,4,9-pentamethyl-5H-pyrazolo[4,3-c]quinoline |
| **51** | 6-fluoro-8-(6-fluoro-1-methylsulfonylindol-4-yl)-1,3,4,4,9-pentamethyl-5H-pyrazolo[4,3-c]quinoline |
| **52** | 6,7-difluoro-8-(5-fluoro-3-methyl-1-indol-7-yl)-1,4,4-trimethyl-5H-pyrazolo[4,3-c]quinoline |
| **53** | 6-fluoro-1,3,9-trimethyl-8-(1-methylsulfonylindol-4-yl)spiro[5H-pyrazolo[4,3-c]quinoline-4,1-cyclobutane] |
| **54** | 6-fluoro-1,3,9-trimethyl-8-(1-methylsulfonylindazol-4-yl)spiro[5H-pyrazolo[4,3-c]quinoline-4,1-cyclobutane] |
| **55** | 6-fluoro-8-(5-fluoro-3-methyl-1H-indol-7-yl)-4,4,9-trimethyl-2,5-dihydropyrazolo[4,3-c]quinoline |
| **56** | 6-fluoro-1,3,9-trimethyl-8-(3-methyl-1H-indol-7-yl)spiro[5H-pyrazolo[4,3-c]quinoline-4,1-cyclobutane] |
| **57** | 6-fluoro-8-(6-fluoro-1-methylsulfonylindol-4-yl)-4,4,9-trimethyl-2-methylsulfonyl-5H-pyrazolo[4,3-c]quinoline |
| **58** | 6-fluoro-8-(6-fluoro-1-methylsulfonylindol-4-yl)-4,4,9-trimethyl-2,5-dihydropyrazolo[4,3-c]quinoline |
| **59** | 6-fluoro-8-(6-fluoro-1-methylsulfonylindazol-4-yl)-4,4,9-trimethyl-2,5-dihydropyrazolo[4,3-c]quinoline |
| **60** | 8-(6-fluoro-1-methylsulfonylindazol-4-yl)-1,4,4,9-tetramethyl-5H-triazolo[4,5-c]quinoline |
| **61** | 7-fluoro-8-(6-fluoro-1-methylsulfonylindol-4-yl)-4,4,9-trimethyl-5H-[1,3]oxazolo[4,5-c]quinoline |
| **62** | 7-fluoro-8-(5-fluoro-3-methyl-1H-indol-7-yl)-4,4,9-trimethyl-5H-[1,3]oxazolo[4,5-c]quinoline |
| **63** | 7-fluoro-8-(6-fluoro-1-methylsulfonyfindazol-4-yl)-4,4,9-trimethyl-5H-[1,3]oxazolo[4,5-c]quinofine |

in the form of the free compound or a physiologically acceptable salt thereof.

The compounds according to the present invention can be synthesized by standard reactions in the field of organic chemistry known to the person skilled in the art or in a manner as described herein (cf. Reaction Scheme 1 below) or analogously. The reaction conditions in the synthesis routes described herein are known to the skilled person and are for some cases also exemplified in the Examples described herein.

Reaction scheme 1:

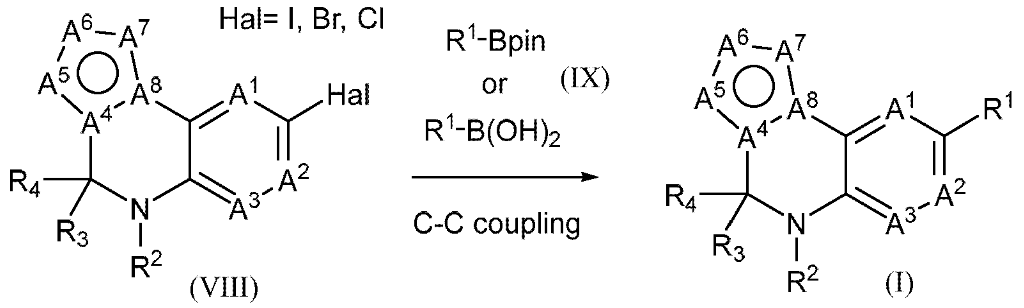


Compounds of the gerenal formula (I) can be obtained from a metal-catalyzed C-C coupling reaction between compounds of the general formula (VIII) and compounds of the general formula (IX). Metal-catalyzed C-C coupling reactions are known in the art (cf. Metal Catalyzed Cross-Coupling Reactions and More, 3 Volume Set Wiley, 2014**;** Angew. Chem. Int. Ed., 2012, 51, 5062 - 5085). Favorable C-C coupling reactions are palladium catalyzed cross coupling reactions (cf. Angew. Chem., 2005, 117, 4516 - 4563), using as favorable palladium-based catalysts $Pd(tBu_3)_2$, $Pd(PPh_3)_4$, Ataphos or $Pd_2(dba)_3$ in combination with XPhos as ligand. Favourable halides for cross coupling of compounds of the general formula (VIII) include I, Br, Cl, most favourably Br. Compounds of the general formula (VIII) can be synthesized following the exemplified synthetic routes. Compounds of the general formula (IX) are either commercially available or can be synthesized following the exemplified synthetic routes.

The compounds according to the present invention can be produced in the manner described here or in an analogous manner.

In a preferred embodiment, the compounds according to the present invention are modulators of the glucocorticoid receptor. In the sense of the present invention, the term "selective modulator of the glucocorticoid receptor (glucocorticoid receptor modulator)" preferably means that the respective compound exhibits in a cellular target engagement assay for agonistic or antagonistic potency on the glucocorticoid receptor an EC50 or IC50 value on the glucocorticoid receptor of at most 15 μM ($10 \cdot 10^{-6}$ mol/L) or at most 10 μM; more preferably at most 1 μM; still more preferably at most 500 nM ($10^{-9}$ mol/L); yet more preferably at most 300 nM; even more preferably at most 100 nM; most preferably at most 10 nM; and in particular at most 1 nM.

The person skilled in the art knows how to test compounds for modulation (agonistic or antagonistic) of the activity of the glucocorticoid receptor. Preferred target engagement assays for testing compounds for their agonistic or antagonistic potency (EC50, IC50) on the glucocorticoid receptor are described herein below:

**Glucocorticoid receptor cell-based assays**

Potential selective glucocorticoid receptor modulators of this intervention can be tested for modulation of the activity of the glucocorticoid receptor using cell-based assays. These assays involve a Chinese hamster ovary (CHO) cell line which contains fragments of the glucocorticoid receptor as well as fusion proteins. The glucocorticoid receptor fragments used are capable of binding the ligand (e.g. beclomethasone) to identify molecules that compete for binding with glucocorticoid receptor ligands. In more detail, the glucocorticoid receptor ligand binding domain is fused to the DNA binding domain (DBD) of the transcriptionfactor GAL4 (GAL4 DBD-GR) and is stably integrated into a CHO cell line containing a GAL4-UAS-Luciferase reporter construct. To identify selective glucocorticoid receptor modulators, the reporter cell line is incubated with the molecules using an 8-point half-log compound dilution curve for several hours. After cell lysis the luminescence that is produced by luciferase after addition of the substrate is detected and EC50 or IC50 values can be calcuated. Engagement of molecules which induce gene expression via glucocortocoid receptor binding to the DNA leads to expression of the luciferase gene under the control of the fusion protein GAL4 DBD-GR and therefore to a dose-dependent increase of the luminescence signal. Binding of molecules which repress beclomethasone-induced gene expression of the luciferase gene under the control of the fusion protein GAL4 DBD-GR leads to a dose-dependent reduction of the luminescence signal.

In a preferred embodiment, the compound according to the present invention exhibits in a cellular target engagement assay for agonistic or antagonistic potency on the glucocorticoid receptor an EC50 or IC50 value on the glucocorticoid receptor of at most 1 μM ($10^{-6}$ mol/L); still more preferably at most 500 nM ($10^{-9}$ mol/L); yet more preferably at most 300 nM; even more preferably at most 100 nM; most preferably at most 50 nM; and in particular at most 10 nM or at most 1 nM.

In a preferred embodiment, the compound according to the present invention exhibits in a cellular target engagement assay for agonistic or antagonistic potency on the glucocorticoid receptor an EC50 or IC50 value on the glucocorticoid receptor in the range of from 0.1 nM ($10^{-9}$ mol/L) to 1000 nM; still more preferably 1 nM to 800 nM; yet more preferably 1 nM to 500 nM; even more preferably 1 nM to 300 nM; most preferably 1 nM to 100 nM; and in particular 1 nM to

80 nM.

Preferably, the compounds according to the present invention are useful as selective modulators of the glucocorticoid receptor.

Therefore, the compounds according to the present invention are preferably useful for the in vivo treatment or prevention of diseases in which participation of the glucocorticoid receptor is implicated.

The present invention therefore further relates to a compound according to the present invention for use in the modulation of glucocorticoid receptor activity.

Therefore, another aspect of the present invention relates to a compound according to the present invention for use in the treatment and/or prophylaxis of a disorder which is mediated at least in part by the glucocorticoid receptor. Still another aspect of the present invention relates to a method of treatment of a disorder which is mediated at least in part by the glucocorticoid receptor comprising the administration of a therapeutically effective amount of a compound according to the present invention to a subject in need thereof, preferably a human.

A further aspect of the invention relates to the use of a compound according to the present invention as medicament. Another aspect of the present invention relates to a pharmaceutical dosage form comprising a compound according to the present invention. Preferably, the pharmaceutical dosage form comprises a compound according to the present invention and one or more pharmaceutical excipients such as physiologically acceptable carriers, additives and/or auxiliary substances; and optionally one or more further pharmacologically active ingredient. Examples of suitable physiologically acceptable carriers, additives and/or auxiliary substances are fillers, solvents, diluents, colorings and/or binders. These substances are known to the person skilled in the art (see H. P. Fiedler, Lexikon der Hilfsstoffe fur Pharmazie, Kosmetik und angrenzende Gebiete, Editio Cantor Aulendoff).

The pharmaceutical dosage form according to the present invention is preferably for systemic, topical or local administration, preferably for oral administration. Therefore, the pharmaceutical dosage form can be in form of a liquid, semisolid or solid, e.g. in the form of injection solutions, drops, juices, syrups, sprays, suspensions, tablets, patches, films, capsules, plasters, suppositories, ointments, creams, lotions, gels, emulsions, aerosols or in multiparticulate form, for example in the form of pellets or granules, if appropriate pressed into tablets, decanted in capsules or suspended in a liquid, and can also be administered as such.

The pharmaceutical dosage form according to the present invention is preferably prepared with the aid of conventional means, devices, methods and processes known in the art. The amount of the compound according to the present invention to be administered to the patient may vary and is e.g. dependent on the patient's weight or age and also on the type of administration, the indication and the severity of the disorder. Preferably 0.001 to 100 mg/kg, more preferably 0.05 to 75 mg/kg, most preferably 0.05 to 50 mg of a compound according to the present invention are administered per kg of the patient's body weight.

The glucocorticoid receptor is believed to have potential to modify a variety of diseases or disorders in mammals such as humans. These include in particular inflammatory diseases.

Another aspect of the present invention relates to a compound according to the present invention for use in the treatment and/or prophylaxis of pain and/or inflammation; more preferably inflammatory pain.

EXAMPLES

The following abbreviations are used in the descriptions of the experiments:

AcOH = acetic acid; Ac = acetyl group; Ataphos = bis(di-tert-butyl(4 dimethylaminophenyl)phosphine)dichloropalladium(II); Ar = argon; BISPIN (or Bis-Pin) = bis(pinacolato)diborane;
Cp* = Pentamethylcyclopentadienyl, dba = dibenzylideneacetone; DCM = dichloromethane; DIPEA = N,N-diisopropylethylamine; DMADMF = N,N-dimethylformamide dimethylacetal; DMAP = 4-(dimethylamino)-pyridine;
DMF = N,N-dimethylformamid; DMSO = dimethylsulfoxid; dppf = 1,1'; bis(diphenylphosphanyl)ferrocene; EtOAc = ethyl acetate; EtOH = ethanol; h = hour; LDA = lithiumdiisopropylamide; LiHMDS = lithium bis(trimethylsilyl)amide; MeOH = methanol; min = minute; n-BuLi = n-butyllithium; pin=(pinacolato)borane; RT = room temperature; Rt = retention time; tert = tertiary; TEA = triethylamine; THF = tetrahydrofuran; p-TSA = paratoluene sulfonic acid; TMSCl = trimethylsilyl chloride; Xantphos= 4,5-bis(diphenylphosphino)-9,9-dimethylxanthene, X-Phos = 2-dicyclohexylphosphino-2',4',6'-triisopropylbiphenyl

The intermediates in Table 1 are commercially available as the corresponding pinacolatoborane and/or as the corresponding boronic acid:

| Name | Structure |
|---|---|
| (3-fluoro-5-methylphenyl)boronic acid **Intermediate A1** | 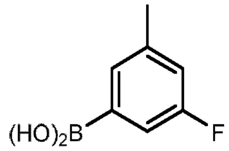 |
| 3-(4,4,5,5-tetramethyl-1,3,2-dioxaborolan-2-yl)-1H-pyrazole **Intermediate A2** | 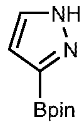 |
| 4-(4,4,5,5-tetramethyl-1,3,2-dioxaborolan-2-yl)-1H-indole **Intermediate A3** | 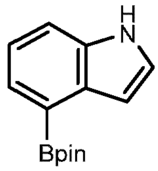 |
| 3-(4,4,5,5-tetramethyl-1,3,2-dioxaborolan-2-yl)pyrazolo[1,5-a]pyrimidine **Intermediate A4** | 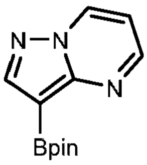 |
| 2-methoxy-3-(4,4,5,5-tetramethyl-1,3,2-dioxaborolan-2-yl)-5-(trifluoromethyl)pyridine **Intermediate A6** | 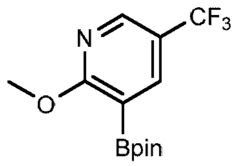 |
| 4-(4,4,5,5-tetramethyl-1,3,2-dioxaborolan-2-yl)-6-(trifluoromethyl)-1H-indole **Intermediate A7** | 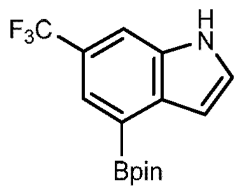 |
| 4-(4,4,5,5-tetramethyl-1,3,2-dioxaborolan-2-yl)-1H-pyrrolo[2,3-b]pyridine **Intermediate A12** | 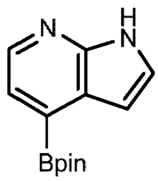 |
| 5-chloro-2-methoxy-3-(4,4,5,5-tetramethyl-1,3,2-dioxaborolan-2-yl)pyridine **Intermediate A15** | 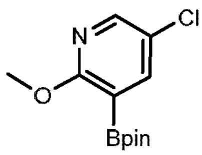 |

Synthesis of 6-fluoro-4-(4,4,5,5-tetramethyl-1,3,2-dioxaborolan-2-yl)-1H-indole **(intermediate A5)**

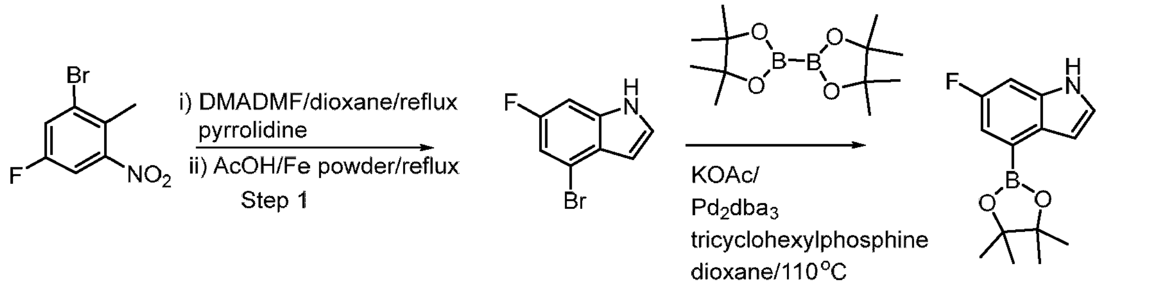


**intermediate A5**

Step 1: To a stirring solution of 2-bromo-4-fluoro-6-nitrotoluene (4.69 g, 20 mmol, leq) in 1,4-dioxane (25 ml) was slowly added N,N-dimethylformamide dimethylacetal (13.3 mL, 100 mmol, 5eq) and pyrrolidine (1.47 mL, 20 mmol, leq). The reaction mixture was then stirred for 18 h at 100°C. The reaction mixture was concentrated to a dark residue. To this residue were added AcOH (30 mL) and iron powder (11 g, 200mmol, 10 eq) and then the reaction mixture was refluxed for 1 h. The reaction mixture was then cooled to RT and then filtered through a celite bed. The filtrate was neutralised by 50% sodium hydroxide solution and then extracted with EtOAc (2 x 100 mL). Combined organic layers was washed with water (100 mL), brine (100 mL), dried over anhydrous $Na_2SO_4$ and evaporated to get the crude which was purified by column chromatography to afford 4-bromo-6-fluoro-1H-indole (1.3 g, 30%) as brown liquid.

Step 2: To a stirring suspension of 4-bromo-6-fluoro-1H-indole (1.1 g, 5.1 mmol, 1 eq), bis(pinacolato)diborane (2.6 g, 10.2 mmol, 2eq) and potassium acetate (2.0 g, 20.4 mmol, 4 eq) in 1,4-dioxan (20 mL) was deoxygenated by Ar for 10 min. $Pd_2(dba)_3$ (0.07 g, 0.07 mmol. 0.015 eq) and tricyclohexylphosphine (0.102 g, 0.36 mmol, 0.07 eq) was then added to the reaction mixture and again deoxygenated by Ar for 10 min. The reaction mixture was then stirred for 14 h at 110°C. The reaction mixture then cooled to RT and then filtered through celite bed. Filtrate was concentrated under reduced pressure to get the crude material which was purified by column chromatography to afford 6-fluoro-4-(4,4,5,5-tetramethyl-1,3,2-dioxaborolan-2-yl)-1H-indole (1.1 g, 82%) as light yellow solid.

Synthesis of 1-(cyclopropylmethyl)-4-(4,4,5,5-tetramethyl-1,3,2-dioxaborolan-2-yl)-1H-indole **(intermediate A8)**

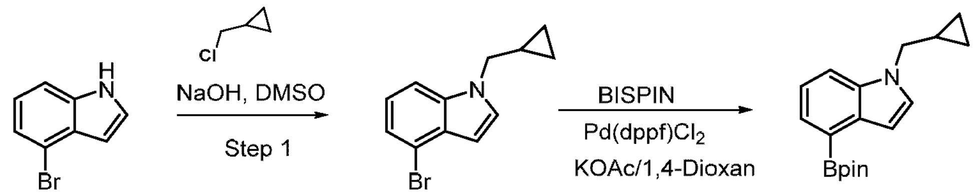


**intermediate A8**

Step 1: Sodium hydroxide (408 mg, 10.2 mmol, 4.0 eq.) was weighed out into a vial under nitrogen atmosphere, followed by the addition of DMSO (6.6 mL). The mixture was allowed to stir at ambient temperature for five minutes, before 4-bromo-1H-indole (500 mg, 2.6 mmol, 1.0 eq.) in DMSO (3.3 mL) was added. The mixture was stirred for 10 minutes, before the dropwise addition of (chloromethyl)cyclopropane (692 mg, 7.7 mmol, 3.0 eq.). The reaction mixture was then heated to 60 °C for 16 hours. Water and EtOAc were then added, the layers were separated, and the aqueous layer was extracted three times with EtOAc. The combined organic layers were washed with brine, dried over $MgSO_4$ and the solvent was removed under reduced pressure to obtain a crude mixture of 4-bromo-1-(cyclopropylmethyl)-1H-indole (685 mg), which was used in the next step without further purification.

Step 2: 1-(cyclopropylmethyl)-4-(4,4,5,5-tetramethyl-1,3,2-dioxaborolan-2-yl)-1H-indole was prepared in a similar manner (use of appropriate reagents and purification methods known to the person skilled in the art) as the synthesis described for **intermediate A18,** step 2. Yield: 814 mg, 77% over two steps.

Synthesis of 1-cyclopropyl-4-(4,4,5,5-tetramethyl-1,3,2-dioxaborolan-2-yl)-1H-indole **(intermediate A10)**

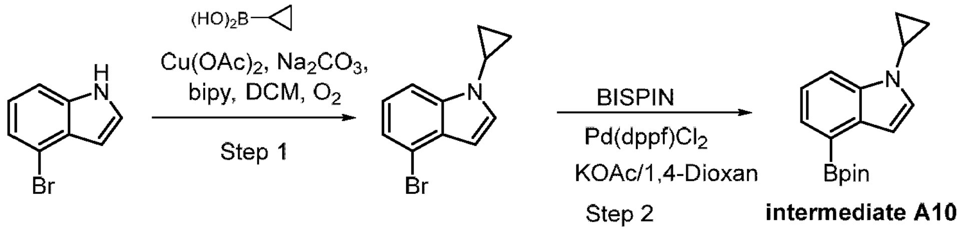


Step 1: 4-Bromo-1H-indole (50 mg, 0.26 mmol, 1.0 eq), cyclopropyl boronic acid (48 mg, 0.59 mmol, 2.2 eq.), $Na_2CO_3$ (81 mg, 0.77 mol, 3.0 eq.), $Cu(OAc)_2$ (46 mg, 0.26 mmol, 1.0 eq.) and 2,2'-bipyridine (40 mg, 0.26 mmol, 1.0 eq.) were eighed out into a microwave vial, a stir bar was added and the vial was sealed. Then DCM (5.7 mL) was added, followed by purging the reaction mixture with oxygen. The reaction mixture was then stirred at ambient temperature for 22 days. Then, 10% $NH_4Cl$ solution was added, the layers were separated and the aqueous layer was repeatedly extracted with DCM. The combined organic layers were then washed with brined, dried over $MgSO_4$ and the solvent was removed under reduced pressure. The obtained residue was purified via silica gel chromatography to yield 39 mg (65%) of 4-bromo-1-cyclopropyl-1H-indole.

Step 2: 1-cyclopropyl-4-(4,4,5,5-tetramethyl-1,3,2-dioxaborolan-2-yl)-1H-indole was prepared in a similar manner (use of appropriate reagents and purification methods known to the person skilled in the art) as the synthesis described for **intermediate A18,** step 2.

Synthesis of 5-fluoro-3-methyl-7-(4,4,5,5-tetramethyl-1,3,2-dioxaborolan-2-yl)-1H-indole (intermediate A13)

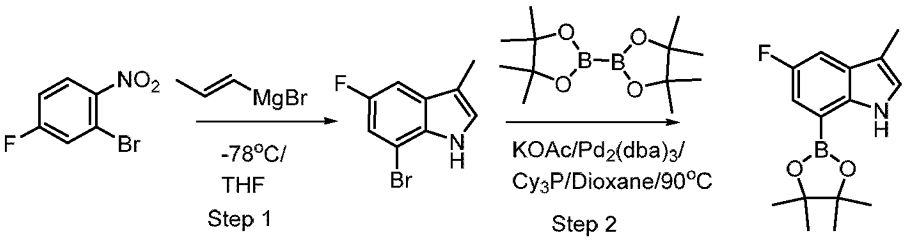


**intermediate A13**

Step 1: To a solution of 7-bromo-5-fluoro-3-methyl-1H-indole (0.5 g, 2.27 mmol, 1 eq.) in THF (20 mL) was added (E)-prop-1-en-1-ylmagnesium bromide (0.5 M in THF) (13.6 mL, 6.818 mmol, 3 eq) at -60°C under nitrogen atmosphere. Then the reaction mixture was stirred at the same temperature for 4 h. The reaction was quenched with saturated ammonium chloride solution at -60°C Then the resulting mixture was extracted with EtOAc (2 x 100 mL), washed with brine solution and concentrated under reduced pressure to give the crude product which was purified by flash column chromatography to afford 7-bromo-5-fluoro-3-methyl-1H-indole (0.3 g, 58%) as dense yellow liquid.

Step 2: To a solution of 7-bromo-5-fluoro-3-methyl-1H-indole (0.8 g, 3.669 mmol, 1 eq) in 1,4-dioxane (15.0 mL) were added KOAC (1.43 g, 14.67 mmol, 4 eq) and bispincolatediborane (1.12 g, 7.33 mmol, 2 eq). The solution was degassed with Ar for 20 min followed by addition of $Pd_2(dba)_3$ (0.16 g, 0.183 mmol, 0.05 eq) and $Cy_3P$ (0.082 g, 0.293 mmol, 0.08 eq). The reaction mixture was refluxed for 16 h. After completion of reaction (monitored by TLC), solvent was evaporated under reduced pressure to get the crude product which was purified by column chromatography to afford 5-fluoro-3-methyl-7-(4,4,5,5-tetramethyl-1,3,2-dioxaborolan-2yl)-1H-indole (0.7 g, 70%), as brown solid.

Synthesis of 6-fluoro-1-(methylsulfonyl)-4-(4,4,5,5-tetramethyl-1,3,2-dioxaborolan-2-yl)-1H-indole **(intermediate A14)**

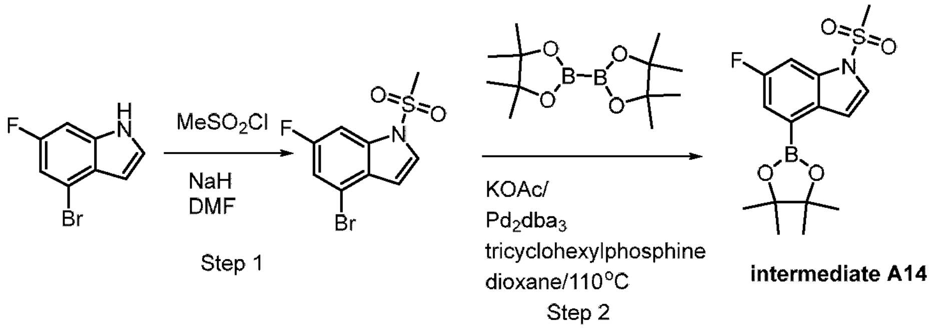


Step1: To a stirring solution of 4-bromo-6-fluoro-1H-indole (0.18 g, 0.841 mmol, 1 eq) in DMF (5 mL) was portion wise added sodium hydride (60%, 0.07 g, 1.68 mmol, 2 eq) at 0°C. The reaction mixture was then stirred for 30 min at RT. Methanesulfonylchloride (0.114 ml, 1.26 mmol, 1.5 eq) was then added to the reaction mixture at 0°C. The reaction mixture was stirred for 2 h at RT. Reaction mixture was diluted with EtOAc (50 mL). Combined organic layers were washed with water (5 x 10 mL), brine (10 mL), dried over anhydrous $Na_2SO_4$ and the solvent was evaporated under reduced pressure. Crude product was purified by column chromatography to afford 4-bromo-6-fluoro-1-(methylsulfonyl)-1H-indole (0.1 g, 41%) as off-white solid.

Step2: To a stirring suspension of 4-bromo-6-fluoro-1-(methylsulfonyl)-1H-indole (1.2 g, 3.53 mmol, 1 eq), bis-pinacolatodiborane(1.79 g, 7.06 mmol, 2 eq) and potassium acetate (1.39 g, 10.62 mmol, 4eq) in 1,4-dioxan (20 mL) was deoxygenated by Ar for 10 min. $Pd_2(dba)_3$ (0.048 g,0.052 mmol. 0.015 eq) and triclyclohexylphosphine (0.071g, 0.25 mmol, 0.07 eq) was then added to the reaction mixture and again deoxygenated by Ar for 10 min. The reaction mixture was stirred for 14 h at 110 °C. The reaction mixture was cooled to RT and then filtered through celite bed. Filtrate was concentrated under reduced pressure to get the crude product which was purified by column chromatography to afford 6-fluoro-1-(methylsulfonyl)-4-(4,4,5,5-tetramethyl-1,3,2-dioxaborolan-2-yl)-1H-indole (1.0 g, 80%) as light yellow solid.

Synthesis of 1-(methylsulfonyl)-4-(4,4,5,5-tetramethyl-1,3,2-dioxaborolan-2-yl)-1H-indazole **(intermediate A18)**

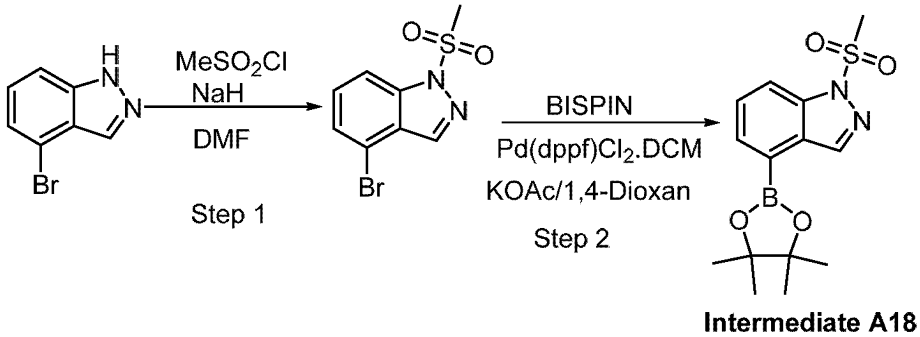


Step 1: To a stirring solution of 4-bromo-1H-indazole (1.0 g, 5.07 mmol, 1 eq) in DMF (25ml) was portion wise added sodium hydride (60%, 0.406 g, 10.152mmol, 2 eq) at 0°C. The reaction mixture was stirred for 30 min at RT. Methanesulfonylchloride (0.59 mL, 7.6 mmol, 1.5 eq) was added to the reaction mixture at 0°C. The reaction mixture was stirred for 2 h at RT. Reaction mixture was diluted with EtOAc (150 mL). Combined organic layers were washed with water (5 x 30 mL), brine (30 mL), dried over anhydrous $Na_2SO_4$ and the solvent was evaporated under reduced pressure. Crude product was purified by column chromatography (230-400 mesh silica gel 10% EtOAc/hexane; $R_f$-value-0.5) to afford 4-bromo-1-(methylsulfonyl)-1H-indazole (0.95 g, 69%) as light yellow solid.

Step 2: To a stirring suspension of 4-bromo-1-(methylsulfonyl)-1H-indazole (0.95, 3.45 mmol, 1 eq), bis(pinacolato)diborane (1.75 g, 6.91 mmol, 2eq) and potassium acetate (1.01 g, 10.36 mmol, 3 eq) in 1,4-dioxane (35 mL) was deoxygenated by Ar for 10 min. $Pd(dppf)Cl_2 \cdot DCM$ (0.141g, 0.1727mmol. 0.05 eq) was added to the reaction mixture and again deoxygenated by Ar for 10 min. The reaction mixture was stirred for 14 h at 110°C. The reaction mixture was cooled to RT and then filtered through celite bed. Filtrate was concentrated under reduced pressure to get the crude material which was purified by column chromatography (230-400 mesh silica gel, 10% EtOAc/hexane; $R_f$-value-0.45) to afford 1-(methylsulfonyl)-4-(4,4,5,5-tetramethyl-1,3,2-dioxaborolan-2-yl)-1H-indazole (0.9 g, 85.4%) as off white solid.

The following intermediates were prepared in a similar manner (use of appropriate reagents and purification methods known to the person skilled in the art) as the synthesis described for **intermediate A18:**

| Intermediate | Structure |
|---|---|
| **A9** | 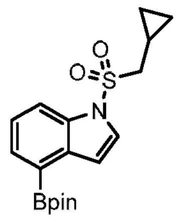 |

Synthesis of 1-(4-(4,4,5,5-tetramethyl-1,3,2-dioxaborolan-2-yl)-1H-indol-1-yl)ethanone **(intermediate A19)**

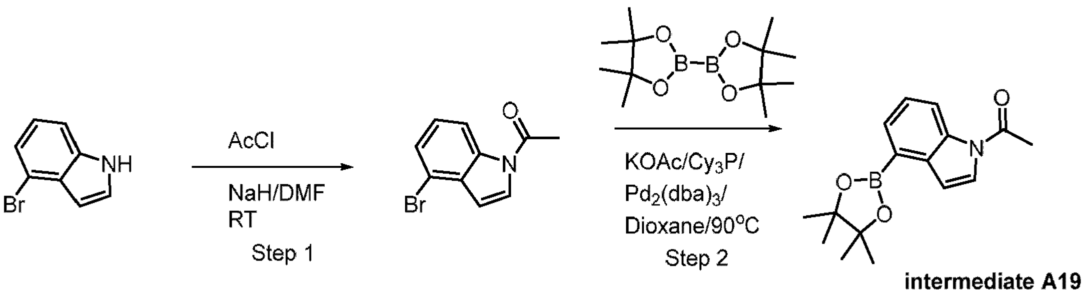


**intermediate A19**

Step 1: To a stirred solution of 4-bromo-1H-indole (0.5 g, 2.55 mmol, 1 eq) in THF (25 mL) was added sodium hydride (60%) (0.122 g, 3.06 mmol, 1.2eq) at 0°C and continued stirred at RT for 30 min. Acetyl chloride (0.02 mL, 3.06 mmol, 1.2 eq) was then added to the reaction mixture and again stirred for another 2 h. The reaction mixture was quenched with water and extracted with EtOAc (2 x 100 mL). Combined organic layers were washed with water (100 mL), brine (100 mL), dried over anhydrous $Na_2SO_4$ and the solvent was evaporated to get the crude product which was purified by column chromatography to afford 1-(4-bromo-1H-indol-1-yl)ethanone (0.55 g, 91%) as brown liquid.

Step 2: To a stirred solution of 1-(4-bromo-1H-indol-1-yl)ethanone (0.55 g, 2.31 mmol, 1 eq), bis(pinacolato) diborane (0.707 g, 4.62 mmol, 2 eq) and potassium acetate (0.680 g, 6.93 mmol, 3 eq) in 1,4-dioxan (20 mL) was deoxygenated by Ar for 10 min. $Pd_2(dba)_3$ (0.106g, 0.1155 mmol, 0.08 eq) and $Cy_3P$ (0.052 g, 0.1848 mmol. 0.08 eq) was then added to the reaction mixture and reflux at 90°C for another 16 h. The reaction mixture was cooled to RT and filtered through celite bed. Filtrate was concentrated under reduced pressure to get the crude material which was purified by column chromatography to afford 1-(4-(4,4,5,5-tetramethyl-1,3,2-dioxaborolan-2-yl)-1H-indol-1-yl)ethanone (0.600 g, 92%) as brown liquid.

Synthesis of 1-(4-(4,4,5,5-tetramethyl-1,3,2-dioxaborolan-2-yl)-1H-indol-1-yl)ethanone **(intermediate A20)**

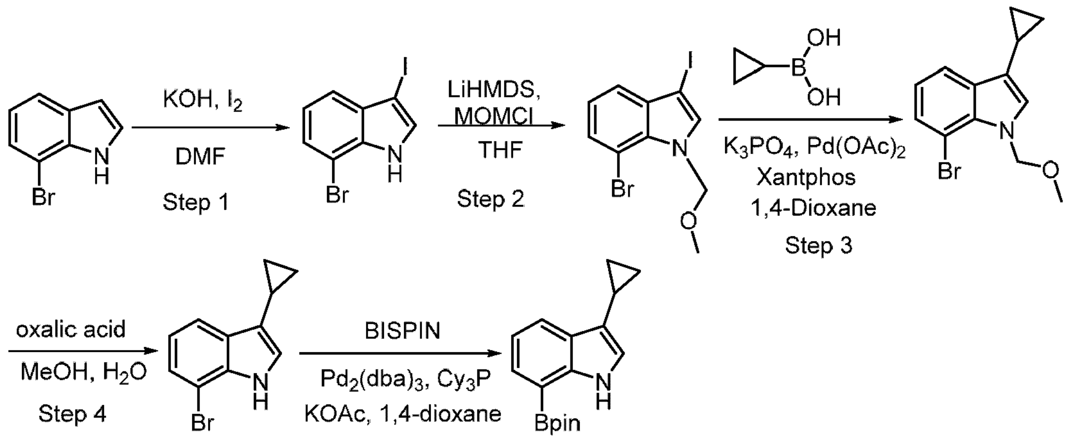


**intermediate A20**

Step 1: To a stirring solution of 7-bromo-5-fluoroindole (7.0 g, 35.7 mmol, 1.0 eq.) in dimethylformamide (145 ml) was added powdered potassium hydroxide (3.0 g, 53.55 mmol, 1.5 eq.). The reaction mixture was then stirred for 30 min at

room temperature. Iodine (10.0 g, 39.28 mmol, 1.1 eq.) was then added to the reaction mixture and and the resulting reaction mixture was then stirred for 2 h at room temperature. The reaction mixture was then diluted with ethyl acetate (1 L) and was washed with water (5x100ml) followed by brine (100 ml). The organic layer was dried over anhydrous $Na_2SO_4$ and evaporated to get the crude product, which was purified by silica gel column chromatography (10% ethyl acetate/hexane; $R_f$-value-0.4) to afford 7-bromo-3-iodo-1H-indole (8.5 g, 74%) as a brown solid.

Step 2: To a stirring solution of 7-bromo-3-iodo-1H-indole (8.5 g, 26.4 mmol, 1.0 eq.) in tetrahydrofuran (150 ml) was dropwise added LiHMDS (1.3 M, 101.5 ml, 132.3 mmol, 5.0 eq.) at -78 °C under an inert atmosphere. The reaction mixture was then stirred for 30 min at this temperature. MOMCl (8.44 g, 105.6 mmol, 4.0 eq) was then added to the reaction mixture at -78 °C. The reaction mixture was then slowly allowed to reach room temperature and was then stirred for 16 h. The reaction mixture was quenches by the addition of a saturated solution of ammonium chloride (100 ml). The organic layer was separated and the aqueous layer was extracted with ethyl acetate (100 ml). The combined organic layers were washesd with brine (100 ml), dried over anhydrous $Na_2SO_4$ and evaporated to get the crude product, which was purified by silica gel column chromatography (10% ethyl acetate/hexane; $R_f$-value-0.5) to afford 7-bromo-3-iodo-1-(methoxymethyl)-1H-indole (9.0 g, 93%) as an off-white solid.

Step 3: A stirred suspension of 7-bromo-3-iodo-1-(methoxymethyl)-1H-indole (5.0 g, 13.66 mmol, 1.0 eq.), cyclopropylbronic acid (3.52 g, 40.98 mmol, 3.0 eq.) and $K_3PO_4$ (8.68 g, 40.98 mmol, 3.0 eq.) in 1,4-dioxane (100 ml) was deoxygenated with argon for 10 min. $Pd(OAc)_2$ (0.153 g, 0.683 mmol, 0.05 eq.) and xantphos (0.79 g, 1.366 mmol, 0.1 eq.) were then added to the reaction mixture, which was again deoxygenated for 10 min. The reaction mixture was then heated to 100 °C for 16 h. The reaction mixture was then cooled to room temperature and was filtered through a celite bed. The filtrate was concentrated under reduced pressure to get the crude material which was purified by silica gel column chromatography (10% ethyl acetate/hexane; $R_f$-value-0.5) to afford 7-bromo-3-cyclopropyl-1-(methoxymethyl)-1H-indole (1.7 g, 44%) as an off-white solid.

Step 4: To a stirring solution of 7-bromo-3-cyclopropyl-1-(methoxymethyl)-1H-indole (2.2 g, 7.87 mmol, 1.0 eq.) in a mixture of methanol and water (3:1) (64 ml) was added oxalic acid (2.12 g, 23.57 mmol, 3.0 eq). The reaction mixture was then heated to 90 °C for 18 h. The reaction mixture was then cooled to room temperature and was concentrated under reduced pressure to get the crude residue, which was diluted with ethyl acetate (200 ml) and was washed with water (2x70 ml) and brine (70 ml). The organic layer was dried over anhydrous $Na_2SO_4$ and evaporated to get the crude product, which was purified by silica gel column chromatography (10% ethyl acetate/hexane; $R_f$-value-0.55) to afford 7-bromo-3-cyclopropyl-1H-indole (1.3 g, 70%) as a colorless liquid.

Step 5: A stirring suspension of 7-bromo-3-cyclopropyl-1H-indole (1.35 g, 5.72 mmol, 1.0 eq.), bis-pinacolatodiborane (2.88 g, 11.44 mmol, 2.0 eq.) and potassium acetate (1.65 g, 17.16 mmol, 3.0 eq.) in 1,4-dioxane (67 ml) was deoxygenated by argon gas for 10 min. $Pd_2(dba)_3$ (0.070 g, 0.085 mmol. 0.015 eq.) and triclyclohexylphosphine (0.12 g, 0.429 mmol, 0.075 eq.) were then added to the reaction mixture, which was again deoxygenated by argon for 10 min. The reaction mixture was then heated to 110 °C for 14 h. The reaction mixture was then cooled to room temperature and was filtered through a celite bed. The filtrate was concentrated under reduced pressure to get the crude material which was purified by column chromatography (20% ethyl acetate/hexane; $R_f$-value-0.6) to afford 3-cyclopropyl-7-(4,4,5,5-tetramethyl-1,3,2-dioxaborolan-2-yl)-1H-indole (0.5 g, 31%) as an off-white solid.

Synthesis of 2-(6-fluoro-4-(4,4,5,5-tetramethyl-1,3,2-dioxaborolan-2-yl)-1H-indol-1-yl)ethanol **(intermediate A21)**

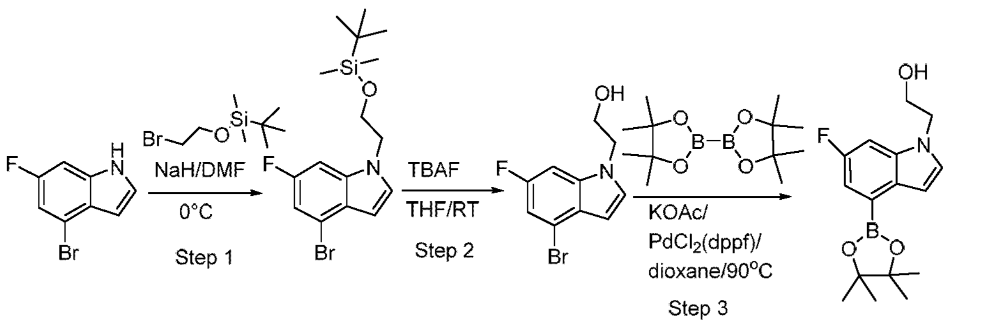


**intermediate A21**

Step 1: To a solution of 4-bromo-6-fluoro-1H-indole (0.5 g, 2.34 mmol, 1 eq.) in DMF (5 mL) was added sodium hydride (0.130 g, 2.80 mmol, 1.2 eq) at 0°C. The solution was stirred at RT for 30 min followed by addition of (2-bromoethoxy)(tert-butyl)dimethylsilane (1.17g, 4.67 mmol, 2.0 eq) and reaction mixture was stirred at RT for 2 h. After completion of reaction (monitored by LCMS), reaction mixture was diluted with EtOAc (20 mL) and organic layer was

washed with cold water (5 x 10 mL), brine (10 mL), dried over anhydrous $Na_2SO_4$ and the solvent was evaporated under reduced pressure. Crude product was purified by column chromatography to afford 4-bromo-1-(2-((tert-butyldimethylsilyl)oxy)ethyl)-6-fluoro-1H-indole (0.85g, 98%) as brown liquid having (2-bromoethoxy)(tert-butyl)dimethylsilane as impurity.

Step 2: To a stirred solution of 4-bromo-1-(2-((tert-butyldimethylsilyl)oxy)ethyl)-6-fluoro-1H-indole (1.3 g, 3.49 mmol, 1 eq.) in THF (15 mL) was added TBAF (3.49 mL) (1M) at RT and the mixture was stirred for 16 h. After completion of reaction (monitored by LCMS & TLC), reaction mixture was diluted with EtOAc (20 mL) and organic layer was washed with cold water (5 x 10 mL), brine (10 mL), dried over anhydrous $Na_2SO_4$ and the solvent was evaporated under reduced pressure. Crude product was purified by column chromatography to afford 2-(4-bromo-6-fluoro-1H-indol-1-yl)ethanol (0.55 g, 61%) as brown liquid.

Step 3: To a stirred solution of 2-(4-bromo-6-fluoro-1H-indol-1-yl)ethanol (0.55 g, 2.13 mmol, 1 eq), bis(pinacolato)diborane (0.647 g, 2.55 mmol, 1.2 eq) and potassium acetate (0.626 g, 6.393 mmol, 3 eq) in 1,4-dioxan (20 mL) was deoxygenated by Ar for 10 min. $PdCl_2(dppf)\cdot DCM$ (0.173 g, 0.213 mmol. 0.1 eq) was then added to the reaction mixture and the mixture was stirred at 90°C for 16 h. After completion of reaction (monitored by TLC), reaction mixture was filtered through celite bed. Filtrate was concentrated under reduced pressure to get the crude product which was used in next step without further purification.

Synthesis of 1-(methylsulfonyl)-4-(4,4,5,5-tetramethyl-1,3,2-dioxaborolan-2-yl)-1H-indole **(intermediate A22)**

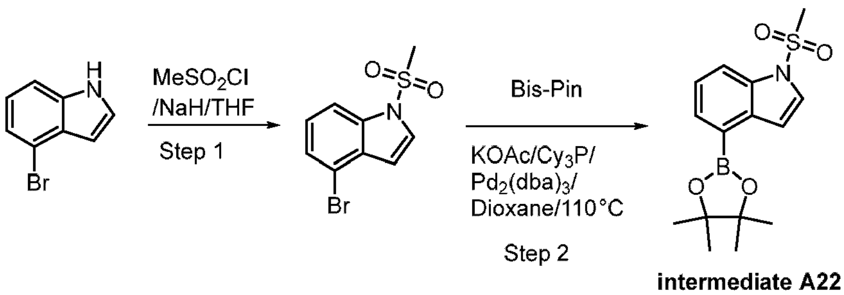


**intermediate A22**

Step 1: To a stirring solution of 4-bromo-1H-indole (1.0 g, 5.1mmol, 1 eq) in DMF (20ml) was portion wise added sodium hydride (60%, 0.245 g, 10.2 mmol, 2 eq) at 0°C. The reaction mixture was then stirred for 30 min at RT. Methanesulfonylchloride (0.584 ml, 7.6 mmol, 1.5 eq) then added to the reaction mixture at 0°C. The reaction mixture was stirred for 2 h at RT. Reaction mixture was diluted with EtOAc (100 mL). Combined organic layers was washed with water (5 x 20 mL), brine (20 mL), dried over anhydrous $Na_2SO_4$ and the solvent was evaporated under reduced pressure. The crude product was purified by column chromatography to afford 4-bromo-1-(methylsulfonyl)-1H-indole (0.532 g, 38%) as off white solid.

Step 2: To a stirring suspension of 1-(methylsulfonyl)-4-(4,4,5,5-tetramethyl-1,3,2-dioxaborolan-2-yl)-1H-indole (0.36g, 1.31mmol, 1eq), bis(pinacolato)diborane (0.66 g, 2.62 mmol, 2eq) and potassium acetate (0.57 g, 5.25 mmol, 4 eq) in 1,4-dioxan (10 L1) was deoxygenated by Ar for 10 min. $Pd_2(dba)_3$ (0.018g, 0.019mmol. 0.015 eq) and tricyclohexylphosphine (0.027 g, 0.094 mmol, 0.072 eq) was then added to the reaction mixture and again deoxygenated by Ar for 10 min. The reaction mixture was then stirred for 14 h at 110°C. The reaction mixture then cooled to RT and then filtered through celite bed. Filtrate was concentrated under reduced pressure to get the crude material which was purified by column chromatography to afford 1-(methylsulfonyl)-4-(4,4,5,5-tetramethyl-1,3,2-dioxaborolan-2-yl)-1H-indole (0.31 g, 73%) as off white solid.

Synthesis of 6-fluoro-1-(methylsulfonyl)-4-(4,4,5,5-tetramethyl-1,3,2-dioxaborolan-2-yl)-1H-indazole **(intermediate A23)**

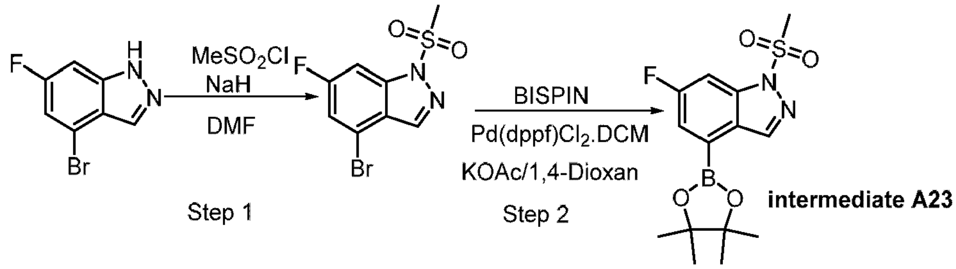


Step 1: To a stirring solution of 4-bromo-6-fluoro-1H-indazole (1.2 g, 5.58 mmol, 1 eq) in DMF (30 mL) was portion wise added sodium hydride (60%, 0.446 g, 11.16mmol, 2 eq) at 0°C. The reaction mixture was then stirred for 30 min at RT. Methanesulfonylchloride (0.65 ml, 8.37 mmol, 1.5 eq) was added to the reaction mixture at 0°C. The reaction mixture was stirred for 2 h at RT. Reaction mixture was diluted with EtOAc (150 mL). Combined organic layers were washed with water (5 x 30 mL), brine (30 mL), dried over anhydrous $Na_2SO_4$ and evaporated under reduced pressure. Crude product was purified by column chromatography (230-400 mesh silica gel 10% EtOAc/hexane; $R_f$-value-0.5) to afford 4-bromo-6-fluoro-1-(methylsulfonyl)-1H-indazole (1.3 g, 80%) as light yellow solid.

Step 2: To a stirring suspension of 4-bromo-6-fluoro-1-(methylsulfonyl)-1H-indazole (1.3, 4.43 mmol, 1eq), bis(pinacolato)diborane (2.25 g, 8.87 mmol, 2 eq) and potassium acetate (1.3 g, 13.3 mmol, 3 eq) in 1,4-dioxane (45 mL) was deoxygenated by Ar for 10 min. $Pd(dppf)Cl_2 \cdot DCM$ (0.18 g, 0.22 mmol. 0.05 eq) and was then added to the reaction mixture and again deoxygenated by Ar for 10 min. The reaction mixture was stirred for 14 h at 110°C. The reaction mixture was cooled to RT and then filtered through celite bed. Filtrate was concentrated under reduced pressure to get the crude material which was purified by column chromatography (230-400 mesh silica gel, 10% EtOAc/hexane; $R_f$-value-0.45) to afford 6-fluoro-1-(methylsulfonyl)-4-(4,4,5,5-tetramethyl-1,3,2-dioxaborolan-2-yl)-1H-indazole (1.1 g, 73%) as off white solid.

Synthesis of 8-bromo-7-fluoro-1,4,4,9-tetramethyl-4,5-dihydroimidazo[1,2-a]quinoxaline **(intermediate B1):**

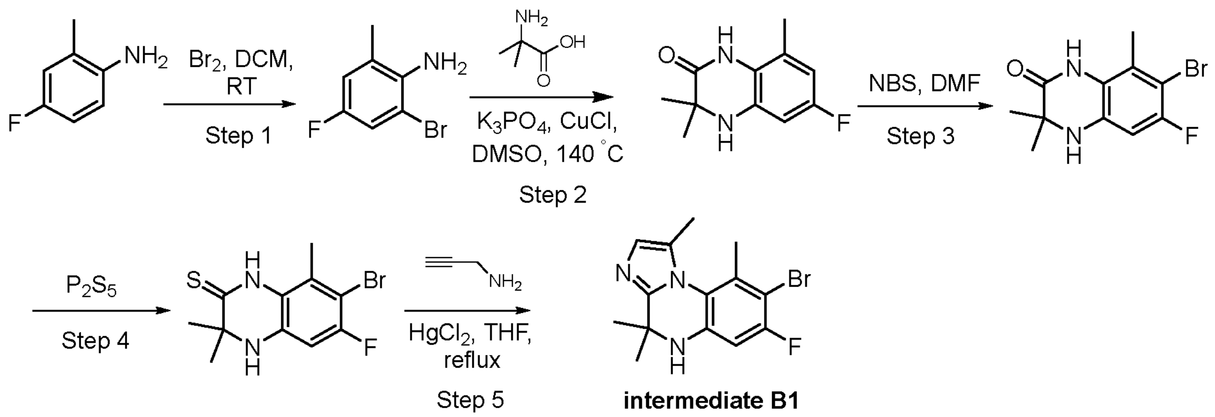


Step 1: To a solution of 4-fluoro-2-methyl-phenylamine (30 g, 0.239 mol) in DMF (450 ml) was added NBS (44.81 g, 0.251 mol) portionwise at -10 °C. The resulting reaction mixture was stirred at room temperature for 16 h. After completion of the reaction (monitored by LCMS), the reaction mixture was diluted with water (1000 ml) and extracted with ethyl acetate (2 x 500 ml). The combined organic layers were washed with water (2 x 500 ml) and brine (250 ml), dried over anhydrous $Na_2SO_4$ and concentrated to afford the crude compound, which was purified by column chromatography (100-200 mesh silica gel; 10% ethyl acetate/hexane) to afford 2-bromo-4-fluoro-6-methylphenylamine (45 g, 92%) as a white solid.

Step 2: To the stirred suspension of 2-bromo-4-fluoro-6-methyl-phenylamine (40 g, 0.19 mol) in dry DMSO (600 ml) was added 2-amino-2-methyl-propionic acid (40.4 g, 0.39 mol) followed by $K_3PO_4$ (83.1 g, 0.39 mol) at room temperature. The resulting reaction mixture was degassed with nitrogen for 30 min, then cuprous chloride (1.93 g, 0.019 mol) was added and reaction mixture was heated to 140 °C for 2 h. After completion of the reaction (monitored by TLC, 20% EA-Hexane, Rf 0.4), the reaction mixture was cooled to room temperature and filtered through celite and the celite bed was washed with ethyl acetate (500 ml). The resulting filtrate was poured into ice cold water (1 L). The resulting aqueous layer was extracted with ethyl acetate (2 x 250 ml). The combined organic layers were washed with water (2 x 500

ml) and brine (250 ml), dried over anhydrous $Na_2SO_4$ and evaporated under reduced pressure to afford the crude compound, which was purified by column chromatography (100-200 mesh silica gel and 20% ethyl acetate/hexane as eluent) to afford 6-fluoro-3,3,8-trimethyl-3,4-dihydro-1H-quinoxalin-2-one (25.8 g, 64%) as a brown solid.

Step 3: To a solution of 6-fluoro-3,3,8-trimethyl-3,4-dihydro-1H-quinoxalin-2-one (8.6 g, 41.34 mmol, 1.0 eq.) in DMF (100 ml) was added NBS (8.83 g, 49.61mmol, 1.2 eq.) portion wise at 0 °C. The reaction mixture was gradually warmed to ambient temperature and was stirred for 3 h. After completion of the reaction (monitored by LCMS), the reaction mixture was diluted with ice water (500 ml) and was extracted with ethyl acetate (2 x 400 ml). The combined organic layers were washed with water (500 ml) and brine (400 ml) and were then dried over anhydrous $Na_2SO_4$. The solvent was evaporated under reduced pressure to get the crude compound which was purified by column chromatography (silica gel; 10% EA-Hexane) to afford 7-bromo-6-fluoro-3,3,8-trimethyl-3,4-dihydro-1H-quinoxalin-2-one (6.2 g, 55%) as a light brown solid.

Step 4: $P_2S_5$ (5.56 g, 25.08 mmol, 1.2 eq.) was added to a mixture of acetonitrile and triethylamine (1:1, 80 ml) and was stirred for 15 min. Then, 7-bromo-6-fluoro-3,3,8-trimethyl-3,4-dihydro-1H-quinoxalin-2-one (6.0 g, 20.90 mmol, 1.0 eq.) was added to the reaction mixture at 0 °C. The reaction mixture was warmed to ambient temperature and was then refluxed for 1h. The reaction mixture was diluted with water (250 ml) and extracted with ethyl acetate (2 x 300 ml). The combined organic layers were washed with water (250 ml) and brine (250 ml) and were dried over sodium sulfate. The solvent was evaporated under reduced pressure to get the crude material which was purified by column chromatography (silica gel 100-200 mesh, 10-15% EA/Hexane) to yield 7-bromo-6-fluoro-3,3,8-trimethyl-3,4-dihydro-1H-quinoxaline-2-thione (5.2 g, 82%) as a light yellow solid.

Step 5: To a solution of 7-bromo-6-fluoro-3,3,8-trimethyl-3,4-dihydro-1H-quinoxaline-2-thione (3.0 g, 9.90 mmol, 1.0 eq.) in THF (40 ml) were added propargylamine (6.3 ml, 99.0 mmol, 10.0 eq.) and $HgCl_2$ (2.7 g, 9.90 mmol, 1.0 eq.) and the reaction mixture was heated to reflux for 16 h. After 16 h, $HgCl_2$ (1.35 g, 0.5 eq.) was added to the reaction mixture and the reaction mixture was again heated to reflux for another 16 h. The reaction mixture was then diluted with ethyl acetate (300 ml), washed with water (150 ml) and brine (200 ml) and dried over sodium sulfate. The solvent was evaporated under reduced pressure to get the crude product which was purified by column chromatography (silica gel; 25-30% EA/Hexane) to yield 8-bromo-7-fluoro-1,4,4,9-tetramethyl-4,5-dihydro-imidazo[1,2-a]quinoxaline (2.1 g, 65%) as an off-white solid.

Synthesis of 8-bromo-7,9-difluoro-1,4,4-trimethyl-4,5-dihydropyrrolo[1,2-a]quinoxaline **(intermediate B2):**

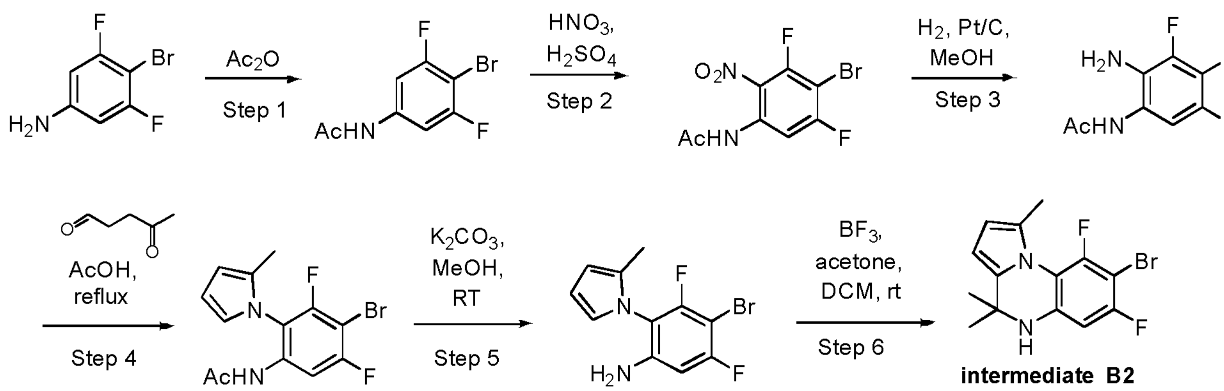

**intermediate B2**

Step 1: 4-Bromo-3,5-difluoro-phenylamine (5 g, 24.02 mmol) was treated with acetic anhydride (2.26 ml, 24.02 mmol) at 0 °C for 30 mins. After completion of the reaction as ensured from TLC, the reaction mixture was poured in ice water, the precipitated solids were filtered off and were washed washed thoroughly with water to afford N-(4-bromo-3,5-difluorophenyl)acetamide (5.3 g, 88%) as a solid.

Step 2: To a suspension of N-(4-bromo-3,5-difluorophenyl)acetamide (5.3 g, 21.19 mmol) in concentrated $HNO_3$ at 0 °C (7.52 ml) was added concentrated $H_2SO_4$ (7.52 ml) dropwise. The reaction mixture was gradually warmed up to room temperature. After ensuring complete consumption of the starting material by TLC (2 h), the reaction mixture was poured into ice water, the precipitated solids were filtered off, washed thoroughly with water and were dried to afford N-(4-bromo-3,5-difluoro-2-nitrophenyl)acetamide (5.1 g, 82%) as a pale yellow solid.

Step 3: A solution of N-(4-bromo-3,5-difluoro-2-nitrophenyl)acetamide (500 mg, 1.69 mmol) in methanol (50 ml) was hydrogenated in a Parr shaker at 50 psi in the presence of 5% platinum on carbon (150 mg). After ensuring complete consumption of starting material by TLC (30 min), the reaction mixture was filtered through a bed of celite and the filtrate was then concentrated under reduced pressure to afford N-(2-amino-4-bromo-3,5-difluorophenyl)acetamide (430 mg, 96%) as a solid.

Step 4: To a solution of N-(2-amino-4-bromo-3,5-difluorophenyl)acetamide (380 mg, 1.433 mmol) in acetic acid (10 ml) was added 4-oxopentanal (144.4 mg, 1.433 mmol) and the mixture was heated to 120 °C for 10 minutes. After consumption of the starting material as evident from TLC (10 min), the reaction mixture was concentrated under reduced pressure and the residual crude material was purified using silica gel chromatography (elution with 4% ethyl acetate: hexane) to afford N-(4-bromo-3,5-difluoro-2-(2-methyl-1H-pyrrol-1-yl)phenyl)acetamide (268 mg, 57%) as a dark brown solid.

Step 5: N-(4-Bromo-3,5-difluoro-2-(2-methyl-1H-pyrrol-1-yl)phenyl)acetamide (420 mg, 1.276 mmol) was taken up in methanol (8 ml) and was treated with potassium carbonate (528.3 mg, 3.828 mmol). After completion of the reaction as ensured from TLC (16 h), the solids were filtered off and were washed thoroughly with methanol. The filtrate was concentrated under reduced pressure and was purified using silica gel chromatography (elution with 3% ethyl acetate: hexane) to afford 4-bromo-3,5-difluoro-2-(2-methyl-1H-pyrrol-1-yl)aniline (289 mg, 79%) as a solid.

Step 6: A solution of 4-bromo-3,5-difluoro-2-(2-methyl-1H-pyrrol-1-yl)aniline (300 mg, 1.044 mmol) in DCM (6 ml) at 0 °C was treated with acetone (0.092 ml, 1.253 mmol), followed by the addition of boron trifluoride etherate (0.088 ml, 0.626 mmol). After ensuring completion of the reaction by TLC (10 mins), the reaction mixture was quenched with saturated sodium bicarbonate solution. The organic part was separated and the aqueous part was extracted with additional DCM. The combined organic extracts were washed with brine, dried over $Na_2SO_4$ and concentrated under reduced pressure. The remains were purified using silica gel chromatography (elution with 2% ethyl acetate: hexane) to afford 8-bromo-7,9-difluoro-1,4,4-trimethyl-4,5-dihydropyrrolo[1,2-a]quinoxaline (270 mg, 79%) as a yellow solid.

Synthesis of 8-bromo-7,9-difluoro-1,4,4-trimethyl-4,5-dihydroimidazo[1,2-a]quinoxaline **(intermediate B3):**

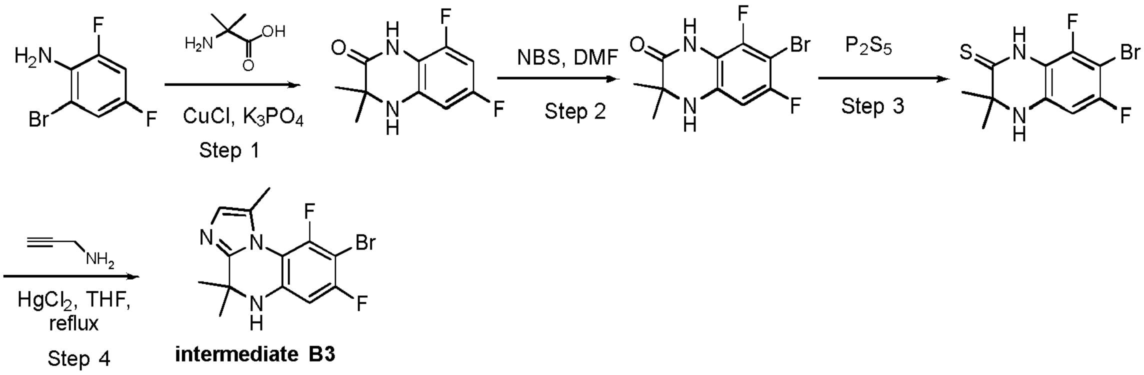


**intermediate B3**

Step 1: To the stirred suspension of 2-bromo-4,6-difluoro-phenylamine (50 g, 0.24 mol) in dry DMSO (1 L) was added 2-amino-2-methyl-propionic acid (49.49 g, 0.48 mol) followed by $K_3PO_4$ (101.88 g, 0.48 mol) at room temperature. The resulting reaction mixture was degassed with nitrogen for 30 min, then cuprous chloride (2.3 g, 0.024 mol) was added and reaction mixture was heated to 130 °C for 16 h. After completion of the reaction (monitored by LCMS), the reaction mixture was cooled to room temperature and was filtered through celite, which was washed with ethyl acetate (1000 ml). The filtrate was poured into ice cold water and the resulting mixture was extracted with MTBE (3 x 1500 ml). The combined organic layers were washed with water (2 x 2500 ml) and brine (1 lit), dried over anhydrous $Na_2SO_4$ and evaporated under reduced pressure to afford the crude compound, which was purified by column chromatography (100-200 mesh silica gel; 30% EA/hexane) to afford 6,8-difluoro-3,3-dimethyl-3,4-dihydro-1H-quinoxalin-2-one (36 g, 71%) as a brown solid.

Step 2: To a solution of 6,8-difluoro-3,3-dimethyl-3,4-dihydro-1H-quinoxalin-2-one (10 g, 47.125 mmol) in DMF (120 ml) was added NBS (9.23 g, 51.837 mmol) portionwise at -10 °C. The resulting reaction mixture was stirred at room temperature for 16 h. After completion of the reaction (monitored by LCMS), the reaction mixture was diluted with ice water (500 ml) and extracted with MTBE (2 x 500 ml). The combined organic layers were washed with water (750 ml) followed by brine (400 ml), dried over anhydrous $Na_2SO_4$ and concentrated to afford the crude compound, which was purified by column chromatography (100-200 mesh silica gel; 20% EA-Hexane) to afford 7-bromo-6,8-difluoro-3,3-dimethyl-3,4-dihydro-1H-quinoxalin-2-one (10 g, 73 %) as a light brown solid.

Step 3: $P_2S_5$ (6.86 g, 30.92 mmol, 1.2 eq.) was added to a mixture of acetonitrile and triethylamine (1:1, 100 ml) and the mixture was stirred for 15 min. Then, 7-bromo-6,8-difluoro-3,3-dimethyl-3,4-dihydro-1H-quinoxalin-2-one (7.5 g, 25.77 mmol, 1.0 eq.) was added to the reaction mixture at 0 °C. The reaction mixture was warmed to ambient temperature and was then refluxed for 3h. The reaction mixture was diluted with water (250 ml) and was then extracted with ethyl acetate (2 x 300 ml). The combined organic layers were washed with water (250 ml) and brine (250 ml) and were dried over sodium sulfate. The solvent was evaporated under reduced pressure to get the crude material which was purified by

column chromatography (silica gel 100-200 mesh, 30% EA/Hexane) to yield 7-bromo-6,8-difluoro-3,3-dimethyl-3,4-dihydro-1H-quinoxaline-2-thione (6.5 g, 21.17 mmol, 82%) as a yellow solid which was contaminated with the corresponding des-bromo compound (~5-10%). The mixture was used as such in the next step without further purification.

Step 4: To a solution of7-bromo-6,8-difluoro-3,3-dimethyl-3,4-dihydro-1H-quinoxaline-2-thione (6.0 g, 19.54 mmol, 1.0 eq.) in THF (100 ml) were added propargylamine (12 ml, 195.4 mmol, 10 eq.) and $HgCl_2$ (5.2 g, 19.54 mmol, 1.0 eq.) and the reaction mixture was heated to reflux for 16 h. After 16 h, $HgCl_2$ (2.6 g, 0.5 eq.) was again added to the reaction mixture and the mixture was heated to reflux for another 16 h. The reaction mixture was then diluted with ethyl acetate (500 ml), washed with water (200 ml) and brine (200 ml) and was dried over sodium sulfate. The solvent was evaporated under reduced pressure to get the crude product which was purified by column chromatography (silica gel; 30% EA/Hexane) and then triturated with DCM-hexane to yield 8-bromo-7,9-difluoro-1,4,4-trimethyl-4,5-dihydro-imidazo[1,2-a]quinoxaline (1.5 g, 23%) as a white solid.

Synthesis of 8-bromo-9-fluoro-1,4,4-trimethyl-4,5-dihydropyrido[3,4-e][1,2,4]triazolo[4,3-a]pyrazine **(intermediate B4):**

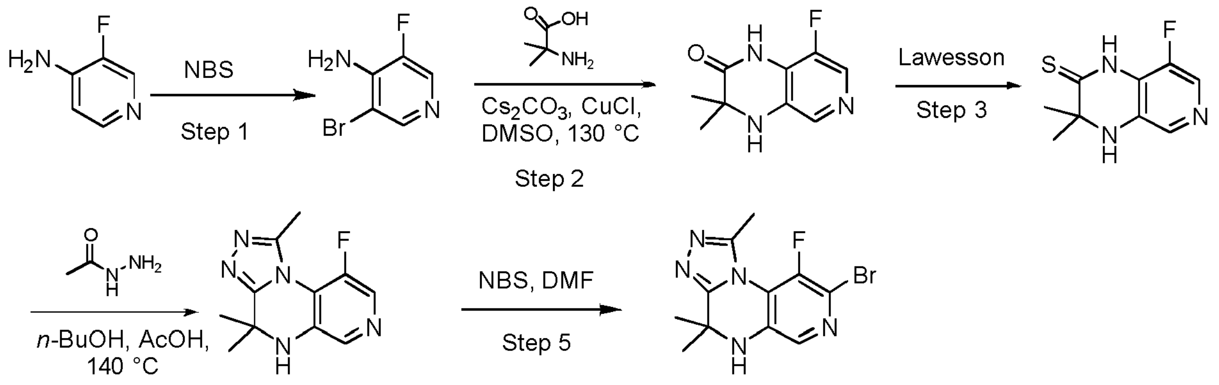


Step 4

**intermediate B4**

Step 1: To a stirred solution of 3-Fluoro-pyridin-4-ylamine (13.0 g, 115.95 mmol) in ACN (270 ml) and added NBS (30.96 g, 173.93 mmol). The reaction was then heated to 80 °C for 4 h. The reaction mixture was concentrated and the obtained crude material was purified by flash column chromatography (20 % Ethyl acetate/Hexane) to afford 3-bromo-5-fluoro-pyridin-4-ylamine (9.0 g, 41%) as an off-white solid.

Step 2: To a stirred solution of 3-bromo-5-fluoro-pyridin-4-ylamine (9.0 g, 47.12 mmol) and 2-amino-2-methyl-propionic acid (9.7 g, 94.24 mmol) in DMSO (170 ml) was added $K_3PO_4$ (20.0 g, 94.24 mmol). The reaction was degassed with argon for 30 minutes before the addition of CuCl (0.47g, 4.71mmol). The reaction mixture was heated to 130 °C for 16 h. The reaction mixture was then cooled to ambient temperature, was diluted with water and extracted with EtOAc (6 x 200 ml). The combined organic layers were dried with anhydrous $Na_2SO_4$, filtered and concentrated under reduced pressure. The residue was cooled and treated with crushed ice and stirred until solids formed. The solid was filtered off and was washed with cold water followed by n-hexane to afford 8-fluoro-3,3-dimethyl-3,4-dihydro-1H-pyrido[3,4-b]pyrazin-2-one (8.2 g, 89%).

Step 3: To a stirred solution of 8-fluoro-3,3-dimethyl-3,4-dihydro-1H-pyrido[3,4-b]pyrazin-2-one (8.2 g, 42.029 mmol) in toluene (90 ml) was added Lawesson's reagent (25.5 g, 63.044 mmol). The reaction was then heated to 120 °C for 7 h. The reaction mixture was concentrated under reduced pressure and the obtained crude material was purified by flash column chromatography (50% Ethyl acetate/Hexane, neutral $Al_2O_3$) to afford 8-fluoro-3,3-dimethyl-3,4-dihydro-1H-pyrido[3,4-b]pyrazine-2-thione (6.5 g, 73%) as a yellow solid.

Step 4: To a stirred solution of 8-fluoro-3,3-dimethyl-3,4-dihydro-1H-pyrido[3,4-b]pyrazin-2-one (6.5 g, 30.7677 mmol) in n-butanol (117 ml) were added acetic acid hydrazide (9.117 g,123.07 mmol) and acetic acid (11.7 ml) at room temperature. The reaction mixture was then heated to 140 °C for 16 h. The reaction mixture was concentrated under reduced pressure and the obtained crude material was purified by column chromatography (60% Ethyl acetate/Hexane, neutral $Al_2O_3$) to afford 9-fluoro-1,4,4-trimethyl-4,5-dihydro-2,3,5,7,9b-pentaaza-cyclopenta[a] naphthalene (2.5 g, 35%) as an off-white solid.

Step 5: To a stirred solution of 9-fluoro-1,4,4-trimethyl-4,5-dihydro-2,3,5,7,9b-pentaaza-cyclopenta[a] naphthalene (1.55 g, 6.652 mmol) in DMF (20 ml) was added dropwise a solution of N-Bromo succinimide (0.710 g, 3.99 mmol) in DMF (10 ml) at -30 C. After addition, the reaction temperature was slowly raised to room temperature and the mixture was

stirred at room temperature for 16 h. The reaction was diluted with EtOAc, and was then washed with ice cold water. The organic layer was dried with anhydrous $Na_2SO_4$, filtered and concentrated. The obtained crude material was purified by column chromatography (50% Ethyl acetate/Hexane, neutral $Al_2O_3$) to afford 8-bromo-9-fluoro-1,4,4-trimethyl-4,5-dihydropyrido[3,4-e][1,2,4]triazolo[4,3-a]pyrazine (415 mg, 20%) as an off-white solid.

Synthesis of 8-bromo-1,4,4,9-tetramethyl-4,5-dihydropyrido[2,3-e][1,2,4]triazolo[4,3-a]pyrazine **(intermediate B5):**

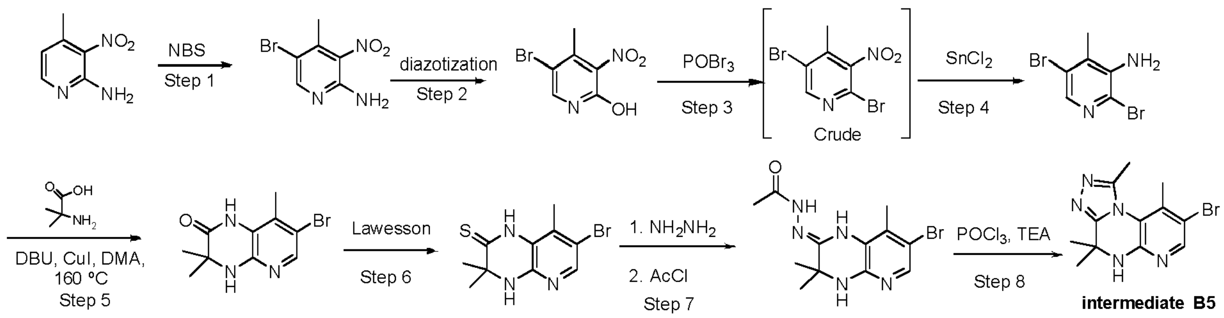


Step 1: To the stirred solution of 4-methyl-3-nitro-pyridin-2-ylamine (25 g, 0.16 mol) in acetonitrile (500 ml) was added NBS (29.2 g, 0.16 mol) portionwise at room temperature. The resulting suspension was stirred at 80 °C for 2 h. After completion of the reaction (monitored by TLC, 20% EA-Hexane, Rf = 0.5) the reaction mixture was concentrated. The obtained residue was diluted with ethyl acetate (500 ml) and was washed with water (3 x 250 ml). The organic layer was washed with water followed by brine, dried over $Na_2SO_4$ and concentrated under reduced pressure to afford 5-bromo-4-methyl-3-nitro-pyridin-2-ylamine (36 g, 94%) as a yellow solid.

Step 2: To the stirred solution of 5-bromo-4-methyl-3-nitro-pyridin-2-ylamine (43 g, 0.18 mol) in TFA:water (654 ml, 2:1) was added $NaNO_2$ (25.5 g, 0.36 mol) portionwise at 0 °C. The resulting suspension was stirred at 0 °C for 4 h. After completion of the reaction (monitored by TLC, 20% EA-Hexane, Rf = 0.4), the reaction mixture was concentrated, the obtained residue was diluted with water (50 ml) and the solids were filtered off. The obtained solid was washed with MTBE and dried under vacuum to afford 5-bromo-4-methyl-3-nitro-pyridin-2-ol (40 g, 92%) as a yellow solid.

Step 3: To the stirred solution of 5-bromo-4-methyl-3-nitro-pyridin-2-ol (20 g, 85.829 mmol) in acetonitrile (400 ml) was added $POBr_3$ (123 g, 429.145 mmol) portionwise at room temperature. The resulting suspension was heated to reflux for 16 hrs. After completion of the reaction (monitored by LCMS), the reaction mixture was concentrated. The obtained residue was diluted with ethyl acetate (500 ml) and was quenched with a saturated aqueous solution of $NaHCO_3$. The organic layer was washed with water followed by brine, dried over $Na_2SO_4$ and concentrated under reduced pressure to afford 2,5-dibromo-4-methyl-3-nitro-pyridine (23 g, crude) as a brown solid. This crude material was used for the next step without further purification.

Step 4: To the stirred solution of 2,5-Dibromo-4-methyl-3-nitro-pyridine (43 g, 0.145 mol) in EtOH (860 ml) was added $SnCl_2 \cdot 2\,H_2O$ (98.1 g, 0.435 mol) portionwise at room temperature. The resulting suspension was heated to reflux for 16 h. After completion of the reaction (monitored by LCMS), the reaction mixture was concentrated, the obtained residue was diluted with ethyl acetate (900 ml) and washed with water (3 x 250 ml). The organic part was washed with brine, dried over $Na_2SO_4$ and concentrated under reduced pressure to afford the crude compound, which was triturated with MTBE-Hexane to afford 2,5-dibromo-4-methyl-pyridin-3-ylamine (20 g, 52%) as a brown solid.

Step 5: To the stirred suspension of 2,5-dibromo-4-methyl-pyridin-3-ylamine (5 g, 18.95 mmol) in dry DMA (100 ml) was added 2-amino-2-methyl-propionic acid (2.9 g, 28.425 mmol) followed by DBU (4.3 g, 28.425 mmol) at room temperature. The resulting reaction mixture was degassed with nitrogen for 30 minutes, then CuI (180 mg, 0.9475 mmol) was added and the reaction mixture was heated to 160 °C for 16 h. After completion of the reaction (monitored by LCMS), the reaction mixture was cooled to room temperature and was filtered through celite. The celite bed was washed with ethyl acetate (100 ml). The resulting filtrate was poured into ice cold water. The resulting aqueous layer was extracted with MTBE (3 x 150 ml). The combined organic layers were washed with water (2 x 150 ml) and brine (100 ml), dried over anhydrous $Na_2SO_4$ and evaporated under reduced pressure to afford the crude compound, which was purified by column chromatography (100-200 mesh silica gel and 30% ethyl acetate/hexane as eluent) to afford 7-romo-3,3,8-trimethyl-3,4-dihydro-1H-pyrido[2,3-b]pyrazin-2-one (3.6 g, 70%) as a brown solid.

Step 6: To a solution of 7-bromo-3,3,8-trimethyl-3,4-dihydro-1H-pyrido[2,3-b]pyrazin-2-one (2 g, 0.74 mmol) in toluene (25 ml) was added Lawesson's reagent (4.48 g, 1.05 mol) at RT and the reaction mixture was then heated to 120 °C for 2 h. After completion of the reaction (monitored by TLC in 20% EA-Hexane, Rf = 0.5), the reaction mixture was concentrated and the obtained solid residue was quenched with sat. $NaHCO_3$ solution (100 ml). The resulting mixture was

extracted with ethyl acetate (3 x 150 ml), the combined organic layers were washed with water (100 ml) and brine (100 ml), dried over anhydrous $Na_2SO_4$ and evaporated to afford the crude compound, which was purified by column chromatography (100-200 mesh silica gel and 20% ethyl acetate/hexane as eluent) to afford 7-bromo-3,3,8-trimethyl-3,4-dihydro-1H-pyrido[2,3-b]pyrazine-2-thione (1.4 g, 66%) as a yellow solid.

Step 7: To a stirring solution of 7-bromo-3,3,8-trimethyl-3,4-dihydro-1H-pyrido[2,3-b]pyrazine-2-thione (1.4 g, 0.491 mmol) in tetrahydrofuran (35 ml) was added dropwise hydrazine hydrate (0.7 ml, 1.47 mol) at 0 °C. The reaction mixture was then stirred at room temperature for 16 h. Triethylamine (3.4 ml, 2.45 mol) followed by acetyl chloride (1.1 ml, 1.47 mol) were added successively to the reaction mixture dropwise at 0 °C and the resulting mixture was stirred for 2 h at room temperature. After completion of the reaction (monitored by LCMS), the reaction mixture was diluted with water (50 ml) and was extracted with 10% MeOH-DCM (5 x 100 ml). The total organic part was washed by brine (100 ml), dried over $Na_2SO_4$ and concentrated under reduced pressure to afford acetic acid (7-bromo-3,3,8-trimethyl-3,4-dihydro-1H-pyrido [2,3-b]pyrazin-2-ylidene)-hydrazide (1.3 g, 81%) as ayellow solid.

Step 8: Acetic acid (7-bromo-3,3,8-trimethyl-3,4-dihydro-1H-pyrido[2,3-b]pyrazin-2-ylidene)-hydrazide (1.1 g, 3.37 mol) in a round bottom flask (25 ml) was cooled to -10 °C, before the dropwise addition of phosphorus oxalylchloride (1.52 ml, 16.86 mmol) to the compound, followed by the dropwise addition of triethyl amine (0.47 ml, 3.37 mol). After the reaction mixture was stirred at -10 °C for 10 minutes and then 10 minutes at room temperature, the reaction was heated to reflux for 4 h. After completion of the reaction (monitored by LCMS), the reaction mixture was cooled to 0 °C and was quenched with crushed ice water (25 ml). The aqueous part was then basified using cold ammonium solution (25 ml) dropwise. The resulting basic aqueous layer was then extracted with ethyl acetate (3 x 50 ml). The combined organic layers were washed with brine (50 ml), dried over anhydrous $Na_2SO_4$ and concentrated under reduced pressure to afford the crude compound, which was purified by trituration using MTBE to afford 8-bromo-1,4,4,9-tetramethyl-4,5-dihydro-2,3,5,6,9b-pentaaza-cyclopenta[a]naphthalene (800 mg, 77%) as an off-white solid. Synthesis of 8-bromo-7,9-difluoro-4,4-dimethyl-4,5-dihydrotetrazolo[1,5-a]quinoxaline **(intermediate B6):**

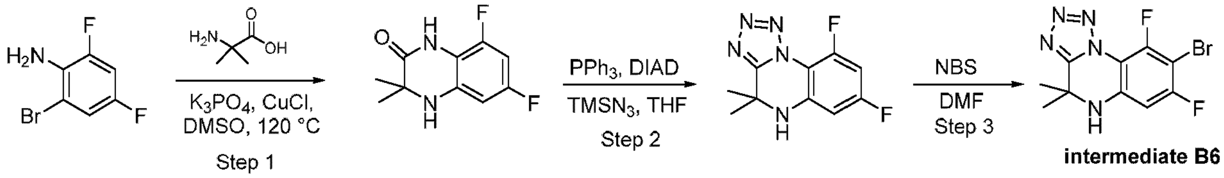

**intermediate B6**

Step 1: To a stirred solution of 2-bromo-4,6-difluoroaniline (10.0 g, 48.076 mmol, 1.0 eq) and 2-aminoisobutyric acid (9.92 g, 96.152 mmol, 2.0 eq) in DMSO was added $K_3PO_4$ (20.41 g, 96.152 mmol, 2.0 eq) under a nitrogen atmosphere. The mixture was degassed for 10 minutes using nitrogen and then CuCl (0.476 g, 4.808 mmol, 0.1 eq) was added. The mixture was heated to 130 °C for 6 h (monitored by TLC). The reaction mixture was then cooled to room temperature and was filtered through a celite pad. The filtrate was diluted with EtOAc and washed with water and brine. The organic layer was dried over $Na_2SO_4$ and concentrated, the obtained crude material was purified via column chromatography (100-200 mesh silica gel, TLC system: EtOAc / hexane (3:7); $R_f$ = 0.3) to give 6,8-difluoro-3,3-dimethyl-3,4-dihydroquinoxalin-2(1H)-one (5.1 g, 50%) as an off-white solid.

Step 2: $PPh_3$ (3.70 g, 14.15 mmol, 2.5 eq) and DIAD (2.78 ml, 14.15 mmol, 2.5 eq) were added to a solution of 6,8-difluoro-3,3-dimethyl-3,4-dihydroquinoxalin-2(1H)-one (1.2 g, 5.66 mmol, 1.0 eq) in THF (40 ml) at 0 °C and the mixture was stirred for 30 minutes. Then the reaction mixture was allowed to warm to room temperature. $TMSN_3$ (1.86 ml, 14.15 mmol, 2.5 eq) was added dropwise and the mixture was stirred for 14 h at room temperature. THF was removed under reduced pressure, the residue was diluted with EtOAc and washed with ice-cooled water. The extracted organic layer was washed with brine, dried over anhydrous $Na_2SO_4$ and concentrated under reduced pressure to obtain the crude product, which was purified by column chromatography (100-200 mesh silica gel, TLC system: EtOAc / hexane (3:7); $R_f$ = 0.35) to give 7,9-difluoro-4,4-dimethyl-4,5-dihydrotetrazolo[1,5-a]quinoxaline (0.75 g, 56%).

Step 3: To a stirred solution of 7,9-difluoro-4,4-dimethyl-4,5-dihydrotetrazolo[1,5-a]quinoxaline (0.700 g, 2.953 mmol, 1.0 eq) in DMF (200 ml) was added NBS (0.525 g, 2.953 mmol, 1.0 eq) portionwise at 0 °C and the reaction mixture was stirred at the same temperature for another hour (monitored by TLC). The reaction mixture was then quenched by adding water, causing precipitation. The solid was filtered off, was washed with water (3x10 ml) and then dried under vacuum to get 8-bromo-7,9-difluoro-4,4-dimethyl-4,5-dihydrotetrazolo[1,5-a]quinoxaline (0.66 g, 71%) as a white solid.

Synthesis of 8-bromo-7-fluoro-4,4-dimethyl-9-(trifluoromethyl)-4,5-dihydrotetrazolo[1,5-a]quinoxaline **(intermediate B7):**

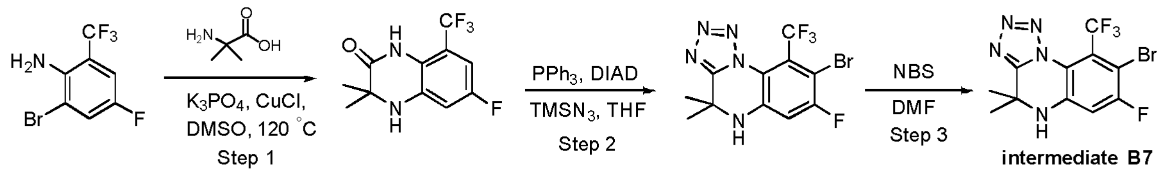


Step 1: To a stirred solution of 2-bromo-4-fluoro-6-(trifluoromethyl)aniline (9.0 g, 35.02 mmol, 1.0 eq) and 2-aminoisobutyric acid (7.22 g, 70.04 mmol, 2.0 eq) in DMSO was added $K_3PO_4$ (14.86 g, 70.04 mmol, 2.0 eq) under nitrogen atmosphere. The mixture was degassed for 10 minutes ($N_2$) and then CuCl (0.346 g, 3.502 mmol, 0.1eq) was added. The mixture was heated to 130 °C for 6 h (monitored by TLC). The reaction mixture was then cooled to room temperature and filtered through a celite pad. The filtrate was diluted with EtOAc and was washed with water and brine. The organic layer was dried over $Na_2SO_4$ and concentrated, the obtained crude resdue was purified by column chromatography (100-200 mesh silica gel, TLC system: EtOAc / hexane (3:7); $R_f$= 0.2) to give 6-fluoro-3,3-dimethyl-8-(trifluoromethyl)-3,4-dihydroquinoxalin-2(1H)-one (4.5 g, 49%).

Step 2: $PPh_3$ (3.0 g, 11.45 mmol, 2.5 eq) and DIAD (2.25 ml, 11.45 mmol, 2.5 eq) were added to a solution of 6-fluoro-3,3-dimethyl-8-(trifluoromethyl)-3,4-dihydroquinoxalin-2(1H)-one (1.2 g, 4.58 mmol, 1.0 eq) in THF (50 mL) at 0 °C and the mixture was stirred for 30 minutes. Then the reaction mixture was allowed to warm to room temperature. $TMSN_3$ (1.5 ml, 11.45 mmol, 2.5 eq) was added dropwise and the mixture was stirred for 14 h at room temperature. THF was then removed under reduced pressure and the residue was diluted with EtOAc and was washed with ice-cooled water. The extracted organic layer was washed with brine, dried over anhydrous $Na_2SO_4$ and concentrated under reduced pressure. The crude product was purified by column chromatography (100-200 mesh silica gel, TLC system: EtOAc / hexane (4:6); $R_f$ = 0.45) to give 8-bromo-7-fluoro-4,4-dimethyl-9-(trifluoromethyl)-4,5-dihydrotetrazolo[1,5-a]quinoxaline (0.75 g, 57%).

Step 3: To a stirred solution of 8-bromo-7-fluoro-4,4-dimethyl-9-(trifluoromethyl)-4,5-dihydrotetrazolo[1,5-a]quinoxaline (0.750 g, 2.61 mmol, 1.0 eq) in DMF (50 ml) was added NBS (0.465 g, 2.61 mmol, 1.0 eq) portionwise at 0 °C and the reaction mixture was stirred at the same temperature for another hour (monitored by TLC). The reaction mixture was then quenched by adding water, causing precipitation of a white solid. The solid was filtered off, was washed with water (3x10 ml) and then dried under vacuum to get 8-bromo-7-fluoro-4,4-dimethyl-9-(trifluoromethyl)-4,5-dihydrotetrazolo[1,5-a]quinoxaline (0.620 g, 65%) as a white solid.

Synthesis of 8-bromo-7-fluoro-1,4,4,9-tetramethyl-4,5-dihydro-1H-[1,2,3]triazolo[4,5-c]quinoline **(intermediate B8)**:

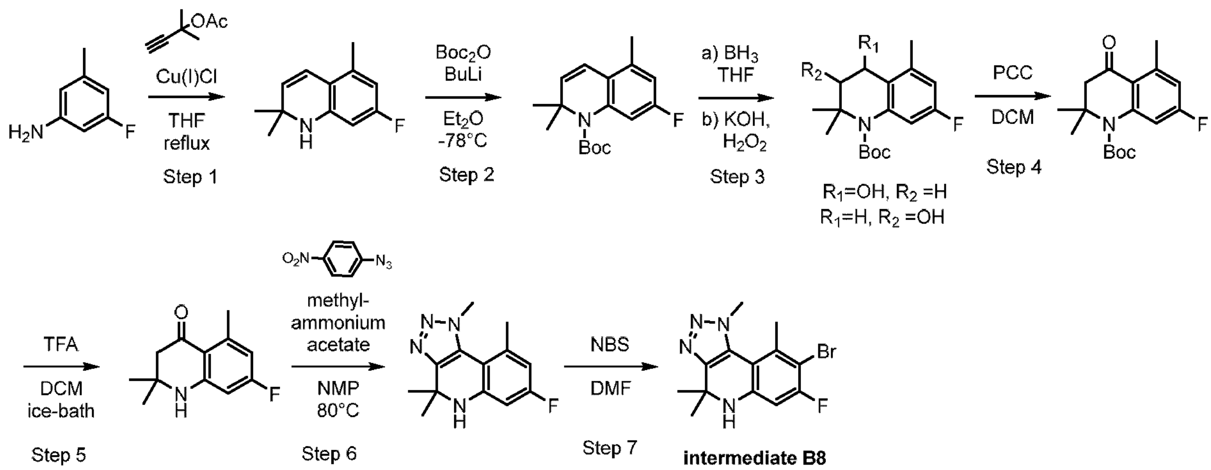


Step 1: Oven-dried glassware was used. CuCl (1.582 g, 15.98 mmol) was added to a solution of 3-fluoro-5-methylaniline (20 g, 160 mmol) and 2-methylbut-3-yn-2-yl acetate (43.2 g (70% pure), 240 mmol) in dry and degassed THF (250 mL). The mixture was stirred at 85 °C overnight. The reaction mixture was quenched with aqueous $NH_4Cl$ (500 mL) and diluted with EtOAc (250 mL). The layers were separated and the aqueous layer was extracted with EtOAc (2x200 mL). The combined organic layers were washed with brine and dried over $Na_2SO_4$. Filtration and *in vacuo* filtrate concentration gave the crude product. Purification by flash chromatography (220 g silica, gradient heptane/EtOAc, 1:0 → 19:1) afforded 7-fluoro-2,2,5-trimethyl-1,2-dihydroquinoline as a brown non-viscous oil (27.9 g (70% pure), 102 mmol, 63%).

Step 2: Heat gun dried glassware was used and the reaction was carried out under an inert atmosphere. A

solution of 7-fluoro-2,2,5-trimethyl-1,2-dihydroquinoline (10 g (70% pure), 36.6 mmol) in dry $Et_2O$ (900 mL) was cooled to -78 °C. Then, 1.6 M BuLi in hexanes (46 mL, 73.6 mmol) was slowly added and stirring was continued at the same temperature for 15 min and at -30°C for another 15 min. The reaction mixture was re-cooled to -78 °C and a solution of $Boc_2O$ (17.5 g, 80 mmol) in dry $Et_2O$ (100 mL) was added dropwise. Then the reaction mixture was stirred at room temperature overnight. Saturated aqueous $NH_4Cl$ (500 mL) was added to the suspension and the mixture was stirred until gas evolution ceased and a clear solution was obtained. The layers were separated and the aqueous layer was extracted with $Et_2O$ (3x50 mL). The organic layers were combined and washed with brine (250 mL) and dried over $Na_2SO_4$. Filtration followed by *in vacuo* concentration gave impure tert-butyl 7-fluoro-2,2,5-trimethylquinoline-1(2H)-carboxylate as a brown oil (19.5 g).

Step 3: Heat gun dried glassware was used and the reaction was carried out under an inert atmosphere. To an ice-bath cooled solution of tert-butyl 7-fluoro-2,2,5-trimethylquinoline-1(2H)-carboxylate (18.8 g, max. 35.3 mmol) in dry THF (400 mL) was added dropwise 1 M $BH_3$ in THF (210 mL, 210 mmol) while vigorously stirring. After complete addition, the mixture was allowed to warm up to room temperature and was stirred overnight. Then, the reaction mixture was cooled in an ice-bath and carefully oxidised by adding 1 M aqueous KOH (300 mL, 300 mmol), followed by 30% aqueous $H_2O_2$ (60 mL, 587 mmol). The mixture was stirred at room temperature for 3 h and was then diluted with water (300 mL) and EtOAc (200 mL). The aqueous layer was extracted with EtOAc (3x100 mL). The combined organic layers were washed with brine and dried over $Na_2SO_4$. Filtration and *in vacuo* filtrate concentration gave the crude product as a mixture of tert-butyl 7-fluoro-4-hydroxy-2,2,5-trimethyl-3,4-dihydroquinoline-1(2H)-carboxylate and tert-butyl 7-fluoro-3-hydroxy-2,2,5-trimethyl-3,4-dihydroquinoline-1(2H)-carboxylate as a brown oil (19.95 g). The isolated material was used as such without further purification.

Step 4: To a solution/suspension of a mixture of tert-butyl 7-fluoro-4-hydroxy-2,2,5-trimethyl-3,4-dihydroquinoline-1(2H)-carboxylate and tert-butyl 7-fluoro-3-hydroxy-2,2,5-trimethyl-3,4-dihydroquinoline-1(2H)-carboxylate (14.8 g, max. 26.3 mmol) in DCM (500 mL) was added PCC (6.02 g, 27.9 mmol). The obtained orange/brown suspension was stirred at room temperature for 2 h. An extra portion of PCC (1.51 g, 7.00 mmol) was added and stirring was continued overnight. Then, celite (ca. 50 g) was added and the reaction mixture was stirred at room temperature for 1 h. The black solid particles were filtered off over a silica column. The filter cake was washed with DCM (3x50 mL). Combined filtrates were *in vacuo* concentrated to obtain the crude product. Purification by flash chromatography (80 g silica, gradient heptane/EtOAc, 1:0 → 19:1) gave a pure batch (325 mg) and an impure batch (1.95 g) of tert-butyl 7-fluoro-2,2,5-trimethyl-4-oxo-3,4-dihydroquinoline-1(2H)-carboxylate. The impure batch was repurified by flash chromatography (120 g silica, gradient heptane/EtOAc, 1:0 → 99:1, then heptane/EtOAc, 19:1) and gave another pure batch of tert-butyl7-fluoro-2,2,5-trimethyl-4-oxo-3,4-dihydroquinoline-1(2H)-carboxylate (1181 mg). Combining the pure batches gave tert-butyl 7-fluoro-2,2,5-trimethyl-4-oxo-3,4-dihydroquinoline-1(2H)-carboxylate as an off-white solid (1.506 g, 4.90 mmol, 19% over three steps).

Step 5: To an ice-bath cooled solution of tert-butyl 7-fluoro-2,2,5-trimethyl-4-oxo-3,4-dihydroquinoline-1(2H)-carboxylate (1.506 g, 4.90 mmol) in DCM (25 mL) was added a solution of TFA (20.64 mL, 269 mmol) in DCM (5 mL). After stirring the mixture at room temperature for 30 min, the reaction mixture was diluted with water (20 mL) and was cooled in an ice-bath. The acidic mixture was carefully alkalised with 2 M aqueous NaOH (135 mL) and saturated aqueous $NaHCO_3$ to pH 8-9. The layers were separated using a phase separator. The aqueous layer was extracted with DCM (3x10 mL). The combined organic layers were *in vacuo* concentrated to isolate 7-fluoro-2,2,5-trimethyl-2,3-dihydroquinolin-4(1H)-one as an orange solid (997 mg, 4.81 mmol, 98%).

Step 6: The conversion of 7-fluoro-2,2,5-trimethyl-2,3-dihydroquinolin-4(1H)-one (911 mg, 4.40 mmol) was performed in 4 batches of 166 mg each (in 5 mL NMP each), 1 batch of 177 mg (in 5 mL NMP) and 1 batch of 70 mg (in 2.5 mL NMP). A typical procedure is shown below. Oven dried glassware was used. To a mixture of 7-fluoro-2,2,5-trimethyl-2,3-dihydroquinolin-4(1H)-one (166 mg, 0.8 mmol), methylammonium acetate (365 mg, 4.0 mmol) and 4-nitrophenyl azide (171 mg, 1.04 mmol) was added dry NMP (5 mL) and after sealing, the vial was stirred at 80 °C for 90 h. The reaction mixture was cooled down to room temperature and combined with other reaction mixtures for work up. The obtained mixture was diluted with EtOAc (200 mL) and brine (1000 mL). The layers were separated and the aqueous layer was extracted with EtOAc (3x100 mL). The combined organic layers were washed with brine (3x200 mL) and dried over $Na_2SO_4$. Filtration and *in vacuo* filtrate concentration gave the crude product. Purification by flash chromatography (120 g silica, EtOAc/heptane, 1:99 → 1:1) gave an impure batch of 7-fluoro-1,4,4,9-tetramethyl-4,5-dihydro-1H-[1,2,3]triazolo[4,5-c]quinoline and the starting material 7-fluoro-2,2,5-trimethyl-2,3-dihydroquinolin-4(1H)-one as a brownish solid (0.5 g, 2.41 mmol, 54% recovery). The impure batch of the target compound was purified further by flash chromatography (12 g silica, DCM followed by heptane/EtOAc, 19:1 - 1:1) and gave 7-fluoro-1,4,4,9-tetramethyl-4,5-dihydro-1H-[1,2,3]triazolo[4,5-c]quinoline as a brownish solid (137 mg, 0.556 mmol, 12%).

Step 7: To an ice-bath cooled solution of 7-fluoro-1,4,4,9-tetramethyl-4,5-dihydro-1H-[1,2,3]triazolo[4,5-c]quinoline (124 mg, 0.493 mmol) in dry DMF (7 mL) was added a solution of NBS (88 mg, 0.493 mmol) in dry DMF (2 mL) and the mixture was stirred at room temperature for 60 min. The reaction mixture was diluted with brine (150 mL) and EtOAc (50 mL). The layers were separated and the aqueous layer was extracted with EtOAc (4x20 mL). The combined organic layers

were washed with brine (3x50 mL) and dried over $Na_2SO_4$. Filtration followed by *in vacuo* filtrate concentration gave the crude product. Purification by flash chromatography (12 g silica, gradient heptane/EtOAc, 20:1 → 1:1) gave 8-bromo-7-fluoro-1,4,4,9-tetramethyl-4,5-dihydro-1H-[1,2,3]triazolo[4,5-c]quinoline as a salmon pink solid (150 mg, 0.461 mmol, 93%).

The following intermediates were prepared in a similar manner (use of appropriate reagents and purification methods known to the person skilled in the art) as the synthesis described for **intermediate B11:**

| Intermediate | Structure |
|---|---|
| **B26** | 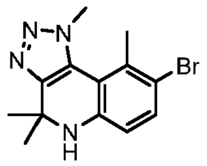 |

Synthesis of 9-bromo-8-fluoro-1,5,5,10-tetramethyl-5,6-dihydropyrazolo[1,5-c]quinazoline **(intermediate B9):**

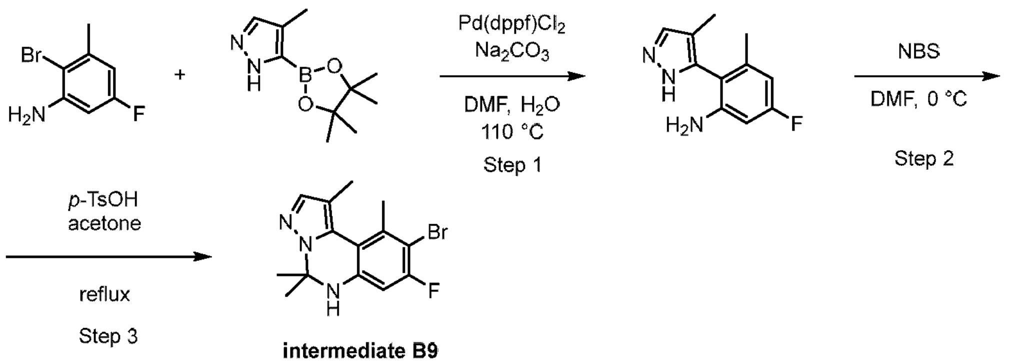


**intermediate B9**

Step 1: In the glovebox stock solutions were prepared of 2-bromo-5-fluoro-3-methylaniline (1.20 g, 5.88 mmol) in degassed DMF (75 mL), 4-methyl-5-(4,4,5,5-tetramethyl-1,3,2-dioxaborolan-2-yl)-1*H*-pyrazole (1.84 g, 8.82 mmol) in degassed DMF (75 mL) and $Na_2CO_3$ (1.25 g, 11.8 mmol) in degassed water (6 mL). These stock solutions were divided over 31 vials. Next, Pd(dppf)$Cl_2$ (28 mg, 0.038 mmol) was added to each vial. The vials were capped, removed from the glovebox and stirred in the fume hood at 110 °C overnight. The 31 batches were combined, diluted with EtOAc (150 mL) and filtered through celite. The filter cake was rinsed with EtOAc (75 mL) and the combined filtrates were concentrated under reduced pressure. The crude product was co-evaporated with toluene (2x20 mL) and was then suspended in EtOAc (25 mL). The suspension was filtered through celite and the filter cake was rinsed with EtOAc (200 mL). The combined filtrate was concentrated under reduced pressure. Purification by flash chromatography (80 g silica, gradient DCM/(7M $NH_3$ in MeOH), 1:0 → 95:5) afforded an impure batch of 5-fluoro-3-methyl-2-(4-methyl-1H-pyrazol-5-yl)aniline. The impure product was combined with other impure batches prepared in a similar fashion (starting with 25-200 mg of 5-fluoro-3-methyl-2-(4-methyl-1H-pyrazol-5-yl)aniline) and was purified further by preparative LC to afford pure 5-fluoro-3-methyl-2-(4-methyl-1H-pyrazol-5-yl)aniline (399 mg, 1.94 mmol, 23%).

Step 2: To an ice-bath cooled solution of 5-fluoro-3-methyl-2-(4-methyl-1H-pyrazol-5-yl)aniline (393 mg, 1.92 mmol) in dry DMF (24 mL) was added dropwise a solution of NBS (341 mg, 1.92 mmol) in dry DMF (8 mL). The mixture was stirred for 1 h and was then combined with another batch which was prepared in a similar fashion (starting from 50 mg (0.24 mmol) of 5-fluoro-3-methyl-2-(4-methyl-1H-pyrazol-5-yl)aniline). The mixture was diluted with brine (100 mL), EtOAc (100 mL) and water (25 mL), and the layers were separated. The aqueous phase was extracted with EtOAc (2x100 mL) and the combined organic layers were washed with brine (2x100 mL), dried over $Na_2SO_4$ and concentrated under reduced pressure to afford crude 4-bromo-5-fluoro-3-methyl-2-(4-methyl-1H-pyrazol-5-yl)aniline (1.56 g).

Step 3: To a solution of 4-bromo-5-fluoro-3-methyl-2-(4-methyl-1H-pyrazol-5-yl)aniline (1.56 g, max. 2.16 mmol) in acetone (50 mL) was added *p*-TsOH $H_2O$ (41 mg, 0.22 mmol) and the solution was stirred at reflux temperature overnight. The mixture was diluted with brine (250 mL) and saturated aqueous $NaHCO_3$ (50 mL), then the acetone was removed under reduced pressure. EtOAc (200 mL) was added and the layers were separated. The aqueous layer was extracted with EtOAc (2x150 mL) and the combined organic layer was washed with brine (3x150 mL), dried over $Na_2SO_4$

and concentrated under reduced pressure. Purification by flash chromatography (24 g silica, gradient heptane/EtOAc, 95:5 → 1:1) and co-evaporation with $Et_2O$ (4x20 mL) afforded 9-bromo-8-fluoro-1,5,5,10-tetramethyl-5,6-dihydropyrazolo [1,5-c]quinazoline (621 mg, 1.92 mmol, 89% over two steps).

Synthesis of 9-bromo-8,10-difluoro-5,5-dimethyl-5,6-dihydropyrazolo[1,5-c]quinazoline **(intermediate B10):**

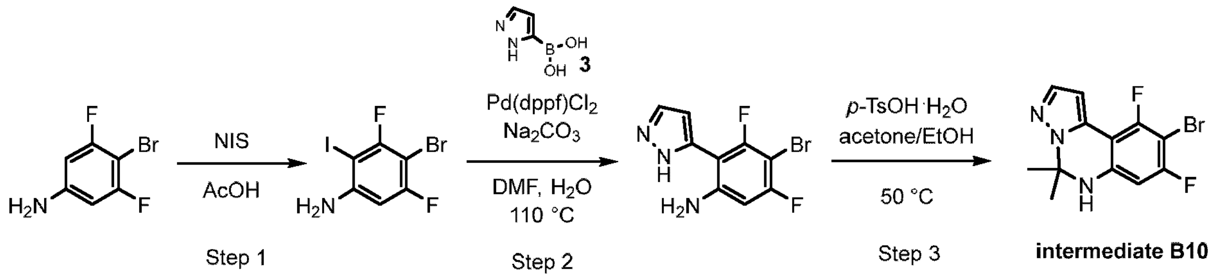


Step 1: To a solution of 4-bromo-3,5-difluoroaniline (5 g, 24.0 mmol) in AcOH (60 mL) was added NIS (5.68 g, 25.2 mmol) and the mixture was stirred for 2 h. Then, the mixture was poured into water (300 mL) and was extracted with EtOAc (2x200 mL). The combined organic layers were washed with aqueous 1 M NaOH (200 mL), aqueous saturated $Na_2S_2O_3$ (100 mL) and brine (300 mL). Next, the organic layers were dried over $Na_2SO_4$, concentrated under reduced pressure and co-evaporated with toluene (2x100 mL). Purification by flash chromatography (120 g silica, gradient heptane/EtOAc, 95:5 → 1:1) afforded a pure batch of 4-bromo-3,5-difluoro-2-iodoaniline (5.99 g, 17.9 mmol, 74%) and an impure batch. The impure batch was purified further by flash chromatography (40 g silica, gradient heptane/EtOAc 1:0 → 3:1) to afford 4-bromo-3,5-difluoro-2-iodoaniline (1.29 g, 3.86 mmol, 16%). Total yield of 4-bromo-3,5-difluoro-2-iodoaniline was 7.28 g (21.8 mmol, 90%).

Step 2: The reaction mixture was prepared in the glovebox. To a suspension of 4-bromo-3,5-difluoro-2-iodoaniline (1.5 g, 4.49 mmol), 1H-pyrazole-5-boronic acid (0.75 g, 6.74 mmol) and $Na_2CO_3$ (0.95 g, 8.98 mmol) in degassed DMF (105 mL) and degassed water (4.2 mL) was added Pd(dppf)$Cl_2$ (0.66 g, 0.898 mmol). The mixture was removed from the glovebox and stirred at 110 °C for 3 h in a pre-heated oil-bath as an open inert system. The mixture was cooled down to room temperature and was diluted with EtOAc (250 mL). Brine (250 mL) and saturated aqueous $NaHCO_3$ (100 mL) were added and the layers were separated. The aqueous phase was extracted with EtOAc (2x250 mL). The combined organic layers were washed with 80% saturated brine (3x500 mL) and saturated brine (500 mL), dried over $Na_2SO_4$(s) and concentrated under reduced pressure to afford crude 4-bromo-3,5-difluoro-2-(1H-pyrazol-5-yl)aniline (2.11 g, max. 4.49 mmol).

Step 3: To a solution of two combined batches of crude 4-bromo-3,5-difluoro-2-(1H-pyrazol-5-yl)aniline (2.33 g, max. 4.94 mmol) in EtOH/acetone (50 mL, 1/1, v/v) was added *p*-TsOH $H_2O$ (94 mg, 0.49 mmol) and the solution was stirred at 50 °C for 30 min. The mixture was cooled down to room temperature. Brine (250 mL) and saturated aqueous $NaHCO_3$ (50 mL) were added and the mixture was extracted with EtOAc (3x200 mL). The combined organic layers were washed with brine (500 mL), dried over $Na_2SO_4$(s) and concentrated under reduced pressure. Purification by flash chromatography (80 g silica, gradient heptane/EtOAc, 95:5 → 1:1) afforded impure 9-bromo-8,10-difluoro-5,5-dimethyl-5,6-dihydropyrazolo[1,5-c]quinazoline, which was further purified by preparative LC. The product containing fractions were combined and MeCN was removed under reduced pressure. The aqueous phase was extracted with EtOAc (3x150 mL) and the combined organic layers were washed with brine (300 mL), dried over $Na_2SO_4$ and concentrated under reduced pressure to afford 9-bromo-8,10-difluoro-5,5-dimethyl-5,6-dihydropyrazolo[1,5-c]quinazoline (831 mg, 2.65 mmol, 53% over two steps).

Synthesis of 2-bromo-6,6,9-trimethyl-5,6-dihydropyrido[3,2-e][1,2,4]triazolo[4,3-a]pyrazine **(intermediate B11):**

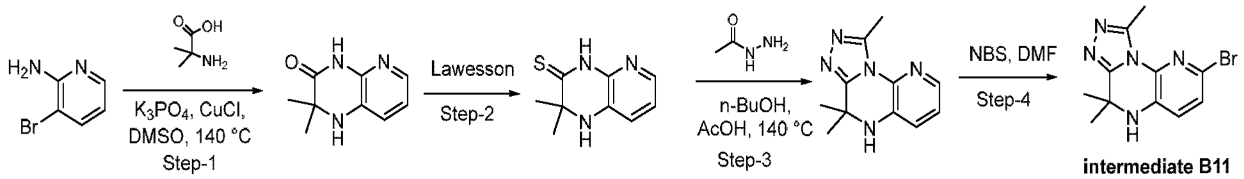

Step 1: A suspension of 3-bromopyridin-2-amine (1 g, 5.78 mmol, 1 eq.) in dry DMSO (18 ml), 2-Amino-2-methyl-propionic acid (1.19 g, 11.56 mmol, 2 eq.) and $K_3PO_4$ (2.45 g, 11.56 mmol, 2 eq.) was degassed with argon for 10 min, before CuCl (0.078 g, 0.578 mmol, 0.1 eq) was added. The reaction mixture was then heated to 140 °C for 16 h. After completion of the reaction it was filtered through a celite bed, which was washed with ethyl acetate (100 ml). The filtrate was diluted with ethyl acetate (100 ml) and was washed with water (3x150 ml) and brine (200 ml), dried over anhydrous $Na_2SO_4$ and evaporated under reduced pressure. The crude residue was purified by column chromatography (100-200 mesh silica gel; 30% ethyl acetate/hexane; $R_f$-value-0.5) to afford 2,2-dimethyl-1,4-dihydropyrido[2,3-b]pyrazin-3(2H)-one (0.5 g, 49%) as a brown solid.

Step 2: To a solution of 2,2-dimethyl-1,4-dihydropyrido[2,3-b]pyrazin-3(2H)-one (0.5 g, 2.82 mmol, 1 eq.) in toluene (10 ml) was added Lawesson's reagent (1.71 g, 4.23 mmol, 1.5 eq.) at RT and the reaction mixture was then refluxed at 120 °C for 40 min. After completion of the reaction (monitored by TLC), the reaction mixture was quenched with sat. $NaHCO_3$ solution (50 ml) followed by extraction with ethyl acetate (3x50 ml). The combined organic layers were washed with water (100 ml) and brine (100 ml), dried over anhydrous $Na_2SO_4$ and evaporated to get the crude residue which was purified by column chromatography (230-400 mesh silica gel; 20% ethyl acetate/hexane; $R_f$-value-0.4) to afford 2,2-dimethyl-1,4-dihydropyrido[2,3-b]pyrazine-3(2H)-thione (0.5 g, 91.9%) as a yellow solid.

Step 3: To a solution of 2,2-dimethyl-1,4-dihydropyrido[2,3-b]pyrazine-3(2H)-thione (6.5 g, 33.67 mmol, 1 eq.) in *n*-BuOH (120 ml) was added acetyl hydrazide (9.96 g, 134.71 mmol, 4 eq) followed by addition of acetic acid (12 ml) and then the reaction mixture was heated to 160 °C for 16 h in a sealed tube. After completion of the reaction (monitored by TLC), the reaction mixture was evaporated under reduced pressure to get the crude material, which was purified by column chromatography (100-200 mesh silica gel; 5% methanol/dichloromethane; $R_f$-value-0.3) to afford 6,6,9-trimethyl-5,6-dihydropyrido[3,2-e][1,2,4]triazolo[4,3-a]pyrazine (5 g, 70.1%) as an off-white solid.

Step 4: To the stirred solution of 6,6,9-trimethyl-5,6-dihydropyrido[3,2-e][1,2,4]triazolo[4,3-a]pyrazine (5 g, 23.25 mmol, 1 eq.) in DMF (60 ml) was added N-bromosuccinimide (4.5 g, 25.58 mmol, 1.1 eq) portionwise. The reaction mixture was allowed to warm to RT and was stirred for 2 h. The reaction mixture was quenched with ice, causing precipitation of a solid, which was filtered off, was dried under reduced pressure and was washed with pentane to afford 2-bromo-6,6,9-trimethyl-5,6-dihydropyrido[3,2-e][1,2,4]triazolo[4,3-a]pyrazine (3 g, 43.9%) as brown solid. The following intermediates were prepared in a similar manner (use of appropriate reagents and purification methods known to the person skilled in the art) as the synthesis described for **intermediate B11:**

| Intermediate | Structure |
|---|---|
| **B12** | 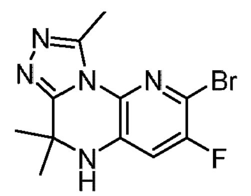 |

Synthesis of 8-bromo-1,4,4,9-tetramethyl-4,5-dihydropyrido[3,4-e][1,2,4]triazolo[4,3-a]pyrazine **(intermediate B13):**

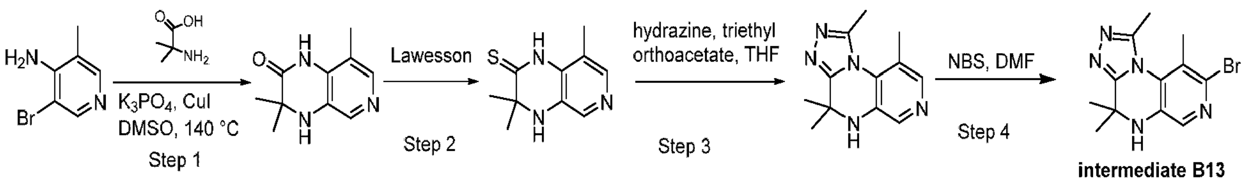


**intermediate B13**

Step 1: A mixture of 3-bromo-5-methylpyridin-4-amine (10 g, 53.47 mmol, 1 eq.), 2-amino-2-methyl-propionic acid (11 g, 106.95 mmol, 2 eq.) and $K_3PO_4$ (22.7 g, 106.95 mmol, 2 eq.) in dry DMSO (100 ml) was degassed with argon for 10 min before the addition of CuI (1 g, 5.347 mmol, 0.1 eq). The reaction mixture was then stirred at 140 °C for 16 h. After completion of the reaction it was filtered through a celite bed, which was washed with ethyl acetate (300 ml). The filtrate was diluted with ethyl acetate (300 ml) and was washed with water (3x500 ml) and brine (500 ml), dried over anhydrous $Na_2SO_4$ and evaporated under reduced pressure. The obtained crude residue was purified by column chromatography (100-200 mesh silica gel; 30% ethyl acetate/hexane; $R_f$-value-0.5) to afford 3,3,8-trimethyl-3,4-dihydropyrido[3,4-b]pyrazin-2(1H)-one (3 g, 29.4%) as a brown solid.

Step 2: To a solution of 3,3,8-trimethyl-3,4-dihydropyrido[3,4-b]pyrazin-2(1H)-one (1.5 g, 7.85 mmol, 1 eq.) in toluene (30 ml) was added Lawesson's reagent (4.76 g, 11.78 mmol, 1.5 eq.) at RT and the reaction mixture was then heated to 120 °C for 40 min. After completion of the reaction (monitored by TLC), the reaction mixture was quenched with sat. $NaHCO_3$ solution (50 ml) followed by extraction with ethyl acetate (3x50 ml). The combined organic layers were washed with water (100 ml) and brine (100 ml), dried over anhydrous $Na_2SO_4$ and evaporated to get the crude material which was purified by column chromatography (230-400 mesh silica gel; 20% ethyl acetate/hexane; $R_f$-value-0.4) to afford 3,3,8-trimethyl-3,4-dihydropyrido[3,4-b]pyrazine-2(1H)-thione (1 g, 61.7%) as a yellow solid.

Step 3: To a solution of 3,3,8-trimethyl-3,4-dihydropyrido[3,4-b]pyrazine-2(1H)-thione (1 g, 4.83 mmol, 1 eq) in THF (15 ml) was added hydrazine hydrate (1.5 ml) at RT. The reaction was then stirred at RT for 5 h. After completion of the reaction (monitored by TLC), the reaction mixture was evaporated under reduced pressure to get the crude material, which was dissolved in triethylorthoacetate (15 ml). The resulting mixture was heated to 130 °C for 16 h. After completion of the reaction (monitored by TLC), the reaction mixture was evaporated under reduced pressure to get the crude material, which was purified by column chromatography to afford 1,4,4,9-tetramethyl-4,5-dihydropyrido[3,4-e][1,2,4]triazolo[4,3-a]pyrazine (0.7 g, 63.6%) as a brown gum.

Step 4: To the stirred solution of 1,4,4,9-tetramethyl-4,5-dihydropyrido[3,4-e][1,2,4]triazolo[4,3-a]pyrazine (1.3 g, 5.67 mmol, 1 eq.) inDMF (15 ml) was added dropwise N-bromosuccinimide (1 g, 5.67 mmol, 1 eq) dissolved in DMF (5 ml) at 55°C. The reaction mixture was stirred at the same temperature for 2 h. After completion of the reaction (monitored by LCMS), the reaction mixture was quenched with ice and was extracted with EtOAc. The combined organic layers were washed water and brine, dried over $Na_2SO_4$, filtered and evaporated under reduced pressure to get the crude material, which was purified by column chromatography to afford 8-bromo-1,4,4,9-tetramethyl-4,5-dihydropyrido[3,4-e][1,2,4]triazolo[4,3-a]pyrazine (0.3 g, 17.6%) as a brown solid.

Synthesis of 8-bromo-6-fluoro-1,4,4,9-tetramethyl-4,5-dihydro-1H-pyrazolo[4,3-c]quinoline **(intermediate B14):**

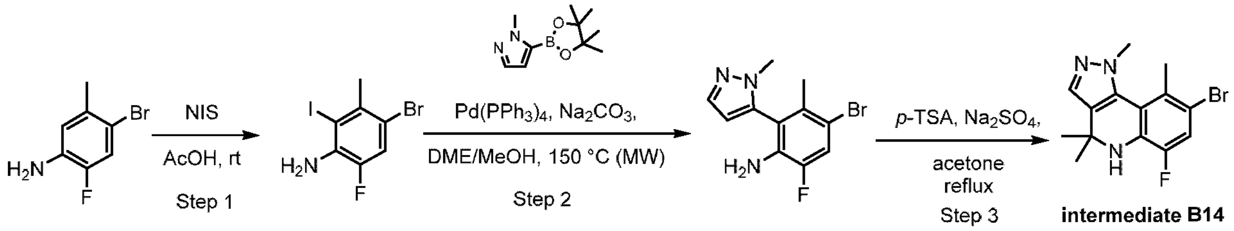


Step 1: To a solution of 4-bromo-2-fluoro-5-methylaniline (5 g, 24.50 mmol) in AcOH (60 mL) was added NIS (5.51 g, 24.50 mmol) and the mixture was stirred at room temperature for 2 h. The mixture was partially concentrated under reduced pressure to ~10 mL and the remainder was poured into water (300 mL). The mixture was then extracted with EtOAc (2x200 mL). The combined organic layers were washed with aqueous 2 M NaOH (150 mL), saturated aqueous $Na_2S_2O_3$ (300 mL) and brine (300 mL), dried over $Na_2SO_4$ and concentrated under reduced pressure. Purification by flash chromatography (120 g silica, gradient heptane/EtOAc, 95:5 → 85:15) afforded 4-bromo-6-fluoro-2-iodo-3-methylaniline (7.80 g, 23.6 mmol, 96%).

Step 2: The following procedure was repeated in three batches. In the glovebox was prepared a suspension of 4-bromo-6-fluoro-2-iodo-3-methylaniline (1.25 g, 3.79 mmol), 1-methyl-1H-pyrazole-5-boronic acid pinacol ester (1.18 g, 5.68 mmol) and $Na_2CO_3$ (1.21 g, 11.37 mmol) in DME (12.5 mL)/MeOH (6.25 mL). $Pd(PPh_3)_4$ (0.44 g, 0.38 mmol) was added, and the vial was capped and removed from the glovebox. The mixture was then stirred at 150 °C for 45 minutes using microwave irradiation. The three batches were combined and water (75 mL), brine (75 mL) and EtOAc (150 mL) were added. The layers were separated and the aqueous phase was extracted with EtOAc (2x 100 mL). The combined organic layers were washed with brine (300 mL), dried over $Na_2SO_4$ and concentrated under reduced pressure. Purification by flash chromatography (220 g silica, gradient heptane/EtOAc, 95:5 → 3:2) afforded 4-bromo-6-fluoro-3-methyl-2-(1-methyl-1H-pyrazol-5-yl)aniline (2.29 g, 8.06 mmol, 71%).

Step 3: A suspension of 4-bromo-6-fluoro-3-methyl-2-(1-methyl-1H-pyrazol-5-yl)aniline (2.29 g, 8.06 mmol), p-TSA (1.533 g, 8.06 mmol) and $Na_2SO_4$ (11.45 g, 81 mmol) in dry acetone (75 mL) was stirred at reflux overnight under a nitrogen atmosphere. The mixture was filtered through celite and the filter cake was washed with acetone (25 mL). The filtrates were combined and concentrated under reduced pressure. Purification by flash chromatography (220 g silica, gradient heptane/EtOAc, 9:1 → 3:2) afforded 8-bromo-6-fluoro-1,4,4,9-tetramethyl-4,5-dihydro-1H-pyrazolo[4,3-c]quinoline (965 mg, 2.98 mmol, 37%).

The following intermediates were prepared in a similar manner (use of appropriate reagents and purification

methods known to the person skilled in the art) as the synthesis described for **intermediate B14:**

| Intermediate | Structure | Reagents |
|---|---|---|
| **B16** | 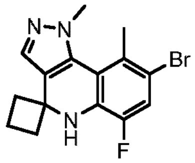 | 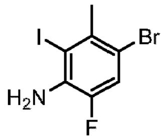 + 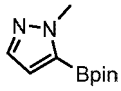 + 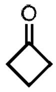 |
| **B17** | 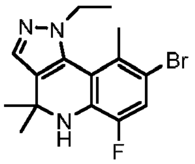 | 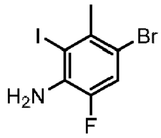 + 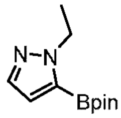 + 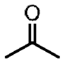 |
| **B18** | 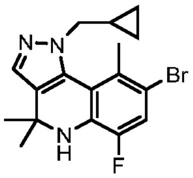 | 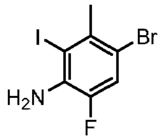 + 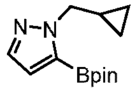 + 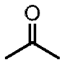 |
| **B19** | 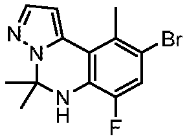 | 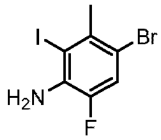 + 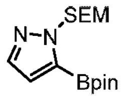 + 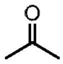 |
| **B20** | 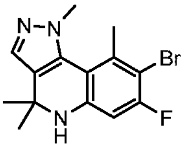 | 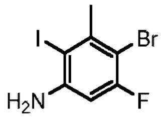 + 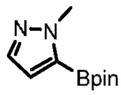 + 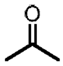 |
| **B22** | 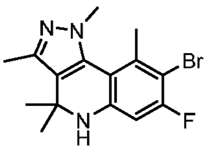 | 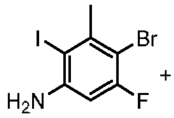 + 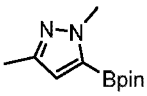 + 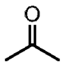 |
| **B23** | 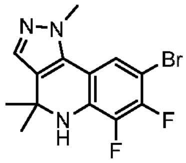 | 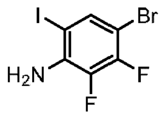 + 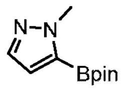 + 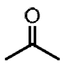 |
| **B24** | 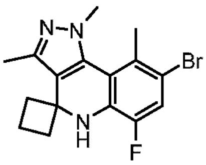 | 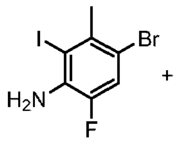 + 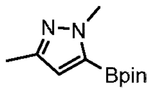 + 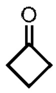 |
| **B28** | 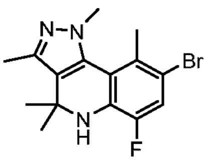 | 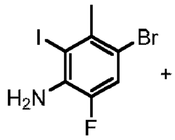 + 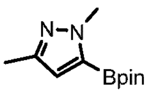 + 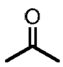 |

Synthesis of 9-bromo-7-fluoro-1,5,5,10-tetramethyl-5,6-dihydro-[1,2,3]triazolo[1,5-c]quinazoline **(intermediate B15):**

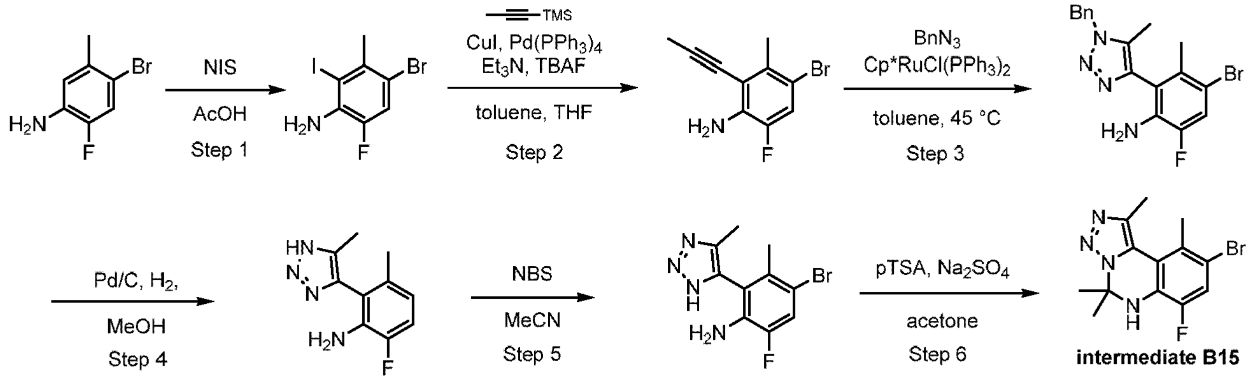


**intermediate B15**

Step 1: To a solution of 4-bromo-2-fluoro-5-methylaniline (15 g, 73.5 mmol) in AcOH (175 mL) was added NIS (16.5 g, 73.5 mmol) and the mixture was stirred at room temperature for 2 h. The mixture was concentrated under reduced pressure and the remainder was poured into $H_2O$ (500 mL), followed by extraction with EtOAc (3x250 mL). The combined organic layers were washed with aqueous 2 M NaOH (300 mL), saturated aqueous $Na_2S_2O_3$ (300 mL) and brine (300 mL), dried over $Na_2SO_4$ and concentrated under reduced pressure. The crude product was filtered over silica using heptane/EtOAc (4:1) as eluent. The product was further purified by crystallisation from hot heptane to afford 4-bromo-6-fluoro-2-iodo-3-methylaniline (21 g, 63.6 mmol, 86%).

Step 2: CuI (2.67 g, 14.00 mmol) was weighed out in the glovebox and was then added to a solution of 4-bromo-6-fluoro-2-iodo-3-methylaniline (15.4 g, 46.7 mmol) in degassed toluene (90 mL) in the fumehood. $Pd(PPh_3)_4$ (2.70 g, 2.33 mmol), degassed $Et_3N$ (21.41 mL, 154 mmol) and degassed 1-(trimethylsilyl)-1-propyne (13.97 mL, 93 mmol) were added followed by the dropwise addition of degassed 1 M TBAF in THF (93 mL, 93 mmol). The mixture was stirred for 5 h before additional degassed 1-(trimethylsilyl)-1-propyne (13.97 mL, 93 mmol) was added followed by the dropwise addition of degassed 1 M TBAF in THF (93 mL, 93 mmol). Stirring was continued overnight. Aqueous 0.5 M HCl (500 mL) was added and the layers were separated. The aqueous phase was extracted with EtOAc (2x300 mL) and the combined organic layers were washed with saturated aqueous $NaHCO_3$ (300 mL) and brine (300 mL), dried over $Na_2SO_4$ and concentrated under reduced pressure. The crude product was coated on hydro-matrix and was purified by gravitational column chromatography (1 kg silica, heptane/EtOAc 1:0 $\rightarrow$ 99:1 (~25 L) to afford impure product. The impure product was again coated on hydro-matrix and was purified in two batches by flash chromatography (440 g silica, heptane) which afforded 4-bromo-6-fluoro-3-methyl-2-(prop-1-yn-1-yl)aniline (6.10 g, 25.2 mmol, 54%).

Step 3: $Cp^*RuCl(PPh_3)_2$ (0.31 g, 0.38 mmol) was weighed in in the glovebox and was then added to a degassed solution of 4-bromo-6-fluoro-3-methyl-2-(prop-1-yn-1-yl)aniline (1.82 g, 7.51 mmol) and benzyl azide (0.938 mL, 7.51 mmol) in toluene (75 mL). The mixture was heated to 45 °C for 16 h and was then cooled down to room temperature and was concentrated under reduced pressure. Purification by flash chromatography (80 g silica, gradient heptane/EtOAc, 95:5 $\rightarrow$ 1:1) afforded 2-(1-benzyl-5-methyl-1H-1,2,3-triazol-4-yl)-4-bromo-6-fluoro-3-methylaniline (1.92 g, 5.12 mmol, 68%).

Step 4: 2-(1-Benzyl-5-methyl-1H-1,2,3-triazol-4-yl)-4-bromo-6-fluoro-3-methylaniline (1.92 g, 5.12 mmol) was dissolved in MeOH (150 mL) by heating with a heatgun and the resulting solution was flushed with nitrogen for 5 min. Next, 10% Pd(C) (0.545 g, 0.512 mmol) was added, the atmosphere was replaced by $H_2$ and the mixture was stirred overnight. The reaction mixture was filtered over celite (pre-rinsed with MeOH) and the filer cake was rinsed with MeOH (2x~25 mL). The filtrates were combined and concentrated under reduced pressure to afford the product as its HBr-salt. The product was partitioned between EtOAc (100 mL), MeOH (5 mL), $H_2O$ (25 mL) and saturated aqueous $NaHCO_3$ (50 mL). The layers were separated and the aqueous phase was extracted with EtOAc (50 mL). The combined organic layers were washed with brine, dried over $Na_2SO_4$ and concentrated under reduced pressure to afford 6-fluoro-3-methyl-2-(5-methyl-1H-1,2,3-triazol-4-yl)aniline (1.0 g, 4.85 mmol, 95%).

Step 5: To a solution of crude 6-fluoro-3-methyl-2-(5-methyl-1H-1,2,3-triazol-4-yl)aniline (1.0 g, 4.85 mmol) in anhydrous MeCN (50 mL) was added NBS (0.863 g, 4.85 mmol) and the mixture was stirred for 1 h. Half saturated aqueous $NaHCO_3$ (100 mL) and EtOAc (50 mL) were added and the layers were separated. The aqueous phase was extracted with EtOAc (2x75 mL) and the combined organic layers were washed with brine (100 mL), dried over $Na_2SO_4$ and concentrated under reduced pressure to afford 4-bromo-6-fluoro-3-methyl-2-(4-methyl-1H-1,2,3-triazol-5-yl)aniline (1.46 g, max. 4.85 mmol).

Step 6: To a solution of crude 4-bromo-6-fluoro-3-methyl-2-(4-methyl-1H-1,2,3-triazol-5-yl)aniline (1.46 g, max.

4.85 mmol) in acetone (dried over 3 Å molsieves, 30 mL) was added $Na_2SO_4$ (17.3 g, 122 mmol) and p-TsOH·$H_2O$ (93 mg, 0.486 mmol) and the mixture was stirred for 30 min. The reaction mixture was poured into saturated aqueous $NaHCO_3$ (50 mL). $H_2O$ (50 mL) and EtOAc (75 mL) were added and the layers were separated. The aqueous phase was extracted with EtOAc (2x75 mL) and the combined organic layers were washed with brine (150 mL), dried over $Na_2SO_4$ and concentrated under reduced pressure. Purification by flash chromatography (24 g silica, gradient heptane/EtOAc 95:5 → 1:1) afforded 9-bromo-7-fluoro-1,5,5,10-tetramethyl-5,6-dihydro-[1,2,3]triazolo[1,5-c]quinazoline (1.32 g, 4.06 mmol, 79% (over 2 steps)).

Synthesis of 8-bromo-6-fluoro-1,4,4,9-tetramethyl-4,5-dihydro-1H-imidazo[4,5-c]quinoline **(intermediate B21):**

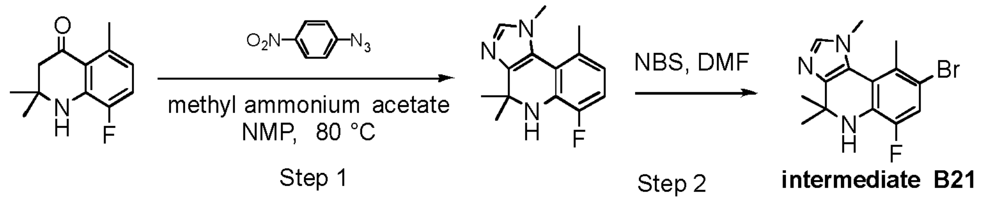


Step 1: To a solution of 8-fluoro-2,2,5-trimethyl-2,3-dihydro-1H-quinolin-4-one (this compound can be prepared in a similar manner (use of appropriate reagents and purification methods known to the person skilled in the art) as the synthesis described for 7-fluoro-2,2,5-trimethyl-2,3-dihydroquinolin-4(1H)-one) (2.5 g, 12 mmol) in dry NMP (25 ml) were added molecular sieves (4 Å, 1 g) and methyl ammonium acetate (5.5 g, 60.3 mmol) followed by 4-nitrophenyl azide (2.5 g, 15.6 mmol) at RT and the reaction mixture was then heated to 80 °C for 3 days. After completion of the reaction (monitored by LCMS), the reaction mixture was diluted with MTBE (100 ml) nd was washed with water (70 ml) followed by brine (70 ml). The organic layer was dried over anhydrous $Na_2SO_4$ and concentrated. The obtained crude residue was purified by column chromatography (silica gel, 100-200 mesh, 40% EtOAc in hexane as eluent) to afford 6-fluoro-1,4,4,9-tetramethyl-4,5-dihydro-1H-imidazo[4,5-c]quinolone (10)(580 mg, 19.7%) as a brownish liquid.

Step 2: To a solution of 6-fluoro-1,4,4,9-tetramethyl-4,5-dihydro-1H-imidazo[4,5-c]quinolone (100 mg, 0.407 mmol) in DMF (8 ml) was slowly added NBS (36.27 mg, 0.203 mmol) dissolved in DMF (2 ml) at 0 °C. The resulting reaction mixture was stirred at 0 °C for 1 h. After completion of the reaction (monitored by LCMS), the reaction mixture was diluted with cold water (10 ml) and extracted with ethyl acetate (2 x 50 ml). The combined organic layers were washed with cold water (2 x 50 ml) followed by cold brine (20 ml), dried over anhydrous $Na_2SO_4$ and concentrated to afford the crude compound. Four batches (300 mg each) ware done in parallel and combined batches were purified by column chromatography (silica gel, 100-200 mesh, 1-1.5% MeOH in DCM as eluent) to afford 8-bromo-6-fluoro-1,4,4,9-tetramethyl-4,5-dihydro-1H-imidazo[4,5-c]quinolone (420 mg, 24.5%) as a brownish solid. Synthesis of 8-bromo-6-fluoro-4,4,9-trimethyl-4,5-dihydro-1H-pyrazolo[4,3-c]quinoline **(intermediate B25):**

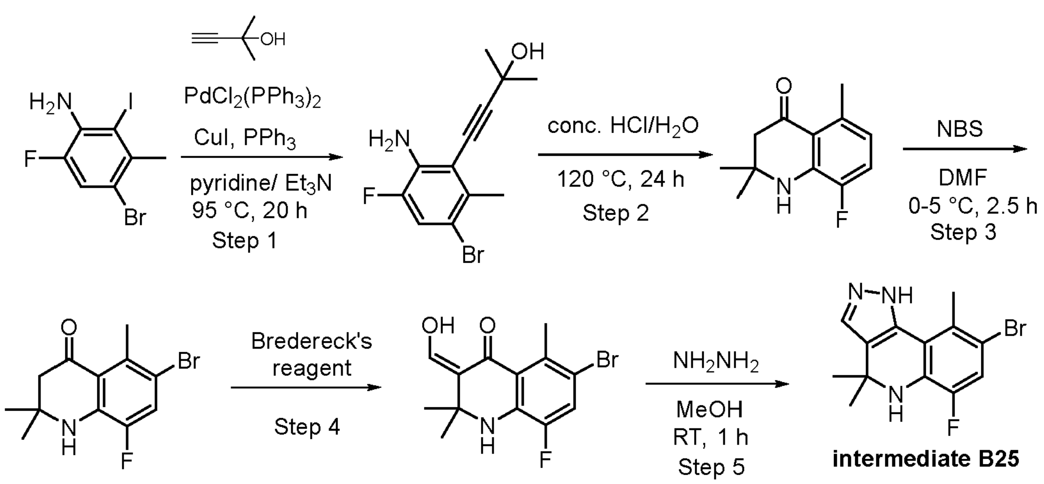


Step 1: To an argon purged solution of 4-bromo-6-fluoro-2-iodo-3-methylaniline (40 g, 121.23 mmol) in $Et_3N$: pyridine (1:1) (200 mL) was added 2-methyl-3-butyn-2-*ol* (17.6 mL, 181.85 mmol) and purging was continued for another 10 min, prior to the addition of CuI (1.15 g, 6.06 mmol), $PPh_3$ (15.9 g, 60.62 mmol) and $PdCl_2(PPh_3)_2$ (4.25 g, 6.06 mmol) at

RT. The resulting mixture was then heated to 90-95 °C for 20 h. The reaction was then quenched with brine (300 mL) and extracted with ethyl acetate (3 × 500 mL). The combined organic layers werr dried over $Na_2SO_4$ and concentrated to get the crude product which was purified by column chromatography (silica gel : 100-200 mesh) using 8-12% of EtOAc in pet-ether as eluent to afford 21 g (60%) of 4-(2-amino-5-bromo-3-fluoro-6-methylphenyl)-2-methylbut-3-yn-2-ol as a pale brown solid. (TLC system: 20% EtOAc in pet-ether, R*f*: 0.15).

Step 2: To a stirred solution of 4-(2-amino-5-bromo-3-fluoro-6-methylphenyl)-2-methylbut-3-yn-2-ol (21 g, 73.39 mmol) in 1,4-dioxane (210 mL) was added conc. HCl:water (1:1) (350 mL). The resulting mixture was then stirred at 120 °C for 20 h. The reaction mixture was cooled to RT, was diluted with EtOAc (500 mL) and ice-water (200 mL) and was then neutralized with solid $NaHCO_3$. The organic layer was separated and the aqueous layer was re-extracted with EtOAc (3 × 200 mL). The combined organic layers were dried over $Na_2SO_4$ and concentrated. The crude product was purified by silica-gel chromatography (100-200 mesh) using 1.5% of EtOAc in pet-ether as an eluent to afford 5.7 g (37%) of 8-fluoro-2,2,5-trimethyl-2,3-dihydroquinolin-4(1H)-one as a pale yellow solid (TLC system: 20% EtOAc in pet-ether, $R_f$: 0.7).

Step 3: To a solution of 8-fluoro-2,2,5-trimethyl-2,3-dihydroquinolin-4(1H)-one (5.7 g, 27.50 mmol) in DMF (40 mL) was added NBS (4.9 g, 27.50 mmol) in small portions at 0 °C and the mixture was stirred at 0-10 °C for 2 h. The reaction was monitered by LC-MS. After completion of the reaction, the reaction was diluted with cold water (100 ml), the precipitated solid was collected by filtration and was washed with water and dried to afford 7.9 g (99%) of 6-bromo-8-fluoro-2,2,5-trimethyl-2,3-dihydroquinolin-4(1H)-one as a pale yellow solid (TLC system: 20% EtOAc in pet-ether, $R_f$: 0.7).

Step 4: To a cold solution of 6-bromo-8-fluoro-2,2,5-trimethyl-2,3-dihydroquinolin-4(1H)-one (5 g, 17.47 mmol) in THF (50 mL) was added Bredereck's reagent (2.5 mL) at 0 °C. The resulting mixture was stirred at reflux (Note: Another two more portions of Bredereck's reagent were added at intervals 18 h and 26 h) for 44 h. The reaction mixture was diluted with EtOAc (500 mL), washed with water (100 mL) and brine, dried over $Na_2SO_4$, and concentrated to give 6 g (crude) of (Z)-6-bromo-8-fluoro-3-(hydroxymethylene)-2,2,5-trimethyl-2,3-dihydroquinolin-4(1H)-one as a brown color gummy mass (TLC system: 10% EtOAc in pet-ether, $R_f$: 0.5).

Step 5: To a cold solution of (Z)-6-bromo-8-fluoro-3-(hydroxymethylene)-2,2,5-trimethyl-2,3-dihydroquinolin-4(1H)-one (6 g, 19.10 mmol) in methanol (120 mL) was added hydrazine hydrate (9.3 mL, 191.00 mmol) at 0 °C and the reaction mixture was stirred at RT for 2 h. The reaction mass was quenched with sat. $NaHCO_3$ solution, and extracted with EtOAc (3 × 500 mL). The combined organic layers were dried over $Na_2SO_4$ and concentrated under reduced pressure (Note: Another 1 g reaction was carried out and was combined with the crude material for work up). The combined crude product was purified on silica-gel (100-200 mesh) using 15%-20% of EtOAc in pet-ether as an eluent to afford 2.4 g (44% over two steps) of 8-bromo-6-fluoro-4,4,9-trimethyl-4,5-dihydro-1H-pyrazolo[4,3-c]quinoline as a pale brown solid (TLC system: 20% EtOAc in pet-ether, $R_f$: 0.2).

Synthesis of 8-bromo-7-fluoro-4,4,9-trimethyl-4,5-dihydro-oxazolo[4,5-c]quinoline **(intermediate B27):**

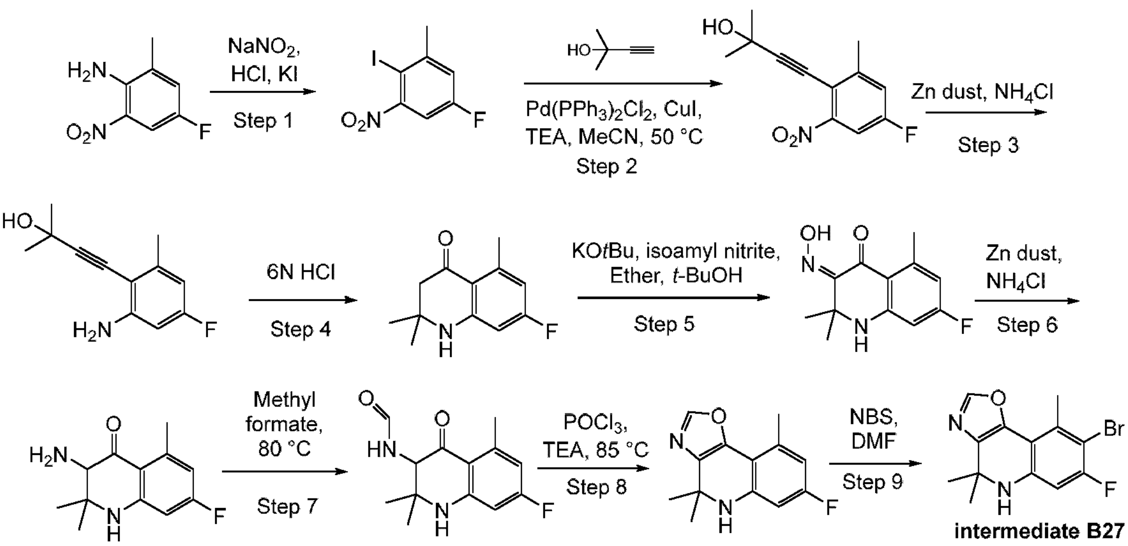


**intermediate B27**

Step 1: 4-Fluoro-2-methyl-6-nitro-phenylamine (50 g, 294.031 mmol) was treated with conc. HCl (80 ml) at 100 °C for 30 min. After that, the reaction mixture was allowed to cool to 0 °C before the dropwise addition of $NaNO_2$ (24.34 g, 352.837 mmol) in water (120 ml) to the reaction mixture at 0 °C. After 15 minutes of stirring, KI (73.218 g, 441.05 mmol) in water (120 ml) was added to the reaction mixture at 0 °C. The reaction mixture was allowed to warm to room temperature

and was then heated to 70 °C for 3 hours. After completion of the reaction (monitored by TLC, 20%EA-Hexane, Rf =0.7), the reaction mixture was partitioned between ethyl acetate and water. The organic layer was washed with brine, dried over $Na_2SO_4$ and concentrated to afford a crude residue, which was purified over column chromatography (using 100-200 mesh silica gel, eluted with 10% EA-Hexane) to afford 74 g (90%) of 5-fluoro-2-iodo-1-methyl-3-nitro-benzene as a yellow solid.

Step 2: To a stirred solution of 5-fluoro-2-iodo-1-methyl-3-nitro-benzene (40 g, 142.334 mmol) in TEA (400 ml) was added 2-methyl-but-3-yn-2-ol (27.844 ml, 284.667 mmol) at room temperature. The reaction mixture was degassed with $N_2$ gas for 15 mins. After that, CuCl (769.79 mg, 5.693 mmol) followed by $Pd(PPh_3)_2Cl_2$ (1.998 g, 2.847 mmol) was added to the reaction mixture at RT. The reaction mixture was stirred for 16 hours at room temperature. After that, the reaction mixture was diluted with DCM (150 ml) and was stirred for 3 hours at reflux temperature. After completion of the reaction (monitored by TLC, 20% EA-Hexane, $R_f$ 0.5), the reaction mixture was partitioned between ethyl acetate and water. The organic layer was washed with brine, dried over $Na_2SO_4$ and was concentrated to afford a crude residue, which was purified via column chromatography (using 100-200 mesh silica gel, eluted with 12% EA-Hexane) to afford 25 g (75%) of 4-(4-fluoro-2-methyl-6-nitro-phenyl)-2-methyl-but-3-yn-2-ol as a reddish liquid.

Step 3: To a stirred solution of 4-(4-fluoro-2-methyl-6-nitro-phenyl)-2-methyl-but-3-yn-2-ol (25 g, 105.383 mmol) in 10% $H_2O$ in MeOH (750 ml) was added $NH_4Cl$ (28.186 g, 526.94 mmol) followed by Zn dust (20.67 g, 316.149 mmol) at room temperature. The reaction mixture was then heated to reflux for 2 hours. After completion of the reaction (monitored by TLC, 20%EA-Hexane, $R_f$ 0.3), the reaction mixture was filtered through a celite bed, which was washed with ethyl acetate. The filtrate was washed with brine, dried over $Na_2SO_4$ and concentrated to afford 23 g of crude 4-(2-amino-4-fluoro-6-methyl-phenyl)-2-methyl-but-3-yn-2-ol as a dark brown solid. This crude material was carried on to the next step without further purification.

Step 4: A mixture of 4-(2-Amino-4-fluoro-6-methyl-phenyl)-2-methyl-but-3-yn-2-ol (23 g, 111.052 mmol) and 6N HCl (230 ml) was heated to 90 °C for 16 hours. After completion of the reaction (monitored by LCMS), the reaction mixture was quenched with sat. $K_2CO_3$ solution and was extracted with ethyl acetate. The combined organic layers were washed with brine, dried over $Na_2SO_4$ and concentrated to afford a crude residue, which was purified via column chromatography (using 100-200 mesh, eluted with 10% EA-Hexane) to afford 15 g (68% over two steps) of 7-fluoro-2,2,5-trimethyl-2,3-dihydro-1H-quinolin-4-one.

Step 5: To a stirred solution of 7-fluoro-2,2,5-trimethyl-2,3-dihydro-1H-quinolin-4-one (2.5 g, 12.070 mmol) in THF (50 ml) was added KOtBu (1M in THF, 24.142 ml, 24.142 mmol) at room temperature. The reaction mixture was stirred for 30 minutes at room temperature. After that, isoamyl nitrite (2.424 ml, 18.105 mmol) was added to the reaction mixture dropwise at room temperature. The reaction mixture was stirred for 16 hours at room temperature. After completion of the reaction (monitored by LCMS), the reaction mixture was quenched with ice water and was extracted with ethyl acetate. The combined organic layers were washed with brine, dried over $Na_2SO_4$ and concentrated to afford 3.3 g of crude 7-fluoro-2,2,5-trimethyl-1,2-dihydro-quinoline-3,4-dione 3-oxime as a reddish brown liquid.

Step 6: To a stirred solution of 7-fluoro-2,2,5-trimethyl-1,2-dihydro-quinoline-3,4-dione 3-oxime (3.3 g, 13.968 mmol) in 10% $H_2O$ in MeOH (100 ml) was added $NH_4Cl$ (3.74 g, 69.84 mmol) followed by Zn dust (2.74 g, 41.905 mmol) at room temperature. The reaction mixture was stirred for 2 hours at room temperature. After completion of the reaction (monitored by TLC, 50%EA-Hexane, $R_f$ 0.3), the reaction mixture was filtered through a celite bed, which was washed with ethyl acetate. The filtrate was washed with brine, dried over $Na_2SO_4$ and concentrated to afford 2.4 g of crude 3-amino-7-fluoro-2,2,5-trimethyl-2,3-dihydro-1H-quinolin-4-one, which was carried on to the next step without further purification.

Step 7: To a stirred solution of 3-amino-7-fluoro-2,2,5-trimethyl-2,3-dihydro-1H-quinolin-4-one (2.4 g, 10.798 mmol) in toluene (24 ml) was added methyl formate (4.66 ml, 75.587 mmol) at room temperature. The reaction mixture was then heated to 80 °C in a sealed tube for 16 hours. After completion of the reaction (monitored by TLC, 40% EA-Hexane, $R_f$ 0.5), the reaction mixture was concentrated to afford a crude residue, which was purified via column chromatography (using 100-200 mesh silica gel, eluted with 15% EA-Hexane) to afford 900 mg of N-(7-fluoro-2,2,5-trimethyl-4-oxo-1,2,3,4-tetrahydro-quinolin-3-yl)-formamide. LCMS of column purified material showed 50% of desired product in the obtained material, which was used as such in the next step.

Step 8: To a stirred solution of N-(7fluoro-2,2,5-trimethyl-4-oxo-1,2,3,4-tetrahydro-quinolin-3-yl)-formamide (800 mg, 3.196 mmol) in $POCl_3$ (1.494 ml, 15.982 mmol) was added TEA (0.449 ml, 3.196 mmol) at room temperature. The reaction mixture was then heated to reflux for 4 hours. After completion of the reaction (monitored by TLC, 40% EA-hexane, $R_f$ 0.7), the reaction mixture was quenched with ice cold sat. $NaHCO_3$ solution, followed by extraction with ethyl acetate. The combined organic layers were washed with brine, dried over $Na_2SO_4$ and concentrated to afford a crude residue, which was purified via column chromatography (using 100-200 mesh silica gel, eluted with 40% EA-Hexane) and later PREP-SFC to afford 300 mg (10.7 % over four steps) of 7-fluoro-4,4,9-trimethyl-4,5-dihydrooxazolo[4,5-c]quinoline.

Step 9: To a stirred solution of 7-fluoro-4,4,9-trimethyl-4,5-dihydro-oxazolo[4,5-c]quinoline (745 mg, 3.207 mmol) in DMF (55 ml) was added NBS (485.16 mg, 2.725 mmol) in DMF (18 ml) portionwise at 0 °C. The reaction was stirred for 1 hour at the same temperature. After completion of the reaction (monitored by TLC, 20% ea-hexane, Rf =0.5), the reaction mixture was quenched with saturated $Na_2S_2O_3$ solution and was extracted with MTBE. The combined

organic layers were washed with sat. $NaHCO_3$ solution followed by brine and were then concentrated under reduced pressure to afford a crude residue, which was purified via flash chromatography (40 g silica column, eluted with 5% EA-Hexane solvent system) to afford 600 mg (60%) of 8-bromo-7-fluoro-4,4,9-trimethyl-4,5-dihydro-oxazolo[4,5-c]quinoline as a light brown solid.

Example 1: 7-fluoro-8-(3-fluoro-5-methylphenyl)-1,4,4,9-tetramethyl-4,5-dihydroimidazo[1,2-a]quinoxaline

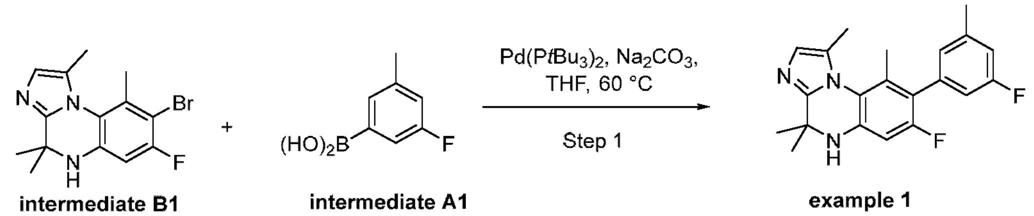


intermediate B1 intermediate A1 example 1

Step 1: 8-Bromo-7-fluoro-1,4,4,9-tetramethyl-4,5-dihydroimidazo[1,2-a]quinoxaline (100 mg, 0.31 mmol, 1.0 eq.), (3-fluoro-5-methylphenyl)boronic acid (142 mg, 0.93 mmol, 3.0 eq.) and $Pd(PtBu_3)_2$ (8 mg, 0.02 mmol, 0.05 eq.) were weighed out into a microwave vial, a stir bar was added, the vial was sealed and purged with nitrogen. Then, THF (2.0 mL) and 2M $Na_2CO_3$ solution (1.0 mL) were added, and the vial was slightly evacuated and backfilled/purged with nitrogen again. The reaction mixture was then heated to 60 °C for 48 hours and was then stirred for 48 hours at ambient temperature. Then, sat. $NaHCO_3$ solution and EtOAc were added, the layers were separated and the aqueous layer was extracted with EtOAc. The combined organic layers were then washed with brine, dried over $MgSO_4$ and the solvent was removed under reduced pressure. The obtained residue was purified via silica gel chromatography (using 4:1 EtOAc/cyclohexane as eluent) and later reverse phase HPLC to obtain 80 mg (73%) of 7-fluoro-8-(3-fluoro-5-methylphenyl)-1,4,4,9-tetramethyl-4,5-dihydroimidazo[1,2-a]quinoxaline. $^1H$ NMR (DMSO-$d_6$) δ: 7.08 - 6.99 (m, 4H), 6.77 (d, 1H), 6.71 (d, 1H), 6.52 (s, 1H), 2.38 (s, 3H), 2.23 (d, 3H), 2.07 (s, 3H), 1.50 - 1.28 (m, 6H).

The following examples were prepared in a similar manner (use of appropriate reagents and purification methods known to the person skilled in the art) as the synthesis described for **example 1:**

| Example Nr. | Structure | Intermediates | Yield % | Data |
|---|---|---|---|---|
| 2 | 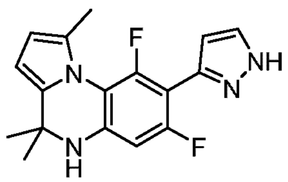 | A2, B2 | 8 | $[M+H]^+$ (m/z): calc. for $C_{17}H_{16}F_2N_4$+H: 315.1, found 315.1 |
| 3 | 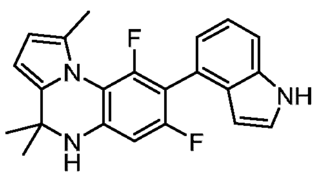 | A3, B2 | 41 | $^1H$ NMR (DMSO-$d_6$) δ: 11.21 (s, 1H), 7.46 (d, 1H), 7.38 (t, 1H), 7.22 - 7.14 (m, 1H), 7.03 (d, 1H), 6.74 - 6.68 (m, 1H), 6.53 (d, 1H), 6.22 (s, 1H), 5.97 (dd, 1H), 5.91 (d, 1H), 2.25 (d, 3H), 1.52 - 1.35 (m, 6H) |
| 4 | 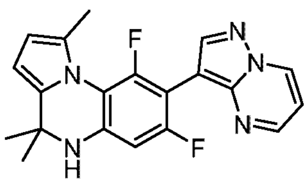 | A4, B2 | 48 | $[M+H]^+$ (m/z): calc. for $C_{20}H_{17}F_2N_5$+H: 366.15 found: 366.1 |

(continued)

| Example Nr. | Structure | Intermediates | Yield % | Data |
|---|---|---|---|---|
| 5 | 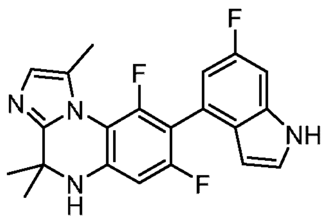 | A5, B3 | 72 | $[M+H]^+$ (m/z): calc. for $C_{21}H_{17}F_3N_4$+H: 383.15 found: 383.0 |
| 7 | 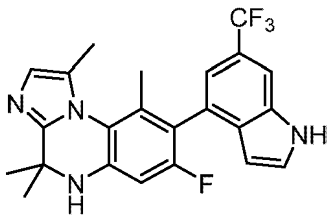 | A7, B1 | 26 | $[M+H]^+$ (m/z): calc. for $C_{23}H_{20}F_4N_4$+H: 429.17 found 429.1 |
| 8 | 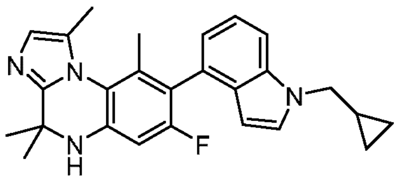 | A8, B1 | 26 | $^1$H NMR (DMSO-$d_6$) δ: 7.59 - 7.52 (m, 1H), 7.47 (d, 1H), 7.23 (dd, 1H), 6.98 (d, 1H), 6.82 - 6.71 (m, 2H), 6.48 (s, 1H), 6.09 (d, 1H), 4.09 (qd, 2H), 2.23 (d, 3H), 1.98 (s, 3H), 1.59 - 1.22 (m, 7H), 0.55 (ddd, 2H), 0.43 (hept, 2H) |
| 9 | 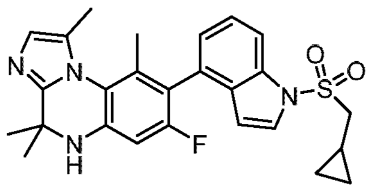 | A9, B1 | 68 | $^1$H NMR (DMSO-$d_6$) δ: 7.90 (d, 1H), 7.60 (d, 1H), 7.46 (dd, 1H), 7.27 (d, 1H), 6.82 - 6.75 (m, 2H), 6.58 (s, 1H), 6.45 (d, 1H), 3.60 (d, 2H),2.23 (d, 3H), 1.97 (s, 3H), 1.58 - 1.28 (m, 6H), 0.83 (tt, 1H), 0.39 - 0.28 (m, 2H), -0.08 (dd, 2H) |
| 10 | 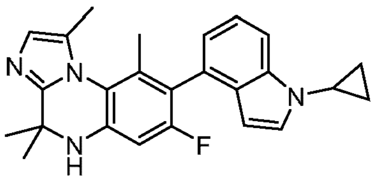 | A13, B1 | 62 | $^1$H NMR (DMSO-$d_6$) δ: 7.60 (d, 1H), 7.34 (d, 1H), 7.27 (dd, 1H), 7.00 (d, 1H), 6.78 (s, 1H), 6.74 (d, 1H), 6.48 (s, 1H), 6.03 (d, 1H), 2.22 (d, 3H), 1.97 (s, 3H), 1.56 - 1.27 (m, 6H), 1.09 (tdd, 2H), 1.04 - 0.95 (m, 2H) |
| 13 | 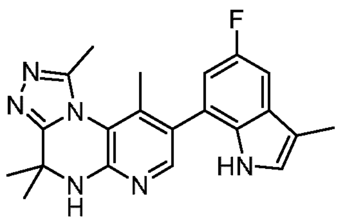 | A14, B5 | 4 | $[M+H]^+$ (m/z): calc. for $C_{21}H_{21}FN_6$+H: 377.19 found 377.3 |
| 21 | 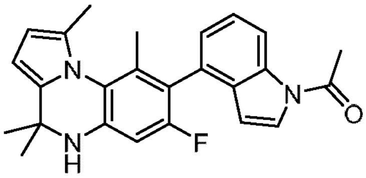 | A19, B2 | 19 | $^1$H NMR (DMSO-$d_6$) δ: 8.44 - 8.38 (m, 1H), 7.91 (dd, 1H), 7.43 (td, 1H), 7.32 (d, 1H), 6.74 (dd, 1H), 6.63 (d, 1H), 6.53 (d, 1H), 6.00 - 5.95 (m, 1H), 5.91 (dd, 1H), 2.68 (d, 3H), 2.25 (d, 3H), 1.46 (s, 3H), 1.38 (s, 3H) |

(continued)

| Example Nr. | Structure | Intermediates | Yield % | Data |
|---|---|---|---|---|
| 24 | 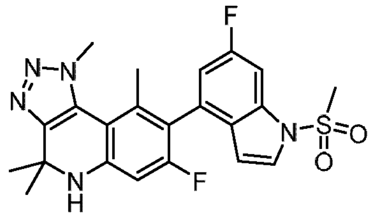 | A14, B8 | 92 | $^1$H NMR (DMSO-$d_6$) δ: 7.71 - 7.65 (m, 1H), 7.61 (dd, 1H), 7.26 (ddd, 1H), 6.75 (s, 1H), 6.67 (d, 1H), 6.58 - 6.42 (m, 1H), 4.10 (d, 3H), 3.56 (d, 3H), 2.22 - 2.12 (m, 3H), 1.54-1.44 (m, 6H) |
| 25 | 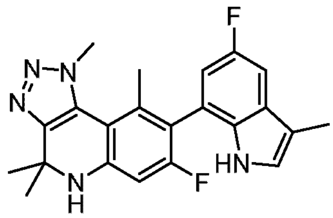 | A13, B8 | 78 | $^1$H NMR (DMSO-$d_6$) δ: 10.61 (d, 1H), 7.26 (dd, 1H), 7.14 (dd, 1H), 6.88 (dd, 1H), 6.73 - 6.59 (m, 2H), 4.13 (s, 3H), 2.26 (d, 3H), 2.13 (s, 3H), 1.55 (s, 3H), 1.45 (s, 3H) |
| 26 | 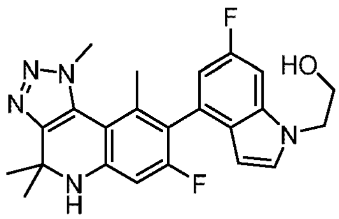 | A21, B8 | 80 | $^1$H NMR (DMSO-$d_6$) δ: 7.42 - 7.33 (m, 2H), 6.88 (dd, 1H), 6.64 (d, 2H), 6.12 (d, 1H), 4.92 (t, 1H), 4.22 (q, 2H), 4.08 (s, 3H), 3.75 (q, 2H), 2.16 (s, 3H), 1.54-1.44 (m, 6H) |
| 27 | 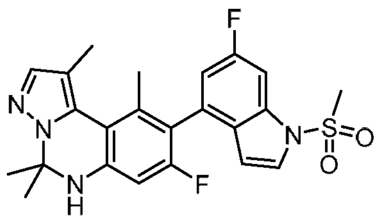 | A14, B9 | 92 | $^1$H NMR (DMSO-$d_6$) δ: 7.67 (dd, 1H), 7.62 (d, 1H), 7.34 (s, 1H), 7.24 (dd, 1H), 7.08 (s, 1H), 6.69 (d, 1H), 6.42 (d, 1H), 3.56 (s, 4H), 2.10-2.05 (m, 6H), 1.62 (m, 6H) |
| 28 | 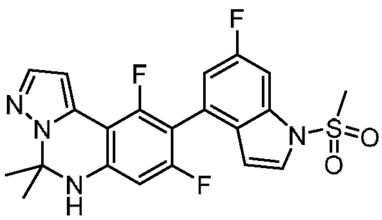 | A14, B10 | 36 | $^1$H NMR (DMSO-$d_6$) δ: 7.71 - 7.67 (m, 2H), 7.64 (d, 1H), 7.59 (d, 1H), 7.29 (dd, 1H), 6.62 (d, 1H), 6.57 (d, 1H), 6.52 (dd, 1H), 3.56 (s, 2H), 1.70 (d, 6H) |
| 32 | 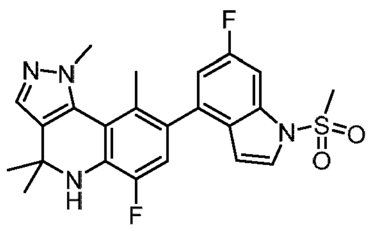 | A14, B14 | 72 | $^1$H NMR (DMSO-$d_6$) δ: 7.67 - 7.60 (m, 2H), 7.44 (s, 1H), 7.25 (dd, 1H), 7.04 (d, 1H), 6.58 (d, 1H), 5.95 (d, 1H), 3.83 (s, 3H), 3.53 (s, 3H), 2.16 (s, 3H), 1.45 (s, 6H) |
| 33 | 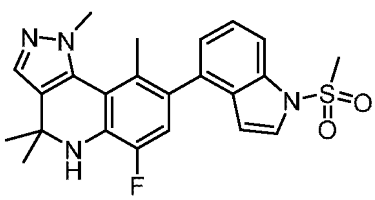 | A22, B14 | 46 | $[M+H]^+$ (m/z): calc. for $C_{23}H_{23}FN_4O_2S+H$: 439.16 found 439.16 |

(continued)

| Example Nr. | Structure | Intermediates | Yield % | Data |
|---|---|---|---|---|
| 34 | 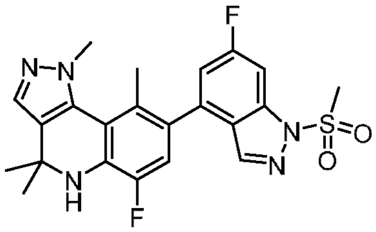 | A23, B14 | 27 | $^1$H NMR (DMSO-$d_6$) δ: 8.47 (d, 1H), 7.72 - 7.66 (m, 1H), 7.48 - 7.42 (m, 2H), 7.14 (d, 1H), 6.06 (d, 1H), 3.88 (s, 3H), 3.56 (s, 3H), 2.21 (s, 3H), 1.46 (s, 6H) |
| 35 | 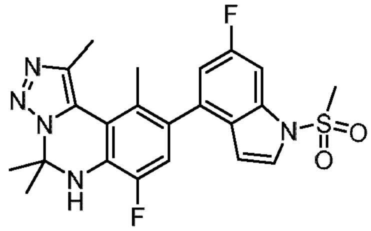 | A14, B15 | 64 | $^1$H NMR (DMSO-$d_6$) δ: 7.69 - 7.62 (m, 2H), 7.26 (dd, 1H), 7.20 (d, 1H), 7.02 (d, 1H), 6.56 (dd, 1H), 3.56 (s, 3H), 2.43 (s, 3H), 2.16 (s, 3H), 1.89 - 1.65 (m, 7H) |
| 36 | 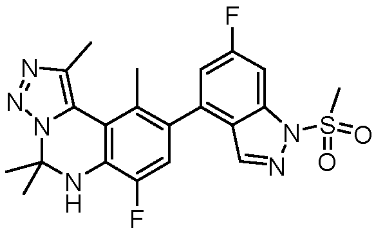 | A23, B15 | 54 | $^1$H NMR (DMSO-$d_6$) δ: 8.45 (d, 1H), 7.74 - 7.69 (m, 1H), 7.44 (dd, 1H), 7.30 (d, 1H), 7.10 (d, 1H), 3.56 (s, 3H), 2.46 (s, 3H), 2.21 (s, 3H), 1.79 (s, 6H) |
| 56 | 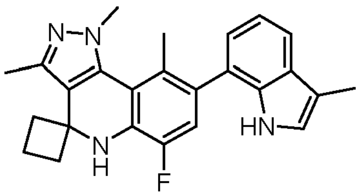 | A11, B24 | 69 | $^1$H NMR (DMSO-$d_6$) δ: 10.49 (d, 1H), 7.48 (dd, 1H), 7.09 - 7.03 (m, 2H), 7.03 - 6.96 (m, 2H), 6.25 (d, 1H), 3.78 (s, 3H), 2.63 - 2.31 (m, 7H), 2.29 (d, 3H), 2.06 (s, 3H), 1.95 - 1.78 (m, 2H) |
| 61 | 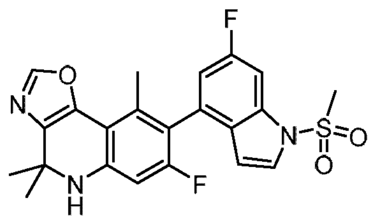 | A14, B27 | 73 | $^1$H NMR (DMSO-$d_6$) δ: 8.38 (d, 1H), 7.64 (ddd, 1H), 7.58 (d, 1H), 7.09 (dd, 1H), 6.77 (s, 1H), 6.44-6.39 (m, 2H), 3.56 (d, 3H), 2.21 (s, 3H), 1.52 (s, 3H), 1.49 (s, 3H) |
| 62 | 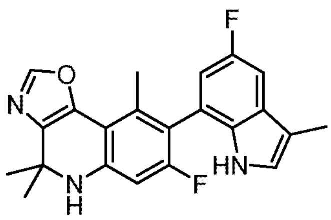 | A13, B27 | 43 | $^1$H NMR (DMSO-$d_6$) δ: 10.45 (d, 1H), 8.37 (s, 1H), 7.25-7.20 (m, 1H), 7.07 (dd, 1H), 6.74 (dd, 1H), 6.70 (s, 1H), 6.40 (d, 1H), 2.25 (d, 3H), 2.17 (s, 3H), 1.52 (s, 3H), 1.50 (s, 3H) |
| 63 | 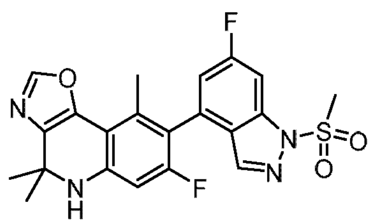 | A23, B27 | 64 | $^1$H NMR (DMSO-$d_6$) δ: 8.40 (s, 1H), 8.29 (d, 1H), 7.69 (ddd, 1H), 7.26 (dd, 1H), 6.87 (s, 1H), 6.44 (d, 1H), 3.58 (s, 3H), 2.25 (s, 3H), 1.53 (s, 3H), 1.50 (s, 3H) |

Example 11: 9-fluoro-1,4,4-trimethyl-8-(3-methyl-1H-indol-7-yl)-4,5-dihydropyrido[3,4-e][1,2,4]triazolo[4,3-a]pyrazine

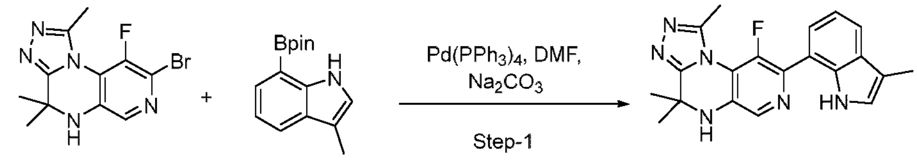


intermediate B11 intermediate A4 example 11

Step 1: To a stirred solution of 8-bromo-9-fluoro-1,4,4-trimethyl-4,5-dihydro-2,3,5,7,9b-pentaaza-cyclopenta[a]naphthalene (100 mg, 0.3203 mmol) and 3-methyl-7-(4,4,5,5-tetramethyl-[1,3,2]dioxaborolan-2-yl)-1H-indole (164.7 mg, 0.6407 mmol, 2 eq) in DMF (10 ml) was added 2M $Na_2CO_3$ solution (3.8 ml). The reaction mixture was degassed with argon for 30 minutes. $Pd(PPh_3)_4$ (52 mg, 0.045 mmol) was added to the reaction mixture and the reaction mixture was heated to 120 °C for 16 h. The reaction mixture was filtered through a celite bed, which was afterwards washed with ethyl acetate. The filtrate was washed with ice cold water followed by brine. The organic layer was dried with anhydrous $Na_2SO_4$, filtered and concentrated. The obtained crude residue was purified by column chromatography (50% Ethyl acetate/Hexane, neutral alumina) to afford 9-fluoro-1,4,4-trimethyl-8-(3-methyl-1H-indol-7-yl)-4,5-dihydro-2,3,5,7,9b-pentaaza-cyclopenta[a]naphthalene (48 mg, 41%) as an off-white solid. $^1H$ NMR (DMSO-$d_6$) δ: 10.82 (s, 1H), 8.30 (s, 1H), 7.58-7.56 (d, 1H), 7.51-7.49 (d, 1H), 7.22 (s, 1H), 7.14-7.10 (m, 2H), 2.64-2.61 (d, 3H), 2.30 (s, 3H), 1.56 (s, 6H).

Example 12: 7,9-difluoro-1,4,4-trimethyl-8-(1H-pyrrolo[2,3-b]pyridin-4-yl)-4,5-dihydropyrrolo[1,2-a]quinoxaline

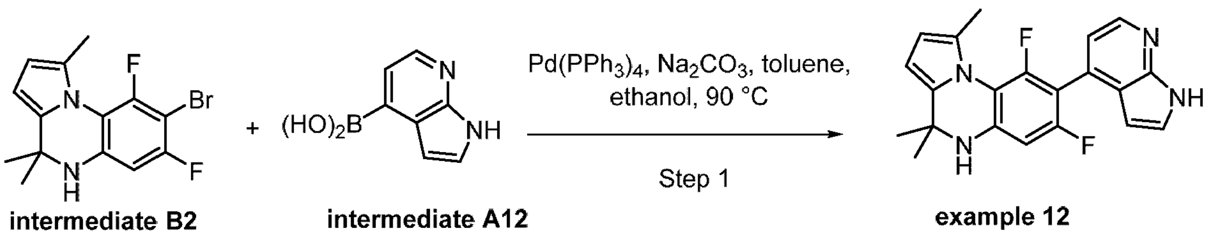


intermediate B2 intermediate A12 example 12

Step 1: 8-Bromo-7,9-difluoro-1,4,4-trimethyl-5H-pyrrolo[1,2-a]quinoxaline (80 mg, 0.24 mmol, 1.0 eq.), 1H-pyrrolo[2,3-b]pyridin-4-ylboronic acid (119 mg, 0.73 mmol, 3.0 eq.) and $Pd(PPh_3)_4$ (14 mg, 0.01 mmol, 0.05 eq.) were weighed out into a microwave vial, a stir bar was added, the vial was sealed and purged with nitrogen. Then, toluene (2.0 mL), 2M $Na_2CO_3$ solution (0.5 mL) and ethanol (0.3 mL) were added, and the vial was purged with nitrogen again. The reaction mixture was then heated to 90 °C for 16 hours. Then, DCM and water were added, and the resulting mixture was filtered through a hydrophobic frit. The organic layer was evaporated and the residue was purified via silica gel chromatography, reverse phase HPLC and finally recrystallization from EtOAc to yield 11 mg (12%) of 7,9-difluoro-1,4,4-trimethyl-8-(1H-pyrrolo[2,3-b]pyridin-4-yl)-4,5-dihydropyrrolo[1,2-a]quinoxaline. $^1H$ NMR (DMSO-$d_6$) δ: 8.29 (d, 1H), 7.52 (t, 1H), 7.12 (d, 1H), 6.83 - 6.65 (m, 2H), 6.31 (d, 1H), 5.95 (dd, 2H), 2.26 (d, 3H), 1.42 (s, 6H)

The following examples were prepared in a similar manner (use of appropriate reagents and purification methods known to the person skilled in the art) as the synthesis described for **example 12:**

| Example Nr. | Structure | Intermediates | Yield % | Data |
|---|---|---|---|---|
| **6** | 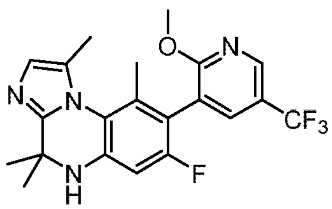 | **A6, B1** | 77 | $^1H$ NMR (DMSO-$d_6$) δ: 8.67 (dd, 1H), 8.15 (d, 1H), 6.79 (d, 1H), 6.71 (d, 1H), 6.62 (s, 1H), 3.96 (s, 3H), 2.22 (d, 3H), 2.03 (s, 3H), 1.52-1.28 (m, 6H) |

(continued)

| Example Nr. | Structure | Intermediates | Yield % | Data |
|---|---|---|---|---|
| 14 | 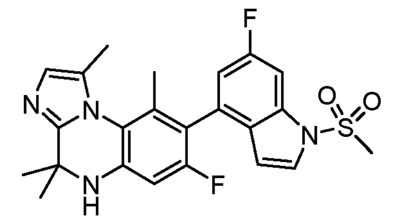 | A14, B1 | 36 | $^1$H NMR (DMSO-$d_6$): δ 7.68-7.63 (m, 2H), 7.25 (d, 1H), 6.76 (d, 1H), 6.64 (s, 1H), 6.44 (s, 1H), 3.57 (s, 3H), 2.19 (s, 3H), 1.99 (s, 3H), 1.44-1.37 (m, 6H). |
| 19 | 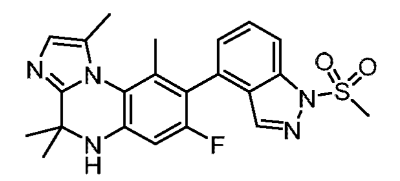 | A18, B1 | 30 | $^1$H NMR (DMSO-$d_6$) δ: 8.33 (s, 1H), 8.03 (d, 1H), 7.75 (dd, 1H), 7.44 (d, 1H), 6.89 - 6.73 (m, 2H), 6.67 (s, 1H), 3.55 (s, 3H), 2.32 - 2.23 (m, 3H), 2.01 (s, 3H), 1.58-1.29 (m, 6H) |
| 20 | 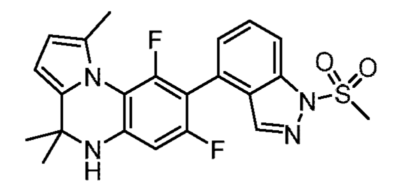 | A18, B2 | 50 | $^1$H NMR (DMSO-$d_6$) δ: 8.47 (s, 1H), 8.05 (d, 1H), 7.80 - 7.70 (m, 1H), 7.50 (d, 1H), 6.82 - 6.74 (m, 2H), 6.04 - 5.96 (m, 1H), 5.92 (d, 1H), 3.55 (s, 3H), 2.27 (d, 3H), 1.43 (s, 6H) |
| 60 | 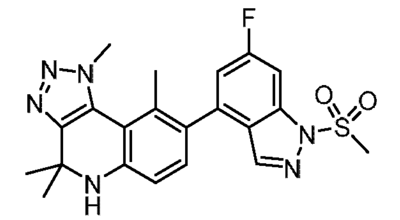 | A23, B26 | 63 | $^1$H NMR (DMSO-$d_6$) δ: 7.62 (dd, 1H), 7.60 (d, 1H), 7.22 (dd, 1H), 7.09 (d, 1H), 6.85 (d, 1H), 6.46 (s, 1H), 4.14 (s, 3H), 3.54 (s, 3H), 2.23 (s, 3H), 1.49 (s, 6H) |

Example 16: 7-fluoro-8-(5-fluoro-3-(tetrahydrofuran-3-yl)-1H-indol-7-yl)-1,4,4,9-tetramethyl-4,5-dihydroimidazo[1,2-a]quinoxaline

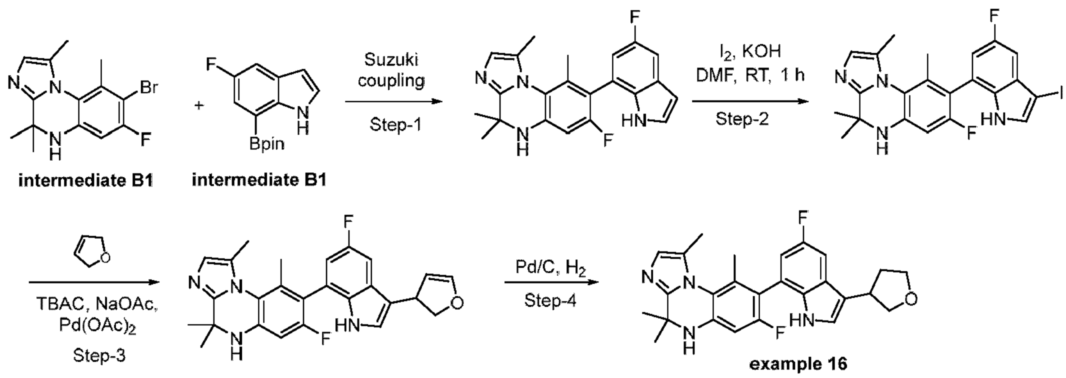


Step 1: 7-fluoro-8-(5-fluoro-1H-indol-7-yl)-1,4,4,9-tetramethyl-4,5-dihydroimidazo[1,2-a]quinoxaline was prepared in a similar manner (use of appropriate reagents and purification methods known to the person skilled in the art) as the synthesis described for example 23.

Step 2: To a stirred solution of 7-fluoro-8-(5-fluoro-1H-indol-7-yl)-1,4,4,9-tetramethyl-4,5-dihydroimidazo[1,2-a]quinoxaline (1.6 g, 4.23 mmol, 1.0 eq) in DMF (40 ml) at 0 °C (40 ml) were added KOH (0.592 g, 10.58 mmol, 2.5 eq) and iodine (1.07 g, 4.23 mmol, 1.0 eq) and the resulting reaction mixture was stirred for 30 min at 0 °C. The reaction mixture was diluted with EtOAc (800 ml), washed with aq. sodium metabisulfite (2 x 300 ml), water (4 x 300 ml) and brine (300 ml), dried

($Na_2SO_4$), filtered and concentrated under reduced pressure to afforded 7-fluoro-8-(5-fluoro-3-iodo-1H-indol-7-yl)-1,4,4,9-tetramethyl-4,5-dihydroimidazo[1,2-a]quinoxaline (1.6 g, 75%). TLC system: 5% MeOH/DCM; $R_f$: 0.3.

Step 3: A solution of 7-fluoro-8-(5-fluoro-3-iodo-1H-indol-7-yl)-1,4,4,9-tetramethyl-4,5-dihydroimidazo[1,2-a] quinoxaline (1.5 g, 2.97 mmol, 1.0 eq.) in DMF (20 ml) was degassed with argon for 20 min followed by the addition of tetrabutylammonium chloride (0.82 g, 2.97 mmol, 1.0 eq.), NaOAc (0.732 g, 8.92 mmol, 3.0 eq.), $Pd(OAc)_2$ (0.066 g, 0.297 mmol, 0.1 eq.) and 2,5-dihydrofuran (2.19 ml, 29.76 mmol, 10 eq.). The reaction mixture was stirred at 50 °C for 48 h. After completion of the reaction (monitored by TLC), the reaction mixture was filtered through a celite bed and the filtrate was concentrated to get the crude product, which was purified by prep-HPLC (10% MeOH/DCM; $R_f$-value-0.3) to afford 8-(3-(2,3-dihydrofuran-3-yl)-5-fluoro-1H-indol-7-yl)-7-fluoro-1,4,4,9-tetramethyl-4,5-dihydroimidazo[1,2-a]quinoxaline (0.35g, 27%) as a white solid.

Step 4: A solution of 8-(3-(2,3-dihydrofuran-3-yl)-5-fluoro-1H-indol-7-yl)-7-fluoro-1,4,4,9-tetramethyl-4,5-dihydroimidazo[1,2-a]quinoxaline (0.25 g, 0.56 mmol, 1.0 eq.) in ethanol (20 ml) was degassed with argon for 10 min followed by the addition of Pd/C (50 mg, 10 wt % loading). The reaction mixture was stirred at RT under hydrogen atmosphere for 3 h. After completion of the reaction (monitored by TLC), the reaction mixture was filtered through a celite bed and the filtrate was concentrated to afford 7-fluoro-8-(5-fluoro-3-(tetrahydrofuran-3-yl)-1H-indol-7-yl)-1,4,4,9-tetramethyl-4,5-dihydroimidazo[1,2-a]quinoxaline (0.23 g, 91%) as a white solid.

$^1H$ NMR (DMSO-$d_6$) δ: δ 10.75 (s, 1H), 7.38 (d, 1H), 7.22 (s, 1H), 6.88 (d, 1H), 6.74 (d, 2H), 6.57 (s, 1H) 4.12 (q, 1H), 3.91-3.96 (m, 1H), 3.82 (q, 1H), 3.55-3.66 (m, 2H), 2.33-2.37 (m ,1H), 2.23 (s, 3H), 2.00-2.07 (m, 1H), 1.94 (s, 3H), 1.46 (s, 3H), 1.37 (s, 3H).

Example 17: 7-fluoro-8-(5-fluoro-3-(prop-1-yn-1-yl)-1H-indol-7-yl)-1,4,4,9-tetramethyl-4,5-dihydroimidazo[1,2-a]quinoxaline

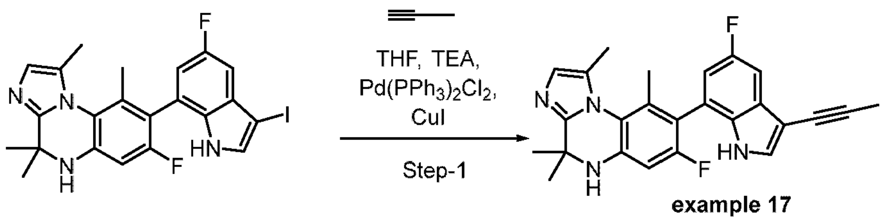


**example 17**

Step 1: A solution of 7-fluoro-8-(5-fluoro-3-iodo-1H-indol-7-yl)-1,4,4,9-tetramethyl-4,5-dihydroimidazo[1,2-a] quinoxaline (0.26 g, 0.515 mmol, 1.0 eq) in THF and TEA (1:1) (10 ml) was deoxygenated with argon gas for 10 min in a sealed tube. $Pd(PPh_3)_2Cl_2$ (0.018g, 0.025 mmol, 0.05 eq) and CuI (0.019g, 0.103 mmol, 0.2 eq) were then added to the reaction mixture, which was again deoxygenated by argon gas for 10 min at -78 °C. In a test tube propyne gas was condensed in TEA (3 ml) at -78 °C. The volume rose to 5ml. The condensed propyne gas was then instantly poured into the reaction mixture at -78 °C. The reaction mixture was then stirred for 2 h at -78 °C and 14 h at room temperature. The reaction mixture was diluted with dichloromethane (50 ml). The organic layer was washed with water (2x20ml) and brine (20ml). The organic layer was dried over anhydrous $Na_2SO_4$, and concentrated under reduced pressure to get the crude material, which was purified by silica gel column chromatography (5% MeOH/DCM; $R_f$-value-0.4) as well as by prep. HPLC to afford compound 7-fluoro-8-(5-fluoro-3-(prop-1-yn-1-yl)-1H-indol-7-yl)-1,4,4,9-tetramethyl-4,5-dihydroimidazo[1,2-a] quinoxaline (0.1 g, 47%) as an off-white solid. $^1H$ NMR (DMSO-$d_6$) δ: 11.27 (s, 1H), 7.57 (d, 1H), 7.26 (dd, 1H), 6.96-7.0 (bd, 1H), 6.74 (d, 2H), 6.59 (s, 1H), 2.23 (s, 3H), 2.08 (d, 3H), 1.94 (s, 3H), 1.44-1.38 (m, 6H).

Example 18: 9-fluoro-8-(6-fluoro-1-(methylsulfonyl)-1H-indol-4-yl)-1,4,4-trimethyl-4,5-dihydropyrido[3,4-e][1,2,4]triazolo[4,3-a]pyrazine

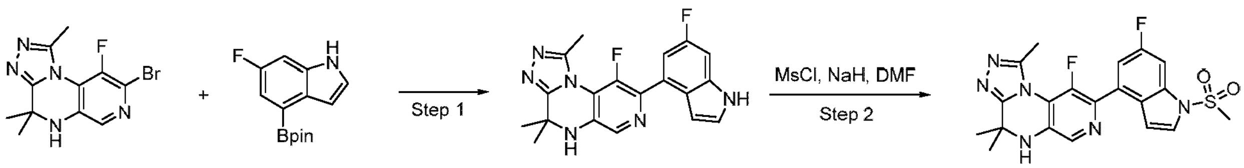


**example 18**

Step 1: 9-Fluoro-8-(6-fluoro-1H-indol-4-yl)-1,4,4-trimethyl-4,5-dihydropyrido[3,4-e][1,2,4]triazolo[4,3-a]pyrazine was prepared in a similar manner (use of appropriate reagents and purification methods known to the person skilled in the art) as the synthesis described for example 1.

Step 2: NaH (60% suspension in mineral oil, 10 mg, 0.25 mmol, 2.0 eq.) was suspended in DMF (2.9 mL) and the mixture was cooled to 0 °C, followed by the addition of 9-fluoro-8-(6-fluoro-1H-indol-4-yl)-1,4,4-trimethyl-4,5-dihydropyrido[3,4-e][1,2,4]triazolo[4,3-a]pyrazine (45 mg, 0.13 mmol, 1.0 eq.). The resulting mixture was stirred for 15 minutes, followed by the addition of methanesulfonyl chloride (14 mg, 0.12 mmol, 1.0 eq.). The resulting mixture was stirred for 70 minutes at 0 °C. Sat. $NaHCO_3$ solution and EtOAc were then added, the layers were separated. The organic layer was washed with water and brine, was dried over $MgSO_4$ and the solvent was removed under reduced pressure. The resulting residue was purified via silica gel chromatography and later reverse phase HPLC to give 6.3 mg (12%) of 9-fluoro-8-(6-fluoro-1-(methylsulfonyl)-1H-indol-4-yl)-1,4,4-trimethyl-4,5-dihydropyrido[3,4-e][1,2,4]triazolo[4,3-a]pyrazine.

$[M+H]^+$ (m/z): calc. for $C_{20}H_{18}F_2N_6O_2S$+H: 444.12, found 445.2.

Example 22: 8-(3-cyclopropyl-1H-indol-7-yl)-7,9-difluoro-4,4-dimethyl-4,5-dihydrotetrazolo[1,5-a]quinoxaline

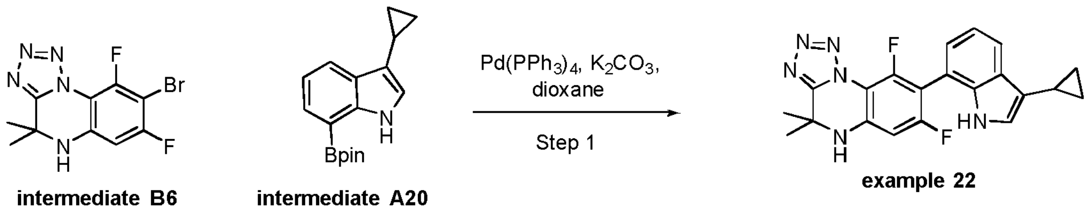


**intermediate B6** **intermediate A20** **example 22**

Step 1: To a solution of 8-bromo-7,9-difluoro-4,4-dimethyl-4,5-dihydrotetrazolo[1,5-a]quinoxaline (0.250 g, 0.793 mmol, 1.0 eq) in dioxane (100 ml) were added $K_2CO_3$ (2M aqueous solution; 0.328 g, 2.38 mmol, 3.0 eq) and 3-cyclopropyl-7-(4,4,5,5-tetramethyl-1,3,2-dioxaborolan-2-yl)-1H-indole (0.254 g, 0.897 mmol, 1.1 eq). The solution was degassed with argon for 10 min followed by addition of $Pd(PPh_3)_4$ (0.045 g, 0.0396 mmol, 0.05 eq). The reaction mixture was then heated for 15 h to 100 °C. After completion of the reaction (monitored by LCMS), the reaction mixture was filtered through a celite pad. The filtrate was concentrated under reduced pressure to get the crude product, which was purified by preparative HPLC to afford 8-(3-cyclopropyl-1H-indol-7-yl)-7,9-difluoro-4,4-dimethyl-4,5-dihydrotetrazolo[1,5-a]quinoxaline (0.070 g, 23%) as a white solid.

$^1H$ NMR (DMSO-$d_6$) δ: 10.66 (s, 1H), 7.68 (d, 1H), 7.57 (s, 1H), 7.12-7.04 (m, 3H), 6.72 (d, 1H), 1.96 (m, 1H), 1.68 (s, 6H), 0.86 (d, 2H), 0.63 (s, 2H).

The following examples were prepared in a similar manner (use of appropriate reagents and purification methods known to the person skilled in the art) as the synthesis described for **example 22:**

| Example Nr. | Structure | InterMediates | Yield % | Data |
|---|---|---|---|---|
| 31 | 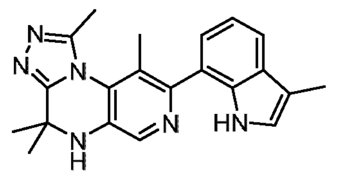 | A11, B13 | 43 | $^1H$ NMR (DMSO-$d_6$) δ: 10.6 (s, 1H), 7.91 (s, 1H), 7.54-7.52 (d, 1H), 7.24 (s, 1H), 7.10-7.06 (m, 2H), 6.99-6.98 (d, 1H), 2.53 (s, 3H), 2.29 (s, 3H), 2.03 (s, 3H), 1.52 (bs, 6H). |

Example 23: 7-fluoro-8-(6-fluoro-1-(methylsulfonyl)-1H-indol-4-yl)-4,4-dimethyl-9-(trifluoromethyl)-4,5-dihydrotetrazolo[1,5-a]quinoxaline

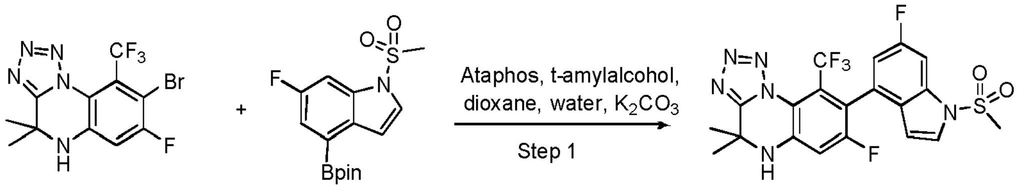


**intermediate B7** **intermediate A14** **example 23**

Step 1: To a solution of 8-bromo-7-fluoro-4,4-dimethyl-9-(trifluoromethyl)-4,5-dihydrotetrazolo[1,5-a]quinoxaline (0.3 g, 0.822 mmol, 1.0 eq) and 6-fluoro-1-(methylsulfonyl)-4-(4,4,5,5-tetramethyl-1,3,2-dioxaborolan-2-yl)-1H-indole (0.334 g, 0.986 mmol, 1.2 eq) in a mixture of [*t*-amyl alcohol (5 ml) /1,4-dioxane (5 ml) / water (0.5 ml)] was added $K_2CO_3$ (0.340 g, 2.466 mmol, 3.0 eq). The solution was then degassed ($N_2$) for 10 minutes followed by the addition of Ataphos catalyst (bis(di-*tert*-butyl(4-dimethylaminophenyl)phosphine)dichloropalladium(II), 0.027 g, 0.0376 mmol, 0.05 eq). The reaction mixture was then heated at 100 °C for 16 h. After completion of the reaction (monitored by LCMS), the reaction mixture was filtered through a celite pad. The filtrate was concentrated under reduced pressure to get the crude product which was purified by preparative HPLC to afford 7-fluoro-8-(6-fluoro-1-(methylsulfonyl)-1H-indol-4-yl)-4,4-dimethyl-9-(trifluoromethyl)-4,5-dihydrotetrazolo[1,5-a]quinoxaline (0.035 g, 9%) as a white solid.

$^1$H NMR (DMSO-$d_6$) δ: 7.91 (s, 1H), 7.72 (d, 1H), 7.64 (d, 1H), 7.24 (dd, 1H), 7.19 (d, 1H), 6.60 (d, 1H), 3.60 (s, 3H), 1.71 (s, 3H), 1.64 (s, 3H).

The following examples were prepared in a similar manner (use of appropriate reagents and purification methods known to the person skilled in the art) as the synthesis described for **example 23:**

| Example Nr. | Structure | InterMediates | Yield % | Data |
|---|---|---|---|---|
| **15** | 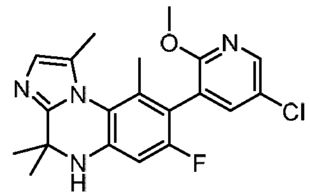 | **A15, B1** | 42 | $^1$H NMR (DMSO-$d_6$) δ: 8.30 (s, 1H), 7.96 (s, 1H), 6.82 (s, 1H), 6.72 (d, 1H), 3.85 (s, 3H), 2.42 (s, 3H), 2.03 (s, 3H), 1.44-1.46 (m, 6H). |
| **29** | 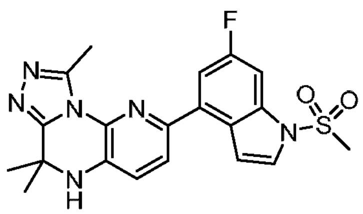 | **A14, B11** | 74 | $^1$H NMR (DMSO-$d_6$) δ: 7.73-7.71 (m, 1H), 7.69-7.68 (m, 1H), 7.65-7.63 (m, 1H), 7.56-7.53 (m, 1H), 7.40-7.38 (m, 1H), 7.28-7.27 (m, 1H), 7.04 (s, 1H), 3.53 (s, 3H), 2.81 (s, 3H), 1.58 (s, 6H). |
| **30** | 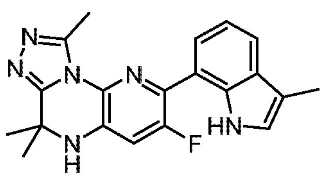 | **A11, B12** | 26 | $^1$H NMR (DMSO-$d_6$) δ: 10.65 (s, 1H), 7.55-7.53 (m, 1H), 7.36-7.33 (m, 1H), 7.24-7.21 (m, 1H), 7.15-7.14 (m, 2H), 7.10-7.06 (m, 1H), 2.67 (s, 3H), 2.29 (s, 3H), 1.59 (s, 6H). |

Example 37: 6'-fluoro-8'-(6-fluoro-1-(methylsulfonyl)-1H-indol-4-yl)-1',9'-dimethyl-1',5'-dihydrospiro[cyclobutane-1,4'-pyrazolo[4,3-c]quinoline]

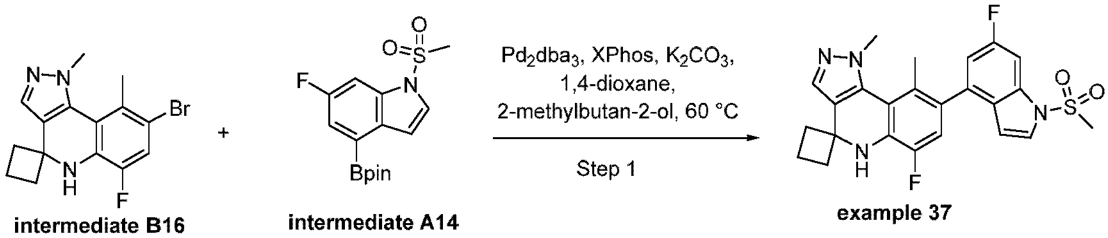


**intermediate B16** **intermediate A14** **example 37**

Step 1: 8-bromo-6-fluoro-1,9-dimethyl-spiro[5H-pyrazolo[4,3-c]quinoline-4,1'-cyclobutane] (50 mg, 0.15 mmol, 1.0 eq.), 6-fluoro-1-methylsulfonyl-4-(4,4,5,5-tetramethyl-1,3,2-dioxaborolan-2-yl)indole (76 mg, 0.22 mmol, 1.5 eq.), $Pd_2dba_3$ (14 mg, 0.015 mmol, 0.1 eq.) and 2-dicyclohexylphosphino-2',4',6'-triisopropylbiphenyl (Xphos, 14 mg, 0.030 mmol, 0.2 eq.) were weighed out into a microwave vial under nitrogen. A stirr bar was added, the vial was sealed. Then, 1,4-dioxane (1.1 mL), 2-methylbutan-2-ol (1.1 mL) and 2M $K_2CO_3$ solution (0.3 mL) were added. Nitrogen gas was bubbled through the reaction mixture for two minutes. The reaction mixture was then heated to 60 °C for 21 hours. Then, DCM and water were added, and the resulting mixture was filtered through a hydrophobic frit. The organic layer was evaporated and the residue was purified via silica gel chromatography to yield 62 mg (89%) of 6'-fluoro-8'-(6-fluoro-1-(methylsulfonyl)-1H-indol-4-yl)-1',9'-dimethyl-1',5'-dihydrospiro[cyclobutane-1,4'-pyrazolo[4,3-c]quinoline].

$^1H$ NMR (DMSO-$d_6$) δ: 7.66 (s, 1H), 7.64 (dd, 1H), 7.62 (d, 1H), 7.27 (dd, 1H), 7.05 (d, 1H), 6.65 - 6.58 (m, 1H), 6.49 (d, 1H), 3.86 (s, 3H), 3.55 (s, 3H), 2.42 (d, 2H), 2.26 (s, 2H), 2.16 (s, 3H), 1.92 - 1.84 (m, 1H), 1.78 (dd, 1H).

The following examples were prepared in a similar manner (use of appropriate reagents and purification methods known to the person skilled in the art) as the synthesis described for **example 37**:

| Example Nr. | Structure | InterMediates | Yield % | Data |
|---|---|---|---|---|
| **38** | 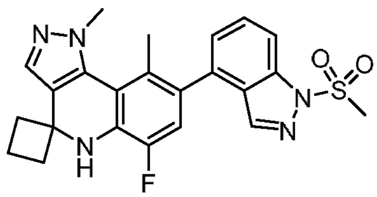 | **A18, B16** | 48 | $^1H$ NMR (DMSO-$d_6$) δ: 8.48 (d, 1H), 8.02 - 7.93 (m, 1H), 7.74 - 7.68 (m, 1H), 7.67 (s, 1H), 7.45 (dd, 1H), 7.10 (d, 1H), 6.51 (d, 1H), 3.91 - 3.87 (m, 3H), 3.57 - 3.50 (m, 3H), 2.50 - 2.41 (m, 2H), 2.27 (d, 2H), 2.17 (s, 3H), 1.95 - 1.84 (m, 1H), 1.82 - 1.75 (m, 1H) |
| **39** | 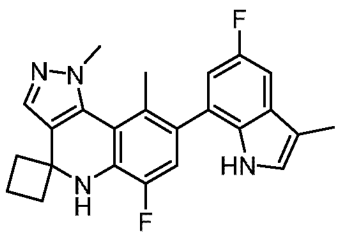 | **A13, B16** | 48 | $^1H$ NMR (DMSO-$d_6$) δ: 10.62 (d, 1H), 7.65 (s, 1H), 7.23 (dd, 1H), 7.16 - 7.12 (m, 1H), 7.04 (d, 1H), 6.89 (dd, 1H), 6.40 (d, 1H), 3.90 (s, 3H), 2.63 - 2.16 (m, 7H), 2.11 (s, 3H), 1.93 - 1.85 (m, 1H), 1.82 - 1.74 (m, 1H) |
| **40** | 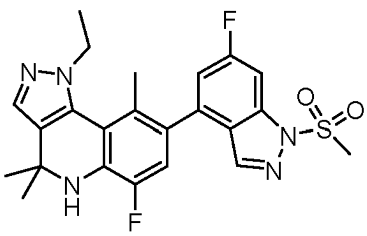 | **A23, B17** | 66 | $^1H$ NMR (DMSO-$d_6$) δ: 8.42 (d, 1H), 7.72 - 7.66 (m, 1H), 7.51 (s, 1H), 7.47 (dd, 1H), 7.13 (d, 1H), 6.03 (d, 1H), 4.13 - 4.06 (m, 2H), 3.57 (s, 3H), 2.22 (s, 3H), 1.45 (s, 6H), 1.41 (t, 3H) |

(continued)

| Example Nr. | Structure | InterMediates | Yield % | Data |
|---|---|---|---|---|
| **41** | 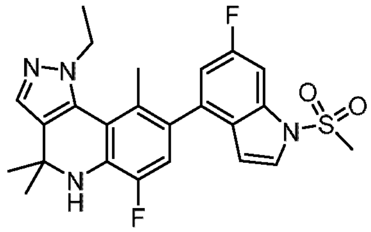 | **A14, B17** | 33 | $^1$H NMR (DMSO-$d_6$) δ: 7.67-7.61 (m,2H), 7.50 (d, 1H), 7.28 (dd, 1H), 7.04 (d, 1H), 6.54 (d, 1H), 5.94 (d, 1H), 4.10 - 4.03 (m, 2H), 3.55 (s, 3H), 2.17 (s, 3H), 1.45 (s, 6H), 1.39 (t, 3H) |
| **42** | 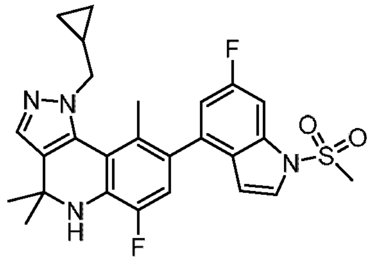 | **A14, B18** | 92 | $^1$H NMR (DMSO-$d_6$) δ: 7.87 (d, 1H), 7.65 (d, 1H), 7.51 -7.44 (m, 2H), 7.29 (d, 1H), 6.99 (d, 1H), 6.45 (d, 1H), 5.89 (d, 1H), 3.98 (s, 2H), 3.50 (s, 3H), 1.45 (s, 7H), 0.43 (dd, 2H), 0.22 (t, 2H) |
| **43** | 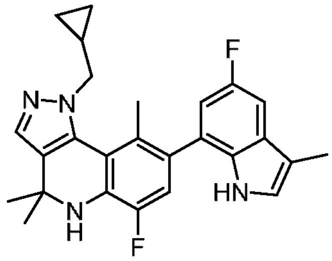 | **A13, B18** | 91 | $^1$H NMR (DMSO-$d_6$) δ: 7.87 (d, 1H), 7.65 (d, 1H), 7.51 -7.44 (m, 2H), 7.29 (d, 1H), 6.99 (d, 1H), 6.45 (d, 1H), 5.89 (d, 1H), 3.98 (s, 2H), 3.50 (s, 3H), 1.45 (s, 7H), 0.43 (dd, 2H), 0.22 (t, 2H) |
| **44** | 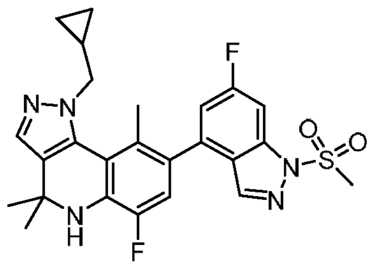 | **A23, B18** | quant. | $^1$H NMR (DMSO-$d_6$) δ: 8.31 (d, 1H), 7.69 (ddd, 1H), 7.50 (s, 1H), 7.45 (dd, 1H), 7.12 (d, 1H), 6.03 (d, 1H), 3.99 (d, 2H), 3.56 (s, 3H), 2.23 (s, 3H), 1.46 (s, 7H), 1.16 (s, 1H), 0.47 - 0.41 (m, 2H), 0.30 - 0.24 (m, 2H) |
| **45** | 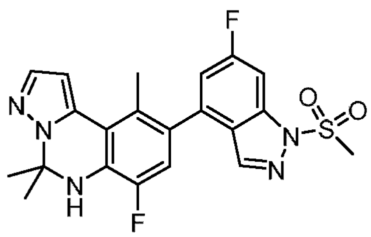 | **A23, B19** | 90 | $^1$H NMR (DMSO-$d_6$) δ: 8.33 (d, 1H), 7.73 - 7.66 (m, 1H), 7.61 (d, 1H), 7.29 (dd, 1H), 7.15 (d, 1H), 6.93 (d, 1H), 6.71 (d, 1H), 3.55 (s, 3H), 2.27 (s, 3H), 1.71 (s, 6H |
| **46** | 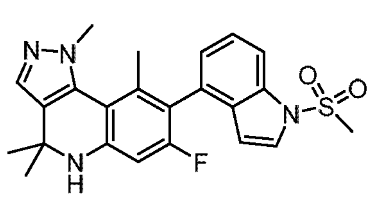 | **A22, B20** | quant. | $^1$H NMR (DMSO-$d_6$) δ: 7.90-7.86 (m, 1H), 7.59 (d, 1H), 7.47 (dd, 1H), 7.40 (s, 1H), 7.28 (d, 1H), 6.63 (d, 1H), 6.48 (d, 1H), 6.37 (s, 1H), 3.77 (s, 3H), 3.49 (s, 3H), 2.10 (s, 3H), 1.43 (s, 3H), 1.38 (s, 3H) |

(continued)

| Example Nr. | Structure | InterMediates | Yield % | Data |
|---|---|---|---|---|
| **47** | 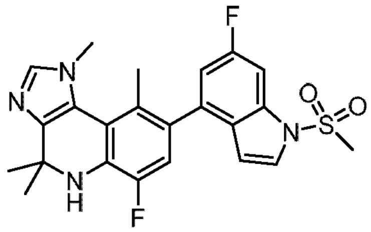 | **A14, B21** | 65 | $^1$H NMR (DMSO-$d_6$) δ: 7.69 (s, 1H), 7.65 - 7.59 (m, 2H), 7.23 (dd, 1H), 6.94 (d, 1H), 6.60 (dd, 1H), 5.90 (d, 1H), 3.72 (s, 3H), 3.54 (s, 3H), 2.15 (s, 3H), 1.43 (s, 6H) |
| **48** | 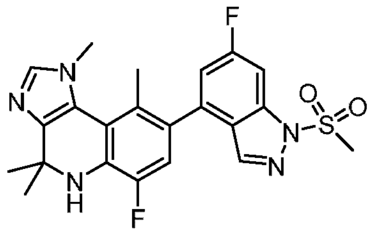 | **A23, B21** | 57 | $^1$H NMR (DMSO-$d_6$) δ: 8.46 (d, 1H), 7.70 (d, 1H), 7.67 (ddd, 1H), 7.40 (dd, 1H), 7.03 (d, 1H), 6.00 (d, 1H), 3.77 (s, 3H), 3.55 (s, 3H), 2.20 (s, 3H), 1.44 (s, 6H) |
| **49** | 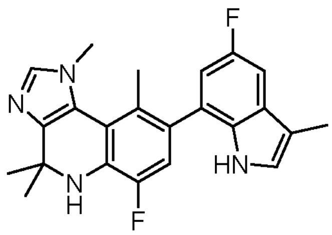 | **A13, B21** | 35 | $^1$H NMR (DMSO-$d_6$) δ: 10.55 (d, 1H), 7.67 (s, 1H), 7.22 (ddd, 1H), 7.13 (dd, 1H), 6.93 (d, 1H), 6.86 (dd, 1H), 5.81 (d, 1H), 3.77 (s, 3H), 2.26 (d, 3H), 2.11 (s, 3H), 1.44 (s, 6H) |
| **50** | 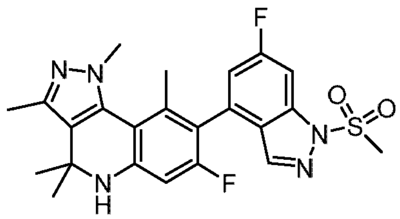 | **A23, B22** | 72 | $^1$H NMR (DMSO-$d_6$) δ: 8.35 (d, 1H), 7.71 (ddd, 1H), 7.45 (dd, 1H), 6.64 (d, 1H), 6.43 (s, 1H), 3.70 (s, 3H), 3.57 (s, 3H), 2.23 (s, 3H), 2.14 (s, 3H), 1.43 (d, 6H) |
| **51** | 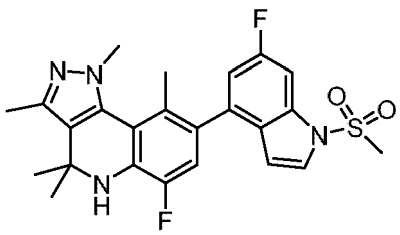 | **A14, B28** | 91 | $^1$H NMR (DMSO-$d_6$) δ: 7.66-7.61 (m,2H), 7.25 (dd, 1H), 7.03 (d, 1H), 6.57 (dd, 1H), 5.81 (d, 1H), 3.72 (s, 3H), 3.54 (s, 3H), 2.25 (s, 3H), 2.13 (s, 3H), 1.48 (s, 6H) |
| **52** | 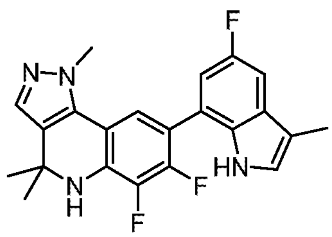 | **A13, B23** | 96 | $^1$H NMR (DMSO-$d_6$) δ: 10.75 (d, 1H), 7.46 (dd, 1H), 7.37 (s, 1H), 7.27 (dd, 1H), 7.17 (dd, 1H), 7.02 (dd, 1H), 6.47 (d, 1H), 4.07 (s, 3H), 2.26 (d, 3H), 1.52 (s, 6H) |

(continued)

| Example Nr. | Structure | InterMediates | Yield % | Data |
|---|---|---|---|---|
| 53 | 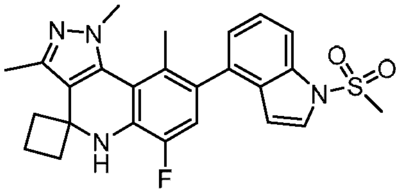 | A22, B24 | 62 | $^1$H NMR (DMSO-$d_6$) δ: 7.87 (dt, 1H), 7.61 (d, 1H), 7.46 (dd, 1H), 7.30 (dd, 1H), 7.01 (d, 1H), 6.59 (dd, 1H), 6.34 (d, 1H), 3.72 (s, 3H), 3.49 (s, 3H), 2.59 - 2.30 (m, 7H), 2.10 (s, 3H), 1.95 - 1.76 (m, 2H) |
| 54 | 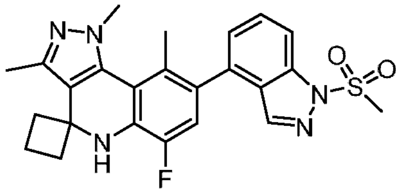 | A18, B24 | 62 | $^1$H NMR (DMSO-$d_6$) δ: 8.46 (d, 1H), 7.99 (d, 1H), 7.70 (dd, 1H), 7.44 (d, 1H), 7.10 (d, 1H), 6.43 (d, 1H), 3.76 (s, 3H), 3.52 (s, 3H), 2.44 (s, 7H), 2.13 (s, 3H), 1.93 - 1.77 (m, 2H) |
| 55 | 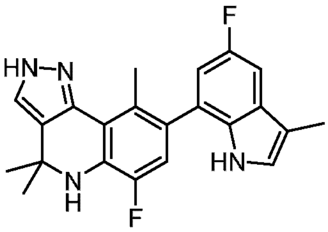 | A13, B25 | 42 | $^1$H NMR (DMSO-$d_6$) δ: 10.37 (s, 1H), 7.68 (s, 1H), 7.22 - 7.17 (m, 1H), 7.10 - 7.06 (m, 1H), 6.85 (d, 1H), 6.78 - 6.73 (m, 1H), 5.64 (s, 1H), 2.41 - 2.37 (m, 3H), 2.27 -2.22 (m, 3H), 1.51 (s, 6H) |
| 58 | 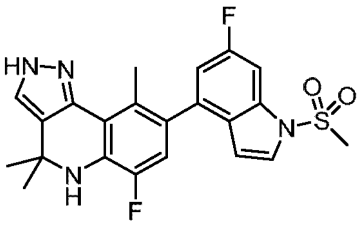 | A14, B25 | 14 | $^1$H NMR (DMSO-$d_6$) δ: 7.69 (s, 1H), 7.65 - 7.56 (m, 2H), 7.11 - 7.07 (m, 1H), 6.87 (d, 1H), 6.43 (d, 1H), 5.73 (d, 1H), 3.54 (s, 3H), 2.42 (s, 3H), 1.53 (s, 3H), 1.49 (s, 3H) |
| 59 | 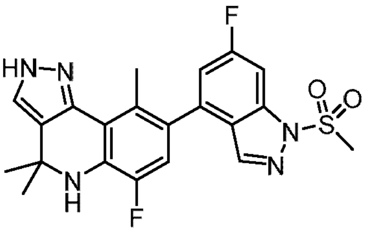 | A23, B25 | 23 | $^1$H NMR (DMSO-$d_6$) δ: 7.62 (dd, 1H), 7.60 (d, 1H), 7.22 (dd, 1H), 7.09 (d, 1H), 6.85 (d, 1H), 6.46 (s, 1H), 4.14 (s, 3H), 3.54 (s, 3H), 2.23 (s, 3H), 1.49 (s, 6H) |

Example 57: 6-fluoro-8-(6-fluoro-1-(methylsulfonyl)-1H-indol-4-yl)-4,4,9-trimethyl-2-(methylsulfonyl)-4,5-dihydro-2H-pyrazolo[4,3-c]quinoline

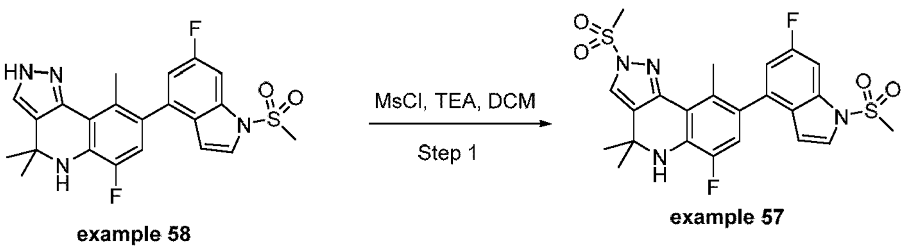


example 58 example 57

Step 1: 6-fluoro-8-(6-fluoro-1-methylsulfonyl-indol-4-yl)-4,4,9-trimethyl-1,5-dihydropyrazolo[4,3-c]quinoline (40

mg, 0.09 mmol, 1.0 eq.) was dissolved in DCM (2.0 mL) followed by the addition of triethylamine (0.063 mL, 0.45 mmol, 5.0 eq.), and the reaction mixture was stirred for 5 minutes at ambient temperature. Then, methansulfonyl chloride (12 mg, 0.10 mmol, 1.1 eq.) was added and the reaction mixture was stirred for one hour, followed by the addition of another aliquot of methansulfonyl chloride (12 mg, 0.10 mmol, 1.1 eq.). The reaction mixture was then stirred at ambient temperature for 16 hours. DCM and water were added, and the resulting mixture was filtered through a hydrophobic frit. The organic part was evaporated and purified via silica gel column chromatography to yield 27 mg (57%) of 6-fluoro-8-(6-fluoro-1-(methylsulfonyl)-1H-indol-4-yl)-4,4,9-trimethyl-2-(methylsulfonyl)-4,5-dihydro-2H-pyrazolo [4,3-c] quinoline.

$^{1}$H NMR (DMSO-$d_6$) δ: 8.32 - 8.26 (m, 1H), 7.63 (dd, 1H), 7.60 (d, 1H), 7.12 (dd, 1H), 7.02 (d, 1H), 6.45 (d, 1H), 6.15 - 6.12 (m, 1H), 3.57 - 3.52 (m, 6H), 2.42 (s, 4H), 1.58 (s, 3H), 1.54 (s, 3H)

## Biological Assays

### Agonistic mode of action on the glucocorticoid receptor

The reporter cell line CHO-Gal4/GR consisted of a chinese hamster ovary (CHO) cell line (*Leibniz Institute DSMZ* - German Collection of Microorganisms and Cell Cultures GmbH: ACC-110) containing a firefly luciferase gene under the control of the GR ligand binding domain fused to the DNA binding domain (DBD) of GAL4 (GAL4 DBD-GR) stably integrated into CHO cells. This cell line was established by stable transfection of CHO cells with a *GAL4*-UAS-Luciferase reporter construct. In a subsequent step the ligand binding domain of the GR cloned into pIRES2-EGFP-GAL4 containing the DNA binding domain of GAL4 from pFA-AT2 was transfected. This fusion construct activated firefly luciferase expression under the control of a multimerized GAL4 upstream activation sequence (UAS). The signal of the emitted luminescence was recorded by the FLIPR$^{TETRA}$. This allowed for specific detection of ligand-induced activation of the GR and therefore for the identification of compounds with agonistic properties. The GAL4/UAS reporter was premixed with a vector that constitutively expressed Renilla luciferase, which served as an internal positive control for transfection efficiency.

The complete culture medium for the assay was:

- DMEM F-12 (1:1) MIXTURE (LONZA cat. N°: BE04-687F/U1) 500mL
- 5 mL of 100 mM Sodium Pyruvate (LONZA cat. N°: BE12-115E)
- 25 mL of 7.5% Sodium Bicarbonate (LONZA cat. N° BE17-613E)
- 6.5 mL of 1 M Hepes (LONZA cat. N°: BE17-737E)
- 5 mL of 100X Penicillin/Streptomycin (LONZA cat. N° DE17-602E)
- 50 mL of Fetal Bovine Serum (Euroclone cat. N° ECS 0180L)
- 0.25 mL of 10mg/mL Puromycin (InvivoGen cat. : ant-pr-1 )
- 0.5 mL of 100 mg/mL Zeocin (InvivoGen cat. : ant-zn-1)

Cryo-preserved CHO-Gal4/GR cells were suspended in complete medium and 5000 cells/25μl/well were seeded into the wells of 384-well polystyrene assay plates (Thermo Scientific, cat.# 4332) and cultured at 37°C, 5% $CO_2$ and 95% humidity. After 24 hours growth medium was carefully removed and replaced by 30μl Opti-MEM (GIBCO, cat.# 31985062) as assay buffer. To test the compounds an 8-point half-log compound dilution curve was generated in 100% DMSO starting from a 2mM stock and compounds were then diluted 1:50 in Opti-MEM. 10μl of compounds were then added to the wells containing 30μl Opti-MEM resulting in a final assay concentration range from 10 μM to 0.003 μM in 0.5% DMSO. Compounds were tested at 8 concentrations in quadruplicate data points. Cells were incubated for 6 hour with compounds and beclometasone (Sigma, cat.# Y0000351) as control compound at 37°C, 5% $CO_2$ and 95% humidity in a total volume of 40 μl. Finally, cells were lysed with 20μl of Triton/Luciferin solution and the signal of the emitted luminescence was recorded at the FLIPR$^{TETRA}$ for 2 minutes.

The relative efficacy of a compound (% effect) was calculated based on the full effect of the agonist beclometasone:

$$\%\ \text{effect} = ((\text{compound} - \text{min})/(\text{max} - \text{min}))\ \text{x}\ 100$$

[min=Opti-MEM only, max=beclometasone]

To calculate $EC_{50}$, max, min and slope factor for each compound a concentration response curve was fitted by plotting %effect versus compound concentration using a 4 parameter logistic equation:

$$y = A + (B\text{-}A)/(1+((10C)/x)D)$$

[A=min y, B=max y, C=log$EC_{50}$, D=slope]

**Antagonistic mode of action on the glucocorticoid receptor**

The reporter cell line CHO-Gal4/GR consisted of a chinese hamster ovary (CHO) cell line (*Leibniz Institute DSMZ* - German Collection of Microorganisms and Cell Cultures GmbH: ACC-110) containing a firefly luciferase gene under the control of the GR ligand binding domain fused to the DNA binding domain (DBD) of GAL4 (GAL4 DBD-GR) stably integrated into CHO cells. This cell line was established by stable transfection of CHO cells with a *GAL4*-UAS-Luciferase reporter construct. In a subsequent step the ligand binding domain of the GR cloned into pIRES2-EGFP-GAL4 containing the DNA binding domain of GAL4 from pFA-AT2 was transfected. This fusion construct activated firefly luciferase expression under the control of a multimerized GAL4 upstream activation sequence (UAS). The signal of the emitted luminescence was recorded by the $FLIPR^{TETRA}$. This allowed for specific detection of antagonistic properties of compounds by measuring the ligand-induced inhibition of beclometasone-activated GR. The GAL4/UAS reporter was premixed with a vector that constitutively expressed Renilla luciferase, which served as an internal positive control for transfection efficiency.

The complete culture medium for the assay was:

- DMEM F-12 (1:1) MIXTURE (LONZA cat. N°: BE04-687F/U1) 500mL
- 5 mL of 100 mM Sodium Pyruvate (LONZA cat. N°: BE12-115E)
- 25 mL of 7.5% Sodium Bicarbonate (LONZA cat. N° BE17-613E)
- 6.5 mL of 1 M Hepes (LONZA cat. N°: BE17-737E)
- 5 mL of 100X Penicillin/Streptomycin (LONZA cat. N° DE17-602E)
- 50 mL of Fetal Bovine Serum (Euroclone cat. N° ECS 0180L)
- 0.25 mL of 10mg/mL Puromycin (InvivoGen cat. : ant-pr-1 )
- 0.5 mL of 100 mg/mL Zeocin (InvivoGen cat. : ant-zn-1)

Cryo-preserved CHO-Gal4/GR cells were suspended in complete medium and 5000 cells/25μl/well were seeded into the wells of 384-well polystyrene assay plates (Thermo Scientific, cat.# 4332) and cultured at 37°C, 5% $CO_2$ and 95% humidity. After 24 hours growth medium was carefully removed and replaced by 20μl Opti-MEM (GIBCO, cat.# 31985062) as assay buffer. For testing compounds an 8-point half-log compound dilution curve was generated in 100% DMSO starting from a 2mM stock and compounds were then diluted 1:50 in Opti-MEM. To test the compounds in the antagonist mode 10μl of compounds were then added to the wells containing 20μl Opti-MEM and incubated for 10 min. After this pre-incubation 10μl of the reference agonist beclometasone (Sigma, cat.# Y0000351) at an EC50 of 2.5 nM were added resulting in a final assay concentration range from 10 μM to 0.003 μM in 0.5% DMSO in a total volume of 40 μl. Compounds were tested at 8 concentrations in quadruplicate data points. Cells were incubated for 6 hour with compounds and mifepristone as control compound (Sigma, cat.# M8046) at 37°C, 5% $CO_2$ and 95% humidity. Finally, cells were lysed with 20μl of Triton/Luciferin solution and the signal of the emitted luminescence was recorded at the $FLIPR^{TETRA}$ for 2 minutes.

The relative efficacy of a compound (% effect) was calculated based on the full effect of the antagonist mifepristone:

$$\%\ \text{effect} = ((\text{compound} - \text{min})/(\text{max} - \text{min}))\ x\ \text{-}100$$

[min=Opti-MEM only, max=mifepristone]

To calculate $IC_{50}$, max, min and slope factor for each compound a concentration response curve was fitted by plotting %effect versus compound concentration using a 4 parameter logistic equation:

$$y = A + (B\text{-}A)/(1+((10C)/x)D)$$

[A=min y, B=max y, C=log$IC_{50}$, D=slope]

In Table 9 below, the IC50 or EC50 ranges of the Examples are summarized which were observed in the agonistic assay or the antagonistic assay described above.

Table 9 (A < 100nM, B = 100nM-1μM, C = 1μM-15μM):

| Ex. # | $IC_{50}$ or $EC_{50}$ | Ex. # | $IC_{50}$ or $EC_{50}$ | Ex. # | $IC_{50}$ or $EC_{50}$ |
|---|---|---|---|---|---|
| **1** | A | **22** | B | **43** | C |

(continued)

| Ex. # | $IC_{50}$ or $EC_{50}$ | Ex. # | $IC_{50}$ or $EC_{50}$ | Ex. # | $IC_{50}$ or $EC_{50}$ |
|---|---|---|---|---|---|
| **2** | B | **23** | A | **44** | C |
| **3** | A | **24** | A | **45** | C |
| **4** | C | **25** | A | **46** | A |
| **5** | A | **26** | A | **47** | B |
| **6** | A | **27** | A | **48** | B |
| **7** | B | **28** | B | **49** | A |
| **8** | A | **29** | B | **50** | A |
| **9** | A | **30** | B | **51** | B |
| **10** | B | **31** | B | **52** | A |
| **11** | A | **32** | A | **53** | B |
| **12** | A | **33** | A | **54** | A |
| **13** | C | **34** | A | **55** | B |
| **14** | B | **35** | B | **56** | A |
| **15** | A | **36** | B | **57** | B |
| **16** | B | **37** | A | **58** | B |
| **17** | B | **38** | A | **59** | B |
| **18** | C | **39** | A | **60** | A |
| **19** | B | **40** | B | **61** | B |
| **20** | B | **41** | A | **62** | B |
| **21** | B | **42** | C | **63** | C |

## Claims

**1.** A compound according to general formula (I),

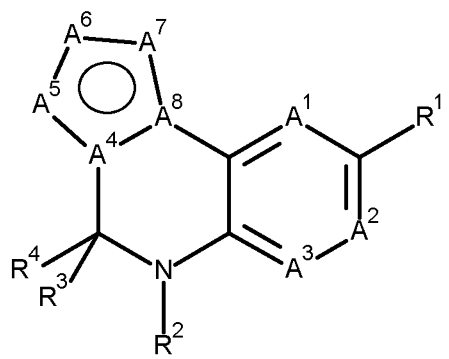

(I),

wherein

$R^1$ represents phenyl or 5 to 10-membered heteroaryl;
$R^2$ represents H;
$R^3$ and $R^4$ independently of one another represent H; $C_{1-10}$-alkyl; or together with the carbon atom joining them, form $C_{3-10}$-cycloalkyl;
$A^1$, $A^2$ and $A^3$ corresponds to embodiment **a, b, c,** or **d:**

| embodiment | $A^1$ | $A^2$ | $A^3$ |
|---|---|---|---|
| **a** | C-$R^5$ | C-$R^6$ | C-$R^7$ |
| **b** | C-$R^5$ | N | C-$R^7$ |
| **c** | C-$R^5$ | C-$R^6$ | N |
| **d** | N | C-$R^6$ | C-$R^7$ |

wherein $R^5$ represents H; F; Cl; Br; I; $C_{1-4}$-alkyl; $C_{3-10}$-cycloalkyl; or O-$C_{1-10}$-alkyl; $R^6$ represents H; F; Cl; Br; I; $C_{1-10}$-alkyl; $C_{3-10}$-cycloalkyl; or O-$C_{1-10}$-alkyl; $R^7$ represents H; F; Cl; Br; I; $C_{1-10}$-alkyl; $C_{3-10}$-cycloalkyl; or O-$C_{1-10}$-alkyl;

$A^4$ represents C or N;

$A^5$ represents O, N, N-$R^8$ or C-$R^8$, wherein $R^8$ represents H; $C_{1-10}$-alkyl; $C_{3-10}$-cycloalkyl; 3 to 7 membered heterocycloalkyl; $S(O)_2$-$C_{1-6}$-alkyl; or $S(O)_2$-$C_{3-10}$-cycloalkyl, wherein $C_{3-10}$-cycloalkyl, or 3 to 7 membered heterocycloalkyl, can optionally be bridged via $C_{1-6}$-alkylene;

$A^6$ represents O, N, N-$R^9$ or C-$R^9$, wherein $R^9$ represents H; $C_{1-10}$-alkyl; $C_{3-10}$-cycloalkyl; 3 to 7 membered heterocycloalkyl; $S(O)_2$-$C_{1-6}$-alkyl; or $S(O)_2$-$C_{3-10}$-cycloalkyl, wherein $C_{3-10}$-cycloalkyl,or 3 to 7 membered heterocycloalkyl, can optionally be bridged via $C_{1-6}$-alkylene;

$A^7$ represents O, N, N-$R^{10}$ or C-$R^{10}$, wherein $R^{10}$ represents H; $C_{1-10}$-alkyl; $C_{3-10}$-cycloalkyl; 3 to 7 membered heterocycloalkyl; $S(O)_2$-$C_{1-6}$-alkyl; or $S(O)_2$-$C_{3-10}$-cycloalkyl, wherein $C_{3-10}$-cycloalkyl, or 3 to 7 membered heterocycloalkyl can optionally be bridged via $C_{1-6}$-alkylene;

$A^8$ represents C or N;

wherein $A^4$, $A^5$, $A^6$, $A^7$ and $A^8$ form a heteroaromatic system; and

wherein if $A^4$ represents C and each of $A^5$, $A^6$ and $A^8$ represent N and $A^7$ represents C-$R^{10}$; then one of $A^1$, $A^2$ and $A^3$ represents N;

wherein $C_{1-4}$-alkyl, $C_{1-6}$-alkyl, $C_{1-10}$-alkyl and $C_{1-6}$-alkylene in each case independently from one another is linear or branched, saturated or unsaturated;

wherein $C_{1-4}$-alkyl, $C_{1-6}$-alkyl, $C_{1-10}$-alkyl, $C_{1-6}$-alkylene, $C_{3-10}$-cycloalkyl and 3 to 7 membered heterocycloalkyl in each case independently from one another are unsubstituted or mono- or polysubstituted with one or more substituents selected from F; Cl; Br; I; CN; $C_{1-6}$-alkyl; $CF_3$; $CF_2H$; $CFH_2$; $CF_2Cl$; $CFCl_2$; C(O)-$C_{1-6}$-alkyl; C(O)-OH; C(O)-O$C_{1-6}$-alkyl; C(O)-$NH_2$; C(O)-N(H)($C_{1-6}$-alkyl); C(O)-N($C_{1-6}$-alkyl)$_2$; OH; =O; $OCF_3$; $OCF_2H$; $OCFH_2$; $OCF_2Cl$; $OCFCl_2$; O-$C_{1-6}$-alkyl; O-C(O)-$C_{1-6}$-alkyl; O-C(O)-O-$C_{1-6}$-alkyl; O-(CO)-N(H)($C_{1-6}$-alkyl); O-C(O)-N($C_{1-6}$-alkyl)$_2$; O-$S(O)_2$-$NH_2$; O-$S(O)_2$-N(H)($C_{1-6}$-alkyl); O-$S(O)_2$-N($C_{1-6}$-alkyl)$_2$; $NH_2$; N(H)($C_{1-6}$-alkyl); N($C_{1-6}$-alkyl)$_2$; N(H)-C(O)-$C_{1-6}$-alkyl; N(H)-C(O)-O-$C_{1-6}$-alkyl; N(H)-C(O)-$NH_2$; N(H)-C(O)-N(H)($C_{1-6}$-alkyl); N(H)-C(O)-N($C_{1-6}$-alkyl)$_2$; N($C_{1-6}$-alkyl)-C(O)-$C_{1-6}$-alkyl; N($C_{1-6}$-alkyl)-C(O)-O-$C_{1-6}$-alkyl; N($C_{1-6}$-alkyl)-C(O)-$NH_2$; N($C_{1-6}$-alkyl)-C(O)-N(H)($C_{1-6}$-alkyl); N($C_{1-6}$-alkyl)-C(O)-N($C_{1-6}$-alkyl)$_2$; N(H)-$S(O)_2$OH; N(H)-$S(O)_2$-$C_{1-6}$-alkyl; N(H)-$S(O)_2$-O-$C_{1-6}$-alkyl; N(H)-$S(O)_2$-$NH_2$; N(H)-$S(O)_2$-N(H)($C_{1-6}$-alkyl); N(H)-$S(O)_2$N($C_{1-6}$-alkyl)$_2$; N($C_{1-6}$-alkyl)-$S(O)_2$-OH; N($C_{1-6}$-alkyl)-$S(O)_2$-$C_{1-6}$-alkyl; N($C_{1-6}$-alkyl)-$S(O)_2$-O-$C_{1-6}$-alkyl; N($C_{1-6}$-alkyl)-$S(O)_2$-$NH_2$; N($C_{1-6}$-alkyl)-$S(O)_2$-N(H)($C_{1-6}$-alkyl); N($C_{1-6}$-alkyl)-$S(O)_2$-N($C_{1-6}$-alkyl)$_2$; $SCF_3$; $SCF_2H$; $SCFH_2$; S-$C_{1-6}$-alkyl; S(O)-$C_{1-6}$-alkyl; $S(O)_2$-$C_{1-6}$-alkyl; $S(O)_2$-OH; $S(O)_2$-O-$C_{1-6}$-alkyl; $S(O)_2$-$NH_2$; $S(O)_2$-N(H)($C_{1-6}$-alkyl); $S(O)_2$-N($C_{1-6}$-alkyl)$_2$; $C_{3-6}$-cycloalkyl; 3 to 7-membered heterocycloalkyl; phenyl; 5 or 6-membered heteroaryl; O-$C_{3-6}$-cycloalkyl; O-(3 to 7-membered heterocycloalkyl); O-phenyl; O-(5 or 6-membered heteroaryl); C(O)-$C_{3-6}$-cycloalkyl; C(O)-(3 to 7-membered heterocycloalkyl); C(O)-phenyl; C(O)-(5 or 6-membered heteroaryl); $S(O)_2$-($C_{3-6}$-cycloalkyl); $S(O)_2$-(3 to 7-membered heterocycloalkyl); $S(O)_2$-phenyl or $S(O)_2$-(5 or 6-membered heteroaryl);

wherein phenyl, and 5 to 10-membered heteroaryl in each case independently from one another are unsubstituted or mono- or polysubstituted with one or more substituents selected from F; Cl; Br; I; CN; $C_{1-6}$-alkyl; $C_{1-6}$-alkenyl; $C_{1-6}$-alkynyl; $C_{1-6}$-alkynyl-C(H)(OH)$CH_3$; $C_{1-6}$-alkynyl-C($CH_3$)$_2$OH; $CF_3$; $CF_2H$; $CFH_2$; $CF_2Cl$; $CFCl_2$; $C_{1-6}$-alkylene-$CF_3$; $C_{1-6}$-alkylene-$CF_2H$; $C_{1-6}$-alkylene-$CFH_2$; $C_{1-6}$-alkylene-OH; $C_{1-6}$-alkylene-$OCH_3$; C(O)-$C_{1-6}$-alkyl; C(O)-OH; C(O)-O$C_{1-6}$-alkyl; C(O)-N(H)(OH); C(O)-$NH_2$; C(O)-N(H)($C_{1-6}$-alkyl); C(O)-N($C_{1-6}$-alkyl)$_2$; OH; $OCF_3$; $OCF_2H$; $OCFH_2$; $OCF_2Cl$; $OCFCl_2$; O-$C_{1-6}$-alkyl; O-$C_{3-6}$-cycloalkyl; O-(3 to 7-membered heterocycloalkyl); $NH_2$; N(H)($C_{1-6}$-alkyl); N($C_{1-6}$-alkyl)$_2$; N(H)-C(O)-$C_{1-6}$-alkyl; N($C_{1-6}$-alkyl)-C(O)-$C_{1-6}$-alkyl; N(H)-C(O)-$NH_2$; N(H)-C(O)-N(H)($C_{1-6}$-alkyl); N(H)-C(O)-N($C_{1-6}$-alkyl)$_2$; N($C_{1-6}$-alkyl)-C(O)-N(H)($C_{1-6}$-alkyl); N($C_{1-6}$-alkyl)-C(O)-N($C_{1-6}$-alkyl)$_2$; N(H)-$S(O)_2$-$C_{1-6}$-alkyl; $SCF_3$; S-$C_{1-6}$-alkyl; S(O)-$C_{1-6}$-alkyl; $S(O)_2$-$C_{1-6}$-alkyl; $S(O)_2$-$C_{3-6}$-cycloalkyl; $S(O)_2$-$C_{1-6}$-alkylene-$C_{3-6}$-cycloalkyl; $S(O)_2$-$NH_2$; $S(O)_2$-N(H)($C_{1-6}$-alkyl); $S(O)_2$-N($C_{1-6}$-alkyl)$_2$; $C_{3-6}$-cycloalkyl; $C_{1-6}$-alkylene-$C_{3-6}$-cycloalkyl; 3 to 7-membered heterocycloalkyl; $C_{1-6}$-alkylene-(3 to 7-membered heterocycloalkyl); phenyl or 5 or 6-membered heteroaryl;

in the form of the free compound or a physiologically acceptable salt thereof.

**2.** The compound according to claim 1, wherein

- $C_{1-4}$-alkyl, $C_{1-6}$-alkyl, $C_{1-10}$-alkyl, $C_{1-6}$-alkylene, $C_{3-10}$-cycloalkyl and 3 to 7 membered heterocycloalkyl in each case independently from one another are unsubstituted or mono- or polysubstituted with one or more substituents selected from F; Cl; Br; I; CN; $C_{1-6}$-alkyl; $CF_3$; $CF_2H$; $CFH_2$; $CF_2Cl$; $CFCl_2$; OH; =O; $OCF_3$; $OCF_2H$; $OCFH_2$; $OCF_2Cl$; $OCFCl_2$; O-$C_{1-6}$-alkyl; $C_{3-6}$-cycloalkyl; or 3 to 7-membered heterocycloalkyl; and/or
- phenyl, and 5 to 10-membered heteroaryl in each case independently from one another are unsubstituted or mono- or polysubstituted with one or more substituents selected from F; Cl; Br; I; CN; $C_{1-6}$-alkyl; $C_{2-6}$-alkinyl, preferably $-C\equiv C-CH_3$; $CF_3$; $CF_2H$; $CFH_2$; $CF_2Cl$; $CFCl_2$; $C_{1-6}$-alkylene-$CF_3$; $C_{1-6}$-alkylene-$CF_2H$; $C_{1-6}$-alkylene-$CFH_2$; C(O)-$C_{1-6}$-alkyl; C(O)-OH; C(O)-O$C_{1-6}$-alkyl; OH; $C_{1-6}$-alkylene-OH; $OCF_3$; $OCF_2H$; $OCFH_2$; $OCF_2Cl$; $OCFCl_2$; O-$C_{1-6}$-alkyl; O-$C_{3-6}$-cycloalkyl; O-(3 to 7-membered heterocycloalkyl); $SCF_3$; S-$C_{1-6}$-alkyl; S(O)-$C_{1-6}$-alkyl; $S(O)_2$-$C_{1-6}$-alkyl; $S(O)_2$-$C_{1-6}$-alkylene-$C_{3-6}$-cycloalkyl; $S(O)_2$-$NH_2$; $S(O)_2$-N(H)($C_{1-6}$-alkyl); $S(O)_2$-N($C_{1-6}$-alkyl)$_2$; $C_{3-6}$-cycloalkyl; $C_{1-6}$-alkylene-$C_{3-6}$-cycloalkyl; 3 to 7-membered heterocycloalkyl; $C_{1-6}$-alkylene-(3 to 7-membered heterocycloalkyl); phenyl or 5 or 6-membered heteroaryl.

**3.** The compound according to claim 1 or 2, wherein

- $R^1$ represents phenyl or 5 to 10-membered heteroaryl which is selected from the group consisting of indolyl, indazolyl, pyridyl, preferably 2-pyridyl, 3-pyridyl or 4-pyridyl, pyrazolyl, pyrazolopyrimidinyl, pyrrolopyridinyl, pyrimidinyl, pyridazinyl, pyrazinyl, pyrrolyl, imidazolyl, oxazolyl, isoxazolyl, thiazolyl, isothiazolyl, furanyl, thienyl (thiophenyl), triazolyl, thiadiazolyl, 4,5,6,7-tetrahydro-2H-indazolyl, 2,4,5,6-tetrahydrocyclo-penta[c]pyrazolyl, benzofuranyl, benzoimidazolyl, benzothienyl, benzothiadiazolyl, benzothiazolyl, benzotriazolyl, benzooxazolyl, benzooxadiazolyl, quinazolinyl, quinoxalinyl, carbazolyl, quinolinyl, dibenzofuranyl, dibenzothienyl, imidazothiazolyl, indolizinyl, isoquinolinyl, naphthyridinyl, oxazolyl, oxadiazolyl, phenazinyl, phenothiazinyl, phthalazinyl, purinyl, phenazinyl, tetrazolyl and triazinyl; and/or
- **(i)** $R^3$ and $R^4$, together with the carbon atom joining them, form $C_{3-10}$-cycloalkyl; or **(ii)** $R^3$ and $R^4$ independently of one another represent H or $C_{1-10}$-alkyl..

**4.** The compound according to any of the preceding claims, wherein

$R^3$ and $R^4$, independently of one another represent H or $-CH_3$; or
($R^3$ and $R^4$, together with the carbon atom joining them, form $C_{3-10}$-cycloalkyl.

**5.** The compound according claim 4, wherein $R^3$ and $R^4$, together with the carbon atom joining them, form cyclobutyl.

**6.** The compound according to any of the preceding claims, wherein

- $R^1$ represents

(i) phenyl or 5 to 10-membered heteroaryl which is selected from the group consisting of indolyl, indazolyl, pyridyl, preferably 2-pyridyl, 3-pyridyl or 4-pyridyl, pyrazolyl, pyrazolopyrimidinyl, pyrrolopyridinyl, pyrimidinyl, pyridazinyl, pyrazinyl, pyrrolyl, imidazolyl, oxazolyl, isoxazolyl, thiazolyl, isothiazolyl, furanyl, thienyl (thiophenyl), triazolyl, thiadiazolyl, 4,5,6,7-tetrahydro-2H-indazolyl, 2,4,5,6-tetrahydrocyclo-penta[c]pyrazolyl, benzofuranyl, benzoimidazolyl, benzothienyl, benzothiadiazolyl, benzothiazolyl, benzotriazolyl, benzooxazolyl, benzooxadiazolyl, quinazolinyl, quinoxalinyl, carbazolyl, quinolinyl, dibenzofuranyl, dibenzothienyl, imidazothiazolyl, indolizinyl, isoquinolinyl, naphthyridinyl, oxazolyl, oxadiazolyl, phenazinyl, phenothiazinyl, phthalazinyl, purinyl, phenazinyl, tetrazolyl and triazinyl; or
**(ii)** phenyl, unsubstituted or mono- or polysubstituted with one or more substituents selected from F; Cl; Br; I; $-CH_3$; $-CH_2-CH_3$; $O-CH_3$; $-CF_3$; $-C_{3-10}$-cycloalkyl; $-CH_2-C_{3-10}$-cycloalkyl; $S(=O)_2-C_{3-10}$-cycloalkyl; $S(=O)_2-CH_2-C_{3-10}$-cycloalkyl; $S(=O)_2-CH_3$; $S(=O)_2-CH_2-CH_3$; $-CH_2-CH_2-O-CH_2-$ (i.e. oxolanyl); $-C\equiv C-CH_3$; $C(=O)-CH_3$; $-CH=CH_2$; $NH_2$; or $-CH_2-CH_2-OH$;

or any of the following structure (II), (III), (IV), (V) or (VI), with the proviso that with respect to structures (II), (III), (IV) and (V) at least one of X and Z is a heteroatom:

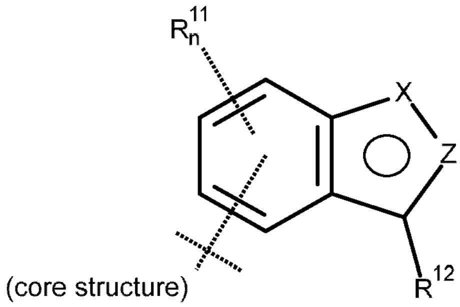
(II)

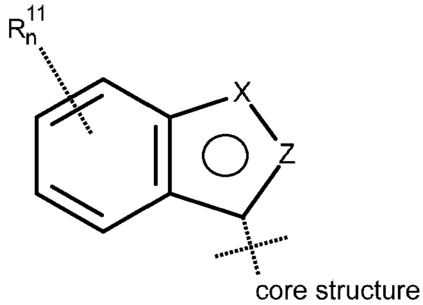
(III)

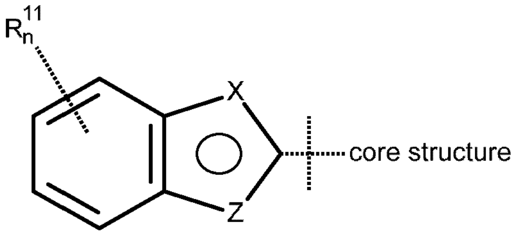
(IV)

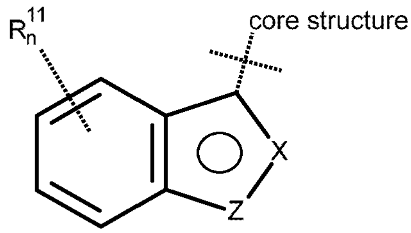
(V)

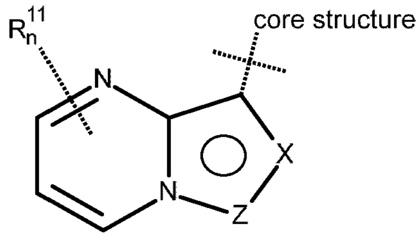
(VI)

wherein

X represents N, N-$R^{13}$ or C-$R^{13}$;
Z represents N, N-$R^{13}$ or C-$R^{13}$;
$R^{11}$, $R^{12}$ and $R^{13}$ represent, independently from one another, H; F; Cl; Br; I; CN; $C_{1-10}$-alkyl; $C_{3-10}$-cycloalkyl; 3 to 7 membered heterocycloalkyl; S(O)-($C_{1-10}$-alkyl); S(O)-($C_{3-10}$-cycloalkyl); S(O)-(3 to 7-membered heterocycloalkyl); $S(O)_2$-($C_{1-10}$-alkyl); $S(O)_2$-($C_{3-10}$-cycloalkyl); $S(O)_2$-(3 to 7-membered heterocycloalkyl); P(O)-($C_{1-10}$-alkyl$)_2$; P(O)($C_{1-10}$-alkyl)($C_{3-10}$-cycloalkyl); P(O)($C_{1-10}$-alkyl)(3 to 7-membered heterocycloalkyl); P(O)-(O-$C_{1-10}$-alkyl$)_2$; P(O)(O-$C_{1-10}$-alkyl)(O-$C_{3-10}$-cycloalkyl); P(O)(O-$C_{1-10}$-alkyl)(O-(3 to 7-membered heterocycloalkyl)); O-$C_{1-10}$-alkyl; S-$C_{1-10}$-alkyl; N(H)($C_{1-10}$-alkyl), N($C_{1-10}$-alkyl$)_2$; C(O)-$C_{1-10}$-alkyl; C(O)-O-$C_{1-10}$-alkyl; C(O)-$NH_2$; C(O)-N(H)($C_{1-10}$-alkyl); C(O)-N($C_{1-10}$-alkyl$)_2$; O-$C_{3-10}$-cycloalkyl; N(H)($C_{3-10}$-cycloalkyl), N($C_{1-10}$-alkyl)($C_{3-10}$-cycloalkyl); C(O)-$C_{3-10}$-cycloalkyl; C(O)-O-$C_{3-10}$-cycloalkyl; C(O)-N(H)($C_{3-10}$-cycloalkyl); C(O)-N($C_{1-10}$-alkyl)($C_{3-10}$-cycloalkyl); O-3 to 7-membered heterocycloalkyl; N(H)(3 to 7-membered heterocycloalkyl), N($C_{1-10}$-alkyl)(3 to 7-membered heterocycloalkyl); C(O)-3 to 7-membered heterocycloalkyl; C(O)-O-(3 to 7-membered heterocycloalkyl); C(O)-N(H)(3 to 7-membered heterocycloalkyl) or C(O)-N($C_{1-10}$-alkyl)(3 to 7-membered heterocycloalkyl); wherein $C_{3-10}$-cycloalkyl and 3 to 7 membered heterocycloalkyl can optionally be bridged via $C_{1-6}$-alkylene; and n represents 0, 1, 2 or 3;
or wherein $R^{11}$, $R^{12}$ and $R^{13}$ represent, independently from one another, F; Cl; Br; I; -$CH_3$; O-$CH_3$; -$CF_3$; -$C_{3-10}$-cycloalkyl; -$CH_2$-$C_{3-10}$-cycloalkyl; $S(=O)_2$-$CH_2$-$C_{3-10}$-cycloalkyl; $S(=O)_2$-$CH_3$; -$CH_2$-$CH_2$-O-$CH_2$-(i.e. oxolanyl); C≡C-$CH_3$; C(=O)-$CH_3$; -$CH_2$-$CH_2$-OH; and n represents 0, 1, 2 or 3.

**7.** The compound according to any of the preceding claims, wherein

- none of $A^1$, $A^2$ and $A^3$ represents N, respectively; and/or
- $R^5$, $R^6$ and $R^7$, independently from one another, represent $CH_3$, F, Cl, $CF_3$, or H.

**8.** The compound according to any of the preceding claims, wherein

- $A^7$ does not represent C-$R^{10}$; or
- none of $A^5$, $A^6$ and $A^7$ represents C-$R^8$, C-$R^9$ or C-$R^{10}$, respectively, and/or at most one of $A^5$, $A^6$ and $A^7$ represents O; or
- at least one of $A^5$, $A^6$ and $A^7$ represents C-$R^8$, C-$R^9$ or C-$R^{10}$, respectively, and/or at most one of $A^5$, $A^6$ and $A^7$ represents O; or
- at least one of $A^5$, $A^6$ and $A^7$ represents N, respectively, and/or at most one of $A^5$, $A^6$ and $A^7$ represents O; or
- at least one of $A^5$, $A^6$ and $A^7$ represents N-$R^8$, N-$R^9$ or N-$R^{10}$, respectively, and/or at most one of $A^5$, $A^6$ and $A^7$ represents O; and/or
- $R^8$, $R^9$ and $R^{10}$, independently from one another, represent $S(O)_2$-$CH_3$, $CH_3$, $CH_2CH_3$, F, $CF_3$, $CH_2$-cyclopropyl, or H.

**9.** The compound according to any of the preceding claims, wherein the definition of $A^5$, $A^6$ and $A^7$ corresponds to embodiment **e, f, g, h, i, j, k, l** or **m**:

| embodiment | $A^5$ | $A^6$ | $A^7$ |
|---|---|---|---|
| **e** | N | C-$R^9$ | C-$R^{10}$ |
| **f** | C-$R^8$ | C-$R^9$ | C-$R^{10}$ |
| **g** | N | N | C-$R^{10}$ |
| **h** | N | N | N |
| **i** | N | N | N-$R^{10}$ |
| **j** | C-$R^8$ | N | N-$R^{10}$ |
| **k** | N | C-$R^9$ | N-$R^{10}$ |
| **l** | C-$R^8$ | N-$R^9$ | N |
| **m** | N | C-$R^9$ | O |

**10.** The compound according to any of the preceding claims, wherein the definition of $A^4$, $A^5$, $A^6$, $A^7$ and $A^8$ corresponds to embodiment **n, o, p, q, r, s, t, u, v, w, x** or **y:**

| embodiment | $A^4$ | $A^5$ | $A^6$ | $A^7$ | $A^8$ |
|---|---|---|---|---|---|
| **n** | C | N | C-$R^9$ | C-$R^{10}$ | N |
| **o** | N | N | C-$R^9$ | C-$R^{10}$ | C |
| **p** | C | C-$R^8$ | C-$R^9$ | C-$R^{10}$ | N |
| **q** | C | N | N | C-$R^{10}$ | N |
| **r** | N | N | N | C-$R^{10}$ | C |
| **s** | C | N | N | N | N |
| **t** | C | N | N | N-$R^{10}$ | C |
| **u** | C | C-$R^8$ | N | N-$R^{10}$ | C |
| **v** | N | N | C-$R^9$ | N-$R^{10}$ | C |
| **w** | C | N | C-$R^9$ | N-$R^{10}$ | C |
| **x** | C | C-$R^8$ | N-$R^9$ | N | C |
| **y** | C | N | C-$R^9$ | O | C |

**11.** The compound according to any of the preceding claims which is selected from the group consisting of

**1** 7-fluoro-8-(3-fluoro-5-methylphenyl)-1,4,4,9-tetramethyl-5H-imidazo[1,2-a]quinoxaline

(continued)

| | |
|---|---|
| **2** | 7,9-difluoro-1,4,4-trimethy1-8-(1H-pyrazol-3-y1)-5H-pyrrolo[1,2-a]quinoxaline |
| **3** | 7,9-difluoro-8-(1H-indol-4-yl)-1,4,4-trimethyl-5H-pyrrolo[1,2-a]quinoxaline |
| **4** | 7,9-difluoro-1,4,4-trimethyl-8-pyrazolo[1,5-a]pyrimidin-3-yl-5H-pyrrolo[1,2-a]quinoxaline |
| **5** | 7,9-difluoro-8-(6-fluoro-1H-indol-4-yl)-1,4,4-trimethyl-5H-imidazo[1,2-a]quinoxaline |
| **6** | 7-fluoro-8-[2-methoxy-5-(trifluoromethyl)pyridin-3-yl]-1,4,4,9-tetramethyl-5H-imidazo[1,2-a]quinoxaline |
| **7** | 7-fluoro-1,4,4,9-tetramethyl-8-[6-(trifluoromethyl)-1H-indol-4-yl]-5H-imidazo[1,2-a]quinoxaline |
| **8** | 8-[1-(cyclopropylmethyl)indol-4-yl]-7-fluoro-1,4,4,9-tetramethyl-5H-imidazo[1,2-a]quinoxaline |
| **9** | 8-[1-(cyclopropylmethylsulfonyl)indol-4-yl]-7-fluoro-l,4,4,9-tetramethyl-5H-imidazo[1,2-a]quinoxaline |
| **10** | 8-(1-cyclopropylindol-4-yl)-7-fluoro-1,4,4,9-tetramethyl-5H-imidazo[1,2-a]quinoxaline |
| **11** | 9-fluoro-1,4,4-trimethyl-8-(3-methyl-1H-indol-7-yl)-4,5-dihydropyrido[3,4-e] [1,2,4]triazolo[4,3-a]pyrazine |
| **12** | 7,9-difluoro-1,4,4-trimethyl-8-(1H-pyrrolo[2,3-b]pyridin-4-yl)-5H-pyrrolo[1,2-a]quinoxaline |
| **13** | 8-(5-fluoro-3-methyl-1H-indol-7-yl)-1,4,4,9-tetramethyl-4,5-dihydropyrido[2,3-e] [1,2,4]triazolo[4,3-a]pyrazine |
| **14** | 7-fluoro-8-(6-fluoro-1-methylsulfonylindol-4-yl)-1,4,4,9-tetramethyl-5H-imidazo[1,2-a]quinoxaline |
| **15** | 8-(5-chloro-2-methoxypyridin-3-yl)-7-fluoro-1,4,4,9-tetramethyl-5H-imidazo[1,2-a]quinoxaline |
| **16** | 7-fluoro-8-[5-fluoro-3-(oxolan-3-yl)-1H-indol-7-yl]-1,4,4,9-tetramethyl-5H-imidazo[1,2-a]quinoxaline |
| **17** | 7-fluoro-8-(5-fluoro-3-prop-1-ynyl-1H-indol-7-yl)-1,4,4,9-tetramethyl-5H-imidazo[1,2-a]quinoxaline |
| **18** | 9-fluoro-8-(6-fluoro-1-(methylsulfonyl)-1H-indol-4-yl)-1,4,4-trimethyl-4,5-dihydropyrido[3,4-e][1,2,4]triazolo [4,3-a]pyrazine |
| **19** | 7-fluoro-1,4,4,9-tetramethyl-8-(1-methylsulfonylindazol-4-yl)-5H-imidazo[1,2-a]quinoxaline |
| **20** | 7,9-difluoro-1,4,4-trimethyl-8-(1-methylsulfonylindazol-4-yl)-5H-pyrrolo[1,2-a]quinoxaline |
| **21** | 1-[4-(7,9-difluoro-1,4,4-trimethyl-5H-pyrrolo[1,2-a]quinoxalin-8-yl)indol-1-yl]ethanone |
| **22** | 8-(3-cyclopropyl-1H-indol-7-yl)-7,9-difluoro-4,4-dimethyl-5H-tetrazolo[1,5-a]quinoxaline |
| **23** | 7-fluoro-8-(6-fluoro-1-methylsulfonylindol-4-yl)-4,4-dimethyl-9-(trifluoromethyl)-5H-tetrazolo[1,5-a]quinoxaline |
| **24** | 7-fluoro-8-(6-fluoro-1-methylsulfonylindol-4-yl)-1,4,4,9-tetramethyl-5H-triazolo[4,5-c]quinoline |
| **25** | 7-fluoro-8-(5-fluoro-3-methyl-1H-indol-7-yl)-1,4,4,9-tetramethyl-5H-triazolo[4,5-c]quinoline |
| **26** | 2-[6-fluoro-4-(7-fluoro-1,4,4,9-tetramethyl-5H-triazolo[4,5-c]quinolin-8-yl)indol-1-yl]ethanol |
| **27** | 8-fluoro-9-(6-fluoro-1-methylsulfonylindol-4-yl)-1,5,5,10-tetramethyl-6H-pyrazolo[1,5-c]quinazoline |
| **28** | 8,10-difluoro-9-(6-fluoro-1-methylsulfonylindol-4-yl)-5,5-dimethyl-6H-pyrazolo[1,5-c]quinazoline |
| **29** | 2-(6-fluoro-1-(methylsulfonyl)-1H-indol-4-yl)-6,6,9-trimethyl-5,6-dihydropyrido[3,2-e][1,2,4]triazolo[4,3-a]pyrazine |
| **30** | 3-fluoro-6,6,9-trimethyl-2-(3-methyl-1H-indol-7-yl)-5,6-dihydropyrido[3,2-e][1,2,4]triazolo[4,3-a]pyrazine |
| **31** | 1,4,4,9-tetramethyl-8-(3-methyl-1H-indol-7-yl)-4,5-dihydropyrido[3,4-e][1,2,4]triazolo[4,3-a]pyrazine |
| **32** | 6-fluoro-8-(6-fluoro-1-methylsulfonylindol-4-yl)-1,4,4,9-tetramethyl-5H-pyrazolo[4,3-c]quinoline |
| **33** | 6-fluoro-1,4,4,9-tetramethyl-8-(1-methylsulfonylindol-4-yl)-5H-pyrazolo[4,3-c]quinoline |
| **34** | 6-fluoro-8-(6-fluoro-1-methylsulfonylindazol-4-yl)-1,4,4,9-tetramethyl-5H-pyrazolo[4,3-c]quinoline |
| **35** | 7-fluoro-9-(6-fluoro-1-methylsulfonylindol-4-yl)-1,5,5,10-tetramethyl-6H-triazolo[1,5-c]quinazoline |
| **36** | 7-fluoro-9-(6-fluoro-1-methylsulfonylindazol-4-yl)-1,5,5,10-tetramethyl-6H-triazolo[1,5-c]quinazoline |
| **37** | 6-fluoro-8-(6-fluoro-1-methylsulfonylindol-4-yl)-1,9-dimethylspiro[5H-pyrazolo[4,3-c]quinoline-4,1-cyclobutane] |
| **38** | 6-fluoro-1,9-dimethyl-8-(1-methylsulfonylindazol-4-yl)spiro[5H-pyrazolo[4,3-c]quinoline-4,1-cyclobutane] |
| **39** | 6-fluoro-8-(5-fluoro-3-methyl-1H-indol-7-yl)-1,9-dimethylspiro[5H-pyrazolo[4,3-c]quinoline-4,1-cyclobutane] |
| **40** | 1-ethyl-6-fluoro-8-(6-fluoro-1-methylsulfonylindazol-4-yl)-4,4,9-trimethyl-5H-pyrazolo[4,3-c]quinoline |
| **41** | 1-ethyl-6-fluoro-8-(6-fluoro-1-methylsulfonylindol-4-yl)-4,4,9-trimethyl-5H-pyrazolo[4,3-c]quinoline |
| **42** | 1-(cyclopropylmethyl)-6-fluoro-8-(6-fluoro-1-methylsulfonylindol-4-yl)-4,4,9-trimethyl-5H-pyrazolo[4,3-c]quinoline |
| **43** | 1-(cyclopropylmethyl)-6-fluoro-8-(5-fluoro-3-methyl-1H-indol-7-yl)-4,4,9-trimethyl-5H-pyrazolo[4,3-c]quinoline |
| **44** | 1-(cyclopropylmethyl)-6-fluoro-8-(6-fluoro-1-methylsulfonylindazol-4-yl)-4,4,9-trimethyl-5H-pyrazolo[4,3-c] quinoline |
| **45** | 7-fluoro-9-(6-fluoro-1-methylsulfonylindazol-4-yl)-5,5,10-trimethyl-6H-pyrazolo[1,5-c]quinazoline |

(continued)

| | |
|---|---|
| **46** | 7-fluoro-1,4,4,9-tetramethyl-8-(1-methylsulfonylindol-4-yl)-5H-pyrazolo[4,3-c]quinoline |
| **47** | 6-fluoro-8-(6-fluoro-1-methylsulfonylindol-4-yl)-1,4,4,9-tetramethyl-5H-imidazo[4,5-c]quinoline |
| **48** | 6-fluoro-8-(6-fluoro-1-methylsulfonylindazol-4-y1)-1,4,4,9-tetramethyl-5H-imidazo[4,5-c]quinoline |
| **49** | 6-fluoro-8-(5-fluoro-3-methyl-1H-indol-7-yl)-1,4,4,9-tetramethyl-5H-imidazo[4,5-c]quinoline |
| **50** | 7-fluoro-8-(6-fluoro-1-methylsulfonylindazol-4-yl)-1,3,4,4,9-pentamethyl-5H-pyrazolo[4,3-c]quinoline |
| **51** | 6-fluoro-8-(6-fluoro-1-methylsulfonyfindol-4-yl)-1,3,4,4,9-pentamethyl-5H-pyrazolo[4,3-c]quinoline |
| **52** | 6,7-difluoro-8-(5-fluoro-3-methyl-1-indol-7-yl)-1,4,4-trimethyl-5H-pyrazolo[4,3-c]quinoline |
| **53** | 6-fluoro-1,3,9-trimethyl-8-(1-methylsulfonylindol-4-yl)spiro[5H-pyrazolo[4,3-c]quinoline-4,1-cyclobutane] |
| **54** | 6-fluoro-1,3,9-trimethyl-8-(1-methylsulfonylindazol-4-yl)spiro[5H-pyrazolo[4,3-c]quinoline-4,1-cyclobutane] |
| **55** | 6-fluoro-8-(5-fluoro-3-methyl-1H-indol-7-yl)-4,4,9-trimethyl-2,5-dihydropyrazolo[4,3-c]quinoline |
| **56** | 6-fluoro-1,3,9-trimethyl-8-(3-methyl-1H-indol-7-yl)spiro[5H-pyrazolo[4,3-c]quinoline-4,1-cyclobutane] |
| **57** | 6-fluoro-8-(6-fluoro-1-methylsulfonylindol-4-yl)-4,4,9-trimethyl-2-methylsulfonyl-5H-pyrazolo[4,3-c]quinoline |
| **58** | 6-fluoro-8-(6-fluoro-1-methylsulfonylindol-4-yl)-4,4,9-trimethyl-2,5-dihydropyrazolo[4,3-c]quinoline |
| **59** | 6-fluoro-8-(6-fluoro-1-methylsulfonylindazol-4-yl)-4,4,9-trimethyl-2,5-dihydropyrazolo[4,3-c]quinoline |
| **60** | 8-(6-fluoro-1-methylsulfonylindazol-4-yl)-1,4,4,9-tetramethyl-5H-triazolo[4,5-c]quinoline |
| **61** | 7-fluoro-8-(6-fluoro-1-methylsulfonylindol-4-yl)-4,4,9-trimethyl-5H-[1,3]oxazolo[4,5-c]quinoline |
| **62** | 7-fluoro-8-(5-fluoro-3-methyl-1H-indol-7-yl)-4,4,9-trimethyl-5H-[1,3]oxazolo[4,5-c]quinoline and |
| **63** | 7-fluoro-8-(6-fluoro-1-methylsulfonylindazol-4-yl)-4,4,9-trimethyl-5H-[1,3]oxazolo[4,5-c]quinoline |

in the form of the free compound or a physiologically acceptable salt thereof.

**12.** A pharmaceutical dosage form comprising a compound according to any of claims 1 to 11.

**13.** The compound according to any of claims 1 to 11 and/or the pharmaceutical dosage form according to claim 12 for use in the treatment and/or prophylaxis of pain and/or inflammation.

**14.** The compound according to any of claims 1 to 11 and/or the pharmaceutical dosage form according to claim 12 for use according to claim 13 in the treatment and/or prophylaxis of inflammatory pain.

## Patentansprüche

**1.** Verbindung gemäß der allgemeinen Formel (I),

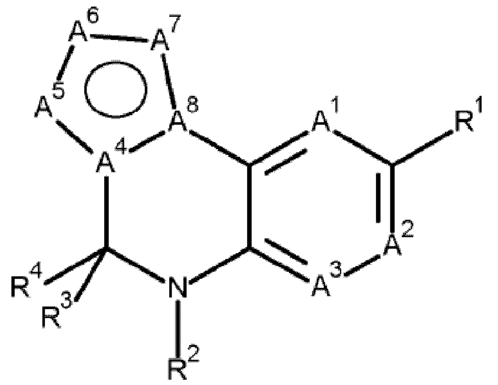

(I),

wobei

$R^1$ für Phenyl oder 5 bis 10-gliedriges Heteroaryl steht;
$R^2$ für H steht;
$R^3$ und $R^4$ unabhängig voneinander für H; $C_{1-10}$-Alkyl stehen; oder zusammen mit dem Kohlenstoffatom, das sie verbindet, $C_{3-10}$-Cycloalkyl bilden;
$A^1$, $A^2$ und $A^3$ Ausführungsform **a, b, c** oder **d** entsprechen:

| Ausführ ungsfor m | $A^1$ | $A^2$ | $A^3$ |
|---|---|---|---|
| **a** | C-$R^5$ | C-$R^6$ | C-$R^7$ |
| **b** | C-$R^5$ | N | C-$R^7$ |
| **c** | C-$R^5$ | C-$R^6$ | N |
| **d** | N | C-$R^6$ | C-$R^7$ |

wobei $R^5$ für H; F; Cl; Br; I; $C_{1-4}$-Alkyl; $C_{3-10}$-Alkyl oder O-$C_{1-10}$-Alkyl steht; $R^6$ für H; F; Cl; Br; I; $C_{1-10}$-Alkyl; $C_{3-10}$-Cycloalkyl oder O-$C_{1-10}$-Alkyl steht; $R^7$ für H; F; Cl; Br; I; $C_{1-10}$-Alkyl; $C_{3-10}$-Cycloalkyl oder O-$C_{1-10}$-Alkyl steht;

$A^4$ für C oder N steht;

$A^5$ für O, N, N-$R^8$ oder C-$R^8$ steht, wobei $R^8$ für H; $C_{1-10}$-Alkyl; $C_{3-10}$-Cycloalkyl; 3- bis 7-gliedriges Heterocycloalkyl; $S(O)_2$-$C_{1-6}$-Alkyl; oder $S(O)_2$-$C_{3-10}$-Cycloalkyl steht, wobei $C_{3-10}$-Cycloalkyl oder 3- bis 7-gliedriges Heterocycloalkyl gegebenenfalls über $C_{1-6}$-Alkylen verbrückt sein können;

$A^6$ für O, N, N-$R^9$ oder C-$R^9$ steht, wobei $R^9$ für H; $C_{1-10}$-Alkyl; $C_{3-10}$-Cycloalkyl; 3- bis 7-gliedriges Heterocycloalkyl; $S(O)_2$-$C_{1-6}$-Alkyl oder $S(O)_2$-$C_{3-10}$-Cycloalkyl steht, wobei $C_{3-10}$-Cycloalkyl oder 3- bis 7-gliedriges Heterocycloalkyl gegebenenfalls über $C_{1-6}$-Alkylen verbrückt sein können;

$A^7$ für O, N, N-$R^{10}$ oder C-$R^{10}$ steht, wobei $R^{10}$ für H; $C_{1-10}$-Alkyl; $C_{3-10}$-Cycloalkyl; 3- bis 7-gliedriges Heterocycloalkyl; $S(O)_2$-$C_{1-6}$-Alkyl oder $S(O)_2$-$C_{3-10}$-Cycloalkyl steht, wobei $C_{3-10}$-Cycloalkyl oder 3- bis 7-gliedriges Heterocycloalkyl gegebenenfalls über $C_{1-6}$-Alkylen verbrückt sein können;

$A^8$ für C oder N steht;

wobei $A^4$, $A^5$, $A^6$, $A^7$ und $A^8$ ein heteroaromatisches System bilden; und

wobei, wenn $A^4$ für C steht und jedes von $A^5$, $A^6$ und $A^8$ für N steht und $A^7$ für C-$R^{10}$ steht, dann eines von $A^1$, $A^2$ und $A^3$ für N steht;

wobei $C_{1-4}$-Alkyl, $C_{1-6}$-Alkyl, $C_{1-10}$-Alkyl and $C_{1-6}$-Alkylen jeweils unabhängig voneinander linear oder verzweigt, gesättigt oder ungesättigt ist;

wobei $C_{1-4}$-Alkyl, $C_{1-6}$-Alkyl, $C_{1-10}$-Alkyl, $C_{1-6}$-Alkylen, $C_{3-10}$-Cycloalkyl und 3- bis 7-gliedriges Heterocycloalkyl in jedem Fall unabhängig voneinander unsubstituiert oder mono- oder polysubstituiert sind mit einem oder mehreren Substituenten ausgewählt aus F; Cl; Br; I; CN; $C_{1-6}$-Alkyl; $CF_3$; $CF_2H$; $CFH_2$; $CF_2Cl$; $CFCl_2$; C(O)-$C_{1-6}$-Alkyl; C(O)-OH; C(O)-O$C_{1-6}$-Alkyl; C(O)-$NH_2$; C(O)-N(H)($C_{1-6}$-Alkyl); C(O)-N($C_{1-6}$-Alkyl)$_2$; OH; =O; $OCF_3$; $OCF_2H$; $OCFH_2$; $OCF_2Cl$; $OCFCl_2$; O-$C_{1-6}$-Alkyl; O-C(O)-$C_{1-6}$-Alkyl; O-C(O)-O-$C_{1-6}$-Alkyl; O-(CO)-N(H)($C_{1-6}$-Alkyl); O-C(O)-N($C_{1-6}$-Alkyl)$_2$; O-S(O)$_2$-$NH_2$; O-S(O)$_2$-N(H)($C_{1-6}$-Alkyl); O-S(O)$_2$-N($C_{1-6}$-Alkyl)$_2$; $NH_2$; N(H)($C_{1-6}$-Alkyl); N($C_{1-6}$-Alkyl)$_2$; N(H)-C(O)-$C_{1-6}$-Alkyl; N(H)-C(O)-O-$C_{1-6}$-Alkyl; N(H)-C(O)-$NH_2$; N(H)-C(O)-N(H)($C_{1-6}$-Alkyl); N(H)-C(O)-N($C_{1-6}$-Alkyl)$_2$; N($C_{1-6}$-Alkyl)-C(O)-$C_{1-6}$-Alkyl; N($C_{1-6}$-Alkyl)-C(O)-O-$C_{1-6}$-Alkyl; N($C_{1-6}$-Alkyl)-C(O)-$NH_2$; N($C_{1-6}$-Alkyl)-C(O)-N(H)($C_{1-6}$-Alkyl); N($C_{1-6}$-Alkyl) -C(O)-N($C_{1-6}$-Alkyl)$_2$; N(H)-S(O)$_2$OH; N(H)-S(O)$_2$-$C_{1-6}$-Alkyl; N(H)-S(O)$_2$-O-$C_{1-6}$-Alkyl; N(H)-S(O)$_2$-$NH_2$; N(H)-S(O)$_2$-N(H)($C_{1-6}$-Alkyl); N(H)-S(O)$_2$N($C_{1-6}$-Alkyl)$_2$; N($C_{1-6}$-Alkyl)-S(O)$_2$-OH; N($C_{1-6}$-Alkyl)-S(O)$_2$-$C_{1-6}$-Alkyl; N($C_{1-6}$-Alkyl)-S(O)$_2$-O-$C_{1-6}$-Alkyl; N($C_{1-6}$-Alkyl)-S(O)$_2$-$NH_2$; N($C_{1-6}$-Alkyl)-S(O)$_2$-N(H)($C_{1-6}$-Alkyl); N($C_{1-6}$-Alkyl)-S(O)$_2$-N($C_{1-6}$-Alkyl)$_2$; $SCF_3$; $SCF_2H$; $SCFH_2$; S-$C_{1-6}$-Alkyl; S(O)-$C_{1-6}$-Alkyl; S(O)$_2$-$C_{1-6}$-Alkyl; S(O)$_2$-OH; S(O)$_2$-O-$C_{1-6}$-Alkyl; S(O)$_2$-$NH_2$; S(O)$_2$-N(H)($C_{1-6}$-Alkyl); S(O)$_2$-N($C_{1-6}$-Alkyl)$_2$; $C_{3-6}$-Cycloalkyl; 3- bis 7-gliedrigem Heterocycloalkyl; Phenyl; 5- oder 6-gliedrigem Heteroaryl; O-$C_{3-6}$-Cycloalkyl; O-(3- bis 7-gliedrigem Heterocycloalkyl); O-Phenyl; O-(5- oder 6-gliedrigem Heteroaryl); C(O)-$C_{3-6}$-Cycloalkyl; C(O)-(3- bis 7-gliedrigem Heterocycloalkyl); C(O)-Phenyl; C(O)-(5- oder 6-gliedrigem Heteroaryl); S(O)$_2$-($C_{3-6}$-Cycloalkyl); S(O)$_2$-(3- bis 7-gliedrigem Heterocycloalkyl); S(O)$_2$-Phenyl oder S(O)$_2$-(5- oder 6-gliedrigem Heteroaryl);

wobei Phenyl und 5- bis 10-gliedriges Heteroaryl in jedem Fall unabhängig voneinander unsubstituiert oder mono- oder polysubstituiert sind mit einem oder mehreren Substituenten ausgewählt aus F; Cl; Br; I; CN; $C_{1-6}$-Alkyl; $C_{1-6}$-Alkenyl; $C_{1-6}$-Alkinyl; $C_{1-6}$-Alkinyl-C(H)(OH)$CH_3$; $C_{1-6}$-Alkinyl-C($CH_3$)$_2$OH; $CF_3$; $CF_2H$; $CFH_2$; $CF_2Cl$; $CFCl_2$; $C_{1-6}$-Alkylen-$CF_3$; $C_{1-6}$-Alkylen-$CF_2H$; $C_{1-6}$-Alkylen-$CFH_2$; $C_{1-6}$-Alkylen-OH; $C_{1-6}$-Alkylen-$OCH_3$; C(O)-$C_{1-6}$-Alkyl; C(O)-OH; C(O)-O$C_{1-6}$-Alkyl; C(O)-N(H)(OH); C(O)-$NH_2$; C(O)-N(H)($C_{1-6}$-Alkyl); C(O)-N($C_{1-6}$-Alkyl)$_2$; OH; $OCF_3$; $OCF_2H$; $OCFH_2$; $OCF_2Cl$; $OCFCl_2$; O-$C_{1-6}$-Alkyl; O-$C_{3-6}$-Cycloalkyl; O-(3- bis 7-gliedrigem Heterocycloalkyl); $NH_2$; N(H)($C_{1-6}$-Alkyl); N($C_{1-6}$-Alkyl)$_2$; N(H)-C(O)-$C_{1-6}$-Alkyl; N($C_{1-6}$-Alkyl)-C(O)-$C_{1-6}$-Alkyl; N(H)-C(O)-$NH_2$; N(H)-C(O)-N(H)($C_{1-6}$-Alkyl); N(H)-C(O)-N($C_{1-6}$-Alkyl)$_2$; N($C_{1-6}$-Alkyl)-C(O)-N(H)($C_{1-6}$-Alkyl); N($C_{1-6}$-Alkyl)-C(O)-N($C_{1-6}$-Alkyl)$_2$; N(H)-S(O)$_2$-$C_{1-6}$-Alkyl; $SCF_3$; S-$C_{1-6}$-Alkyl; S(O)-$C_{1-6}$-Alkyl; S(O)$_2$-$C_{1-6}$-Alkyl; S(O)$_2$-$C_{3-6}$-Cycloalkyl; S(O)$_2$-$C_{1-6}$-Alkylen-C-$_{3-6}$-Cycloalkyl; S(O)$_2$-$NH_2$; S(O)$_2$-N(H)($C_{1-6}$-Alkyl); S(O)$_2$-N($C_{1-6}$-Alkyl)$_2$; $C_{3-6}$-Cycloalkyl; $C_{1-6}$-Alkylen-$C_{3-6}$-cycloalkyl; 3- bis 7-gliedrigem Heterocycloalkyl; $C_{1-6}$-Alkylen-(3- bis 7-gliedrigem Heterocycloalkyl); Phenyl oder 5- oder 6-gliedrigem Heteroaryl;
in Form der freien Verbindung oder eines physiologisch verträglichen Salzes davon.

2. Verbindung nach Anspruch 1, wobei

$C_{1-4}$-Alkyl, $C_{1-6}$-Alkyl, $C_{1-10}$-Alkyl, $C_{1-6}$-Alkylen, $C_{3-10}$-Cycloalkyl und 3- bis 7-gliedriges Heterocycloalkyl in jedem Fall unabhängig voneinander unsubstituiert oder mono- oder polysubstituiert sind mit einem oder mehreren Substituenten ausgewählt aus F; Cl; Br; I; CN; $C_{1-6}$-Alkyl; $CF_3$; $CF_2H$; $CFH_2$; $CF_2Cl$; $CFCl_2$; OH; =O; $OCF_3$; $OCF_2H$; $OCFH_2$; $OCF_2Cl$; $OCFCl_2$; O-$C_{1-6}$-Alkyl; $C_{3-6}$-Cycloalkyl oder 3- bis 7-gliedrigem Heterocycloalkyl; und/oder
Phenyl und 5- bis 10-gliedriges Heteroaryl in jedem Fall unabhängig voneinander unsubstituiert oder mono- oder polysubstituiert sind mit einem oder mehreren Substituenten ausgewählt aus F; Cl; Br; I; CN; $C_{1-6}$-Alkyl; $C_{2-6}$-Alkinyl, vorzugsweise -C≡C-$CH_3$; $CF_3$; $CF_2H$; $CFH_2$; $CF_2Cl$; $CFCl_2$; $C_{1-6}$-Alkylen-$CF_3$; $C_{1-6}$-Alkylen -$CF_2H$; $C_{1-6}$-Alkylen-$CFH_2$; C(O)-$C_{1-6}$-Alkyl; C(O)-OH; C(O)-O$C_{1-6}$-Alkyl; OH; $C_{1-6}$-Alkylen-OH; $OCF_3$; $OCF_2H$; $OCFH_2$; $OCF_2Cl$; $OCFCl_2$; O-$C_{1-6}$-Alkyl; O-$C_{3-6}$-Cycloalkyl; O-(3- bis 7-gliedrigem Heterocycloalkyl); $SCF_3$; S-$C_{1-6}$-Alkyl; S(O)-$C_{1-6}$-Alkyl; $S(O)_2$-$C_{1-6}$-Alkyl; $S(O)_2$-$C_{1-6}$-Alkylen-$C_{3-6}$-Cycloalkyl; $S(O)_2$-$NH_2$; $S(O)_2$-N(H)($C_{1-6}$-Alkyl); $S(O)_2$-N($C_{1-6}$-Alkyl)$_2$; $C_{3-6}$-Cycloalkyl; $C_{1-6}$-Alkylen-$C_{3-6}$-cycloalkyl; 3- bis 7-gliedrigem Heterocycloalkyl; $C_{1-6}$-Alkylen-(3- bis 7-gliedrigem Heterocycloalkyl); Phenyl oder 5- oder 6-gliedrigem Heteroaryl.

3. Verbindung nach Anspruch 1 oder 2, wobei
$R^1$ für Phenyl oder 5- bis 10-gliedriges Heteroaryl steht, das ausgewählt ist aus der Gruppe bestehend aus Indolyl, Indazolyl, Pyridyl, vorzugsweise 2-Pyridyl, 3-Pyridyl oder 4-Pyridyl, Pyrazolyl, Pyrazolopyrimidinyl, Pyrrolopyridinyl, Pyrimidinyl, Pyridazinyl, Pyrazinyl, Pyrrolyl, Imidazolyl, Oxazolyl, Isoxazolyl, Thiazolyl, Isothiazolyl, Furanyl, Thienyl (Thiophenyl), Triazolyl, Thiadiazolyl, 4,5,6,7-Tetrahydro-2H-indazolyl, 2,4,5,6-Tetrahydrocyclo-penta[c]pyrazolyl, Benzofuranyl, Benzoimidazolyl, Benzothienyl, Benzothiadiazolyl, Benzothiazolyl, Benztriazolyl, Benzooxazolyl, Benzooxadiazolyl, Chinazolinyl, Chinoxalinyl, Carbazolyl, Chinolinyl, Dibenzofuranyl, Dibenzothienyl, Imidazothiazolyl, Indolizinyl, Isochinolinyl, Naphthyridinyl, Oxazolyl, Oxadiazolyl, Phenazinyl, Phenothiazinyl, Phthalazinyl, Purinyl, Phenazinyl, Tetrazolyl und Triazolyl und/oder

**(i)** $R^3$ und $R^4$ zusammen mit dem Kohlenstoffatom, das sie verbindet $C_{3-10}$-Cycloalkyl bilden, oder **(ii)** $R^3$ und $R^4$ unabhängig voneinander für H oder $C_{1-10}$-Alkyl stehen.

4. Verbindung nach einem der vorhergehenden Ansprüche, wobei $R^3$ und $R^4$ unabhängig voneinander für H oder -$CH_3$ stehen; oder $R^3$ und $R^4$ zusammen mit dem Kohlenstoffatom, das sie verbindet, $C_{3-10}$-Cycloalkyl bilden.

5. Verbindung nach Anspruch 4, wobei $R^3$ und $R^4$ zusammen mit dem Kohlenstoffatom, das sie verbindet, Cyclobutyl bilden.

6. Verbindung nach einem der vorhergehenden Ansprüche, wobei $R^1$ für folgendes steht:

**(i)** Phenyl oder 5- bis 10-gliedriges Heteroaryl, das ausgewählt ist aus der Gruppe bestehend aus Indolyl, Indazolyl, Pyridyl, vorzugsweise 2-Pyridyl, 3-Pyridyl oder 4-Pyridyl, Pyrazolyl, Pyrazolopyrimidinyl, Pyrrolopyridinyl, Pyrimidinyl, Pyridazinyl, Pyrazinyl, Pyrrolyl, Imidazolyl, Oxazolyl, Isoxazolyl, Thiazolyl, Isothiazolyl, Furanyl, Thienyl (Thiophenyl), Triazolyl, Thiadiazolyl, 4,5,6,7-Tetrahydro-2H-indazolyl, 2,4,5,6-Tetrahydrocyclo-penta[c]pyrazolyl, Benzofuranyl, Benzoimidazolyl, Benzothienyl, Benzothiadiazolyl, Benzothiazolyl, Benztriazolyl, Benzooxazolyl, Benzooxadiazolyl, Chinazolinyl, Chinoxalinyl, Carbazolyl, Chinolinyl, Dibenzofuranyl, Dibenzothienyl, Imidazothiazolyl, Indolizinyl, Isochinolinyl, Naphthyridinyl, Oxazolyl, Oxadiazolyl, Phenazinyl, Phenothiazinyl, Phthalazinyl, Purinyl, Phenazinyl, Tetrazolyl und Triazinyl oder
(ii) Phenyl, das unsubstituiert oder mono- oder polysubstituiert ist mit einem oder mehreren Substituenten ausgewählt aus F; Cl; Br; I; -$CH_3$; -$CH_2$-$CH_3$; O-$CH_3$; -$CF_3$; -$C_{3-10}$-Cycloalkyl; -$CH_2$-$C_{3-10}$-Cycloalkyl; S(=O)$_2$-$C_{3-10}$-Cycloalkyl; S (=O)$_2$-$CH_2$-$C_{3-10}$-Cycloalkyl; S(=O)$_2$-$CH_3$; S(=O)$_2$-$CH_2$-$CH_3$; -$CH_2$-$CH_2$-O-$CH_2$- (d. h. Oxolanyl); - C≡C-$CH_3$; C(=O)-$CH_3$; -CH=$CH_2$; $NH_2$ oder -$CH_2$-$CH_2$-OH; oder eine der folgenden Strukturen (II), (III), (IV), (V) oder (VI), mit der Maßgabe, dass in Bezug auf die Strukturen (II), (III), (IV) oder (V) mindestens eines von X und Z ein Heteroatom ist:

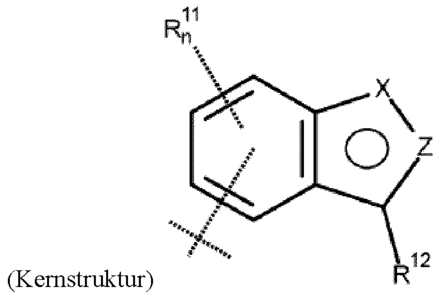
(II)

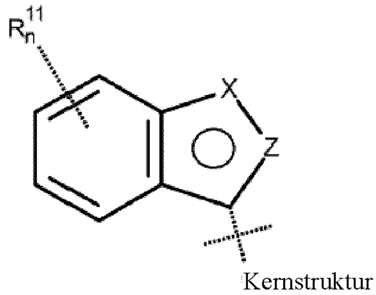
(III)

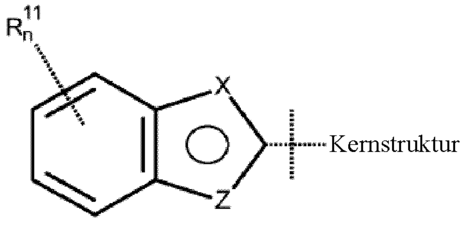
(IV)

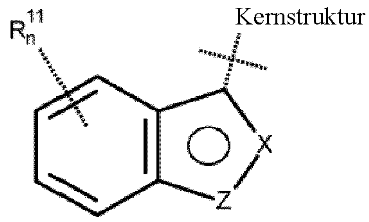
(V)

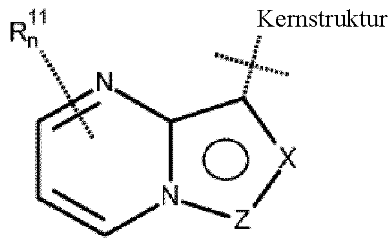
(VI)

wobei

X für N, N-$R^{13}$ oder C-$R^{13}$ steht;
Z für N, N-$R^{13}$ oder C-$R^{13}$ steht;
$R^{11}$, $R^{12}$ und $R^{13}$ unabhängig voneinander für H; F; Cl; Br; I; CN; $C_{1-10}$-Alkyl; $C_{3-10}$-Cycloalkyl; 3- bis 7-gliedriges Heterocycloalkyl; S(O)-($C_{1-10}$-Alkyl); S(O)-($C_{3-10}$-Cycloalkyl); S(O)- (3- bis 7-gliedriges Heterocycloalkyl) ; S $(O)_2$-($C_{1-10}$-Alkyl); $S(O)_2$-($C_{3-10}$-Cycloalkyl); $S(O)_2$-(3- bis 7-gliedriges Heterocycloalkyl) ; P(O)-$(C_{1-10}\text{-Alkyl})_2$; P(O)($C_{1-10}$-Alkyl)($C_{3-10}$-Cycloalkyl) ; P(O)($C_{1-10}$-Alkyl)(3- bis 7-gliedriges Heterocycloalkyl); P(O)-$(O\text{-}C_{1-10}\text{-Alkyl})_2$; P(O)(O-$C_{1-10}$-Alkyl)(O-$C_{3-10}$-Cycloalkyl); P(O)(O-$C_{1-10}$-Alkyl)(O-(3- bis 7-gliedriges Heterocycloalkyl)); O-$C_{1-10}$-Alkyl; S-$C_{1-10}$-Alkyl; N(H)($C_{1-10}$-Alkyl), N $(C_{1-10}\text{-Alkyl})_2$; C(O)-$C_{1-10}$-Alkyl; C(O)-O-$C_{1-10}$-Alkyl; C(O)-$NH_2$; C (O) -N (H) ($C_{1-10}$-Alkyl); C(O)-N $(C_{1-10}\text{-Alkyl})_2$; O-$C_{3-10}$-Cycloalkyl; N (H) ($C_{3-10}$-Cycloalkyl), N($C_{1-10}$-Alkyl)($C_{3-10}$-Cycloalkyl) ; C(O)-$C_{3-10}$-Cycloalkyl; C(O)-O-$C_{3-10}$-Cycloalkyl; C(O)-N (H) ($C_{3-10}$-Cycloalkyl); C(O)-N($C_{1-10}$-Alkyl)($C_{3-10}$-Cycloalkyl); O-3-bis 7-gliedriges Heterocycloalkyl; N(H) (3- bis 7-gliedriges Heterocycloalkyl), N($C_{1-10}$-Alkyl)(3- bis 7-gliedriges Heterocycloalkyl); C(O)-3- bis 7-gliedriges Heterocycloalkyl; C(O)-O-(3- bis 7-gliedriges Heterocycloalkyl); C(O)-N(H) (3- bis 7-gliedriges Heterocycloalkyl) oder C(O)-N($C_{1-10}$-Alkyl)(3- bis 7-gliedriges Heterocycloalkyl) stehen; wobei $C_{3-10}$-Cycloalkyl und 3- bis 7-gliedriges Heterocycloalkyl gegebenenfalls über $C_{1-6}$-Alkylen verbrückt sein können; und n für 0, 1, 2 oder 3 steht; oder wobei $R^{11}$, $R^{12}$ und $R^{13}$ unabhängig voneinander für F; Cl; Br; I; -$CH_3$; O-$CH_3$; -$CF_3$; -$C_{3-10}$-Cycloalkyl; -$CH_2$-$C_{3-10}$-Cycloalkyl; $S(=O)_2$-$CH_2$-$C_{3-10}$-Cycloalkyl; $S(=O)_2$-$CH_3$; -$CH_2$-$CH_2$-O-$CH_2$- (d. h. Oxolanyl); C≡C-$CH_3$; C(=O)-$CH_3$; -$CH_2$-$CH_2$-OH stehen; und n für 0, 1, 2 oder 3 steht.

**7.** Verbindung nach einem der vorhergehenden Ansprüche, wobei keines von $A^1$, $A^2$ und $A^3$ für N steht; und/oder
$R^5$, $R^6$ und $R^7$ unabhängig voneinander für $CH_3$, F, Cl, $CF_3$ oder H stehen.

**8.** Verbindung nach einem der vorhergehenden Ansprüche, wobei $A^7$ nicht für C-$R^{10}$ steht; oder

keines von $A^5$, $A^6$ und $A^7$ für C-$R^8$, C-$R^9$ oder C-$R^{10}$ steht, und/oder höchstens eines von $A^5$, $A^6$ und $A^7$für O steht; oder
mindestens eines von $A^5$, $A^6$ und $A^7$ für C-$R^8$, C-$R^9$ oder C-$R^{10}$ steht, und/oder höchstens eines von $A^5$, $A^6$ und $A^7$ für O steht; oder
mindestens eines von $A^5$, $A^6$ und $A^7$ für N steht und/oder höchstens eines von $A^5$, $A^6$ und $A^7$ für O steht; oder
mindestens eines von $A^5$, $A^6$ und $A^7$ für N-$R^8$, N-$R^9$ oder N-$R^{10}$ steht und/oder höchstens eines von $A^5$, $A^6$ und $A^7$ für O steht; und/oder
$R^8$, $R^9$ und $R^{10}$ unabhängig voneinander für $S(O)_2$-$CH_3$, $CH_3$, $CH_2CH_3$, F, $CF_3$, $CH_2$-Cyclopropyl oder H stehen.

**9.** Verbindung nach einem der vorhergehenden Ansprüche, wobei die Definition von $A^5$, $A^6$ und $A^7$ der Ausführungsform **e, f, g, h, i, j, k, l** oder **m** entspricht:

| Ausführ ungsfor m | $A^5$ | $A^6$ | $A^7$ |
|---|---|---|---|
| **e** | N | C-$R^9$ | C-$R^{10}$ |
| **f** | C-$R^8$ | C-$R^9$ | C-$R^{10}$ |
| **g** | N | N | C-$R^{10}$ |
| **h** | N | N | N |
| **i** | N | N | N-$R^{10}$ |
| **J** | C-$R^8$ | N | N-$R^{10}$ |
| **k** | N | C-$R^9$ | N-$R^{10}$ |
| **l** | C-$R^8$ | N-$R^9$ | N |
| **m** | N | C-$R^9$ | O |

**10.** Verbindung nach einem der vorhergehenden Ansprüche, wobei die Definition von $A^4$, $A^5$, $A^6$, $A^7$ und $A^8$ Ausführungsform **n, o, p, q, r, s, t, u, v, w, x** oder **y** entspricht:

| Ausführ ungsfor m | $A^4$ | $A^5$ | $A^6$ | $A^7$ | $A^8$ |
|---|---|---|---|---|---|
| **n** | C | N | C-$R^9$ | C-$R^{10}$ | N |
| **o** | N | N | C-$R^9$ | C-$R^{10}$ | C |
| **p** | C | C-$R^8$ | C-$R^9$ | C-$R^{10}$ | N |
| **q** | C | N | N | C-$R^{10}$ | N |
| **r** | N | N | N | C-$R^{10}$ | C |
| **S** | C | N | N | N | N |
| **t** | C | N | N | N-$R^{10}$ | C |
| **u** | C | C-$R^8$ | N | N-$R^{10}$ | C |
| **v** | N | N | C-$R^9$ | N-$R^{10}$ | C |
| **w** | C | N | C-$R^9$ | N-$R^{10}$ | C |
| **x** | C | C-$R^8$ | N-$R^9$ | N | C |
| **y** | C | N | C-$R^9$ | O | C |

**11.** Verbindung nach einem der vorhergehenden Ansprüche ausgewählt aus der Gruppe bestehend aus

**1** 7-Fluor-8-(3-fluor-5-methylphenyl)-1,4,4,9-tetramethyl-5H-imidazo[1,2-a]chinoxalin
**2** 7,9-Difluor-1,4,4-trimethyl-8-(1H-pyrazol-3-yl)-5H-pyrrolo[1,2-a]chinoxalin
**3** 7,9-Difluor-8-(1H-indol-4-yl)-1,4,4-trimethyl-5H-pyrrolo[1,2-a]chinoxalin
**4** 7,9-Difluor-1,4,4-trimethyl-8-pyrazolo[1,5-a]pyrimidin-3-yl-5H-pyrrolo[1,2-a]chinoxalin
**5** 7,9-Difluoro-8-(6-fluor-1H-indol-4-yl)-1,4,4-trimethyl-5H-imidazo[1,2-a]chinoxalin

**6** 7-Fluor-8-[2-methoxy-5-(trifluormethyl)pyridin-3-yl]-1,4,4,9-tetramethyl-5H-imidazo[1,2-a]chinoxalin
**7** 7-Fluor-1,4,4,9-tetramethyl-8-[6-(trifluormethyl)-1H-indol-4-yl]-5H-imidazo[1,2-a]chinoxalin
**8** 8-[1-(Cyclopropylmethyl)indol-4-yl]-7-fluor-1,4,4,9-tetramethyl-5H-imidazo[1,2-a]chinoxalin
**9** 8-[1-(Cyclopropylmethylsulfonyl)indol-4-yl]-7-fluor-1,4,4,9-tetramethyl-5H-imidazo[1,2-a]chinoxalin
**10** 8-(1-Cyclopropylindol-4-yl)-7-fluor-1,4,4,9-tetramethyl-5H-imidazo[1,2-a]chinoxalin
**11** 9-Fluor-1,4,4-trimethyl-8-(3-methyl-1H-indol-7-yl)-4,5-dihydropyrido[3,4-e][1,2,4]triazolo[4,3-a]pyrazin
**12** 7,9-Difluor-1,4,4-trimethyl-8-(1H-pyrrolo[2,3-b]pyridin-4-yl)-5H-pyrrolo[1,2-a]chinoxalin
**13** 8-(5-Fluor-3-methyl-1H-indol-7-yl)-1,4,4,9-tetramethyl-4,5-dihydropyrido[2,3-e][1,2,4]triazolo[4,3-a]pyrazin
**14** 7-Fluor-8-(6-fluor-1-methylsulfonylindol-4-yl)-1,4,4,9-tetramethyl-5H-imidazo[1,2-a]chinoxalin
**15** 8-(5-Chlor-2-methoxypyridin-3-yl)-7-fluor-1,4,4,9-tetramethyl-5H-imidazo[1,2-a]chinoxalin
**16** 7-Fluor-8-[5-fluor-3-(oxolan-3-yl)-1H-indol-7-yl]-1,4,4,9-tetramethyl-5H-imidazo[1,2-a]chinoxalin
**17** 7-Fluor-8-(5-fluor-3-prop-1-inyl-1H-indol-7-yl)-1,4,4,9-tetramethyl-5H-imidazo[1,2-a]chinoxalin
**18** 9-Fluor-8-(6-fluor-1-(methylsulfonyl)-1H-indol-4-yl)-1,4,4-trimethyl-4,5-dihydropyrido[3,4-e][1,2,4]triazolo[4,3-a]pyrazin
**19** 7-Fluor-1,4,4,9-tetramethyl-8-(1-methylsulfonylindazol-4-yl)-5H-imidazo[1,2-a]chinoxalin
**20** 7,9-Difluor-1,4,4-trimethyl-8-(1-methylsulfonylindazol-4-yl)-5H-pyrrolo[1,2-a]chinoxalin
**21** 1-[4-(7,9-Difluor-1,4,4-trimethyl-5H-pyrrolo[1,2-a]chinoxalin-8-yl)indol-1-yl]ethanon
**22** 8-(3-Cyclopropyl-1H-indol-7-yl)-7,9-difluor-4,4-dimethyl-5H-tetrazolo[1,5-a]chinoxalin
**23** 7-Fluor-8-(6-fluor-1-methylsulfonylindol-4-yl)-4,4-dimethyl-9-(trifluormethyl)-5H-tetrazolo[1,5-a]chinoxalin
**24** 7-Fluor-8-(6-fluor-1-methylsulfonylindol-4-yl)-1,4,4,9-tetramethyl-5H-triazolo[4,5-c]chinolin
**25** 7-Fluor-8-(5-fluor-3-methyl-1H-indol-7-yl)-1,4,4,9-tetramethyl-5H-triazolo[4,5-c]chinolin
**26** 2-[6-Fluor-4-(7-fluor-1,4,4,9-tetramethyl-5H-triazolo[4,5-c]chinolin-8-yl)indol-1-yl]ethanol
**27** 8-Fluor-9-(6-fluor-1-methylsulfonylindol-4-yl)-1,5,5,10-tetramethyl-6H-pyrazolo[1,5-c]chinazolin
**28** 8,10-Difluor-9-(6-fluor-1-methylsulfonylindol-4-yl)-5,5-dimethyl-6H-pyrazolo[1,5-c]chinazolin
**29** 2-(6-Fluor-1-(methylsulfonyl)-1H-indol-4-yl)-6,6,9-trimethyl-5,6-dihydropyrido[3,2-e][1,2,4]triazolo[4,3-a]pyrazin
**30** 3-Fluor-6,6,9-trimethyl-2-(3-methyl-1H-indol-7-yl)-5,6-dihydropyrido[3,2-e][1,2,4]triazolo[4,3-a]pyrazin
**31** 1,4,4,9-Tetramethyl-8-(3-methyl-1H-indol-7-yl)-4,5-dihydropyrido[3,4-e][1,2,4]triazolo[4,3-a]pyrazin
**32** 6-Fluor-8-(6-fluor-1-methylsulfonylindol-4-yl)-1,4,4,9-tetramethyl-5H-pyrazolo[4,3-c]chinolin
**33** 6-Fluor-1,4,4,9-tetramethyl-8-(1-methylsulfonylindol-4-yl)-5H-pyrazolo[4,3-c]chinolin
**34** 6-Fluor-8-(6-fluor-1-methylsulfonylindazol-4-yl)-1,4,4,9-tetramethyl-5H-pyrazolo[4,3-c]chinolin
**35** 7-Fluor-9-(6-fluor-1-methylsulfonylindol-4-yl)-1,5,5,10-tetramethyl-6H-triazolo[1,5-c]chinazolin
**36** 7-Fluor-9-(6-fluor-1-methylsulfonylindazol-4-yl)-1,5,5,10-tetramethyl-6H-triazolo[1,5-c]chinazolin
**37** 6-Fluor-8-(6-fluor-1-methylsulfonylindol-4-yl)-1,9-dimethylspiro[5H-pyrazolo[4,3-c]chinolin-4,1-cyclobutan]
**38** 6-Fluor-1,9-dimethyl-8-(1-methylsulfonylindazol-4-yl)spiro[5H-pyrazolo[4,3-c]chinolin-4,1-cyclobutan]
**39** 6-Fluor-8-(5-fluor-3-methyl-1H-indol-7-yl)-1,9-dimethylspiro[5H-pyrazolo[4,3-c]chinolin-4,1-cyclobutan]
**40** 1-Ethyl-6-fluor-8-(6-fluor-1-methylsulfonylindazol-4-yl)-4,4,9-trimethyl-5H-pyrazolo[4,3-c]chinolin
**41** 1-Ethyl-6-fluor-8-(6-fluor-1-methylsulfonylindol-4-yl)-4,4,9-trimethyl-5H-pyrazolo[4,3-c]chinolin
**42** 1-(Cyclopropylmethyl)-6-fluor-8-(6-fluor-1-methylsulfonylindol-4-yl)-4,4,9-trimethyl-5H-pyrazolo[4,3-c]chinolin
**43** 1-(Cyclopropylmethyl)-6-fluor-8-(5-fluor-3-methyl-1H-indol-7-yl)-4,4,9-trimethyl-5H-pyrazolo[4,3-c]chinolin
**44** 1-(Cyclopropylmethyl)-6-fluor-8-(6-fluor-1-methylsulfonylindazol-4-yl)-4,4,9-trimethyl-5H-pyrazolo[4,3-c]chinolin
**45** 7-Fluor-9-(6-fluor-1-methylsulfonylindazol-4-yl)-5,5,10-trimethyl-6H-pyrazolo[1,5-c]chinazolin
**46** 7-Fluor-1,4,4,9-tetramethyl-8-(1-methylsulfonylindol-4-yl)-5H-pyrazolo[4,3-c]chinolin
**47** 6-Fluor-8-(6-fluor-1-methylsulfonylindol-4-yl)-1,4,4,9-tetramethyl-5H-imidazo[4,5-c]chinolin
**48** 6-Fluor-8-(6-fluor-1-methylsulfonylindazol-4-yl)-1,4,4,9-tetramethyl-5H-imidazo[4,5-c]chinolin
**49** 6-Fluor-8-(5-fluor-3-methyl-1H-indol-7-yl)-1,4,4,9-tetramethyl-5H-imidazo[4,5-c]chinolin
**50** 7-Fluor-8-(6-fluor-1-methylsulfonylindazol-4-yl)-1,3,4,4,9-pentamethyl-5H-pyrazolo[4,3-c]chinolin
**51** 6-Fluor-8-(6-fluor-1-methylsulfonylindol-4-yl)-1,3,4,4,9-pentamethyl-5H-pyrazolo[4,3-c]chinolin
**52** 6,7-Difluor-8-(5-fluor-3-methyl-1-indol-7-yl)-1,4,4-trimethyl-5H-pyrazolo[4,3-c]chinolin
**53** 6-Fluor-1,3,9-trimethyl-8-(1-methylsulfonylindol-4-yl)spiro[5H-pyrazolo[4,3-c]chinolin-4,1-cyclobutan]
**54** 6-Fluor-1,3,9-trimethyl-8-(1-methylsulfonylindazol-4-yl)spiro[5H-pyrazolo[4,3-c]chinolin-4,1-cyclobutan]
**55** 6-Fluor-8-(5-fluor-3-methyl-1H-indol-7-yl)-4,4,9-trimethyl-2,5-dihydropyrazolo[4,3-c]chinolin
**56** 6-Fluor-1,3,9-trimethyl-8-(3-methyl-1H-indol-7-yl)spiro[5H-pyrazolo[4,3-c]chinolin-4,1-cyclobutan]
**57** 6-Fluor-8-(6-fluor-1-methylsulfonylindol-4-yl)-4,4,9-trimethyl-2-methylsulfonyl-5H-pyrazolo[4,3-c]chinolin
**58** 6-Fluor-8-(6-fluor-1-methylsulfonylindol-4-yl)-4,4,9-trimethyl-2,5-dihydropyrazolo[4,3-c]chinolin
**59** 6-Fluor-8-(6-fluor-1-methylsulfonylindazol-4-yl)-4,4,9-trimethyl-2,5-diliydropyrazolo[4,3-c]chinolin

**60** 8-(6-Fluor-1-methylsulfonylindazol-4-yl)-1,4,4,9-tetramethyl-5H-triazolo[4,5-c]chinolin
**61** 7-Fluor-8-(6-fluor-1-methylsulfonylindol-4-yl)-4,4,9-trimethyl-5H-[1,3]oxazolo[4,5-c]chinolin
**62** 7-Fluor-8-(5-fluor-3-methyl-1H-indol-7-yl)-4,4,9-trimethyl-5H-[1,3]oxazolo[4,5-c]chinolin und
**63** 7-Fluor-8-(6-fluor-1-methylsulfonylindazol-4-yl)-4,4,9-trimethyl-5H-[1,3]oxazolo[4,5-c]chinolin

in Form der freien Verbindung oder eines physiologisch verträglichen Salzes davon.

**12.** Pharmazeutische Darreichungsform, umfassend eine Verbindung nach einem der Ansprüche 1 bis 11.

**13.** Verbindung nach einem der Ansprüche 1 bis 11 und/oder pharmazeutische Darreichungsform nach Anspruch 12 zur Verwendung bei der Behandlung und/oder Prophylaxe von Schmerz und/oder Entzündung.

**14.** Verbindung nach einem der Ansprüche 1 bis 11 und/oder pharmazeutische Darreichungsform nach Anspruch 12 zur Verwendung nach Anspruch 13 bei der Behandlung und/oder Prophylaxe von inflammatorischen Schmerzen.

**Revendications**

**1.** Composé selon la formule générale (I),

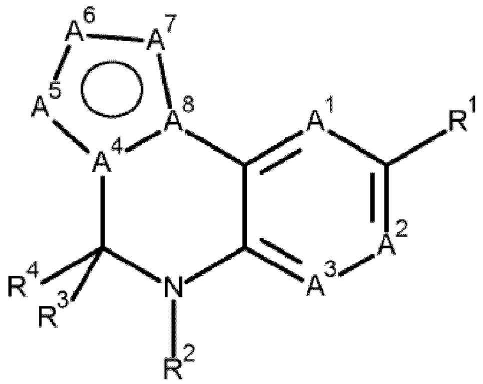

(I), (I),

dans lequel

$R^1$ représente phényle ou hétéroaryle à 5 à 10 chaînons ;
$R^2$ représente H ;
$R^3$ et $R^4$ représentent indépendamment l'un de l'autre H ; alkyle en $C_{1-10}$ ; ou forment avec l'atome de carbone qui les relie cycloalkylke en $C_{3-10}$ ;
$A^1$, $A^2$ et $A^3$ correspondent aux modes de réalisation **a, b, c** ou **d** :

| Mode de réalisation : | $A^1$ | $A^2$ | $A^3$ |
|---|---|---|---|
| **a** | C-$R^5$ | C-$R^6$ | C-$R^7$ |
| **b** | C-$R^5$ | N | C-$R^7$ |
| **c** | C-$R^5$ | C-$R^6$ | N |
| **d** | N | C-$R^6$ | C-$R^7$ |

dans lequel $R^5$ représente H ; F ; Cl ; Br ; I ; alkyle en $C_{1-4}$ ; cycloalkyle en $C_{3-10}$ ; ou O-alkyle en $C_{1-10}$ ; $R^6$ représente H ; F ; Cl ; Br ; I ; alkyle en $C_{1-10}$ ; cycloalkyle en $C_{3-10}$ ; ou O-alkyle en $C_{1-10}$ ; $R^7$ représente H ; F ; Cl ; Br ; I ; alkyle en $C_{1-10}$ ; cycloalkyle en $C_{3-10}$ ; ou O-alkyle en $C_{1-10}$ ;
$A^4$ représente C ou N ;
$A^5$ représente O, N, N-$R^8$ ou C-$R^8$, dans lequel $R^8$ représente H ; alkyle en $C_{1-10}$ ; cycloalkyle en $C_{3-10}$ ; hétérocycloalkyle à 3 à 7 chaînons ; $S(O)_2$-alkyle en $C_{1-6}$ ; ou $S(O)_2$-cycloalkyle en $C_{3-10}$, dans lequel cycloalkyle en $C_{3-10}$ ou hétérocycloalkyle à 3 à 7 chaînons peut éventuellement être ponté via un alkylène en $C_1$-6 ;

$A^6$ représente O, N, N-$R^9$ ou C-$R^9$, dans lequel $R^9$ représente H ; alkyle en $C_{1-10}$ ; cycloalkyle en $C_{3-10}$ ; hétérocycloalkyle à 3 à 7 chaînons ; $S(O)_2$-alkyle en $C_{1-6}$ ; ou $S(O)_2$-cycloalkyle en $C_{3-10}$ dans lequel cycloalkyle en $C_{3-10}$ ou hétérocycloalkyle à 3 à 7 chaînons peut éventuellement être ponté via un alkylène en $C_1$-6 ;

$A^7$ représente O, N, N-$R^{10}$ ou C-$R^{10}$, dans lequel $R^{10}$ représente H ; alkyle en $C_{1-10}$ ; cycloalkyle en $C_{3-10}$ ; hétérocycloalkyle à 3 à 7 chaînons ; $S(O)_2$-alkyle en $C_{1-6}$ ; ou $S(O)_2$-cycloalkyle en $C_{3-10}$ dans lequel cycloalkyle en $C_{3-10}$ ou hétérocycloalkyle à 3 à 7 chaînons peut éventuellement être ponté via un alkylène en $C_1$-6 ;

$A^8$ représente C ou N ;

dans lequel $A^4$, $A^5$, $A^6$, $A^7$ et $A^8$ forment un système hétéroaromatique ; et

dans lequel si $A^4$ représente C et chacun des $A^5$, $A^6$ et $A^8$ représente N et $A^7$ représente $CR^{10}$ ; alors l'un des $A^1$, $A^2$ et $A^3$ représente N ;

dans lequel alkyle en $C_{1-4}$, alkyle en $C_{1-6}$, alkyle en $C_{1-10}$ et alkylène en $C_{1-6}$ dans chaque cas indépendamment les uns des autres sont linéaires ou ramifiés, saturés ou insaturés ;

dans lequel alkyle en $C_{1-4}$, alkyle en $C_{1-6}$, alkyle en $C_{1-10}$, alkylène en $C_{1-6}$, cycloalkyle en $C_{3-10}$ et hétérocycloalkyle à 3 à 7 chaînons dans chaque cas indépendamment les uns des autres sont non substitués ou mono- ou polysubstitués par un ou plusieurs substituants choisis parmi F ; Cl ; Br ; I ; CN ; alkyle en $C_{1-6}$ ; $CF_3$ ; $CF_2H$ ; $CFH_2$ ; $CF_2Cl$ ; $CFCl_2$ ; C(O)-alkyle en $C_{1-6}$ ; C(O)-OH ; C(O)-O-alkyle en $C_{1-6}$ ; C(O)-$NH_2$ ; C(O)-N (H) (alkyle en $C_{1-6}$) ; C(O)-N(alkyle en $C_{1-6}$)$_2$ ; OH ; =O ; $OCF_3$ ; $OCF_2H$ ; $OCFH_2$ ; $OCF_2Cl$ ; $OCFCl_2$ ; O-alkyle en $C_{1-6}$ ; O-C(O)-alkyle en $C_{1-6}$ ; O-C(O)-O-alkyle en $C_{1-6}$ ; O-(CO)-N (H)(alkyle en $C_{1-6}$) ; O-C(O)-N(alkyle en $C_{1-6}$)$_2$ ; O-$S(O)_2$-$NH_2$ ; O-$S(O)_2$-N(H)(alkyle en $C_{1-6}$) ; O-S(O) $_2$-N(alkyle en $C_{1-6}$)$_2$ ; $NH_2$ ; N (H) (alkyle en $C_{1-6}$) ; N (alkyle en $C_{1-6}$)$_2$ ; N(H)-C(O)-alkyle en $C_{1-6}$ ; N (H) -C(O)-O-alkyle en $C_{1-6}$ ; N(H)-C(O)-$NH_2$ ; N(H)-C(O)-N(H)(alkyle en $C_{1-6}$) ; N(H)-C(O)-N(alkyle en $C_{1-6}$)$_2$ ; N(alkyle en $C_{1-6}$)-C(O) -alkyle en $C_{1-6}$ ; N(alkyle en $C_{1-6}$)-C(O)-O-alkyle en $C_{1-6}$ ; N(alkyle en $C_{1-6}$)-C(O)-$NH_2$ ; N(alkyle en $C_{1-6}$)-C(O)-N(H) (alkyle en $C_{1-6}$) ; N(alkyle en $C_{1-6}$)-C(O)-N(alkyle en $C_{1-6}$)$_2$ ; N(H)-$S(O)_2$OH ; N(H)-$S(O)_2$-alkyle en $C_{1-6}$ ; N(H)-$S(O)_2$-O-alkyle en $C_{1-6}$ ; N(H)-$S(O)_2$-$NH_2$ ; N(H)-$S(O)_2$-N(H)(alkyle en $C_{1-6}$) ; N(H)-$S(O)_2$N(alkyle en $C_{1-6}$)$_2$ ; N (alkyle en $C_{1-6}$)-S $(O)_2$-OH ; N (alkyle en $C_{1-6}$)-S $(O)_2$-alkyle en $C_{1-6}$ ; N(alkyle en $C_{1-6}$)-$S(O)_2$-O-alkyle en $C_{1-6}$ ; N(alkyle en $C_{1-6}$)-S (O) $_2$-$NH_2$ ; N (alkyle en $C_{1-6}$)-S $(O)_2$-N(H) (alkyle en $C_{1-6}$) ; N (alkyle en $C_{1-6}$)-$S(O)_2$-N(alkyle en $C_{1-6}$)$_2$ ; $SCF_3$ ; $SCF_2H$ ; $SCFH_2$ ; S-alkyle en $C_{1-6}$ ; S (O) -alkyle en $C_{1-6}$ ; $S(O)_2$-alkyle en $C_{1-6}$ ; $S(O)_2$-OH ; S $(O)_2$-O-alkyle en $C_{1-6}$ ; S $(O)_2$-$NH_2$ ; S $(O)_2$-N(H) (alkyle en $C_{1-6}$) ; S $(O)_2$-N(alkyle en $C_{1-6}$)$_2$ ; cycloalkyle en $C_{3-6}$ ; hétérocycloalkyle à 3 à 7 chaînons ; phényle ; hétéroaryle de 5 ou 6 chaînons ; O-cycloalkyle en $C_{3-6}$ ; O-(hétérocycloalkyle à 3 à 7 chaînons) ; O-phényle ; O-(hétéroaryle de 5 ou 6 chaînons) ; C(O)-cycloalkyle en $C_{3-6}$ ; C(O)-(hétérocycloalkyle à 3 à 7 chaînons) ; C(O)-phényle ; C(O)-(hétéroaryle de 5 ou 6 chaînons) ; $S(O)_2$-(cycloalkyle en $C_{3-6}$) ; S $(O)_2$-(hétérocycloalkyle à 3 à 7 chaînons) ; $S(O)_2$-phényle ou $S(O)_2$-(hétéroaryle de 5 ou 6 chaînons) ;

dans lequel phényle et hétéroaryle à 5 à 10 chaînons dans chaque cas indépendamment l'un de l'autre sont non substitués ou mono- ou polysubstitués par un ou plusieurs substituants choisis parmi F ; Cl ; Br ; I ; CN ; alkyle en $C_{1-6}$ ; alcényle en $C_{1-6}$ ; alcynyle en $C_{1-6}$ ; (alcynyl en $C_{1-6}$)-C(H) $(OH)CH_3$ ; (alcynyl en $C_{1-6}$)-$C(CH_3)_2OH$ ; $CF_3$ ; $CF_2H$ ; $CFH_2$ ; $CF_2Cl$ ; $CFCl_2$ ; (alkylène en $C_{1-6}$)-$CF_3$ ; (alkylène en $C_{1-6}$)-$CF_2H$ ; (alkylène en $C_{1-6}$)-$CFH_2$ ; (alkylène en $C_{1-6}$)-OH ; (alkylène en $C_{1-6}$)-$OCH_3$ ; C (O)-alkyle en $C_{1-6}$ ; C(O)-OH ; C(O)-O-alkyle en $C_{1-6}$ ; C(O)-N(H)(OH) ; C(O)-$NH_2$ ; C(O)-N(H)(alkyle en $C_{1-6}$) ; C(O)-N(alkyle en $C_{1-6}$)$_2$ ; OH ; $OCF_3$ ; $OCF_2H$ ; $OCFH_2$ ; $OCF_2Cl$ ; $OCFCl_2$ ; O-alkyle en $C_{1-6}$ ; O-cycloalkyle en $C_{3-6}$ ; O-(hétérocycloalkyle à 3 à 7 chaînons) ; $NH_2$ ; N (H) (alkyle en $C_{1-6}$) ; N(alkyle en $C_{1-6}$)$_2$ ; N(H)-C(O)-alkyle en $C_{1-6}$ ; N(alkyl en $C_{1-6}$)-C(O)-alkyle en $C_{1-6}$ ; N(H)-C(O)-$NH_2$ ; N(H)-C(O)-N(H) (alkyle en $C_{1-6}$) ; N(H)-C(O)-N(alkyle en $C_{1-6}$)$_2$ ; N(alkyl en $C_{1-6}$)-C(O)-N(H) (alkyle en $C_{1-6}$) ; N(alkyl en $C_{1-6}$)-C(O)-N(alkyle en $C_{1-6}$)$_2$ ; N(H)-$S(O)_2$-alkyle en $C_{1-6}$ ; $SCF_3$ ; S-alkyle en $C_{1-6}$ ; S(O)-alkyle en $C_{1-6}$ ; $S(O)_2$-alkyle en $C_{1-6}$ ; $S(O)_2$-cycloalkyle en $C_{3-6}$ ; $S(O)_2$- (alkylène en $C_{1-6}$)-cycloalkyle en $C_{3-6}$ ; $S(O)_2$-$NH_2$ ; $S(O)_2$-N(H)(alkyle en $C_{1-6}$) ; $S(O)_2$-N(alkyle en $C_{1-6}$)$_2$ ; cycloalkyle en $C_{3-6}$ ; (alkylène en $C_{1-6}$)-cycloalkyle en $C_{3-6}$ ; hétérocycloalkyle à 3 à 7 chaîpons ; (alkylène en $C_{1-6}$)-(héérocycloalkyle à 3 à 7 chaînons) ; phényle hétéroaryle à 5 ou 6 chaînons ;

sous la forme du composé libre ou d'un sel physiologiquement acceptable de celui-ci.

**2.** Composé selon la revendication 1, dans lequel

alkyle en $C_{1-4}$, alkyle en $C_{1-6}$, alkyle en $C_{1-10}$, alkylène en $C_{1-6}$, cycloalkyle en $C_{3-10}$ et hétérocycloalkyle à 3 à 7 chaînons sont chacun indépendamment les uns des autres non substitués ou mono- ou polysubstitués par un ou plusieurs substituants choisis parmi F ; Cl ; Br ; I ; CN ; alkyle en $C_{1-6}$ ; $CF_3$ ; $CF_2H$ ; $CFH_2$ ; $CF_2Cl$ ; $CFCl_2$ ; OH ; =O ; $OCF_3$ ; $OCF_2H$ ; $OCFH_2$ ; $OCF_2Cl$ ; $OCFCl_2$ ; O-alkyle en $C_{1-6}$ ; cycloalkyle en $C_{3-6}$ ; ou hétérocycloalkyle à 3 à 7 chaînons ; et/ou

phényle et hétéroaryle à 5 à 10 chaîpons dans chaque cas indépendamment les uns des autres sont non substitués ou mono- ou polysubstitués par un ou plusieurs substituants choisis parmi F ; Cl ; Br ; I ; CN ; alkyle en $C_{1-6}$ ; alcynyle en $C_{2-6}$, de préférence -C≡C-$CH_3$ ; $CF_3$ ; $CF_2H$ ; $CFH_2$ ; $CF_2Cl$ ; $CFCl_2$ ; (alkylène en $C_{1-6}$)-$CF_3$ ;

(alkylène en $C_{1-6}$)-$CF_2H$ ; (alkylène en $C_{1-6}$)-$CFH_2$ ; C(O)-alkyle en $C_{1-6}$ ; C(O)-OH ; C(O)-O-alkyle en $C_{1-6}$ ; OH ; (alkylène en $C_{1-6}$)-OH ; $OCF_3$ ; $OCF_2H$ ; $OCFH_2$ ; $OCF_2Cl$ ; $OCFCl_2$ ; O-alkyle en $C_{1-6}$ ; O-cycloalkyle en $C_{3-6}$ ; O-(hétérocycloalkyle à 3 à 7 chaînons) ; $SCF_3$ ; S-alkyle en $C_{1-6}$ ; S(O)-alkyle en $C_{1-6}$ ; $S(O)_2$-alkyle en $C_{1-6}$ ; $S(O)_2$-(alkylène en $C_{1-6}$)-cycloalkyle en $C_{3-6}$ ; S $(0)_2$-$NH_2$ ; $S(O)_2$-N(H)(alkyle en $C_{1-6}$) ; $S(O)_2$-N(alkyle en $C_{1-6})_2$ ; cycloalkyle en $C_{3-6}$ ; (alkylène en $C_{1-6}$)-cycloalkyle en $C_{3-6}$ ; hétérocycloalkyle à 3 à 7 chaînons ; (alkylène en $C_{1-6}$)-(hétérocycloalkyle à 3 à 7 chaînons) ; phényle ou hétéroaryle à 5 ou 6 chaînons.

**3.** Composé selon la revendication 1 ou 2, dans lequel

- $R^1$ représente phényle ou hétéroaryle de 5 à 10 chaînons qui est choisi dans le groupe constitué par indolyle, indazolyle, pyridyle, de préférence 2-pyridyle, 3-pyridyle ou 4-pyridyle, pyrazolyle, pyrazolopyrimidinyle, pyrrolopyridinyle, pyrimidinyle, pyridazinyle, pyrazinyle, pyrrolyle, imidazolyle, oxazolyle, isoxazolyle, thiazolyle, isothiazolyle, furanyle, thiényle (thiophényle), triazolyle, thiadiazolyle, 4,5,6,7-tétrahydro-2H-indazolyle, 2,4,5,6-tétrahydrocyclopenta[c]pyrazolyle, benzofuranyle, benzoimidazolyle, benzothiényle, benzothiadiazolyle, benzothiazolyle, benzotriazolyle, benzooxazolyle, benzooxadiazolyle, quinazolinyle, quinoxalinyle, carbazolyle, quinoléinyle, dibenzofuranyle, dibenzothiényle, imidazothiazolyle, indolizinyle, isoquinoléinyle, naphtyridinyle, oxazolyle, oxadiazolyle, phénazinyle, phénothiazinyle, phtalazinyle, purinyle, phénazinyle, tétrazolyle et triazinyle ; et/ou
- (i) $R^3$ et $R^4$, avec l'atome de carbone qui les relie, forment cycloalkyle en $C_{3-10}$ ; ou (ii) $R^3$ et $R^4$ indépendamment l'un de l'autre représentent H ou alkyle en $C_{1-10}$.

**4.** Composé selon l'une quelconque des revendications précédentes, dans lequel

$R^3$ et $R^4$, indépendamment l'un de l'autre, représentent H ou - $CH_3$ ; ou
($R^3$ et $R^4$, ensemble avec l'atome de carbone qui les relie, forment cycloalkyle en $C_{3-10}$.

**5.** Composé selon la revendication 4, dans lequel $R^3$ et $R^4$, ensemble avec l'atome de carbone qui les relie, forment cyclobutyle.

**6.** Composé selon l'une quelconque des revendications précédentes, dans lequel

- $R^1$ représente

(i) phényle ou hétéroaryle de 5 à 10 chaînons qui est choisi dans le groupe constitué par indolyle, indazolyle, pyridyle, de préférence 2-pyridyle, 3-pyridyle ou 4-pyridyle, pyrazolyle, pyrazolopyrimidinyle, pyrrolopyridinyle, pyrimidinyle, pyridazinyle, pyrazinyle, pyrrolyle, imidazolyle, oxazolyle, isoxazolyle, thiazolyle, isothiazolyle, furanyle, thiényle (thiophényle), triazolyle, thiadiazolyle, 4,5,6,7-tétrahydro-2H-indazolyle, 2,4,5,6-tétrahydrocyclopenta[c]pyrazolyle, benzofuranyle, benzoimidazolyle, benzothiényle, benzothiadiazolyle, benzothiazolyle, benzotriazolyle, benzooxazolyle, benzooxadiazolyle, quinazolinyle, quinoxalinyle, carbazolyle, quinoléinyle, dibenzofuranyle, dibenzothiényle, imidazothiazolyle, indolizinyle, isoquinoléinyle, naphtyridinyle, oxazolyle, oxadiazolyle, phénazinyle, phénothiazinyle, phtalazinyle, purinyle, phénazinyle, tétrazolyle et triazinyle ; ou
(ii) phényle, non substitué ou mono- ou polysubstitué par un ou plusieurs substituants choisis parmi F ; Cl ; Br ; I ; -$CH_3$ ; - $CH_2$-$CH_3$ ; O-$CH_3$ ; -$CF_3$ ; cycloalkyle en $C_{3-10}$ ; -$CH_2$-cycloalkyle en $C_{3-10}$ ; $S(=O)_2$-cycloalkyle en $C_{3-10}$ ; $S(=O)_2$-$CH_2$-cycloalkyle en $C_{3-10}$ ; $S(=O)_2$-$CH_3$ ; $S(=O)_2$-$CH_2$-$CH_3$ ; -$CH_2$-$CH_2$-O-$CH_2$- (c'est-à-dire oxolanyle) ; -C≡C-$CH_3$ ; C(=O)-$CH_3$ ; -CH=$CH_2$ ; $NH_2$ ; ou -$CH_2$-$CH_2$-OH ;

ou l'une quelconque des structures suivantes (II), (III), (IV), (V) ou (VI), à condition qu'en ce qui concerne les structures (II), (III), (IV) et (V) au moins l'un de X et Z soit un hétéroatome :

(structure de base) 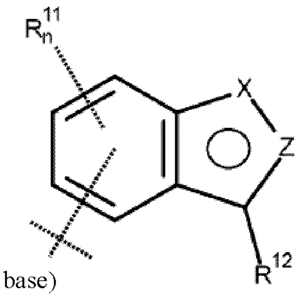 (II)

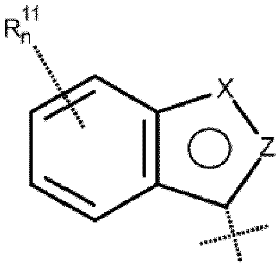 structure de base (III)

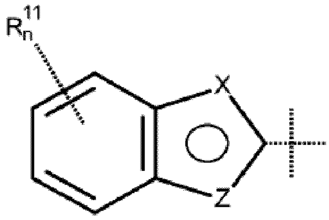 structure de base (IV)

structure de base 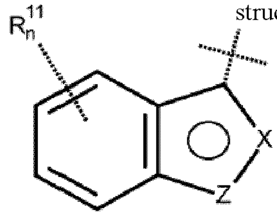 (V)

structure de base 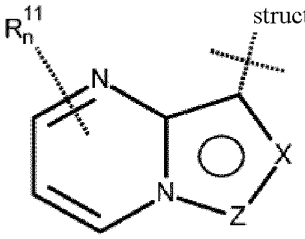 (VI)

dans lequel

X représente N, N-$R^{13}$ ou C-$R^{13}$ ;
Z représente N, N-$R^{13}$ ou C-$R^{13}$ ;
$R^{11}$, $R^{10}$ et $R^{13}$ représentent indépendamment les uns des autres H ; F ; Cl ; Br ; I ; CN ; alkyle en $C_{1-10}$ ; cycloalkyle en $C_{3-10}$ ; hétérocycloalkyle à 3 à 7 chaînons ; S(O)-(alkyle en $C_{1-10}$) ; S(O)-(cycloalkyle en $C_{3-10}$) ; S(O)-(hétérocycloalkyle à 3 à 7 chaînons) ; $S(O)_2$-(alkyle en $C_{1-10}$) ; $S(O)_2$-(cycloalkyle en $C_{3-10}$) ; $S(O)_2$-(hétérocycloalkyle à 3 à 7 chaînons) ; P(O)-(alkyle en $C_{1-10})_2$ ; P(O)(alkyl en $C_{1-10}$)(cycloalkyle en $C_{3-10}$) ; P(O)(alkyl en $C_{1-10}$)(hétérocycloalkyle à 3 à 7 chaînons) ; P(O)-(O-alkyle en $C_{1-10})_2$ ; P(O)(O-alkyl en $C_{1-10}$) (O-cycloalkyle en $C_{3-10}$) ; P(O)(O-alkyl en $C_{1-10}$)(O-(hétérocycloalkyle à 3 à 7 chaînons)) ; O-alkyle en $C_{1-10}$ ; S-alkyle en $C_{1-10}$ ; N(H)(alkyle en $C_{1-10}$), N(alkyle en $C_{1-10})_2$ ; C(O)-alkyle en $C_{1-10}$ ; C(O)-O-alkyle en $C_{1-10}$ ; C(O)-$NH_2$ ; C(O)-N(H)(alkyle en $C_{1-10}$) ; C(O)-N(alkyle en $C_{1-10})_2$ ; O-cycloalkyle en $C_{3-10}$ ; N(H)(cycloalkyle en $C_{3-10}$), N(alkyl en $C_{1-10}$) (cycloalkyle en $C_{3-10}$) ; C(O)-cycloalkyle en $C_{3-10}$ ; C(O)-O-cycloalkyle en $C_{3-10}$ ; C(O)-N(H)(cycloalkyle en $C_{3-10}$) ; C(O)-N(alkyl en $C_{1-10}$)(cycloalkyle en $C_{3-10}$) ; O-hétérocycloalkyle à 3 à 7 chaînons ; N(H)(hétérocycloalkyle à 3 à 7 chaînons), N(alkyl en $C_{1-10}$)(hétérocycloalkyle à 3 à 7 chaînons) ; C(O)-hétérocycloalkyle à 3 à 7 chaînons ; C(O)-O-(hétérocycloalkyle à 3 à 7 chaînons) ; C(O)-N(H)(hétérocycloalkyle à 3 à 7 chaînons) ou C(O)-N(alkyl en $C_{1-10}$)(hétérocycloalkyle à 3 à 7 chaînons) ; dans lequel cycloalkyle en $C_{3-10}$ et hétéorcycloalkyle à 3 à 7 chaînons peuvent être éventuellement pontés via un alkylène en $C_{1-6}$ ; et n représente 0, 1, 2 ou 3 ;
ou dans lequel $R^{11}$, $R^{12}$ et $R^{13}$ représentent, indépendamment les uns des autres, F ; Cl ; Br ; I ; -$CH_3$ ; O-$CH_3$ ; -$CF_3$ ; cycloalkyle en $C_{3-10}$ ; -$CH_2$-cycloalkyle en $C_{3-10}$ ; $S(=O)_2$-$CH_2$-cycloalkyle en $C_{3-10}$ ; $S(=O)_2$-$CH_3$ ; -$CH_2$-$CH_2$-O-$CH_2$- (c'est-à-dire oxolanyle) ; C≡C-$CH_3$ ; C(=O)-$CH_3$ ; -$CH_2$-$CH_2$-OH ; et n représente 0, 1, 2 ou 3.

**7.** Composé selon l'une quelconque des revendications précédentes, dans lequel

- aucun des $A^1$, $A^2$ et $A^3$ ne représente N, respectivement ; et/ou

- $R^5$, $R^6$ et $R^7$, indépendamment les uns des autres, représentent $CH_3$, F, Cl, $CF_3$ ou H.

**8.** Composé selon l'une quelconque des revendications précédentes, dans lequel

- $A^7$ ne représente pas C-$R^{10}$ ; ou
- aucun des $A^5$, $A^6$ et $A^7$ ne représente respectivement C-$R^8$, C-$R^9$ ou C-$R^{10}$, et/ou au plus un des $A^5$, $A^6$ et $A^7$ représente O ; ou
- au moins un de $A^5$, $A^6$ et $A^7$ représente respectivement C-$R^8$, C-$R^9$ ou C-$R^{10}$, et/ou au plus un des $A^5$, $A^6$ et $A^7$ représente O ; ou
- au moins un de $A^5$, $A^6$ et $A^7$ ne représente respectivement N, et/ou au plus un de $A^5$, $A^6$ et $A^7$représente O ; ou
- au moins un de $A^5$, $A^6$ et $A^7$ représente respectivement C-$R^8$, C-$R^9$ ou C-$R^{10}$, et/ou au plus un des $A^5$, $A^6$ et $A^7$ représente O ; et/ou
- $R^8$, $R^9$ et $R^{10}$ représentent indépendamment les uns des autres $S(O)_2$-$CH_3$, $CH_3$, $CH_2CH_3$, F, $CF_3$, $CH_2$-cyclopropyle ou H.

**9.** Composé selon l'une quelconque des revendications précédentes, dans lequel la définition de $A^5$, $A^6$ et $A^7$ correspond au mode de réalisation **e, f, g, h, i, j, k, l** ou **m :**

| mode de réalisation | $A^5$ | $A^6$ | $A^7$ |
|---|---|---|---|
| **e** | N | C-$R^9$ | C-$R^{10}$ |
| **f** | C-$R^8$ | C-$R^9$ | C-$R^{10}$ |
| **g** | N | N | C-$R^{10}$ |
| **h** | N | N | N |
| **i** | N | N | N-$R^{10}$ |
| **J** | C-$R^8$ | N | N-$R^{10}$ |
| **k** | N | C-$R^9$ | N-$R^{10}$ |
| **l** | C-$R^8$ | N-$R^9$ | N |
| **m** | N | C-$R^9$ | O |

**10.** Composé selon l'une quelconque des revendications précédentes, dans lequel la définition de $A^4$, $A^5$, $A^6$, $A^7$ et $A^8$ correspond au mode de réalisation **n, o, p, q, r, s, t, u, v, w, x** ou **y :**

| mode de réalisation | $A^4$ | $A^5$ | $A^6$ | $A^7$ | $A^8$ |
|---|---|---|---|---|---|
| n | C | N | C-$R^9$ | C-$R^{10}$ | N |
| o | N | N | C-$R^9$ | C-$R^{10}$ | C |
| p | C | C-$R^8$ | C-$R^9$ | C-$R^{10}$ | N |
| q | C | N | N | C-$R^{10}$ | N |
| r | N | N | N | C-$R^{10}$ | C |
| s | C | N | N | N | N |
| t | C | N | N | N-$R^{10}$ | C |
| u | C | C-$R^8$ | N | N-$R^{10}$ | C |
| v | N | N | C-$R^9$ | N-$R^{10}$ | C |
| w | C | N | C-$R^9$ | N-$R^{10}$ | C |
| x | C | C-$R^8$ | N-$R^9$ | N | C |
| y | C | N | C-$R^9$ | O | C |

**11.** Composé selon l'une quelconque des revendications précédentes choisi dans le groupe constitué de

**1** 7-fluoro-8-(3-fluoro-5-méthylphényl)-1,4,4,9-tétraméthyl-5H-imidazo[1,2-a]quinoxaline
**2** 7,9-difluoro-1,4,4-triméthyl -8-(1H-pyrazol-3-yl)-5H-pyrrolo[1,2-a]quinoxaline
**3** 7,9-difluoro-8-(1H-indol-4-yl)-1,4,4-triméthyl-5H-pyrrolo[1,2-a]quinoxaline
**4** 7,9-difluoro-1,4,4-triméthyl-8-pyrazolo[1,5-a]pyrimidin-3-yl-5H-pyrrolo[1,2-a]quinoxaline
**5** 7,9-difluoro-8-(6-fluoro-1H-indol-4-yl)-1,4,4-triméthyl-5H-imidazo[1,2-a]quinoxaline
**6** 7-fluoro-8-[2-méthoxy-5-(trifluorométhyl)pyridin-3-yl]-1,4,4,9-tétraméthyl-5H-imidazo[1,2-a]quinoxaline
**7** 7-fluoro-1,4,4,9-tétraméthyl-8-[6-(trifluorométhyl)-1H-indol-4-yl]-5H-imidazo[1,2-a]quinoxaline
**8** 8-[1-(cyclopropylméthyl)indol-4-yl]-7-fluoro-1,4,4,9-tétraméthyl-5H-imidazo[1,2-a]quinoxaline
**9** 8-[1-(cyclopropylméthylsulfonyl)indol-4-yl]-7-fluoro-1,4,4,9-tétraméthyl-5H-imidazo[1,2-a]quinoxaline
**10** 8-(1-cyclopropylindol-4-yl)-7-fluoro-1,4,4,9-tétraméthyl-5H-imidazo[1,2-a]quinoxaline
**11** 9-fluoro-1,4,4-triméthyl-8-(3-méthyl-1H-indol-7-yl)-4,5-dihydropyrido[3,4-e][1,2,4]triazolo[4,3-a]pyrazine
**12** 7,9-difluoro-1,4,4-triméthyl-8-(1H-pyrrolo[2,3-b]pyridin-4-yl)-5H-pyrrolo[1,2-a]quinoxaline
**13** 8-(5-fluoro-3-méthyl-1H-indol-7-yl)-1,4,4,9-tétraméthyl-4,5-dihydropyrido[2,3-e][1,2,4]triazolo[4,3-a]pyrazine
**14** 7-fluoro-8-(6-fluoro-1-méthylsulfonylindol-4-yl)-1,4,4,9-tétraméthyl-5H-imidazo[1,2-a]quinoxaline
**15** 8-(5-chloro-2-méthoxypyridin-3-yl)-7-fluoro-1,4,4,9-tétraméthyl-5H-imidazo[1,2-a]quinoxaline
**16** 7-fluoro-8-[5-fluoro-3-(oxolan-3-yl)-1H-indol-7-yl]-1,4,4,9-tétraméthyl-5H-imidazo[1,2-a]quinoxaline
**17** 7-fluoro-8-(5-fluoro-3-prop-1-ynyl-1H-indol-7-yl)-1,4,4,9-tétraméthyl-5H-imidazo[1,2-a]quinoxaline
**18** 9-fluoro-8-(6-fluoro-1-(méthylsulfonyl)-1H-indol-4-yl)-1,4,4-triméthyl-4,5-dihydropyrido[3,4-e][1,2,4]triazolo[4,3-a]pyrazine
**19** 7-fluoro-1,4,4,9-tétraméthyl-8-(1-méthylsulfonylindazol-4-yl)-5H-imidazo[1,2-a]quinoxaline
**20** 7,9-difluoro-1,4,4-triméthyl-8-(1-méthylsulfonylindazol-4-yl)-5H-pyrrolo[1,2-a]quinoxaline
**21** 1-[4-(7,9-difluoro-1,4,4-triméthyl-5H-pyrrolo[1,2-a]quinoxalin-8-yl)indol-1-yl]éthanone
**22** 8-(3-cyclopropyl-1H-indol-7-yl)-7,9-difluoro-4,4-diméthyl-5H-tétrazolo[1,5-a]quinoxaline
**23** 7-fluoro-8-(6-fluoro-1-méthylsulfonylindol-4-yl)-4,4-diméthyl-9-(trifluorométhyl)-5H-tétrazolo[1,5-a]quinoxaline
**24** 7-fluoro-8-(6-fluoro-1-méthylsulfonylindol-4-yl)-1,4,4,9-tétraméthyl-5H-triazolo[4,5-c]quinoline
**25** 7-fluoro-8-(5-fluoro-3-méthyl-1H-indol-7-yl)-1,4,4,9-tétraméthyl-5H-triazolo[4,5-c]quinoline
**26** 2-[6-fluoro-4-(7-fluoro-1,4,4,9-tétraméthyl-5H-triazolo[4,5-c]quinolin-8-yl)indol-1-yl]éthanol
**27** 8-fluoro-9-(6-fluoro-1-méthylsulfonylindol-4-yl)-1,5,5,10-tétraméthyl-6H-pyrazolo[1,5-c]quinazoline
**28** 8,10-difluoro-9-(6-fluoro-1-méthylsulfonylindol-4-yl)-5,5-diméthyl-6H-pyrazolo[1,5-c]quinazoline
**29** 2-(6-fluoro-1-(méthylsulfonyl)-1H-indol-4-yl)-6,6,9-triméthyl-5,6-dihydropyrido[3,2-e][1,2,4]triazolo[4,3-a]pyrazine
**30** 3-fluoro-6,6,9-triméthyl-2-(3-méthyl-1H-indol-7-yl)-5,6-dihydropyrido[3,2-e][1,2,4]triazolo[4,3-a]pyrazine
**31** 1,4,4,9-tétraméthyl-8-(3-méthyl-1H-indol-7-yl)-4,5-dihydropyrido[3,4-e][1,2,4]triazolo[4,3-a]pyrazine
**32** 6-fluoro-8-(6-fluoro-1-méthylsulfonylindol-4-yl)-1,4,4,9-tétraméthyl-5H-pyrazolo[4,3-c]quinoline
**33** 6-fluoro-1,4,4,9-tétraméthyl-8-(1-méthylsulfonylindol-4-yl)-5H-pyrazolo[4,3-c]quinoline
**34** 6-fluoro-8-(6-fluoro-1-méthylsulfonylindazol-4-yl)-1,4,4,9-tétraméthyl-5H-pyrazolo[4,3-c]quinoline
**35** 7-fluoro-9-(6-fluoro-1-méthylsulfonylindol-4-yl)-1,5,5,10-tétraméthyl-6H-triazolo[1,5-c]quinazoline
**36** 7-fluoro-9-(6-fluoro-1-méthylsulfonylindazol-4-yl)-1,5,5,10-tétraméthyl-6H-triazolo[1,5-c]quinazoline
**37** 6-fluoro-8-(6-fluoro-1-méthylsulfonylindol-4-yl)-1,9-diméthylspiro[5H-pyrazolo[4,3-c]quinoline-4,1-cyclobutane]
**38** 6-fluoro-1,9-diméthyl-8-(1-méthylsulfonylindazol-4-yl)spiro[5H-pyrazolo[4,3-c]quinoline-4,1-cyclobutane]
**39** 6-fluoro-8-(5-fluoro-3-méthyl-1H-indol-7-yl)-1,9-diméthylspiro[5H-pyrazolo[4,3-c]quinoline-4,1-cyclobutane]
**40** 1-éthyl-6-fluoro-8-(6-fluoro-1-méthylsulfonylindazol-4-yl)-4,4,9-triméthyl-5H-pyrazolo[4,3-c]quinoline
**41** 1-éthyl-6-fluoro-8-(6-fluoro-1-méthylsulfonylindol-4-yl)-4,4,9-triméthyl-5H-pyrazolo[4,3-c]quinoline
**42** 1-(cyclopropylméthyl)-6-fluoro-8-(6-fluoro-1-méthylsulfonylindol-4-yl)-4,4,9-triméthyl-5H-pyrazolo[4,3-c]quinoline
**43** 1-(cyclopropylméthyl)-6-fluoro-8-(5-fluoro-3-méthyl-1H-indol-7-yl)-4,4,9-triméthyl-5H-pyrazolo[4,3-c]quinoline
**44** 1-(cyclopropylméthyl)-6-fluoro-8-(6-fluoro-1-méthylsulfonylindazol-4-yl)-4,4,9-triméthyl-5H-pyrazolo[4,3-c]quinoline
**45** 7-fluoro-9-(6-fluoro-1-méthylsulfonylindazol-4-yl)-5,5,10-triméthyl-6H-pyrazolo[1,5-c]quinazoline
**46** 7-fluoro-1,4,4,9-tétraméthyl -8-(1-méthylsulfonylindol-4-yl)-5H-pyrazolo[4,3-c]quinoline
**47** 6-fluoro-8-(6-fluoro-1-méthylsulfonylindol-4-yl)-1,4,4,9-tétraméthyl-5H-imidazo[4,5-c]quinoline
**48** 6-fluoro-8-(6-fluoro-1-méthylsulfonylindazol-4-yl)-1,4,4,9-tétraméthyl-5H-imidazo[4,5-c]quinoline
**49** 6-fluoro-8-(5-fluoro-3-méthyl-1H-indol-7-yl)-1,4,4,9-tétraméthyl-5H-imidazo[4,5-c]quinoline
**50** 7-fluoro-8-(6-fluoro-1-méthylsulfonylindazol-4-yl)-1,3,4,4,9-pentaméthyl-5H-pyrazolo[4,3-c]quinoline

**51** 6-fluoro-8-(6-fluoro-1-méthylsulfonylindol-4-yl)-1,3,4,4,9-pentaméthyl-5H-pyrazolo[4,3-c]quinoline
**52** 6,7-difluoro-8-(5-fluoro-3-méthyl-1-indol-7-yl)-1,4,4-triméthyl-5H-pyrazolo[4,3-c]quinoline
**53** 6-fluoro-1,3,9-triméthyl -8-(1-méthylsulfonylindol-4-yl)spiro[5H-pyrazolo[4,3-c]quinoline-4,1-cyclobutane]
**54** 6-fluoro-1,3,9-triméthyl -8-(1-méthylsulfonylindazol-4-yl)spiro[5H-pyrazolo[4,3-c]quinoline-4,1-cyclobutane]
**55** 6-fluoro-8-(5-fluoro-3-méthyl-1H-indol-7-yl)-4,4,9-triméthyl -2,5-dihydropyrazolo[4,3-c]quinoline
**56** 6-fluoro-1,3,9-triméthyl -8-(3-méthyl-1H-indol-7-yl)spiro[5H-pyrazolo[4,3-c]quinoline-4,1-cyclobutane]
**57** 6-fluoro-8-(6-fluoro-1-méthylsulfonylindol-4-yl)-4,4,9-triméthyl-2-méthylsulfonyl-5H-pyrazolo[4,3-c]quinoline
**58** 6-fluoro-8-(6-fluoro-1-méthylsulfonylindol-4-yl)-4,4,9-triméthyl -2,5-dihydropyrazolo[4,3-c]quinoline
**59** 6-fluoro-8-(6-fluoro-1-méthylsulfonylindazol-4-yl)-4,4,9-triméthyl -2,5-dihydroxypyrazolo[4,3-c]quinoline
**60** 8-(6-fluoro-1-méthylsulfonylindazol-4-yl) -1,4,4,9-tétraméthyl-5H-triazolo[4,5-c]quinoline
**61** 7-fluoro-8-(6-fluoro-1-méthylsulfonylindol-4-yl)-4,4,9-triméthyl-5H-[1,3]oxazolo[4,5-c]quinoline
**62** 7-fluoro-8-(5-fluoro-3-méthyl-1H-indol-7-yl)-4,4,9-triméthyl-5H-[1,3]oxazolo[4,5-c]quinoline et
**63** 7-fluoro-8-(6-fluoro-1-méthylsulfonylindazol-4-yl)-4,4,9-triméthyl-5H-[1,3]oxazolo[4,5-c]quinoline

sous la forme du composé libre ou d'un sel physiologiquement acceptable de celui-ci.

**12.** Forme posologique pharmaceutique comprenant un composé selon l'une quelconque des revendications 1 à 11.

**13.** Composé selon l'une quelconque des revendications 1 à 11 et/ou forme posologique pharmaceutique selon la revendication 12 pour une utilisation dans le traitement et/ou la prophylaxie de la douleur et/ou de l'inflammation.

**14.** Composé selon l'une quelconque des revendications 1 à 11 et/ou forme posologique pharmaceutique selon la revendication 12 pour une utilisation selon la revendication 13 dans le traitement et/ou la prophylaxie de la douleur et/ou de l'inflammation.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009035067 A
- WO 2017034006 A
- EP 1995242 A
- EP 3342768 A
- WO 2020016452 A
- WO 2020016453 A


### Non-patent literature cited in the description


- **HAPGOOD JP. et al.** *Pharmacol Ther.*, September 2016, vol. 165, 93-113
- **BUTTGEREIT F.** *Clin Exp Rheumatol.*, July 2015, vol. 33 (92), 529-33
- **HARTMANN K. et al.** *Physiol Rev.*, April 2016, vol. 96 (2), 409-47
- **DE BOSSCHER K et al.** *Trends Pharmacol Sci.*, January 2016, vol. 37 (1), 4-16
- **BUTTGEREIT F. et al.** *JAMA*, 2016, vol. 315 (22), 2442-2458
- **LIU D. et al.** *Allergy Asthma Clin Immunol.*, 15 August 2013, vol. 9 (1), 30
- Metal Catalyzed Cross-Coupling Reactions and More. Set Wiley, 2014, vol. 3
- Angew. Chem. Int. Ed. 2012, vol. 51, 5062-5085
- Angew. Chem.. 2005, vol. 117, 4516-4563